(19) Europäisches Patentamt
European Patent Office
Office européen des brevets

(11) EP 2 701 704 B1

(12) # EUROPEAN PATENT SPECIFICATION

(45) Date of publication and mention of the grant of the patent:
**13.04.2016 Bulletin 2016/15**

(21) Application number: **12777002.2**

(22) Date of filing: **27.04.2012**

(51) Int Cl.:
***A61K 31/4427*** *(2006.01)* ***A61K 31/435*** *(2006.01)*
***A61P 25/00*** *(2006.01)*

(86) International application number:
**PCT/US2012/035425**

(87) International publication number:
**WO 2012/149295 (01.11.2012 Gazette 2012/44)**

(54) **METHODS OF ADMINISTERING ANATABINE TO TREAT AUTISM SPECTRUM DISORDERS AND SEIZURE DISORDERS**

VERFAHREN ZUR VERABREICHUNG VON ANATABIN ZUR BEHANDLUNG VON AUTISMUSSPEKTRUMSSTÖRUNGEN UND -ANFÄLLEN

PROCÉDÉ D'ADMINISTRATION D'ANATABINE POUR TRAITER DES TROUBLES DU SPECTRE DE L'AUTISME ET DES TROUBLES DE CRISE D'ÉPILEPSIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2011 US 201161480258 P**
**28.04.2011 US 201161480271 P**

(43) Date of publication of application:
**05.03.2014 Bulletin 2014/10**

(73) Proprietor: **RCP Development, Inc.**
**Sarasota, FL 34243 (US)**

(72) Inventor: **WILLIAMS , Jonnie R., JR.**
**Glen Allen**
**Virginia 23060 (US)**

(74) Representative: **Tombling, Adrian George et al**
**Withers & Rogers LLP**
**4 More London Riverside**
**London SE1 2AU (GB)**

(56) References cited:
**WO-A1-99/62531** **WO-A2-01/87288**
**US-A- 5 276 043** **US-A- 5 840 906**

- **PETER DOBELIS ET AL.: 'GABAergic Systems Modulate Nicotinic Receptor- Mediated Seizures in Mice' THE JOURNAL OF PHARMACOLOGY AND EXPERIMENTAL THERAPEUTICS vol. 306, no. 3, 2003, pages 1159 - 1166, XP055130657**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 701 704 B1

## Description

### TECHNICAL FIELD

This disclosure relates generally to methods and compositions for treating Autism Spectrum Disorders and seizure disorders.

### BRIEF DESCRIPTION OF THE DRAWINGS

**FIG. 1.** Graph showing effects of anatabine (AN) on TNFα-induced NFκB activity in *vitro.* See Example 1.

**FIG. 2.** Graph showing effects of a crude extract of smokeless tobacco on TNFα-induced NFκB activity *in vitro.* See Example I.

**FIG. 3.** Graph showing effects of nicotine and of an alkaloid extract of smokeless tobacco on TNFα-induced NFκB activity *in vitro.* See Example I.

**FIG. 4.** Graph showing the results of a cytotoxicity assay measuring release of lactate dehydrogenase (LDH) using supernatant from the cells assayed in **FIG. 1.** See Example 2.

**FIG. 5.** Graph showing the results of a cytotoxicity assay using supernatant from the cells assayed in **FIG. 2.** See Example 2.

**FIG. 6.** Graph showing the results of a cytotoxicity assay using supernatant from the cells assayed in **FIG. 3.** See Example 2.

**FIG. 7.** Graph showing concentrations in rat plasma as a function of time of anatabine and nicotine after a single intravenous bolus injection.

**FIG. 8.** Graph showing concentrations of anatabine and nicotine in rat plasma as a function of time (semi-log).

**FIG. 9.** Graph showing $AUC_{0\rightarrow\infty}$ versus dose for both anatabine and nicotine in male and female rats.

**FIG. 10.** Graph showing concentrations of anatabine or nicotine in rat brain extracts following a single intravenous bolus dose.

**FIG. 11.** Graph showing mean concentration of anatabine and nicotine in rat brain extracts 0.5 hours after a single intravenous bolus dose.

**FIG. 12.** Nicotine product ion scan.

**FIG. 13.** Nicotine sample chromatogram.

**FIG. 14.** Anatabine product ion scan.

**FIG. 15.** Anatabine sample chromatogram.

**FIG. 16.** Nicotine-d3 product ion scan.

**FIG. 17.** Nicotine-d3 sample chromatogram.

**FIG. 18.** Anatabine-d4 product ion scan.

**FIG. 19.** Anatabine-d4 sample chromatogram.

**FIG. 20.** Graph showing mean body weights (± Std Dev) for each treatment group and gender.

**FIGS. 21A-21B.** Graphs showing mean (± SEM) concentration of anatabine in plasma for male or female rats. **FIG. 21A,** 0.6 mg/kg body weight (BW); **FIG. 21B,** 6.0 mg/kg BW.

**FIGS. 22A-22B.** Graphs showing mean (± SEM) concentration of anatabine in plasma for male and female rats combined. **FIG. 22A,** 0.6 mg/kg BW; **FIG. 22B,** 6.0 mg/kg BW.

**FIGS. 23A-23B.** Graphs showing mean (± SEM), maximal (Cp, max), and minimal (Cp, min) concentrations of anatabine in plasma for male or female rats. **FIG. 23A,** 0.6 mg/kg BW; **FIG. 23B,** 6.0 mg/kg BW.

**FIG. 24.** Graph showing effect of S-(-)-anatabine on TNFα-induced NFκB activity in *vitro.*

**DETAILED DESCRIPTION**

This disclosure describes methods of using a composition comprising an isolated form of a compound of Formula I (*e.g.*, anatabine or S-(-)-anatabine or a pharmaceutically acceptable salt thereof) to treat Autism Spectrum Disorders and seizure disorders (*i.e.,* any condition characterized by seizures, described in more detail below).

Some aspects involve administering compounds of Formula I:

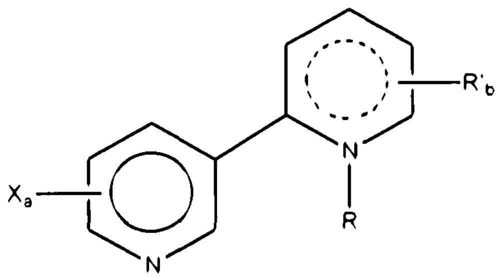

Formula I

wherein:

R represents hydrogen or $C_1$ - $C_5$ alkyl;

R’ represents hydrogen or $C_1$ - $C_7$ alkyl; and

X represents halogen or $C_1$ - $C_7$ alkyl.

The dotted line within the piperidine ring represents a carbon/carbon or carbon/nitrogen double bond within that ring, or two conjugated double bonds within that ring. One of the two conjugated double bonds can be a carbon/nitrogen double bond, or both of the conjugated double bonds can be carbon/carbon double bonds. When a carbon/nitrogen double bond is present, R is absent; and either (i) "a" is an integer ranging from 1-4, usually 1-2, and "b" is an integer ranging from 0-8, usually 0-4; or (ii) "a" is an integer ranging from 0-4, usually 0-2, and "b" is an integer ranging from 1-8, usually 1-4. When a carbon/nitrogen double bond is not present, R is present; "a" is an integer ranging from 0-4, usually 1-2; and "b" is an integer ranging from 0-8, usually 0-4 or 1-2. The term "alkyl," as used herein, encompasses both straight chain and branched alkyl. The term "halogen" encompasses fluorine (F), chlorine (Cl), bromine (Br), and iodine (I).

Compounds of Formula I may be present in the form of racemic mixtures or, in some cases, as isolated enantiomers as illustrated below in Formulas IA and IB.

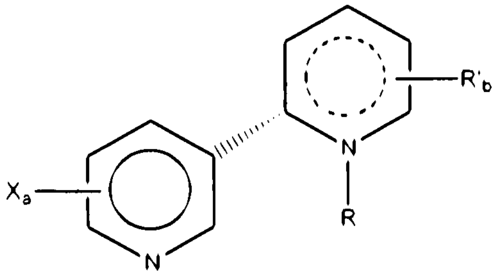

Formula IA

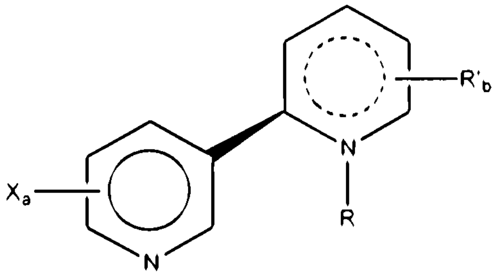

Formula IB

Formula IA represents the S-(-)-enantiomer and Formula IB the R-(+)-enantiomer.

An example of a compound of Formula I is anatabine. An example of a compound of Formula IA is S-(-)-anatabine, and an example of compound of Formula IB is R-(+)-anatabine.

Anatabine is an alkaloid present in tobacco and, in lower concentrations, in a variety of foods, including green tomatoes, green potatoes, ripe red peppers, tomatillos, and sundried tomatoes. Without being bound by this explanation, data presented in Examples I and 2 below indicate that anatabine reduces transcription mediated by nuclear factor κB (NFκB). NFκB is a transcription factor which operates in cells involved in inflammatory and immune reactions.

**Autism Spectrum Disorders**

Autism spectrum disorders (ASDs) are pervasive neurodevelopmental disorders diagnosed in early childhood when acquired skills are lost or the acquisition of new skills becomes delayed. ASDs onset in early childhood and are associated with varying degrees of dysfunctional communication and social skills, in addition to repetitive and stereotypic behaviors. In many cases (25%-50%), a period of seemingly normal development drastically shifts directions as acquired skills are lost or the acquisition of new skills becomes delayed. Examples of Autism Spectrum Disorders include "classical" autism, Asperger's syndrome, Rett syndrome, childhood disintegrative disorder, and atypical autism otherwise known as pervasive developmental disorder not otherwise specified (PDD-NOS).

Autism is a childhood psychosis originating in infancy and characterized by a wide spectrum of psychological symptoms that progress with age (*e.g.*, lack of responsiveness in social relationships, language abnormality, and a need for constant environmental input). It generally appears in children between the ages of two and three years and gives rise to a loss of the development previously gained by the child. Autistic individuals are at increased risk of developing seizure disorders, such as epilepsy.

Excess inflammation has been found in the colon, esophagus, and duodenum of patients with autism, and postmortem studies have also shown an increase in the expression of several markers for neuroinflammation (see Table 1). Proinflammatory cytokines, including TNFα and IL-1, are overproduced in a subset of autistic patients, indicating that these patients had excessive innate immune responses and/or aberrant production of regulatory cytokines for T cell responses (*e.g.*, 20030148955. Isolated forms of anatabine, including S-(-)-anatabine, or salts of such isolated forms are particularly useful for treating disorders comprising an "NFκB-mediated inflammatory component," *i.e.* inflammation characterized by, caused by, resulting from, or affected by NFκB-mediated transcription. Thus, a compound of Formula I (*e.g.*, anatabine or S-(-)-anatabine or a pharmaceutically acceptable salt thereof) in isolated form may be useful in treating or reducing a symptom of an ASD. Use of isolated forms of anatabine avoids the toxicity associated with tobacco, tobacco extracts, alkaloid extracts, and nicotine.

**Seizure Disorders**

Neuroinflammation is a well-established response to central nervous system injury (Minghetti, Curr Opin Neurol 2005; 18:315-21). Human pathologic, *in vitro,* and *in vivo* studies of Alzheimer's disease have implicated a glia-mediated neuroinflammatory response both in the pathophysiology of the disease (Mrak & Griffin, Neurobiol Aging 26:349-54, 2005) and as treatment target (Hu et al., Bioorgan Med Chem Lett 17:414-18, 2007; Ralay et al., J Neurosci 26:662-70, 2006; Craft et al., Exp Opin Therap Targets 9:887-900, 2005). Microglial activation leading to overexpression of IL-1 has been proposed as the pivotal step in initiating a self propagating cytokine cycle culminating in neurodegeneration (Mrak & Griffin, Neurobiol Aging 26:349-54, 2005; Sheng et al., Neurobiol Aging 17:761-66, 1996). IL-1β and pro-inflammatory cytokines may function in epilepsy as pro-convulsant signaling molecules independent of such a cycle (Vezzani et al., Epilepsia 43:S30-S35, 2002), which provides a potential therapeutic target in epilepsy and other seizure disorders (Vezzani & Granata, Epilepsia 46:1724-43, 2005).

In some embodiments an isolated form of anatabine or S-(-)-anatabine or a pharmaceutically acceptable salt of such an isolated form is administered to treat seizures, including the generalized and partial seizures.

As described in The Pharmacological Basis of Therapeutics, 9th ed., (McGraw-Hill), there are two classes of seizures: partial seizures and generalized seizures. Partial seizures consist of focal and local seizures. Partial seizures are further classified as simple partial seizures, complex partial seizures and partial seizures secondarily generalized. Generalized seizures are classified as convulsive and nonconvulsive seizures. They are further classified as absence (previously referred to as 'petit mal') seizures, atypical absence seizures, myoclonic seizures, clonic seizures, tonic seizures, tonic-clonic seizures, and atonic seizures.

Generalized Seizures

Generalized seizures include infantile spasms, absence seizures, tonic-clonic seizures, atonic seizures, and myoclonic seizures. Abnormal motor function and a loss of consciousness are major features of these seizures. A patient may also experience an aura of sensory, autonomic, or psychic sensations. The aura may include paresthesia, a rising epigastric sensation, an abnormal smell, a sensation of fear, or a dejavu sensation. A generalized seizure is often followed by a postictal state, in which a patient may sleep deeply, be confused, and/or have a headache or muscle ache. Todd's paralysis (limb weakness contralateral to the seizure focus) may be present in the postictal state.

Infantile spasms are characterized by frequent flexion and adduction of the arms and forward flexion of the trunk, usually of short duration. They occur only in the first 5 years of life.

Typical absence seizures (also known as petit mal seizures) are characterized by a loss of consciousness with eyelid fluttering, typically for 10-30 seconds or more. There may or may not be a loss of axial muscle tone. Convulsions are absent; instead, patients abruptly stop activity, then abruptly resume it, often without realizing that a seizure has occurred. Absence seizures are genetic. They occur predominantly in children, often frequently throughout the day.

Atypical absence seizures occur as part of the Lennox-Gastaut syndrome, a severe form of epilepsy. They last longer than typical absence seizures and jerking or automatic movements are more pronounced.

Atonic seizures occur most often in children, usually as part of Lennox-Gastaut syndrome. They are characterized by a complete loss of muscle tone and consciousness.

Tonic seizures also occur most often in children, usually as part of Lennox-Gastaut syndrome. They are characterized by tonic (sustained) contraction of axial and proximal muscles, usually during sleep, and last 10 to 15 seconds. In longer tonic seizures a few, rapid clonic jerks may occur at the end of the seizure.

Tonic-clonic seizures, also known as grand mal seizures, may be primarily or secondarily generalized. A patient experiencing a primarily generalized tonic-clonic seizure will often cry out, then lose consciousness and fall. Tonic contractions then begin, followed by clonic (rapidly alternating contraction and relaxation) motion of muscles of the extremities, trunk, and head. A patient may lose urinary and fecal continence, bite his tongue, and froth at the mouth. Seizures usually last 1 to 2 min. There is no aura. Secondarily generalized tonic-clonic seizures begin with a simple partial or complex partial seizure, and then progress to a generalized seizure.

Myoclonic seizures are characterized by brief, rapid jerks of a limb, several limbs, or the trunk. They may be repetitive, leading to a tonic-clonic seizure. The jerks may be bilateral or unilateral. Consciousness is not lost unless the seizures progress into a generalized tonic-clonic seizure.

Juvenile myoclonic epilepsy is an epilepsy syndrome characterized by myoclonic, tonic-clonic, and absence seizures. Patients are usually adolescents. Seizures typically begin with bilateral, synchronous myoclonic jerks, followed in 90% by generalized tonic-clonic seizures. They often occur on rising in the morning. A third of patients may experience absence seizures.

Febrile seizures are associated with fever, but not intracranial infection. Benign febrile seizures are characterized by generalized tonic-clonic seizures of brief duration. Such seizures are common in children, affecting up to four percent of children younger than six years of age. Complicated febrile seizures are characterized by focal seizures lasting more

than fifteen minutes or occurring more than twice in twenty four hours. Two percent of children with febrile seizures develop a subsequent seizure disorder. The risk is greater in children with complicated febrile seizures, preexisting neurologic abnormalities, onset before age 1 yr, or a family history of seizure disorders.

Status epilepticus is a seizure disorder characterized by tonic-clonic seizure activity lasting more than five to ten minutes, or two or more seizures between which patients do not fully regain consciousness. If untreated, seizures lasting more than sixty minutes may cause brain damage or death.

Complex partial status epilepticus and absence status epilepticus are characterized by prolonged episodes of mental status changes. Generalized convulsive status epilepticus may be associated with abrupt withdrawal of anticonvulsants or head trauma.

Partial Seizures

Simple partial seizures are characterized by motor, sensory, or psychomotor symptoms without loss of consciousness. Seizures in different parts of the brain often produce distinct symptoms.

An aura often precedes complex partial seizures. Patients are usually aware of their environment but may experience impaired consciousness. Patients may also experience oral automatisms (involuntary chewing or lip smacking), hand or limb automatisms (automatic purposeless movements), utterance of unintelligible sounds, tonic or dystonic posturing of the extremity contralateral to the seizure focus, head and eye deviation, usually in a direction contralateral to the seizure focus, and bicycling or pedaling movements of the legs, especially where the seizure emanates from the medial frontal or orbitofrontal head regions. Motor symptoms subside after one or two minutes, and confusion and disorientation one to two minutes later. Postictal amnesia is common.

Epilepsy is an important example of a seizure disorder. "Epilepsy" describes a group of central nervous system disorders that are characterized by recurrent seizures that are the outward manifestation of excessive and/or hypersynchronous abnormal electrical activity of neurons of the cerebral cortex and other regions of the brain. This abnormal electrical activity can be manifested as motor, convulsion, sensory, autonomic, or psychic symptoms.

Hundreds of epileptic syndromes have been defined as disorders characterized by specific symptoms that include epileptic seizures. These include, but are not limited to, absence epilepsy, psychomotor epilepsy, temporal lobe epilepsy, frontal lobe epilepsy, occipital lobe epilepsy, parietal lobe epilepsy, Lennox-Gastaut syndrome, Rasmussen's encephalitis, childhood absence epilepsy, Ramsay Hunt Syndrome type II, benign epilepsy syndrome, benign infantile encephalopathy, benign neonatal convulsions, early myoclonic encephalopathy, progressive epilepsy and infantile epilepsy. A patient may suffer from any combination of different types of seizures. Partial seizures are the most common, and account for approximately 60% of all seizure types.

Examples of generalized seizures which may be treated include infantile spasms, typical absence seizures, atypical absence seizures, atonic seizures, tonic seizures, tonic-clonic seizures, myoclonic seizures, and febrile seizures. Examples of partial seizures which may be treated include simple partial seizures affecting the frontal lobe, contralateral frontal lobe, supplementary motor cortex, the insula, the Insular-orbital-frontal cortex, the anteromedial temporal lobe, the amygdala (including the opercular and/or other regions), the temporal lobe, the posterior temporal lobe, the amygdala, the hippocampus, the parietal lobe (including the sensory cortex and/or other regions), the occipital lobe, and/or other regions of the brain.

In some embodiments an isolated form of anatabine or S-(-)-anatabine or a pharmaceutically acceptable salt of such an isolated form is administered to treat an epileptic syndrome including, but not limited to, absence epilepsy, psychomotor epilepsy, temporal lobe epilepsy, frontal lobe epilepsy, occipital lobe epilepsy, parietal lobe epilepsy, Lennox-Gastaut syndrome, Rasmussen's encephalitis, childhood absence epilepsy, Ramsay Hunt Syndrome type II, benign epilepsy syndrome, benign infantile encephalopathy, benign neonatal convulsions, early myoclonic encephalopathy, progressive epilepsy and infantile epilepsy.

An isolated form of a compound of Formula I, IA, or IB (*e.g.*, anatabine or S-(-)-anatabine) or a pharmaceutically acceptable salt of such an isolated form may also be useful for treating the aura that accompanies seizures. Thus, impaired consciousness, oral automatisms, hand or limb automatisms, utterance of unintelligible sounds, tonic or dystonic posturing of extremities, head and eye deviation, bicycling or pedaling movements of the legs and other symptoms that comprise the aura also may be treated.

Neonatal seizures are associated with later neurodevelopmental and cognitive deficits including mental retardation, autism, and epilepsy, and it is estimated that up to 40% of cases of autism suffer from epilepsy or have a history of or seizures earlier in life. Accordingly, important target patients are infants, particularly neonates, and persons with a personal or family a history of seizure, mental retardation or autism.

This disclosure also provides methods and compositions for treating a patient post-seizure. In one embodiment, an isolated form of a compound of Formula I, IA, or IB (*e.g.*, anatabine or S-(-)-anatabine) or a pharmaceutically acceptable salt of such an isolated form is administered in conjunction with a second therapeutic agent, such as a neurotransmitter receptor inhibitor (*e.g.*, an inhibitor of an AMPA receptor, NMDA receptor GABA receptor, chloride cotransporters, or

metabatropic glutamate receptor), a kinase/phosphatase inhibitor (*e.g.*, an inhibitor of calmodulin kinase II (CamK II), protein kinase A (PKA), protein kinase C (PKC), MAP Kinase, Src kinase, ERK kinase or the phosphatase calcineurin), and/or a protein translation inhibitor.

Calmodulin kinase II (CamK II) inhibitors include KN-62, W-7, HA-1004, HA-1077, and staurosporine. Protein kinase A (PKA) inhibitors include H-89, HA-1004, H-7, H-8, HA-100, PKI, and staurosporine.

Protein kinase C (PKC) inhibitors include competitive inhibitors for the PKC ATP-binding site, including staurosporine and its bisindolylmaleimide derivitives, Ro-31-7549, Ro-31-8220, Ro-31-8425, Ro-32-0432 and Sangivamycin; drugs which interact with the PKC's regulatory domain by competing at the binding sites of diacylglycerol and phorbol esters, such as calphostin C, Safingol, D-erythro-Sphingosine; drugs which target the catalytic domain of PKC, such as chelerythrine chloride, and Melittin; drugs which inhibit PKC by covalently binding to PKC upon exposure to UV lights, such as dequalinium chloride; drugs which specifically inhibit Ca-dependent PKC such as Go6976, Go6983, Go7874 and other homologs, polymyxin B sulfate; drugs comprising competitive peptides derived from PKC sequence; and [0056]PKC inhibitors such as cardiotoxins, ellagic acid, HBDDE, 1-O-Hexadecyl-2-O-methyl-rac-glycerol, Hypercin, K-252, NGIC-1, Phloretin, piceatannol, and Tamoxifen citrate.

MAP kinase inhibitors include SB202190 and SB203580. SRC kinase inhibitors include PP1, PP2, Src Inhibitor No. 5, SU6656, and staurosporine. ERK kinase inhibitors include PD 98059, SL327, olomoucine, and 5-Iodotubercidin. Calcineurin inhibitors include tacrolimus and cyclosporine.

Protein translation inhibitors include mTOR inhibitors, such as rapamycin, CCI-779 and RAD 001.

## Methods of Treatment; Pharmaceutical Compositions

"Treat" as used herein refers to reducing a symptom of the inflammation or resulting ASD or seizure disorder but does not require complete cure, either of the inflammation or the disorder. "Reduction of a symptom" includes but is not limited to elimination of the symptom as well as reduction in frequency, severity, or duration of the symptom. Reduction of a symptom can be recognized subjectively by the individual or an observer of the individual or can be confirmed by clinical and/or laboratory findings. Patients who can be treated include adults, teenagers, children, and neonates.

An isolated form of a compound of Formula I, IA, or IB (*e.g.*, anatabine or S-(-)-anatabine or a pharmaceutically acceptable salt thereof) is administered to the individual at a dose sufficient to reduce a symptom of an Autism Spectrum Disorder or at a dose sufficient to reduce a symptom of a seizure disorder. Doses typically range from about 1 μg/kg to about 7 mg/kg body weight (*e.g.,* about I, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, or 5 μg/kg or about 0.1, 0.2, 0.3, 0.4, 0.5, 0.6, 0.7, 0.8, 0.9, I, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, or 5 mg/kg), about 1.5 μg/kg to about 5 μg/kg, about 1 μg/kg to about 10 μg/kg, about 0.01 mg/kg to about 7 mg/kg body weight, about 0.1 mg/kg to about 5 mg/kg; about 0.1 mg/kg to about 2 mg/kg, about I mg/kg to about 3 mg/kg, about 0.5 mg/kg to about 2 mg/kg, about 1 mg/kg to about 2 mg/kg, about 3 mg/kg to about 5 mg/kg, about 2 mg/kg to about 4 mg/kg, about 2 mg/kg to about 5 mg/kg, or about 0.5 mg/kg to about 1.5 mg/kg. Certain factors may influence the dose sufficient to reduce a symptom of a disorder (*i.e.,* an effective dose), including the severity of the disease or disorder, previous treatments, the general health, age, and/or weight of the individual, the frequency of treatments, the rate of release from the composition, and other diseases present. This dose may vary according to factors such as the disease state, age, and weight of the subject. For example, higher doses may be administered for treatments involving conditions which are at an advanced stage and/or life-threatening. Dosage regimens also may be adjusted to provide the optimum therapeutic response.

In some embodiments an isolated form of anatabine or a pharmaceutically acceptable salt thereof is administered. In some embodiments the anatabine is S-(-)-anatabine. In some embodiments tablets comprising about 600 μg anatabine citrate or S-(-)-anatabine citrate are administered from once to 25 times daily (*e.g.*, 1, 2, 3, 4, 5, 6, 7, 8, 9, 10, 11, 12, 13, 14, 15, 16, 17, 18, 19, 20, 21, 22, 23, 24, or 25) times daily. In other embodiments, the administered tablets comprise about 600 μg of a compound of Formula I, IA, or IB.

In some embodiments the dose sufficient to reduce the symptom of the disorder can include a series of treatments. For example, an individual can be treated with a dose of an isolated form of anatabine or S-(-)-anatabine or a salt thereof several times per day (*e.g.*, 2-12 or 4-10 times per day), once daily, or less frequently such as 1-6 times per week. In other embodiments, the compound administered is a compound of Formula I, IA, or IB, which is administered several times per day (*e.g.*, 2-12 or 4-10 times per day), once daily, or less frequently such as 1-6 times per week. Treatments may span between about 1 to 10 weeks (*e.g.*, between 2 to 8 weeks, between 3 to 7 weeks, for about 1, 2, 3, 4, 5, 6, 7, 8, 9, or 10 weeks). It will also be appreciated that a dose regimen used for treatment may increase or decrease over the course of a particular treatment.

In some embodiments an isolated form of anatabine or S-(-)-anatabine or a pharmaceutically acceptable salt of an isolated form of anatabine or S-(-)-anatabine can be administered to reduce the risk of developing an ASD (*i.e.,*

prophylactically). In other embodiments an isolated compound of Formula I, IA, or IB is administered. One can readily identify individuals with an increased risk or family history of a disorder. Other recognized indices of elevated risk of certain disorders can be determined by standard clinical tests or medical history.

Methods of isolating anatabine, including S-(-)-anatabine, are disclosed, for example, in PCT/US11/29613. In some embodiments anatabine is prepared via a benzophenoneimine pathway, as described in co-pending and commonly owned Application No. 12/729,346, filed March 23, 2010, the disclosure of which is incorporated herein by reference in its entirety. As an alternative to preparing anatabine synthetically, anatabine can be obtained by extraction from tobacco or other plants, such as members of the Solanaceae family, such as datura, mandrake, belladonna, capsicum, potato, nicotiana, eggplant, and petunia.

In some embodiments, an isolated form of anatabine or S-(-)-anatabine can be provided as one or more pharmaceutically acceptable (or food grade) salts of anatabine or S-(-)-anatabine. In other embodiments an isolated form of a compound of Formula I, IA, or IB is provided as one or more pharmaceutically acceptable (or food grade) salts of the compound. In general, salts may provide improved chemical purity, stability, solubility, and/or bioavailability relative to anatabine in its native form or to another compound of Formula I, IA, or IB. Non-limiting examples of possible anatabine salts are described in P. H. Stahl et al., Handbook of Pharmaceutical Salts. Properties, Selection and Use, Weinheim/Zürich:Wiley-VCH/VHCA, 2002, including salts of 1-hydroxy-2-naphthoic acid, 2,2-dichloroacetic acid, 2-hydroxyethanesulfonic acid, 2-oxoglutaric acid, 4-acetamidobenzoic acid, 4-aminosalicylic acid, acetic acid, adipic acid, ascorbic acid (L), aspartic acid (L), benzenesulfonic acid, benzoic acid, camphoric acid (+), camphor-10-sulfonic acid (+), capric acid (decanoic acid), caproic acid (hexanoic acid), caprylic acid (octanoic acid), carbonic acid, cinnamic acid, citric acid, cyclamic acid, dodecylsulfuric acid, ethane-1,2-disulfonic acid, ethanesulfonic acid, formic acid, fumaric acid, galactaric acid, gentisic acid, glucoheptonic acid (D), gluconic acid (D), glucuronic acid (D), glutamic acid, glutaric acid, glycerophosphoric acid, glycolic acid, hippuric acid, hydrobromic acid, hydrochloric acid, isobutyric acid, lactic acid (DL), lactobionic acid, lauric acid, maleic acid, malic acid (- L), malonic acid, mandelic acid (DL), methanesulfonic acid, naphthalene-1,5-disulfonic acid, naphthalene-2-sulfonic acid, nicotinic acid, nitric acid, oleic acid, oxalic acid, palmitic acid, pamoic acid, phosphoric acid, proprionic acid, pyroglutamic acid (- L), salicylic acid, sebacic acid, stearic acid, succinic acid, sulfuric acid, tartaric acid (+ L), thiocyanic acid, toluenesulfonic acid (p), and undecylenic acid.

In some aspects, a pharmaceutical composition for use in the disclosed methods comprises an isolated compound of Formula I, IA, or IB (*e.g.*, anatabine or S-(-)-anatabine) and a pharmaceutically acceptable vehicle, diluent, or carrier. An "isolated form" of, *e.g.*, anatabine or S-(-)-anatabine," as used herein, refers to anatabine or S-(-)-anatabine that either has been prepared synthetically or has been substantially separated from plant materials in which it occurs naturally.

Isolated forms of anatabine or S-(-)-anatabine or pharmaceutically acceptable salts of anatabine or S-(-)-anatabine can be provided together with other ingredients, for example, in the form of an elixir, a beverage, a chew, a tablet, a lozenge, a gum, and the like. In other embodiments an isolated form of a compound of Formula I, IA, or IB is so provided. In one embodiment, for example, a beverage may be in the form of a bottled water product containing about 100 ml to about 2,000 ml purified water and from about 0.00001 to about 0.0001 wt% of a water-soluble salt of anatabine or S-(-)-anatabine or of a compound of Formula I, IA, or IB. Additional inactive ingredients may be added to improve product characteristics, such as taste, color/clarity, and/or stability. The bottled water product may also contain other beneficial components, such as vitamins, proteinaceous ingredients, or the like.

Pharmaceutical compositions may be formulated together with one or more acceptable pharmaceutical or food grade carriers or excipients. As used herein, the term "acceptable pharmaceutical or food grade carrier or excipient" means a non-toxic, inert solid, semisolid or liquid filler, diluent, encapsulating material or formulation auxiliary of any type. For example, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

The pharmaceutical composition may be prepared by any suitable technique and is not limited by any particular method for its production. For example, anatabine or S-(-)-anatabine can be combined with excipients and a binder, and then granulated. The granulation can be dry-blended any remaining ingredients, and compressed into a solid form such as a tablet. In other embodiments a compound of Formula I, IA, or IB is so provided.

The pharmaceutical compositions may be administered by any suitable route. For example, the compositions may be administered orally, parenterally, by inhalation spray, topically, rectally, nasally, buccally, vaginally, via an implanted reservoir, or ingested as a dietary supplement or food. The term parenteral as used herein includes subcutaneous,

intracutaneous, intravenous, intramuscular, and intracranial injection or infusion techniques. Most often, the pharmaceutical compositions are readily administered orally and ingested.

The pharmaceutical compositions may contain any conventional non-toxic pharmaceutically-acceptable carriers, adjuvants or vehicles. In some cases, the pH of the formulation may be adjusted with acceptable pharmaceutical or food grade acids, bases or buffers to enhance the stability of the formulated composition or its delivery form.

Liquid dosage forms for oral administration include acceptable pharmaceutical or food grade emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylsulfoxide (DMSO) dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Solid dosage forms for oral administration include capsules, tablets, lozenges, pills, powders, and granules. In such solid dosage forms, the active compound is mixed with at least one inert, acceptable pharmaceutical or food grade excipient or carrier such as sodium citrate or dicalcium phosphate and/or a) fillers or extenders such as starches, lactose, sucrose, glucose, mannitol, and silicic acid, b) binders such as carboxymethylcellulose, alginates, gelatin, polyvinylpyrrolidone, sucrose and acacia, c) humectants such as glycerol, d) disintegrating agents such as agaragar, calcium carbonate, potato or tapioca starch, alginic acid, certain silicates, and sodium carbonate, e) solution retarding agents such as paraffin, f) absorption accelerators such as quaternary ammonium compounds, g) wetting agents such as cetyl alcohol and glycerol monostearate, h) absorbents such as kaolin and bentonite clay, i) lubricants such as talc, calcium stearate, magnesium stearate, solid polyethylene glycols, sodium lauryl sulfate, and mixtures thereof, and j) sweetening, flavoring, perfuming agents, and mixtures thereof. In the case of capsules, lozenges, tablets and pills, the dosage form may also comprise buffering agents.

The solid dosage forms of tablets, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract or, optionally, in a delayed or extended manner. Examples of embedding compositions which can be used include polymeric substances and waxes. Tablet formulations for extended release are also described in U.S. Pat. No. 5,942,244.

Each reference cited in this disclosure is incorporated herein in its entirety. The following examples illustrate but do not limit the scope of the disclosure set forth above.

## EXAMPLE 1

### NFκB-mediated transcription assays; Cytotoxicity assays

The effect of a range of doses of anatabine, nicotine, crude extract of smokeless tobacco, and alkaloid extract of smokeless tobacco was examined in an NFκB luciferase assay (inhibition of TNFα-induced NFκB activity). The smokeless tobacco used in these experiments was plain long-leaf Copenhagen tobacco purchased from a local vendor. Crude extract was extracted with methanol and water and clarified by centrifugation and filtration. The alkaloid extract was prepared from sodium hydroxide and methanol extraction, organic phase separation and purification. All treatment samples were prepared as a function of weight (μg/ml), and all samples were diluted in DMSO. Dilutions were made immediately before cell culture treatments and, in all cases, the final amount of DMSO did not exceed 1% in cell culture media.

Human endothelial kidney cells (HEK293) transfected with an NFκB luciferase reporter were challenged with TNFα for three hours, then samples were applied to the challenged cells. The results are shown in **FIGS. 1-3.**

Cytotoxicity assays using the supernatants from the treated cells were conducted using an LDH Cytotoxicity Detection Kit (Roche) according to the manufacturer's instructions. The results are shown in **FIGS. 4-6.**

As shown in **FIG. 1,** TNFα induces an increase in NFκB-mediated transcription of luciferase; administration of anatabine can reduce this transcription to control levels without cellular toxicity (**FIG. 4).** Crude extracts of smokeless tobacco, while not toxic to cells **(FIG. 5),** do not reduce TNFα-induced NFκB-mediated transcription (**FIG. 2).** Although not suitable for administration as pharmaceuticals, both nicotine and an alkaloid extract of smokeless tobacco reduce TNFα-induced NFκB-mediated transcription (**FIG. 3**); at higher doses, the alkaloid extract demonstrates pronounced cytotoxicity **(FIG. 6).**

## EXAMPLE 2

### Materials and methods

**Animals.** Male and female Sprague-Dawley rats (~ 200-250 grams) were obtained from Charles River Laboratories Inc., Wilmington, MA and used in compliance with the Animal Welfare Act, the Guide for the Care and Use of Laboratory Animals, and the Office of Laboratory Animal Welfare. Upon receipt at the vivarium, rats were examined by trained personnel to ensure acceptable health status. Rats were acclimated for at least 5 days prior to use.

Rats were housed 3 per cage. Cage size met or exceeded the requirements set forth by the Institute for Animal Laboratory Research Guide for the Care and Use of Laboratory Animals. The rats were kept in a room maintained at 64-84 °F (22-24 °C) with humidity set at 40-70 %. The room was illuminated with fluorescent lights timed to give a 12 hour-light, 12 hour-dark cycle. Standard rodent diet (PharmaServ lab diet 5001) and water were available for all rats. The feed was analyzed by the supplier, detailing nutritional information and levels of specified contaminants.

**Test compounds.** The following compounds were tested in the examples below:

(-) Nicotine hydrogen tartrate (Sigma Aldrich: N5260 Lot# 098K0676) 35.1 % (w/w) nicotine;
(R, S) Anatabine tartrate (2:3) (Toronto Research Chemicals, A637510, Lot# 9-BH W-79-2) 41.6% (w/w) Anatabine;
(+/-)-nicotine-3'-d3 (Toronto Research Chemicals: N412423, Lot# 9-BCC-114-2);
(R,S)-Antabine-2,4,5,6-d4 (Toronto Research Chemicals: A637505, Lot# 6-SG-82-1); and
anatabine polacrilex (Emerson Resource Inc. lot # JK02-145); purity was 5.18 % as per Certificate of Analysis

Certificates of analyses for anatabine and nicotine indicated 98% and 100% purity, respectively. Anatabine was stored at 4 °C in a desiccated environment (silica), protected from light. Nicotine was stored at room temperature. Vehicle was sterile phosphate buffered saline (PBS) (Amresco lot#2819B188).

**Supplies.** The following were obtained from Becton Dickinson, Franklin Lakes, NJ: MICROTAINER® Brand Tubes ($K_2$) EDTA (lot# 9050883); serum separator blood collection tubes (lot# 9104015); and sodium citrate blood collection tubes (lot# 8310564). Ten percent neutral-buffered formalin was from Sigma Aldrich, St. Louis, MO (batch# 019K4386).

## EXAMPLE 3

### Toxicokinetic evaluation of single doses of anatabine and nicotine in Sprague-Dawley Rats

This example reports evaluation of the toxicokinetics of anatabine and nicotine following a single intravenous injection in Sprague-Dawley rats.

### Summary

Anatabine was administered as a single intravenous (i.v.) injection at doses of 0.10, 0.75, or 1.0 mg/kg. Nicotine was administered as a single intravenous injection at a dose of 0.4 mg/kg. Six rats (3 males and 3 females) were dosed per dose group. Blood was collected for plasma at 15, 30, 60, 90, 120, 240, 360, 480, and 1440 minutes post i.v. administration. At the 1440 minute time point, animals were euthanized and perfused, and brains were removed and then homogenized. Plasma and brain homogenates were stored at -80 °C until analysis.

An additional 48 rats (24 males and 24 females) received a single intravenous dose via the tail vein at the same doses as mentioned above. At 30 and 360 minutes post administration, 6 rats (3 males and 3 females) per dose group, per time point were euthanized, bled via cardiac puncture, and perfused, and brains were collected. The brains were homogenized. Blood was spun, and plasma was collected. Plasma and brain homogenate were stored at -80 °C.

Both anatabine and nicotine can be measured in rat plasma and brain following a single bolus i.v. dose. The concentration of anatabine in plasma is dose-related. Both compounds are also rapidly cleared from plasma; however, the elimination half-life of anatabine is approximately 2- to 2.5-fold greater than that of nicotine ($t_{1/2}$, 1.64 to 1.68 hr for anatabine compared to 0.67 hr for nicotine). The apparent volume of distribution ($V_D$) for anatabine is also significantly greater than that of nicotine.

At all doses of anatabine the elimination half-life ($t_{1/2}$), mean residence time (MRT), and exposures ($AUC_{0\to\infty}$) tended to be higher for female rats compared to male rats; however, only at the highest dose of anatabine (1.0 mg/kg) was this difference statistically significant. At this dose level, the elimination half-life ($t_{1/2}$) of anatabine in females was 1.84 hr compared to 1.44 hr for males; mean residence time (MRT) was 2.80 hr for females compared to 2.18 hr for males; and exposure ($AUC_{0\to\infty}$) was 788.9 ng·hr/mL for females compared to 631.3 ng·hr/mL for males.

Anatabine and nicotine rapidly appear in brain tissue following i.v. administration, and the concentration of

anatabine is dose-dependent. At each dose level the mean concentration of anatabine appeared to be higher in the brains of female animals compared to males; however, the differences were not statistically significant.

Anatabine tartrate (2:3) or nicotine bitartrate was dissolved to the appropriate concentrations in sterile PBS for the i.v. formulations (Table 2). The dosing solutions for each test compound were prepared on the basis of the relative content of the anatabine or nicotine base so that the final concentrations are reflective of the actual base concentration. Four aliquots of each dose level formulation were collected and stored at -80 °C. The test compound, corresponding dose level, number of animals, and sample collection times for Phase I of the study are shown in Table 3. The test compound, corresponding dose level, number of animals, and sample collection times for Phase II of the study are shown in Table 4. The physical signs of each animal were monitored following administration of the test compound.

The animals were weighed prior to dosing and received a single i.v. dose of either test compound at a volume of 5 mL/kg. Blood was collected via the venus plexus (retro-orbital) into tubes containing ($K_2$) EDTA. No more than 0.5 mL was collected per time point. For the 1440-minute time point of Phase I or the 30- and 360-minute time points of Phase II, the animals were euthanized, bled via cardiac puncture, and perfused. The brain was removed, weighed, homogenized in sterile 0.9% saline at a volume equal to its weight, and stored at -80 °C.

Plasma was separated according to the instructions for MICROTAINER® brand collection tubes (3 minutes, 2000x g). Plasma was decanted into microfuge tubes and stored at -80° C. Remaining test compound was stored at -80 °C.

**Analytical Methods**

The signal was optimized for each compound by electrospray ionization (ESI) positive or negative ionization mode. A single ion mode (SIM) scan was used to optimize the Fragmentor for the precursor ion and a product ion analysis was used to identify the best fragment for analysis and to optimize the collision energy. The fragment which gave the most sensitive and specific signal was chosen.

**Sample preparation.** Plasma and brain samples were treated with three volumes of methanol containing internal standard at I μM (either (+/-)-nicotine-3'-$d_3$ for nicotine or (*R,S*)-Antabine-2,4,5,6-$d_4$ for anatabine), incubated 10 min at 4 °C, and centrifuged. The amount of the test agent in the supernatant was determined by liquid chromatography tandem mass spectrometry (LC/MS/MS).

**Analysis.** Samples were analyzed by LC/MS/MS using an Agilent 6410 mass spectrometer coupled with an Agilent 1200 high pressure liquid chromatography (HPLC) and a CTC PAL chilled autosampler, all controlled by MassHunter software (Agilent). After separation on a hydrophilic interaction liquid chromatography (HILIC) HPLC column (Sepax) using an acetonitrile-ammonium acetate/acetic acid gradient system, peaks were analyzed by mass spectrometry (MS) using ESI ionization in multiple reaction monitoring (MRM) mode. MassHunter software was used to calculate the concentration of the test compounds in samples from the peak area using the appropriate calibration curves.

**Recovery.** Recovery standards were prepared by spiking blank matrix (plasma or brain homogenate) prior to deproteination or after with 23, 62, or 1667 ng/mL of test compound. Deproteination was done by adding 3 columns of methanol containing internal standard with centrifugation to pellet the precipitated protein. Recovery was calculated by dividing the area ratio (peak area of compound over internal standard of the precipitated sample over the recovery standard multiplied by 100. For example: area ratio of spiked plasma/area ratio of spiked deproteinated plasma x 100.

**Calibration samples.** Calibration curves were determined for both rat plasma and brain homogenate. Calibration samples were prepared by diluting a 50x stock solution of the test compound in PBS with blank matrix to the appropriate concentration and these samples were prepared as described above in the sample preparation. Stock solutions were prepared by serial dilution as shown in Table 5.

**Results**

**Physical Signs.** All males and two females that received nicotine at 0.4 mg/kg experienced tremors immediately post dose and recovered within 2 to 4 minutes. One male (7C) and two females (8A and 8C) in this group also experienced labored breathing which lasted 2 to 4 minutes post dose. The same male (7C) was lethargic and recovered approximately 8 minutes post dose. All other animals in each dose group appeared normal following the administration of the test compounds.

**Method Development.** Table 6 shows the results of the LC/MS/MS method development for the determination of the appropriate ionization conditions and the mass to charge ratios (m/z) of the parent and product ions for anatabine and nicotine, and their deuterated analogues. The indicated product m/z ratios were used for the analysis of the relevant test samples.

The product ion spectra and sample chromatograms for each compound in Table 6 are shown in **FIGS. 12-19.** The limits of detection (LOD) of anatabine and nicotine and their lower (LLQ) and upper (ULQ) limits of quantitation were derived from the appropriate calibration curves for each test compound and are shown in Table 7.

Table 8 provides data on the per cent recovery of each test compound from either rat plasma or brain as a

function of the given concentration. Except for the anatabine sample at the LOD and the nicotine samples in rat brain, recovery was generally greater than 90 per cent.

### Analysis of Dosing Solutions

Table 9 summarizes the analyses of the dosing solutions used in this study. The per cent differences between the actual and expected concentrations are shown. Except for the lowest dose of anatabine, which was 70 % of the expected concentration, the actual concentrations of test compounds were within 20 % of the expected levels.

### Plasma Phormacokinetic Results & Analysis

Table 14 lists the plasma concentrations of anatabine and nicotine for all animals at each time point. Table 15 summarizes this data in terms of the mean plasma concentrations of the test compound at each time point for males, females and both genders combined. This data is presented graphically in **FIG. 7** and **FIG. 8** (semi-log plot). The 24-hr data points from all treatment groups were below the limits of quantitation. Between approximately 6 and 8 hours the plasma concentrations of nicotine and anatabine (0.1 mg/kg) were below the limits of quantitation.

Table 10 and Table 11 provide comparisons for several pharmacokinetic parameters between the different treatment groups and between male and female animals. Both nicotine and anatabine can be measured in rat plasma following a single i.v. bolus and their concentrations appear to be dose-related. The elimination half-life ($t_{1/2}$) for each of the anatabine treatment groups was significantly greater than that for the nicotine treatment group (2.1 x to 2.5 x greater; 0.67 hr for nicotine compared to 1.44 to 1.68 hr for anatabine). The elimination half-lives were similar among the anatabine treatment groups. The longer half-life for anatabine is reflected in the longer mean residence times (MRT), which are about 2-fold longer for anatabine compared to nicotine. Finally, the apparent volume of distribution ($V_D$) was lower for the nicotine group compared to the anatabine treatment groups. Amongst the anatabine treatment groups, $V_D$ was significantly greater for the 0.1 mg/kg dose group compared to either of the two higher doses; however, it is not known whether this is a real difference or whether it is due to variability and the fewer number of measurable data points at the low dose.

Table 11 shows a comparison of these same parameters between male and female rats within each treatment group. There were no statistically significant differences between males and females except in the highest anatabine treatment group (1.0 mg/kg) where the females exhibited a longer elimination half-life and therefore, longer mean residence time than the males ($t_{1/2}$, 1.84 ± 0.16 hr and MRT, 2.80 ± 0.24 hr, females compared to $t_{1/2}$, 1.44 ± 0.08 and MRT, 2.18 ± 0.12 hr, males). This difference is apparent for all treatment groups, although it only achieved statistical significance in the highest anatabine group. The females in this treatment group also displayed a much greater overall exposure ($AUC_{0\rightarrow\infty}$) to anatabine than the male animals. This difference is depicted in **FIG. 9,** which shows the dose-exposure relationship for anatabine and nicotine. Overall, there appears to be a linear response between dose and exposure for anatabine; it is not possible to determine if the female animals display a non-linear response at high doses of anatabine.

**FIG. 11** shows the dose-concentration response for the 0.5-hour time point for males and females at each dose level. It appears that the brain levels of anatabine begin to level off between 0.75 mg/kg and 1.0 mg/kg.

Table 14 lists the concentrations of anatabine and nicotine in the brain extracts for all animals at each time point. Table 15 summarizes this data in terms of the mean concentrations of the test compound per gram of brain tissue at each time point for males, females and both genders combined. This data is presented graphically in **FIG. 10** and in tabular form in Table 12. After the 6-hour time point most concentrations were below the limits of quantitation; however, the test compound concentration was quantifiable in several samples at 24-hours.

**FIG. 11** shows the dose-concentration response for the 0.5-hour time point for males and females at each dose level. It appears that the brain levels of anatabine begin to level off between 0.75 mg/kg and 1.0 mg/kg.

### Discussion

All males and two females that received nicotine at 0.4 mg/kg experienced tremors immediately post dose; however they recovered within 2 to 4 minutes. One male (7C) and two females (8A and 8C) in this group also experienced labored breathing which lasted 2 to 4 minutes post dose. The same male (7C) was lethargic and recovered approximately 8 minutes post dose. All animals in each of the anatabine dose groups appeared normal immediately following administration of the test compounds and no obvious adverse signs were observed.

Both nicotine and anatabine can be measured in rat plasma following a single, bolus, i.v. dose and their concentrations appear to be dose-related. The elimination half-life of anatabine is approximately 2- to 2.5-fold greater than that of nicotine, and this is also reflected in a longer mean residence time, which is approximately twice as long as that for nicotine. The 24-hr data points from all treatment groups were below the limits of quantitation and it appears that

at the doses selected, the test compounds are cleared from rat plasma between 8 and 24 hours post-administration.

The apparent volume of distribution ($V_D$) was also significantly lower for the nicotine group compared to the anatabine treatment groups. Amongst the anatabine treatment groups, $V_D$ was significantly greater for the 0.1 mg/kg dose group compared to either of the two higher doses; however, it is not known whether this is a real difference or whether it is due to variability and the fewer number of measurable data points at the low dose.

When comparisons between male and female animals were conducted for these same parameters, within each treatment group, there were no statistically significant differences observed except for the highest anatabine treatment group (1.0 mg/kg) where the females exhibited a longer elimination half-life and therefore, longer mean residence time than the males ($t_{1/2}$, 1.84 $\pm$ 0.16 hr and MRT, 2.80 $\pm$ 0.24 hr, females compared to $t_{1/2}$, 1.44 $\pm$ 0.08 and MRT, 2.18 $\pm$ 0.12 hr, males). In fact, these differences between male and female animals were apparent for all treatment groups, although statistical significance was achieved only at the highest anatabine dose tested. The females in this treatment group also displayed a much greater overall exposure ($AUC_{0\rightarrow\infty}$) to anatabine than the male animals. Overall, there is a linear response between dose and plasma concentrations or exposure to anatabine in both male and female rats; although the response appears to be somewhat greater in female animals and is more pronounced at the higher dose levels. It is not possible to determine from the data if the female animals display a non-linear response at higher doses of anatabine.

Both anatabine and nicotine rapidly appear in brain tissue following i.v. administration. The concentrations of anatabine are dose-dependent but appear to level off between 0.75 mg/kg and 1.0 mg/kg. This observation is based on the levels measured only at the 0.5-hour time point and a greater number of time points are required for a more thorough evaluation. There were no statistically significant differences in the concentrations of either test compound in brain between male and female animals; however at each dose level the mean concentrations in the brains of females tended to be somewhat higher.

## EXAMPLE 4

### Toxicokinetic evaluation of single doses of anatabine and nicotine with a 14-day observation period

This example reports the evaluation of the toxicity of anatabine or nicotine for a period of fourteen days following a single intravenous injection in Sprague-Dawley rats. The toxicity of anatabine and nicotine was evaluated after a single intravenous (i.v.) injection in the rat. Anatabine was administered as a single intravenous injection at doses of 0.10, 0.75, or 1.5 mg/kg. Nicotine was administered as a single intravenous injection at a dose of 1.50 mg/kg. One control group of animals received a single i.v. dose of the vehicle at 5 mL/kg. Ten rats (5 males and 5 females) were dosed per group. Due to animal mortality in the nicotine-dosed group, the surviving animals were taken off study and a separate nicotine tolerability study was conducted. One female received a single i.v. dose of 1.25 mg/kg, and 3 females received a single i.v. dose of 1.0 mg/kg. Following the tolerability study, a group of 5 males and 5 females received a single i.v. dose of nicotine at 0.75 mg/kg.

All rats dosed with vehicle or anatabine, and the animals dosed with 0.75 mg/kg of nicotine were observed daily for 14 days. Body weight and food consumption was measured daily for 14 days. On day 15, urine was collected on all surviving animals. The animals were euthanized and bled via cardiac puncture, and blood was collected for analysis. Tissues were collected, weighed, evaluated for gross abnormalities, and stored in 10% neutral-buffered formalin.

All groups appeared normal immediately after dosing except for the animals dosed with 1.5 mg/kg of anatabine and those dosed with 1.5 mg/kg of nicotine. Both males and females dosed with 1.5 mg/kg of anatabine experienced tremors upon compound administration. The animals appeared normal by 15 minutes post dose. Upon completion of the 1.5 mg/kg dose of nicotine, tremors and rigidity were observed in all dosed animals. The tremors were more severe in the females. One male did not survive, whereas the other 4 appeared normal after 15 minutes. Three females were dosed and two died within 5 minutes of dosing; the remaining 2 females were not dosed due to the morbidity in the group. The surviving animals from this group were removed from study. These results suggest that both anatabine and nicotine affect both the peripheral and central nervous systems.

During the tolerability study, all rats (1 female dosed with 1.25 mg/kg of nicotine and 3 females dosed with 1.0 mg/kg of nicotine) experienced severe tremors upon completion of dosing, but all returned to normal by 20 minutes post dose. These animals were not included in the 14-day observation period.

Both males and females dosed with nicotine at 0.75 mg/kg experienced tremors upon compound administration but returned to normal within 15-20 minutes post dose. One male and two females died post dose. Surviving animals in all groups appeared normal throughout the 14-day observation period. The body weights for both male and female rats in the nicotine group were lower than those in the control and anatabine treatment groups; however, these were still within the study-specified range. Consequently body weight gain for this treatment group was also somewhat lower than the vehicle controls. Food consumption was similar among the groups over the 14-day period; however, consumption by males treated with 0.1 mg/kg or 1.5 mg/kg anatabine appeared to be somewhat higher than animals in the control

group. This is not considered to be a treatment-related effect.

Hematology and blood chemistries for male and female animals were analyzed and evaluated for differences between the individual treatment groups and the relevant vehicle controls. All treatment groups showed no significant differences relative to the controls and/or the values were well within the normal ranges expected for this species. Similarly, no notable differences in any of the urinalysis parameters were observed between animals treated with either anatabine or nicotine, relative to the controls.

Anatabine or nicotine was dissolved to the appropriate concentrations in sterile PBS for the i.v. formulations (see Table 16). The dosing solutions for each test compound were prepared on the basis of the relative content of the anatabine or nicotine base so that the final concentrations reflect the actual base concentrations. Four aliquots of each dose formulation were collected and stored at -80 °C. The test compound, corresponding dose level, number of animals, and frequency of observations are shown in Table 17.

The animals were weighed prior to dosing and received a single i.v. dose via the lateral tail vein of either test compound or vehicle at a volume of 5 mL/kg. Due to animal mortality in the nicotine-dosed group (1.5 mg/kg), the surviving animals were taken off study and a separate nicotine tolerability study was conducted.

**Nicotine Tolerability Study**

One female rat was dosed intravenously with 1.25 mg/kg of nicotine, and three females were received 1.0 mg/kg intravenously. Following the tolerability study, an additional group was added to the study. Five males and five females received a single intravenous dose of nicotine at 0.75 mg/kg. All animals were observed daily. Body weight and food consumption was measured daily, with any abnormal observations noted. Average daily body weights and food consumption was tabulated with standard deviation calculated.

On day 15, urine was collected on all surviving animals for urinalysis. The animals were euthanized and bled via cardiac puncture. Blood was collected for hematology, clinical chemistry, and coagulation analysis. Tissues were collected, weighed, and stored in 10% neutral-buffered formalin for possible future analysis. The tests and tissues collected are summarized in Table 18.

**Results**

**Dosing Solution Analysis**

Table 19 summarizes the dosing solutions used during the conduct of this study. The per cent differences between the actual and expected concentrations of the test compounds are shown. The actual concentrations were within 20 per cent of the expected levels.

**General Observations**

All groups appeared normal immediately after dosing except for the animals dosed with 1.5 mg/kg of anatabine and those dosed with 1.5 mg/kg of nicotine. Both males and females dosed with 1.5 mg/kg of anatabine experienced tremors upon compound administration. The animals appeared normal by 15 minutes post dose. Following administration of the 1.5 mg/kg dose of nicotine, tremors and rigidity were observed in all animals. The tremors were more severe in the females. One male did not survive, whereas the other 4 appeared normal after 15 minutes. Three females in this group were dosed and two died within 5 minutes of dosing; the remaining 2 females were not treated due to the observed morbidity in the group. The surviving animals from this group were removed from the study.

During the tolerability study, all rats (1 female dosed with 1.25 mg/kg of nicotine and 3 females dosed with 1.0 mg/kg of nicotine) experienced severe tremors upon completion of dosing, but all returned to normal by 20 minutes post dose. These animals were not included in the 14-day observation period.

Both males and females dosed with nicotine at 0.75 mg/kg experienced tremors upon compound administration, but returned to normal within 15-20 minutes post dose. One male and two females died post dose.

Surviving animals in all groups appeared normal throughout the 14-day observation period.

**Body Weights, Growth Rates and Food Consumption**

The daily measured body weights for each animal are tabulated in Tables 28A-F and the average daily food consumption is summarized in Tables 29A, B. These data are summarized in Table 20 for the average weight gain over the 14-day observation period and the average daily food consumption, by treatment group and gender. **FIG. 20** shows the mean body weights of animals in each treatment group on the day of dosing (Day 0) and for each day, thereafter.

The average weight gains for animals in each treatment group over the 14-day observation period were similar

to those in the vehicle control group, except for the nicotine-dosed group of male animals that exhibited weight gains that were significantly lower than the controls. The mean increase in the weight of females of the nicotine-dosed group was also lower than that of the vehicle control, though not statistically significant at the 5 per cent level. It should be noted that the mean weights of the male and female animals in the nicotine-treated group at Day 0 were lower than their corresponding genders in the vehicle control. The difference for males was statistically significant (Vehicle: 234.6 $\pm$ 9.9 g *versus* Nicotine: 216.0 $\pm$ 6.2 g; *p*=0.014), although that for females was not (Vehicle: 209.8 $\pm$ 7.3 g versus Nicotine: 195.3 $\pm$ 10.4 g; *p*=0.058).

The average daily food consumption per animal was statistically higher in the males of the 0.1 mg/kg and 1.5 mg/kg anatabine treatment groups. This difference is not considered to be clinically significant or related to any treatment effects.

Overall, although some differences in the changes in weight and food consumption were statistically significant, they are not considered to be treatment-related.

**Necropsy Observations and Organ Weights**

Upon necropsy and organ collection no noticeable differences or abnormalities were observed between the vehicle-dosed animals and the test compound-dosed animals. Individual organ weights can be found in Table 36. Several statistically significant differences in organ weights were noted (see Table 21 and Table 22); however, they do not appear to be dose-related and likely due to the small sample sizes and variability in the organ collection. In general, several organ weights tended to be lower in the nicotine-treated group, although this observation is likely related to the lower animal weights in this group relative to the controls.

**Hematology and Coagulation Parameters**

Plasma samples collected for hematology were analyzed, and individual values for the various parameters for each animal are listed in Table 31 (normal ranges, Table 30) and these are summarized in terms of descriptive statistics in Table 23A, Table 23B, and Table 24. Also shown are statistical comparisons between the vehicle controls and the various treatment groups, subdivided by gender.

In general, there were few significant differences between the treatment groups and the vehicle control group for either gender. Female rats in 0.1 mg/kg anatabine group showed a small but statistically significant decrease in mean corpuscular hemoglobin concentration (MCHC) relative to the control; however, the values are still within the normal range for this species. Similarly, females in the 1.5 mg/kg anatabine and 0.75 mg/kg nicotine treatment groups showed small, but statistically significant decreases in mean corpuscular volume (MCV) and mean corpuscular hemoglobin (MCH), although these values were still within the normal range for this species as well.

Males and females in the 0.75 mg/kg and 1.5 mg/kg anatabine groups showed a statistically significant decrease in reticulocyte count compared to the control animals; however, these values are also well within the normal range for this parameter.

There were no notable differences in red blood cells, white blood cells, platelet counts, lymphocyte, monocyte, eosinophil and basophil counts, or neutrophil segmentation.

Individual values for the coagulation parameters activated partial thromboplastin (aPTT) and prothrombin times (PT) for each animal are listed in Table 34 (normal ranges, Table 30). These are summarized in terms of descriptive statistics in Table 25. Also shown are statistical comparisons between the vehicle controls and the various treatment groups, subdivided by gender. There were no significant differences in aPTT or PT between the vehicle control and each of the anatabine treatment groups; although the aPTT values for all these groups were outside the normal range. In both male and female animals of the nicotine group, however, aPTT was significantly lower relative to the vehicle control group, indicative of faster clotting times due to the intrinsic, contact activation pathway. The origin of this difference is not known, although the values are within the normal range for this species.

**Clinical Chemistry**

Plasma samples collected for blood chemistries were analyzed, and individual values for the various parameters for each animal are listed in Table 33 (normal ranges, Table 32), and these are summarized in terms of descriptive statistics in Tables 26A, 26B, 27A, and 27B. Also shown are statistical comparisons between the vehicle controls and the various treatment groups, subdivided by gender.

Values for all clinical chemistry parameters were within the respective normal ranges. There were several parameters where statistically significant differences were noted between treatment groups and controls. Specifically, males treated with anatabine at 0.75 mg/kg and 1.5 mg/kg showed slight increases in albumin levels, as did females treated with 0.1 mg/kg and 0.75 mg/kg anatabine, but not at 1.5 mg/kg. Total protein was slightly increased in males in

all anatabine treatment groups and the nicotine group relative to vehicles controls. In females, total protein was somewhat higher only in the 0.1 mg/kg anatabine and nicotine groups. Finally, as with total protein, globulins were marginally higher at all anatabine dose levels and the nicotine dose group in males. Globulins were also slightly higher in females in the 0.1 mg/kg anatabine and nicotine groups. The higher globulin levels, but not albumin, in the nicotine group is reflected in slightly lower A/G ratios, for both genders. Nevertheless, all the reported values for albumin, globulins and total protein were within the normal range for this species. There were small, but statistically significant differences noted for calcium levels in males in the nicotine-treated group and for sodium levels in males at 0.75 mg/kg and 1.5 mg/kg anatabine and females in the 1.5 mg/kg anatabine treatment groups. The values are well within normal ranges and therefore, not clinically significant.

**Urinalysis**

Individual values of the urinalysis parameters for each animal are listed in Table 35. There were no notable differences between the active treatment groups and controls and the observations are all consistent with those expected for this species.

**Discussion**

The toxicity of anatabine and nicotine was evaluated after a single intravenous (i.v.) injection in the rat. Anatabine was administered at doses of 0.10, 0.75, or 1.5 mg/kg. Nicotine was administered at a dose of 1.50 mg/kg, initially; however, due to mortality and significant adverse effects observed at this dose and at lower doses of 1.0 mg/kg and 1.25 mg/kg, a separate group was included in the study and dosed with nicotine at 0.75 mg/kg. One group of animals received a single i.v. dose of the vehicle at 5 mL/kg. Ten rats (5 males and 5 females) were dosed per group.

All rats dosed with vehicle or anatabine, and the animals dosed with 0.75 mg/kg of nicotine were observed daily for 14 days. Body weight and food consumption was measured daily for 14 days. On day 15, urine was collected on all surviving animals. The animals were euthanized, bled via cardiac puncture, and blood was collected for analysis. Tissues were collected, weighed, any gross abnormalities were noted, and stored in 10% neutral-buffered formalin for possible future analysis.

All animals, at all dose levels of anatabine, survived the study; however, those in the 1.5 mg/kg anatabine group experienced tremors and shaking immediately after test compound administration, which lasted for approximately 15 minutes post-treatment. In the nicotine treatment group (0.75 mg/kg), one male animal and 2 females died following test compound administration, and all animals experienced tremors and shaking for up to 20 minutes post-administration. These results suggest that both anatabine and nicotine affect both the peripheral and central nervous systems.

The growth rates and food consumption in all anatabine treatment groups were similar to their appropriate male or female vehicle controls. Male rats in the nicotine treatment group had a slightly lower growth rate; however, this is unlikely to be related to the test compound. This group of animals began the study at a lower average weight than males in the control or anatabine treatment groups. The food consumption in males, in the 0.1 mg/kg and 1.5 mg/kg anatabine groups was somewhat higher than controls and although the result was statistically significant it is not likely to be related to an effect of the test compound.

At necropsy, no noticeable differences or gross abnormalities were observed in any of the organs collected between the vehicle-treated and the test compound-treated animals. Several statistically significant differences in organ weights were noted; however, they do not appear to be dose-related and are likely due to the small sample sizes and the inherent variability associated with organ collection. The weights of heart, liver and kidneys in males, and thymus and heart in females of the nicotine-treated group were significantly lower than those of the corresponding vehicle controls; however, this observation is likely related to the lower overall animal weights in this group relative to the controls.

The hematology parameters for all treatment groups and genders were within the normal ranges expected for this species or displayed no significant differences when compared to the vehicle controls. Activated partial thromboplastin and prothrombin times were similar for all anatabine treatment groups relative to the controls; however, they were higher than the expected normal range. Both males and females in the nicotine group displayed significantly shorter clotting times via the intrinsic or contact activation pathway (aPTT) compared to the relevant control animals; however, the values were within the normal ranges for this species. Clotting times via the extrinsic or tissue factor pathway as determined by prothrombin times (PT) were normal.

Values for all clinical chemistry parameters were within the respective normal ranges or showed no differences relative to the vehicle control group.

Evaluation of the individual urinalysis parameters for each animal showed no notable differences between the active treatment groups and controls.

## EXAMPLE 5

### Toxicokinetic evaluation in Sprague-Dawley rats of oral multi-dose administration of anatabine

This example reports the results of an evaluation of the pharmacokinetics of anatabine following multiple oral doses in Sprague-Dawley rats.

### Summary

The plasma pharmacokinetic profile of orally administered anatabine was investigated in the rat. This study consisted of two groups of 8 animals each, 4 males and 4 females. One group received a total of 0.6 mg anatabine per kilogram body weight (BW) and the second group received 6.0 mg anatabine per kilogram BW in three, divided, oral, doses of 0.2 mg/kg BW (0.6 mg total) or 2.0 mg/kg BW (6.0 mg total). The test compound was administered as anatabine polacrilex and each dose was administered at 0, 4, and 8 hours and was administered in a volume of 5 mL/kg BW. Blood was collected for plasma at 30, 60, 240, 270, 300, 480, 540, 600, 720 and 1440 minutes post initial dose.

All animals in both treatment groups appeared normal immediately following each administration of the test compound and no adverse signs were observed for the duration of the observation and plasma sampling period.

The mean time to maximal plasma concentration following the first two oral doses ranged from 0.50 to 0.88 ± 0.25 hr. There were no significant differences between gender or dose group. After the third dose of test compound, the mean time to maximal plasma concentration ranged from 1.00 to 2.00 ± 1.41 hr. Within each dose group there were no significant differences in $C_{p, max}$ between males and females and nor was there any significant change in this parameter over time. In females of the high dose group $C_{p, max}$ appeared to increase from 259.8 ± 35.4 ng/mL to 374.8 ± 122.9 ng/mL; however, the trend was not statistically significant.

There were two, observable, minima following the first two oral doses of anatabine polacrilex. In general, the minima were not significantly different from one another over time, except for females of the high dose group, which increased from 51.5 ± 26.0 ng/mL to 180 ± 31 ng/mL.

The total exposure, elimination half-lives, mean transit times and mean absorption times did not differ significantly between male and female rats within the two treatment groups. When these data are combined and grouped according to dose level the total exposure is significantly greater at the high dose as would be expected; however, the terminal elimination half-life is also significantly higher in the 6.0 mg/kg B W group compared to the 0.6 mg/kg B W dose group.

The overall elimination half-life of anatabine following the first oral dose was 1.93 ± 0.73 hr, the mean transit time was 3.01 ± 1.25 hr and the mean absorption time was 0.56 ± 1.25 hr. The mean absorption time of 0.56 compares favorably with the calculated $T_{max}$ values following the first two doses and indicates that the absorption of anatabine occurs within the first 30 to 60 minutes after oral administration.

Anatabine was stored at 4 °C, protected from light. The vehicle was sterile phosphate buffered saline (PBS) (Amresco).The test compound was formulated in sterile phosphate buffered saline (PBS) based on the content of anatabine base in the anatabine polacrilex. Two formulations were prepared; one for each of the two treatment groups. The test compound was formulated for each treatment group just prior to the first dose administration and constantly stirred until dosing was completed (Table 37). Four aliquots of each dose level formulation were collected and stored at - 80 °C. The test compound, corresponding dose level, and number of animals are shown in Table 38. The sample collection times are shown in Table 39.

The physical signs of each animal were monitored following administration of the test compound.

The animals were weighed prior to dosing and received three doses p.o. of test compound at a volume of 5 mL/kg. Blood was collected via the venus plexus (retro-orbital) into tubes containing ($K_2$) EDTA. No more than 0.5 mL was collected per time point. For the 1440-minute time point the animals were euthanized, and bled via cardiac puncture.

Plasma was separated as per package instructions for MICROTAINER® brand collection tubes (3 minutes, 2000x g). Plasma was decanted into microfuge tubes and stored at -80° C. Remaining test compound was placed at -80 °C.

**Sample preparation.** Plasma samples were treated with three volumes of methanol containing internal standard at 1 μM (*R,S*)-Antabine-2,4,5,6-$d_4$), incubated 10 min at 4 °C, and centrifuged. The amount of the test agent in the supernatant was determined by LC/MS/MS.

**Analysis.** Samples were analyzed by LC/MS/MS using an Agilent 6410 mass spectrometer coupled with an Agilent 1200 high pressure liquid chromatography (HPLC) and a CTC PAL chilled autosampler, all controlled by MassHunter software (Agilent). After separation on a Hydrophilic interaction liquid chromatography (HILIC) HPLC column (Sepax) using an acetonitrile-ammonium acetate/acetic acid gradient system, peaks were analyzed by mass spectrometry (MS) using ESI ionization in multiple reaction monitoring (MRM) mode. MassHunter software was used to calculate the concentration of the test compounds in samples from the peak area using the appropriate calibration curves.

**Calibration samples.** Calibration curves were determined in rat plasma. Calibration samples were prepared

by diluting a 50x stock solution of the test compound in PBS with blank matrix to the appropriate concentration and these samples were prepared as described above in the sample preparation. Stock solutions were prepared by serial dilution as shown in Table 40.

**Data Analysis.** Descriptive statistics were calculated for all pharmacokinetic parameters. Elimination half-lives ($t_{1/2}$) were calculated by linear regression of logarithmically transformed plasma concentration data for each period between doses and following the final dose.

Total areas under the plasma concentration curves (AUC) and under the first moment curves (AUMC) were calculated using linear trapezoidal summation across all concentration time points as well as for intervals between each dose administration and following the final dose. For the interval following the first oral dose of anatabine polacrilex, mean transit times (MTT) were calculated from the corresponding ratio of AUMC to AUC. Mean absorption times (MAT) were calculated according to the following relation:

$$MAT = MTT - MRT,$$

where MRT represents the mean residence time. This was calculated from the mean residence times.

The statistical comparison of parameters between male and female animals was made using a two-tailed, unpaired, *t*-test with a 95 per cent confidence interval. Repeated-measures analysis of variance (ANOVA) was used for multiple comparisons of $C_{p,\ max}$ involving successive determinations on the same group of animals.

**Results**

**Physical Signs.** No adverse events were observed.

**Method Development.** Table 41 shows the results of the LC/MS/MS method development for the determination of the appropriate ionization conditions and the mass to charge ratios (m/z) of the parent and product ions for anatabine and its deuterated analogue as determined above. The indicated product m/z ratios were used for the analysis of the relevant test samples.

See Example 3 for the product ion spectra and sample chromatograms for each compound in Table 41. The limits of detection (LOD), lower (LLQ), and upper (ULQ) limits of quantitation was derived from the calibration curve and are shown in Table 42.

**Analysis of Dosing Solutions.** Table 43 provides a summary of the analyses of the dosing solutions used during the conduct of this study. The per cent differences between the actual and expected concentrations are shown. The lowest dose of anatabine, which was 63% of the expected concentration and the high dose was 84% of the expected level.

**Plasma Pharmacokinetic Results & Analysis. FIG. 21A and FIG. 21B** show the mean plasma anatabine concentration-time curves for male and female rats in each of the two dose groups: 0.6 mg/kg (**FIG. 21A**) and 6.0 mg/kg BW (**FIG. 21B**). **FIG. 22A** and **FIG. 22B** show the same data with the values from both males and females combined. In each instance, three plasma concentration maxima can be observed corresponding to the administration of the three divided doses of anatabine polacrilex at 0, 4 and 8 hours. Similarly, two anatabine plasma concentration minima are found prior to administration of the final dose.

The mean maxima and minima anatabine plasma concentrations ($C_{p,\ max}$, $C_{p,\ min}$) for males and females in each dose group are recorded in Table 44 along with the mean time to maximal concentration following each of the three doses ($T_{max}$). Statistical comparisons between male and female animals within each dose group revealed no significant differences in any of the parameters, except for the second plasma concentration minimum ($C_{p,min(2)}$) in both treatment groups; 15.3 ± 5.5 ng/mL versus 7.5 ± 1.7 ng/mL in the 0.6 mg/kg BW treatment group, and 93 ± 16 ng/mL versus 180 ± 31 ng/mL in the 6.0 mg/kg BW treatment group. **FIG. 23A** and **FIG. 23B** show the data in Table 44 plotted as a function of time.

The times to reach maximal concentration generally occurred within 0.5 hr and 1.0 hr post administration in both treatment groups and for both genders, following doses one and two (see Table 45). After the third dose, $t_{max(3)}$ was generally between 1.0 and 2.0 hours post-administration; however, it should be noted that the earliest sampling point was at 1 hr following this dose.

Table 45 shows a comparison of the plasma concentration maxima and minima over time for male and female rats in both treatment groups. There were no statistically significant changes in any of these parameters except for the plasma concentration minima for female rats in the high dose group; $C_{p,\ min}$ increased from 51.5 ± 26.0 ng/mL to 180.0 ± 30.7 ng/mL.

The mean exposures (AUC), elimination half-lives ($t_{1/2}$), mean transit times (MTT) and mean absorption times (MAT) are reported in Table 46 for male and female animals in the two treatment groups. There are no significant

differences between the genders in any parameter, at either dose level.

When the male and female data are combined, as shown in Table 47, there is a significant difference in total exposure as would be expected as a consequence of the two different dose levels ($AUC_{0\rightarrow\infty}$; 285 ± 77 ng·hr/mL versus 3496 ± 559 ng·hr/mL). There is also a significant difference in the terminal elimination half-life between the two treatment groups ($t_{1/2,\ terminal}$; 1.79 ± 0.64 hr versus 4.53 ± 1.77 hr), where $t_{1/2,\ terminal}$ refers to the elimination half-life following the final dose of anatabine polacrilex.

As there were no significant differences in the calculated elimination half-life, mean transit times and mean absorption times between treatment groups following the first dose of the test compound ($t_{1/2,\ 0\rightarrow 4}$, $MTT_{0\rightarrow 4}$, and $MAT_{0\rightarrow 4}$, respectively), the data at both dose levels were combined for males and females (see Table 48). There were no significant differences in these parameters between genders.

Table 49 provides animal weights and dosing times. Table 50 provides measured concentrations of anatabine in rat plasma samples at each time point. Table 51 provides mean concentration and description statistics of anatabine in plasma samples at each time point.

The data from both genders are also combined to give corresponding overall values. The calculated mean elimination half-life ($t_{1/2,\ 0\rightarrow 4}$) is 1.93 ± 0.73 hr, the mean transit time ($MTT_{0\rightarrow 4}$) is 3.01 ± 1.25 hr, and the mean absorption time ($MAT_{0\rightarrow 4}$) is 0.56 ± 1.25 hr.

**Discussion**

This study evaluated the pharmacokinetics of anatabine in male and female Sprague-Dawley rats following the repeat-dose administration of anatabine polacrilex by oral gavage at two different dose levels. Anatabine was administered at 0.6 mg/kg BW in three, divided, doses of 0.2 mg/kg BW, or at 6.0 mg/kg BW in three, divided, doses of 2.0 mg/kg BW. Each dose was separated by an interval of four hours. All animals in both treatment groups appeared normal immediately following each administration of the test compound and no adverse signs were observed for the duration of the observation and plasma sampling period.

Anatabine concentrations can be measured in rat plasma following single and repeat oral dosing. The mean time to maximal plasma concentration following the first two oral doses ranged from 0.50 to 0.88 ± 0.25 hr. There were no significant differences between gender or dose group. After the third dose of test compound, the mean time to maximal plasma concentration ranged from 1.00 to 2.00 ± 1.41 hr; although in this instance the first time point measured was at one hour post-dose and therefore, it is possible that actual maximum occurred prior to this time. Within each dose group there were no significant differences in $C_{p,\ max}$ between males and females, nor was there any significant change in this parameter over time. In females of the high dose group $C_{p,\ max}$ appeared to increase from 259.8 ± 35.4 ng/mL to 374.8 ± 122.9 ng/mL; however, the trend was not statistically significant.

There were also two, observable, minima following the first two oral doses of anatabine polacrilex. In general, the minima were not significantly different from one another over time, except for females of the high dose group, which increased from 51.5 ± 26.0 ng/mL to 180 ± 31 ng/mL. Overall, these results suggest that with a 4-hour dosing interval, and after eight hours, near steady-state conditions appear have been achieved in male animals, whereas in females this may not yet be the case.

Within the two treatment groups, the total exposure, elimination half-lives, mean transit times and mean absorption times did not differ significantly between male and female rats. When these data are combined and grouped according to dose level the total exposure is significantly greater at the high dose as would be expected; however, the terminal elimination half-life is also significantly higher in the 6.0 mg/kg BW group compared to the 0.6 mg/kg B W dose group. The reason for this difference is not apparent since the mean transit times and mean absorption times did not differ significantly.

The elimination half-life, mean transit time and mean absorption time following the first oral dose of the test compound are the most reliable estimates of these parameters since the plasma concentration data are not confounded by carry-over amounts from a previous dose. The overall elimination half-life of anatabine following the first oral dose was 1.93 ± 0.73 hr, the mean transit time was 3.01 ± 1.25 hr and the mean absorption time was 0.56 ± 1.25 hr. The mean absorption time (also often called mean arrival time) of 0.56 compares favorably with the calculated $T_{max}$ values following the first two doses and indicates that the absorption of anatabine occurs within the first 30 to 60 minutes after oral administration.

**EXAMPLE 6**

**Effect of S-(-)-anatabine on TNFα-induced NFκB activity *in vitro***

The effect of S-(-)-anatabine on TNFα-induced NFκB activity *in vitro* was determined as described in Example I. NFkB activity was stimulated with 20ng/ml of TNFα, then varying doses of a racemic mixture of anatabine or S(-)-anatabine were applied to the challenged cells. The data were plotted as a percentage of the TNFα-induced NFκB activity and are shown in **FIG. 24.** In this assay the $IC_{50}$ for the racemic mixture of anatabine is approximately 600μg/ml, whereas the $IC_{50}$ for the S-(-)-enantiomer is approximately 330μg/ml.

## EXAMPLE 7

### Use of anatabine to treat autism and a seizure disorder

A 10-year old male patient, who was diagnosed with autism and a seizure disorder, had brain surgery and began rehabilitation the following month. About 4 months later, in addition to continuing rehabilitation, he began a course of treatment with 1.0 mg of anatabine three times per day. Over the course of 3½ weeks the frequency of the patient's seizures decreased from one per day to approximately one per week. The patient also experienced cognitive benefits beginning approximately one week after the start of the anatabine treatment, with noticeable improvements daily. These benefits included improved communication and language skills and the ability to focus.

## EXAMPLE 8

### Use of anatabine in a patient with high functioning autism

Anatabine was used to treat a 12-year old male patient weighing 140 pounds and described as having high functioning autism. The patient is mainstreamed in school and functions well intellectually, but is somewhat emotionally labile (limbic system dysfunction) and has difficulty coping with stressful and emotional situations. After taking 2 mg of anatabine citrate three times a day for three days, the patient experienced an incident in school which in the past would have resulted in acting out, cognitive perseveration, dramatic retellings of the incident and difficulty in expressing his thoughts and feelings. This incident however, was notable for its lack of drama. The patient demonstrated a relaxed demeanor when relating the facts of the incident. He was able to put it into perspective and "let it go."

Table 1.

| | |
|---|---|
| | Wakefield et al., Lancet 351, 351-52, 1998; Wakefield et al., Lancet 351, 637-41, 1998), and postmortem studies have also shown an increase in the expression of several markers for neuroinflammation (Vargas et al., Ann Neurol 57, 67-81, 2004) |

Table 2. Dosing solutions

| Test compound | Percentage content of anatabine or nicotine base | Dose level (mg/kg) | Test compound concentration(total) (mg/mL) | Test compound concentration (base) (mg/mL) | Injection volume (mL/kg) |
|---|---|---|---|---|---|
| Anatabine | 41.6 | 0.10 | 0.048 | 0.020 | 5 |
| Anatabine | 41.6 | 0.75 | 0.36 | 0.15 | 5 |
| Anatabine | 41.6 | 1.0 | 0.48 | 0.20 | 5 |
| Nicotine | 35.1 | 0.4 | 0.23 | 0.081 | 5 |

Table 3. Phase I

| Test compound | Route | Dose level (mg/kg) | Number of animals (M/F) | Collection times (minutes)[a] |
|---|---|---|---|---|
| Anatabine | i.v. | 0.10 | 3/3 | 15, 30, 60, 90, 120, 240, 360, 480,1440 |
| Anatabine | i.v. | 0.75 | 3/3 | 15, 30, 60, 90, 120, 240, 360, 480, 1440 |
| Anatabine | i.v. | 1.0 | 3/3 | 15, 30, 60, 90, 120, 240, 360, 480, 1440 |
| Nicotine | i.v. | 0.4 | 3/3 | 15, 30, 60, 90, 120, 240, 360, 480, 1440 |
| [a] Plasma samples were collected at all time points,. Brain tissue was collected at 1440 minutes. | | | | |

Table 4. Phase II

| Test compound | Route | Dose level (mg/kg) | Number of animals per time point (M/F) | Collection times (minutes)[a] |
|---|---|---|---|---|
| Anatabine | i.v. | 0.10 | 3/3 | 30, 360 |
| Anatabine | i.v. | 0.75 | 3/3 | 30,360 |
| Anatabine | i.v. | 1.0 | 3/3 | 30, 360 |
| Nicotine | i.v. | 0.4 | 3/3 | 30, 360 |
| [a] Plasma samples and brain tissue were collected at all time points. | | | | |

Table 5. Calibration Curve Concentrations

| nominal concentration (ng/mL) | stock concentration (μg/mL) |
|---|---|
| 5000 | 250 |
| 1667 | 83.3 |
| 555.5 | 27.8 |
| 185.2 | 9.3 |
| 61.7 | 3.1 |
| 20.6 | 1.0 |
| 6.9 | 0.34 |
| 2.3 | 0.11 |
| 0.76 | 0.038 |
| 0.25 | 0.013 |

Table 6. LC/MS/MS ionization conditions and identity of parent and product ions

| Compound | MW | Polarization | Precursor m/z | Product m/z | Collision energy (V) |
|---|---|---|---|---|---|
| Anatabine | 160.2 | Positive | 161.1 | 115.1 | 28 |
| Nicotine | 162.3 | Positive | 163.1 | 117.1 | 28 |
| (+/-)-nicotine-3'-$d_3$ | 165.25 | Positive | 166.1 | 118 | 28 |
| (*R*,*S*)-Antabine-2,4,5,6-$d_4$ | 164.24 | Positive | 165.1 | 148.1 | 20 |

Table 7. Limits of Detection and Calibration Curves

| Sample | Limit of Detection (LOD) (ng/mL) | Lower Limit of Quantitation (LLQ) (ng/mL) | Upper Limit of Quantitation (ULQ) (ng/mL) |
|---|---|---|---|
| Anatabine in rat plasma | 0.76 | 2.3 | 5000 |
| Anatabine in rat brain | 0.76 | 2.3 | 5000 |
| Nicotine in rat plasma | 0.76 | 2.3 | 5000 |
| Nicotine in rat brain | 0.76 | 2.3 | 5000 |

Table 8. Recovery from Plasma

| | % Recovery at Given Concentrations | | |
|---|---|---|---|
| Sample | 2.3 ng/mL | 62 ng/mL | 1667 ng/mL |
| Anatabine in rat plasma | 74 | 96 | ND[a] |
| Anatabine in rat brain | ND | 96 | 90 |
| Nicotine in rat plasma | ND | 105 | 104 |
| Nicotine in rat brain | ND | 78 | 84 |
| [a]ND - not determined; two points per condition were evaluated for measuring recovery. | | | |

Table 9. Dosing Solution Analysis

| Compound | Dose (mg/kg) | Expected Concentration (mg/mL) | Actual Concentration (mg/mL) | Actual Concentration relative to Expected (%) |
|---|---|---|---|---|
| Nicotine | 0.4 | 0.081 | 0.096 | 118.5 |
| Nicotine | 0.4 | 0.081 | 0.096 | 118.5 |
| Anatabine | 0.1 | 0.02 | 0.014 | 70 |
| Anatabine | 0.75 | 0.15 | 0.135 | 90 |
| Anatabine | 1 | 0.2 | 0.177 | 88.5 |
| Anatabine | 0.1 | 0.02 | 0.015 | 75 |
| Anatabine | 0.75 | 0.15 | 0.133 | 88.7 |
| Anatabine | 1 | 0.2 | 0.162 | 81 |

Table 10. Statistical Comparison of the Pharmacokinetic Parameters

<table>
<tr><td rowspan="2">Parameter</td><td colspan="2">Nicotine (0.4 mg/kg)</td><td colspan="2">Anatabine (0.1 mg/kg)</td><td colspan="2">Anatabine (0.75 mg/kg)</td><td>Anatabine (1.0 mg/kg)</td></tr>
<tr><td>M+F</td><td>p</td><td>M+F</td><td>p</td><td>M+F</td><td>p</td><td>M+F</td></tr>
<tr><td>$AUC_{0\rightarrow\infty}$ (ng·hr/mL)</td><td>156.5 ± 32.6</td><td></td><td>35.0 ± 11.6</td><td></td><td>504.4 ± 63.5</td><td></td><td>710.07 ± 88.4</td></tr>
<tr><td rowspan="3">$t_{1/2}$ (hr)</td><td rowspan="3">0.67 ± 0.07</td><td>0.003[a]</td><td rowspan="3">1.44 ± 0.48</td><td>0.25[d]</td><td rowspan="3">1.68 ± 0.09</td><td>069[f]</td><td rowspan="3">1.64 ± 0.25</td></tr>
<tr><td><0.001[b]</td><td>0.39[e]</td><td></td></tr>
<tr><td><0.001[c]</td><td></td><td></td></tr>
<tr><td rowspan="3">MRT (hr)</td><td rowspan="3">1.22 ± 0.13</td><td>0.004[a]</td><td rowspan="3">2.29 ± 0.07</td><td>0.35[d]</td><td rowspan="3">2.58 ± 0.14</td><td>0.63[f]</td><td rowspan="3">2.49 ± 0.38</td></tr>
<tr><td><0.001[b]</td><td>0.55[e]</td><td></td></tr>
<tr><td><0001[c]</td><td></td><td></td></tr>
</table>

(continued)

| Parameter | Nicotine (0.4 mg/kg) | | Anatabine (0.1 mg/kg) | | Anatabine (0.75 mg/kg) | | Anatabine (1.0 mg/kg) |
|---|---|---|---|---|---|---|---|
| | M+F | p | M+F | p | M+F | p | M+F |
| $V_D$ (L/kg) | 2.06 ± 0.13 | <0.001[a] | 6.08 ± 0.45 | <0.001[d] | 3.33 ± 0.13 | 0.15[f] | 3.08 ± 0.16 |
| | | <0.001[b] | | <0.001[e] | | | |
| | | <0.001[c] | | | | | |

[a] Nicotine *vs.* Anatabine (0.1 mg/kg)
[b] Nicotine *vs.* Anatabine (0.75 mg/kg)
[c] Nicotine vs. Anatabine (1.0 mg/kg)
[d] Anatabine (0.1 mg/kg) vs. Anatabine (0.75 mg/kg)
[e] Anatabine (0.1 mg/kg) vs. Anatabine (1.0 mg/kg)
[f] Anatabine (0.75 mg/kg) vs. Anatabine (1.0 mg/kg)

Table 11. Comparison of Pharmacokinetic Parameters (± Std Dev) between male and female animals in each Treatment Group

| Parameter | Nicotine (0.4 mg/kg) | | | Anatabine (0.1 mg/kg) | | | Anatabine (0.75 mg/kg) | | | Anatabine (1.0 mg/kg) | | |
|---|---|---|---|---|---|---|---|---|---|---|---|---|
| | male | female | p | male | female | p | male | female | p | male | female | p |
| $AUC_{0\rightarrow\infty}$ (ng·hr/mL) | 140.5 ± 5.8 | 172.6 ± 43.1 | 0.27 | 32.0 ± 7.7 | 37.9 ± 15.9 | 0.59 | 464.4 ± 36.2 | 544.5 ± 62.7 | 0.13 | 631.3 ± 28.2 | 788.9 ± 9.3 | <0.001 |
| $t_{1/2}$ (hr) | 0.66 ± 0.11 | 0.68 ± 0.02 | 0.81 | 1.25 ± 0.32 | 1.64 ± 0.59 | 0.37 | 1.64 ± 0.12 | 1.73 ± 0.03 | 0.28 | 1.44 ± 0.08 | 1.84 ± 0.16 | 0.02 |
| MRT (hr) | 1.21 ± 0.20 | 1.22 ± 0.05 | 0.90 | 2.03 ± 0.41 | 2.55 ± 0.93 | 0.42 | 2.50 ± 0.19 | 2.65 ± 0.01 | 0.25 | 2.18 ± 0.12 | 2.80 ± 0.24 | 0.02 |
| $V_D$(L/kg) | 2.10 ± 0.20 | 1.91 ± 0.17 | 0.54 | 5.29 ± 0.49 | 6.52 ± 0.66 | 0.22 | 3.48 ± 0.18 | 3.19 ± 0.15 | 0.38 | 3.01 ± 0.12 | 3.16 ± 0.15 | 0.29 |

Table 12. Mean (ng/g ± Std Dev) concentrations of nicotine and anatabine in rat brain extracts following a single, bolus, i.v. dose

| Treatment Group | Nicotine | | Anatabine | | Anatabine | | Anatabine | |
|---|---|---|---|---|---|---|---|---|
| | 0.4 mg/kg | | 0.1 mg/kg | | 0.75 mg/kg | | 1.0 mg/kg | |
| Time (hr) | Male | Female | Male | Female | Male | Female | Male | Female |
| 0.5 | 94.3 ± 20.6 | 120.0 ± 3.0 | 20.0 ± 1.4 | 29.7 ± 4.5 | 276.0 ± 37.0 | 314.7 ± 67.9 | 323.0 ± 35.9 | 346.0 ± 56.7 |
| 6 | 6.0 ± 1.4 | 5.0 | 3.0 | 3.7 ± 1.5 | 21.3 ± 5.1 | 21.0 ± 6.2 | 5.0 ± 2.6 | 6.0 ± 28 |
| 24 | 6.3 ± 2.3 | 7.0 | 3.0 | 2.0 | <LLQ | <LLQ | 2.5 ± 0.7 | <LLQ |

Table 13A.

Rat PK i.v. dose - Brain Collection

Compound: Anatabine, Nicotine  Dose: 5 mL/kg

Route: i.v., PBS

| | | [illegible] Dose [illegible] | | | 24 hr | | |
|---|---|---|---|---|---|---|---|
| Cmpnd | Rat | B.W. (g) | volume (mL) | time | time | Brain wt (g) | Volume (mL) |
| 1 MALE Anatabine 0.1 mg/kg | A | 245 | 1.23 | 8:17 | 8:17 | 1.74 | 1.74 |
| | B | 247 | 1.23 | 8:19 | 8:19 | 1.88 | 1.88 |
| | C | 236 | 1.20 | 8:21 | 8:21 | 1.80 | 1.80 |
| 2 FEMALE Anatabine 0.1 mg/kg | A | 205 | 1.03 | 8:23 | 8:23 | 1.68 | 1.68 |
| | B | 211 | 1.05 | 8:25 | 8:25 | 1.83 | 1.83 |
| | C | 202 | 1.00 | 8:27 | 8:27 | 1.66 | 1.66 |
| 3 MALE Anatabine 0.75 mg/kg | A | 255 | 1.28 | 8:29 | 8:29 | 1.90 | 1.90 |
| | B | 223 | 1.13 | 8:31 | 8:31 | 1.92 | 1.82 |
| | C | 242 | 1.20 | 8:33 | 8:33 | 1.84 | 1.84 |
| 4 FEMALE Anatabine 0.75 mg/kg | A | 204 | 1.03 | 8:35 | 8:35 | 1.82 | 1.82 |
| | B | 205 | 1.03 | 8:37 | 8:37 | 1.82 | 1.82 |
| | C | 210 | 1.05 | 8:39 | 8:39 | 1.71 | 1.71 |
| 5 MALE Anatabine 1.0 mg/kg | A | 242 | 1.20 | 8:41 | 8:41 | 1.83 | 1.83 |
| | B | 251 | 1.25 | 8:43 | 8:43 | 1.85 | 1.85 |
| | C | 246 | 1.23 | 8:45 | 8:45 | 1.90 | 1.90 |
| 6 FEMALE Anatabine 1.0 mg/kg | A | 213 | 1.08 | 8:47 | 8:47 | 1.95 | 1.95 |
| | B | 218 | 1.10 | 8:49 | 8:49 | 1.75 | 1.75 |
| | C | 219 | 1.10 | 8:51 | 8:51 | 1.91 | 1.91 |
| 7 MALE Nicotine 0.4 mg/kg | A | 242 | 1.20 | 8:54 | 8:54 | 2.03 | 2.03 |
| | B | 241 | 1.20 | 8:56 | 8:56 | 1.96 | 1.98 |
| | C | 252 | 1.25 | 8:58 | 8:58 | 1.86 | 1.86 |
| 8 FEMALE Nicotine 0.4 mg/kg | A | 219 | 1.10 | 9:00 | 9:00 | 1.82 | 1.82 |
| | B | 215 | 1.08 | 9:02 | 9:02 | 1.97 | 1.87 |
| | C | 220 | 1.10 | 9:04 | 9:04 | 1.87 | 1.87 |
| Control Male | 1 | N/A | N/A | N/A | N/A | 1.83 | 1.83 |
| | 2 | N/A | N/A | N/A | N/A | 1.94 | 1.94 |
| | 3 | N/A | N/A | N/A | N/A | 1.82 | 1.82 |

Table 13B. Animal Weights and Dosing Times

**Rat PK i.v. dose - Brain Collection**

Dose 5 mL/kg     Compound: Anatabine, Nicotine

Route: i.v., PBS

| Compound | Rat | B.W. (g) | volume (mL) | time | time | Brain wt (g) | Volume (mL) |
|---|---|---|---|---|---|---|---|
| | | Dose | | | 0.5 hour | | |
| 1 | D | 254 | 1.30 | 12:38 | 13:08 | 1.58 | 1.58 |
| MALE | E | 270 | 1.35 | 12:41 | 13:11 | 1.54 | 1.54 |
| Anatabine 0.1 mg/kg | F | 252 | 1.26 | 12:45 | 13:15 | 1.69 | 1.69 |
| 2 | D | 220 | 1.10 | 12:46 | 13:16 | 1.78 | 1.78 |
| FEMALE | E | 206 | 1.03 | 12:50 | 13:20 | 1.54 | 1.54 |
| Anatabine 0.1 mg/kg | F | 214 | 1.08 | 12:52 | 13:22 | 1.69 | 1.69 |
| 3 | D | 259 | 1.30 | 12:56 | 13:26 | 1.70 | 1.70 |
| MALE | E | 266 | 1.33 | 12:58 | 13:28 | 1.66 | 1.66 |
| Anatabine 0.75 mg/kg | F | 264 | 1.33 | 13:03 | 13:33 | 1.46 | 1.46 |
| 4 | D | 208 | 1.05 | 13:05 | 13:35 | 1.81 | 1.81 |
| FEMALE | E | 219 | 1.10 | 13:09 | 13:39 | 1.84 | 1.84 |
| Anatabine 0.75 mg/kg | F | 223 | 1.13 | 13:12 | 13:42 | 1.69 | 1.69 |
| 5 | D | 276 | 1.38 | 13:16 | 13:46 | 2.00 | 2.00 |
| MALE | E | 252 | 1.25 | 13:19 | 13:49 | 1.84 | 1.84 |
| Anatabine 1.0 mg/kg | F | 255 | 1.28 | 13:22 | 13:52 | 1.68 | 1.68 |
| 6 | D | 212 | 1.05 | 13:25 | 13:55 | 1.66 | 1.66 |
| FEMALE | E | 225 | 1.13 | 13:29 | 13:59 | 1.56 | 1.56 |
| Anatabine 1.0 mg/kg | F | 236 | 1.18 | 13:32 | 14:02 | 1.79 | 1.79 |
| 7 | D | 273 | 1.38 | 13:36 | 14:06 | 1.76 | 1.76 |
| MALE | E | 256 | 1.28 | 13:39 | 14:09 | 1.72 | 1.72 |
| Nicotine 0.1 mg/kg | F | 263 | 1.33 | 13:44 | 14:14 | 1.77 | 1.77 |
| 8 | D | 212 | 1.05 | 13:48 | 14:18 | 1.75 | 1.75 |
| FEMALE | E | 213 | 1.05 | 13:52 | 14:22 | 1.81 | 1.81 |
| Nicotine 0.4 mg/kg | F | 231 | 1.15 | 13:59 | 14:29 | 1.90 | 1.90 |

Table 14. Measured Concentrations of Anatabine and Nicotine in Rat Brain Extracts and Plasma Samples at Each Time Point

| Compound | Dose Group | Tissue | Rat ID | Time Point (hr) | Concentration (ng/mL) |
|---|---|---|---|---|---|
| Nicotine | Nicotine 0.4 mg/kg | Rat Brain | 7D | 0.5 | 110 |
| Nicotine | Nicotine 0.4 mg/kg | Rat Brain | 7E | 0.5 | 71 |
| Nicotine | Nicotine 0.4 mg/kg | Rat Brain | 7F | 0.5 | 102 |

(continued)

| Compound | Dose Group | Tissue | Rat ID | Time Point (hr) | Concentration (ng/mL) |
|---|---|---|---|---|---|
| Nicotine | Nicotine 0.4 mg/kg | Rat Brain | 7G | 6 | 5 |
| Nicotine | Nicotine 0.4 mg/kg | Rat Brain | 7H | 6 | <LLQ |
| Nicotine | Nicotine 0.4 mg/kg | Rat Brain | 71 | 6 | 7 |
| Nicotine | Nicotine 0.4 mg/kg | Rat Brain | 7A | 24 | 5 |
| Nicotine | Nicotine 0.4 mg/kg | Rat Brain | 7B | 24 | 5 |
| Nicotine | Nicotine 0.4 mg/kg | Rat Brain | 7C | 24 | 9 |
| Nicotine | Nicotine 0.4 mg/kg | Rat Brain | 8D | 0.5 | 117 |
| Nicotine | Nicotine 0.4 mg/kg | Rat Brain | 8E | 0.5 | 120 |
| Nicotine | Nicotine 0.4 mg/kg | Rat Brain | 8F | 0.5 | 123 |
| Nicotine | Nicotine 0.4 mg/kg | Rat Brain | 8G | 6 | <LLQ |
| Nicotine | Nicotine 0.4 mg/kg | Rat Brain | 8H | 6 | <LLQ |
| Nicotine | Nicotine 0.4 mg/kg | Rat Brain | 81 | 6 | 5 |
| Nicotine | Nicotine 0.4 mg/kg | Rat Brain | 8A | 24 | <LLQ |
| Nicotine | Nicotine 0.4 mg/kg | Rat Brain | 8B | 24 | 7 |
| Nicotine | Nicotine 0.4 mg/kg | Rat Brain | 8C | 24 | <LLQ |
| | | | | | |
| Anatabine | Anatabine 0.1 mg/kg | Rat Brain | ID | 0.5 | <LLQ |
| Anatabine | Anatabine 0.1 mg/kg | Rat Brain | 1E | 0.5 | 19 |
| Anatabine | Anatabine 0.1 mg/kg | Rat Brain | 1F | 0.5 | 21 |
| Anatabine | Anatabine 0.1 mg/kg | Rat Brain | 1G | 6 | 3 |
| Anatabine | Anatabine 0.1 mg/kg | Rat Brain | 1H | 6 | <LLQ |
| Anatabine | Anatabine 0.1 mg/kg | Rat Brain | 1I | 6 | <LLQ |
| Anatabine | Anatabine 0.1 mg/kg | Rat Brain | 1A | 24 | 3 |
| Anatabine | Anatabine 0.1 mg/kg | Rat Brain | 1B | 24 | <LLQ |
| Anatabine | Anatabine 0.1 mg/kg | Rat Brain | 1C | 24 | <LLQ |
| Anatabine | Anatabine 0.1 mg/kg | Rat Brain | 2D | 0.5 | 34 |
| Anatabine | Anatabine 0.1 mg/kg | Rat Brain | 2E | 0.5 | 25 |
| Anatabine | Anatabine 0.1 mg/kg | Rat Brain | 2F | 0.5 | 30 |

(continued)

| Compound | Dose Group | Tissue | Rat ID | Time Point (hr) | Concentration (ng/mL) |
|---|---|---|---|---|---|
| Anatabine | Anatabine 0.1 mg/kg | Rat Brain | 2G | 6 | 5 |
| Anatabine | Anatabine 0.1 mg/kg | Rat Brain | 2H | 6 | 4 |
| Anatabine | Anatabine 0.1 mg/kg | Rat Brain | 21 | 6 | 2 |
| Anatabine | Anatabine 0.1 mg/kg | Rat Brain | 2A | 24 | 2 |
| Anatabine | Anatabine 0.1 mg/kg | Rat Brain | 2B | 24 | <LLQ |
| Anatabine | Anatabine 0.1 mg/kg | Rat Brain | 2C | 24 | <LLQ |
| Anatabine | Anatabine 0.75 mg/kg | Rat Brain | 3D | 0.5 | 266 |
| Anatabine | Anatabine 0.75 mg/kg | Rat Brain | 3E | 0.5 | 317 |
| Anatabine | Anatabine 0.75 mg/kg | Rat Brain | 3F | 0.5 | 245 |
| Anatabine | Anatabine 0.75 mg/kg | Rat Brain | 3G | 6 | 17 |
| Anatabine | Anatabine 0.75 mg/kg | Rat Brain | 3H | 6 | 27 |
| Anatabine | Anatabine 0.75 mg/kg | Rat Brain | 31 | 6 | 20 |
| Anatabine | Anatabine 0.75 mg/kg | Rat Brain | 3A | 24 | <LLQ |
| Anatabine | Anatabine 0.75 mg/kg | Rat Brain | 3B | 24 | <LLQ |
| Anatabine | Anatabine 0.75 mg/kg | Rat Brain | 3C | 24 | <LLQ |
| Anatabine | Anatabine 0.75 mg/kg | Rat Brain | 4D | 0.5 | 393 |
| Anatabine | Anatabine 0.75 mg/kg | Rat Brain | 4E | 0.5 | 272 |
| Anatabine | Anatabine 0.75 mg/kg | Rat Brain | 4F | 0.5 | 279 |
| Anatabine | Anatabine 0.75 mg/kg | Rat Brain | 4G | 6 | 16 |
| Anatabine | Anatabine 0.75 mg/kg | Rat Brain | 4H | 6 | 28 |
| Anatabine | Anatabine 0.75 mg/kg | Rat Brain | 41 | 6 | 19 |
| Anatabine | Anatabine 0.75 mg/kg | Rat Brain | 4A | 24 | <LLQ |

(continued)

| Compound | Dose Group | Tissue | Rat ID | Time Point (hr) | Concentration (ng/mL) |
|---|---|---|---|---|---|
| Anatabine | Anatabine 0.75 mg/kg | Rat Brain | 4B | 24 | <LLQ |
| Anatabine | Anatabine 0.75 mg/kg | Rat Brain | 4C | 24 | <LLQ |
| Anatabine | Anatabine 1.0 mg/kg | Rat Brain | 5D | 0.5 | 349 |
| Anatabine | Anatabine 1.0 mg/kg | Rat Brain | 5E | 0.5 | 338 |
| Anatabine | Anatabine 1.0 mg/kg | Rat Brain | 5F | 0.5 | 282 |
| Anatabine | Anatabine 1.0 mg/kg | Rat Brain | 5G | 6 | 3 |
| Anatabine | Anatabine 1.0 mg/kg | Rat Brain | 5H | 6 | 4 |
| Anatabine | Anatabine 1.0 mg/kg | Rat Brain | 51 | 6 | 8 |
| Anatabine | Anatabine 1.0 mg/kg | Rat Brain | 5A | 24 | 3 |
| Anatabine | Anatabine 1.0 mg/kg | Rat Brain | 5B | 24 | 2 |
| Anatabine | Anatabine 1.0 mg/kg | Rat Brain | 5C | 24 | <LLQ |
| Anatabine | Anatabine 1.0 mg/kg | Rat Brain | 6D | 0.5 | 362 |
| Anatabine | Anatabine 1.0 mg/kg | Rat Brain | 6E | 0.5 | 393 |
| Anatabine | Anatabine 1.0 mg/kg | Rat Brain | 6F | 0.5 | 283 |
| Anatabine | Anatabine 1.0 mg/kg | Rat Brain | 6G | 6 | <LLQ |
| Anatabine | Anatabine 1.0 mg/kg | Rat Brain | 6H | 6 | 8 |
| Anatabine | Anatabine 1.0 mg/kg | Rat Brain | 61 | 6 | 4 |
| Anatabine | Anatabine 1.0 mg/kg | Rat Brain | 6A | 24 | <LLQ |
| Anatabine | Anatabine 1.0 mg/kg | Rat Brain | 6B | 24 | <LLQ |
| Anatabine | Anatabine 1.0 mg/kg | Rat Brain | 6C | 24 | <LLQ |
| | | | | | |
| Nicotine | Nicotine 0.4 mg/kg | Rat Plasma | 7A | 0.25 | 194 |
| Nicotine | Nicotine 0.4 mg/kg | Rat Plasma | 7B | 0.25 | 156 |

(continued)

| Compound | Dose Group | Tissue | Rat ID | Time Point (hr) | Concentration (ng/mL) |
|---|---|---|---|---|---|
| Nicotine | Nicotine 0.4 mg/kg | Rat Plasma | 7C | 0.25 | 145 |
| Nicotine | Nicotine 0.4 mg/kg | Rat Plasma | 7A | 0.5 | 123 |
| Nicotines | Nicotine 0.4 mg/kg | Rat Plasma | 7B | 0.5 | 85 |
| Nicotine | Nicotine 0.4 mg/kg | Rat Plasma | 7C | 0.5 | 90 |
| Nicotine | Nicotine 0.4 mg/kg | Rat Plasma | 7D | 0.5 | 187 |
| Nicotine | Nicotine 0.4 mg/kg | Rat Plasma | 7E | 0.5 | 118 |
| Nicotine | Nicotine 0.4 mg/kg | Rat Plasma | 7F | 0.5 | 157 |
| Nicotine | Nicotine 0.4 mg/kg | Rat Plasma | 7A | 1 | 72 |
| Nicotine | Nicotine 0.4 mg/kg | Rat Plasma | 7B | 1 | 67 |
| Nicotine | Nicotine 0.4 mg/kg | Rat Plasma | 7C | 1 | 68 |
| Nicotine | Nicotine 0.4 mg/kg | Rat Plasma | 7A | 1.5 | 33 |
| Nicotine | Nicotine 0.4 mg/kg | Rat Plasma | 7B | 1.5 | 30 |
| Nicotine | Nicotine 0.4 mg/kg | Rat Plasma | 7C | 1.5 | 44 |
| Nicotine | Nicotine 0.4 mg/kg | Rat Plasma | 7A | 2 | 21 |
| Nicotine | Nicotine 0.4 mg/kg | Rat Plasma | 7B | 2 | 32 |
| Nicotine | Nicotine 0.4 mg/kg | Rat Plasma | 7C | 2 | 21 |
| Nicotine | Nicotine 0.4 mg/kg | Rat Plasma | 7A | 4 | <LLQ |
| Nicotine | Nicotine 0.4 mg/kg | Rat Plasma | 7B | 4 | <LLQ |
| Nicotine | Nicotine 0.4 mg/kg | Rat Plasma | 7C | 4 | <LLQ |
| Nicotine | Nicotine 0.4 mg/kg | Rat Plasma | 7A | 6 | <LLQ |
| Nicotine | Nicotine 0.4 mg/kg | Rat Plasma | 7B | 6 | <LLQ |
| Nicotine | Nicotine 0.4 mg/kg | Rat Plasma | 7C | 6 | <LLQ |
| Nicotine | Nicotine 0.4 mg/kg | Rat Plasma | 7G | 6 | <LLQ |
| Nicotine | Nicotine 0.4 mg/kg | Rat Plasma | 7H | 6 | <LLQ |
| Nicotine | Nicotine 0.4 mg/kg | Rat Plasma | 7I | 6 | <LLQ |
| Nicotine | Nicotine 0.4 mg/kg | Rat Plasma | 7A | 8 | <LLQ |
| Nicotine | Nicotine 0.4 mg/kg | Rat Plasma | 7B | 8 | <LLQ |
| Nicotine | Nicotine 0.4 mg/kg | Rat Plasma | 7C | 8 | <LLQ |
| Nicotine | Nicotine 0.4 mg/kg | Rat Plasma | 7A | 24 | <LLQ |
| Nicotine | Nicotine 0.4 mg/kg | Rat Plasma | 7B | 24 | <LLQ |
| Nicotine | Nicotine 0.4 mg/kg | Rat Plasma | 7C | 24 | <LLQ |
| Nicotine | Nicotine 0.4 mg/kg | Rat Plasma | 8A | 0.25 | 175 |
| Nicotine | Nicotine 0.4 mg/kg | Rat Plasma | 8B | 0.25 | 145 |
| Nicotine | Nicotine 0.4 mg/kg | Rat Plasma | 8C | 0.25 | 184 |
| Nicotine | Nicotine 0.4 mg/kg | Rat Plasma | 8A | 0.5 | 111 |
| Nicotine | Nicotine 0.4 mg/kg | Rat Plasma | 8B | 0.5 | 95 |
| Nicotine | Nicotine 0.4 mg/kg | Rat Plasma | 8C | 0.5 | 125 |

(continued)

| Compound | Dose Group | Tissue | Rat ID | Time Point (hr) | Concentration (ng/mL) |
|---|---|---|---|---|---|
| Nicotine | Nicotine 0.4 mg/kg | Rat Plasma | 8D | 0.5 | 180 |
| Nicotine | Nicotine 0.4 mg/kg | Rat Plasma | 8E | 0.5 | 157 |
| Nicotine | Nicotine 0.4 mg/kg | Rat Plasma | 8F | 0.5 | 160 |
| Nicotine | Nicotine 0.4 mg/kg | Rat Plasma | 8A | 1 | 67 |
| Nicotine | Nicotine 0.4 mg/kg | Rat Plasma | 8B | 1 | 72 |
| Nicotine | Nicotine 0.4 mg/kg | Rat Plasma | 8C | 1 | 107 |
| Nicotine | Nicotine 0.4 mg/kg | Rat Plasma | 8A | 1.5 | 49 |
| Nicotine | Nicotine 0.4 mg/kg | Rat Plasma | 8B | 1.5 | 37 |
| Nicotine | Nicotine 0.4 mg/kg | Rat Plasma | 8C | 1.5 | 64 |
| Nicotine | Nicotine 0.4 mg/kg | Rat Plasma | 8A | 2 | 25 |
| Nicotine | Nicotine 0.4 mg/kg | Rat Plasma | 8B | 2 | 24 |
| Nicotine | Nicotine 0.4 mg/kg | Rat Plasma | 8C | 2 | 46 |
| Nicotine | Nicotine 0.4 mg/kg | Rat Plasma | 8A | 4 | 3 |
| Nicotine | Nicotine 0.4 mg/kg | Rat Plasma | 8B | 4 | <LLQ |
| Nicotine | Nicotine 0.4 mg/kg | Rat Plasma | 8C | 4 | 4 |
| Nicotine | Nicotine 0.4 mg/kg | Rat Plasma | 8A | 6 | <LLQ |
| Nicotine | Nicotine 0.4 mg/kg | Rat Plasma | 8B | 6 | <LLQ |
| Nicotine | Nicotine 0.4 mg/kg | Rat Plasma | 8C | 6 | <LLQ |
| Nicotine | Nicotine 0.4 mg/kg | Rat Plasma | 8G | 6 | <LLQ |
| Nicotine | Nicotine 0.4 mg/kg | Rat Plasma | 8H | 6 | <LLQ |
| Nicotine | Nicotine 0.4 mg/kg | Rat Plasma | 81 | 6 | <LLQ |
| Nicotine | Nicotine 0.4 mg/kg | Rat Plasma | 8A | 8 | <LLQ |
| Nicotine | Nicotine 0.4 mg/kg | Rat Plasma | 8B | 8 | <LLQ |
| Nicotine | Nicotine 0.4 mg/kg | Rat Plasma | 8C | 8 | <LLQ |
| Nicotine | Nicotine 0.4 mg/kg | Rat Plasma | 8A | 24 | <LLQ |
| Nicotine | Nicotine 0.4 mg/kg | Rat Plasma | 8B | 24 | <LLQ |
| Nicotine | Nicotine 0.4 mg/kg | Rat Plasma | 8C | 24 | <LLQ |
| Anatabine | Anatabine 0.1 mg/kg | Rat Plasma | 1A | 0.25 | 24 |
| Anatabine | Anatabine 0.1 mg/kg | Rat Plasma | 1B | 0.25 | 14 |
| Anatabine | Anatabine 0.1 mg/kg | Rat Plasma | 1C | 0.25 | 20 |
| Anatabine | Anatabine 0.1 mg/kg | Rat Plasma | 1A | 0.5 | 16 |
| Anatabine | Anatabine 0.1 mg/kg | Rat Plasma | 1B | 0.5 | 17 |
| Anatabine | Anatabine 0.1 mg/kg | Rat Plasma | 1C | 0.5 | 13 |

(continued)

| Compound | Dose Group | Tissue | Rat ID | Time Point (hr) | Concentration (ng/mL) |
|---|---|---|---|---|---|
| Anatabine | Anatabine 0.1 mg/kg | Rat Plasma | 1D | 0.5 | 30 |
| Anatabine | Anatabine 0.1 mg/kg | Rat Plasma | 1E | 0.5 | 32 |
| Anatabine | Anatabine 0.1 mg/kg | Rat Plasma | 1F | 0.5 | 33 |
| Anatabine | Anatabine 0.1 mg/kg | Rat Plasma | 1A | 1 | 10 |
| A natabine | Anatabine 0.1 mg/kg | Rat Plasma | 1B | 1 | 16 |
| Anatabine | Anatabine 0.1 mg/kg | Rat Plasma | 1C | 1 | 11 |
| Anatabine | Anatabine 0.1 mg/kg | Rat Plasma | 1A | 1.5 | 7 |
| Anatabine | Anatabine 0.1 mg/kg | Rat Plasma | 1B | 1.5 | <LLQ |
| Anatabine | Anatabine 0.1 mg/kg | Rat Plasma | 1C | 1.5 | 6 |
| Anatabine | Anatabine 0.1 mg/kg | Rat Plasma | 1A | 2 | 6 |
| Anatabine | Anatabine 0.1 mg/kg | Rat Plasma | 1B | 2 | 8 |
| Anatabine | Anatabine 0.1 mg/kg | Rat Plasma | 1C | 2 | 5 |
| Anatabine | Anatabine 0.1 mg/kg | Rat Plasma | 1A | 4 | 3 |
| Anatabine | Anatabine 0.1 mg/kg | Rat Plasma | 1B | 4 | 3 |
| Anatabine | Anatabine 0.1 mg/kg | Rat Plasma | 1C | 4 | <LLQ |
| Anatabine | Anatabine 0.1 mg/kg | Rat Plasma | 1A | 6 | <LLQ |
| Anatabine | Anatabine 0.1 mg/kg | Rat Plasma | 1B | 6 | <LLQ |
| Anatabine | Anatabine 0.1 mg/kg | Rat Plasma | 1C | 6 | <LLQ |
| Anatabine | Anatabine 0.1 mg/kg | Rat Plasma | 1G | 6 | 7 |
| Anatabine | Anatabine 0.1 mg/kg | Rat Plasma | 1H | 6 | 4 |
| Anatabine | Anatabine 0.1 mg/kg | Rat Plasma | 1I | 6 | 4 |
| Anatabine | Anatabine 0.1 mg/kg | Rat Plasma | 1A | 8 | <LLQ |

(continued)

| Compound | Dose Group | Tissue | Rat ID | Time Point (hr) | Concentration (ng/mL) |
|---|---|---|---|---|---|
| Anatabine | Anatabine 0.1 mg/kg | Rat Plasma | 1B | 8 | <LLQ |
| Anatabine | Anatabine 0.1 mg/kg | Rat Plasma | 1C | 8 | <LLQ |
| Anatabine | Anatabine 0.1 mg/kg | Rat Plasma | 1A | 24 | <LLQ |
| Anatabine | Anatabine 0.1 mg/kg | Rat Plasma | 1B | 24 | <LLQ |
| Anatabine | Anatabine 0.1 mg/kg | Rat Plasma | 1C | 24 | <LLQ |
| Anatabine | Anatabine 0.1 mg/kg | Rat Plasma | 2D | 0.5 | 31 |
| Anatabine | Anatabine 0.1 mg/kg | Rat Plasma | 2E | 0.5 | 30 |
| Anatabine | Anatabine 0.1 mg/kg | Rat Plasma | 2F | 0.5 | 34 |
| Anatabine | Anatabine 0.1 mg/kg | Rat Plasma | 2G | 6 | 3 |
| Anatabine | Anatabine 0.1 mg/kg | Rat Plasma | 2H | 6 | 4 |
| Anatabine | Anatabine 0.1 mg/kg | Rat Plasma | 21 | 6 | 4 |
| Anatabine | Anatabine 0.1 mg/kg | Rat Plasma | 2A | 0.25 | 15 |
| Anatabine | Anatabine 0.1 mg/kg | Rat Plasma | 2B | 0.25 | 21 |
| Anatabine | Anatabine 0.1 mg/kg | Rat Plasma | 2C | 0.25 | 14 |
| Anatabine | Anatabine 0.1 mg/kg | Rat Plasma | 2A | 0.5 | 16 |
| Anatabine | Anatabine 0.1 mg/kg | Rat Plasma | 2B | 0.5 | 13 |
| Anatabine | Anatabine 0.1 mg/kg | Rat Plasma | 2C | 0.5 | 10 |
| Anatabine | Anatabine 0.1 mg/kg | Rat Plasma | 2A | 1 | 12 |
| Anatabine | Anatabine 0.1 mg/kg | Rat Plasma | 2B | 1 | 12 |
| Anatabine | Anatabine 0.1 mg/kg | Rat Plasma | 2C | 1 | 9 |
| Anatabine | Anatabine 0.1 mg/kg | Rat Plasma | 2A | 1.5 | 14 |
| Anatabine | Anatabine 0.1 mg/kg | Rat Plasma | 2B | 1.5 | 12 |

(continued)

| Compound | Dose Group | Tissue | Rat ID | Time Point (hr) | Concentration (ng/mL) |
|---|---|---|---|---|---|
| Anatabine | Anatabine 0.1 mg/kg | Rat Plasma | 2C | 1.5 | 7 |
| Anatabine | Anatabine 0.1 mg/kg | Rat Plasma | 2A | 2 | 8 |
| Anatabine | Anatabine 0.1 mg/kg | Rat Plasma | 2B | 2 | 8 |
| Anatabine | Anatabine 0.1 mg/kg | Rat Plasma | 2C | 2 | 4 |
| Anatabine | Anatabine 0.1 mg/kg | Rat Plasma | 2A | 4 | 3 |
| Anatabine | Anatabine 0.1 mg/kg | Rat Plasma | 2B | 4 | 5 |
| Anatabine | Anatabine 0.1 mg/kg | Rat Plasma | 2C | 4 | <LLQ |
| Anatabine | Anatabine 0.1 mg/kg | Rat Plasma | 2A | 6 | <LLQ |
| Anatabine | Anatabine 0.1 mg/kg | Rat Plasma | 2B | 6 | 3 |
| Anatabine | Anatabine 0.1 mg/kg | Rat Plasma | 2C | 6 | <LLQ |
| Anatabine | Anatabine 0.1 mg/kg | Rat Plasma | 2A | 8 | <LLQ |
| Anatabine | Anatabine 0.1 mg/kg | Rat Plasma | 2B | 8 | <LLQ |
| Anatabine | Anatabine 0.1 mg/kg | Rat Plasma | 2C | 8 | <LLQ |
| Anatabine | Anatabine 0.1 mg/kg | Rat Plasma | 2A | 24 | <LLQ |
| Anatabine | Anatabine 0.1 mg/kg | Rat Plasma | 2B | 24 | <LLQ |
| Anatabine | Anatabine 0.1 mg/kg | Rat Plasma | 2C | 24 | <LLQ |
| Anatabine | Anatabine 0.75 mg/kg | Rat Plasma | 3A | 0.25 | 216 |
| Anatabine | Anatabine 0.75 mg/kg | Rat Plasma | 3B | 0.25 | 162 |
| Anatabine | Anatabine 0.75 mg/kg | Rat Plasma | 3C | 0.25 | 223 |
| Anatabine | Anatabine 0.75 mg/kg | Rat Plasma | 3A | 0.5 | 176 |
| Anatabine | Anatabine 0.75 mg/kg | Rat Plasma | 3B | 0.5 | 176 |
| Anatabine | Anatabine 0.75 mg/kg | Rat Plasma | 3C | 0.5 | 190 |

(continued)

| Compound | Dose Group | Tissue | Rat ID | Time Point (hr) | Concentration (ng/mL) |
|---|---|---|---|---|---|
| Anatabine | Anatabine 0.75 mg/kg | Rat Plasma | 3D | 0.5 | 342 |
| Anatabine | Anatabine 0.75 mg/kg | Rat Plasma | 3E | 0.5 | 271 |
| Anatabine | Anatabine 0.75 mg/kg | Rat Plasma | 3F | 0.5 | 292 |
| Anatabine | Anatabine 0.75 mg/kg | Rat Plasma | 3A | 1 | 153 |
| Anatabine | Anatabine 0.75 mg/kg | Rat Plasma | 3B | 1 | 146 |
| Anatabine | Anatabine 0.75 mg/kg | Rat Plasma | 3C | 1 | 156 |
| Anatabine | Anatabine 0.75 mg/kg | Rat Plasma | 3A | 1.5 | 120 |
| Anatabine | Anatabine 0.75 mg/kg | Rat Plasma | 3B | 1.5 | 124 |
| Anatabine | Anatabine 0.75 mg/kg | Rat Plasma | 3C | 1.5 | 136 |
| Anatabine | Anatabine 0.75 mg/kg | Rat Plasma | 3A | 2 | 73 |
| Anatabine | Anatabine 0.75 mg/kg | Rat Plasma | 3B | 2 | 74 |
| Anatabine | Anatabine 0.75 mg/kg | Rat Plasma | 3C | 2 | 104 |
| Anatabine | Anatabine 0.75 mg/kg | Rat Plasma | 3A | 4 | 29 |
| Anatabine | Anatabine 0.75 mg/kg | Rat Plasma | 3B | 4 | 36 |
| Anatabine | Anatabine 0.75 mg/kg | Rat Plasma | 3C | 4 | 39 |
| Anatabine | Anatabine 0.75 mg/kg | Rat Plasma | 3A | 6 | 15 |
| Anatabine | Anatabine 0.75 mg/kg | Rat Plasma | 3B | 6 | 14 |
| Anatabine | Anatabine 0.75 mg/kg | Rat Plasma | 3C | 6 | 13 |
| Anatabine | Anatabine 0.75 mg/kg | Rat Plasma | 3G | 6 | 15 |
| Anatabine | Anatabine 0.75 mg/kg | Rat Plasma | 3H | 6 | 31 |
| Anatabine | Anatabine 0.75 mg/kg | Rat Plasma | 31 | 6 | 20 |
| Anatabine | Anatabine 0.75 mg/kg | Rat Plasma | 3A | 8 | 8 |

(continued)

| Compound | Dose Group | Tissue | Rat ID | Time Point (hr) | Concentration (ng/mL) |
|---|---|---|---|---|---|
| Anatabine | Anatabine 0.75 mg/kg | Rat Plasma | 3B | 8 | 11 |
| Anatabine | Anatabine 0.75 mg/kg | Rat Plasma | 3C | 8 | 8 |
| Anatabine | Anatabine 0.75 mg/kg | Rat Plasma | 3A | 24 | <LLQ |
| Anatabine | Anatabine 0.75 mg/kg | Rat Plasma | 3B | 24 | <LLQ |
| Anatabine | Anatabine 0.75 mg/kg | Rat Plasma | 3C | 24 | <LLQ |
| Anatabine | Anatabine 0.75 mg/kg | Rat Plasma | 4A | 0.25 | 204 |
| Anatabine | Anatabine 0.75 mg/kg | Rat Plasma | 4B | 0.25 | 226 |
| Anatabine | Anatabine 0.75 mg/kg | Rat Plasma | 4C | 0.25 | 207 |
| Anatabine | Anatabine 0.75 mg/kg | Rat Plasma | 4A | 0.5 | 166 |
| Anatabine | Anatabine 0.75 mg/kg | Rat Plasma | 4B | 0.5 | 229 |
| Anatabine | Anatabine 0.75 mg/kg | Rat Plasma | 4C | 0.5 | 175 |
| Anatabine | Anatabine 0.75 mg/kg | Rat Plasma | 4D | 0.5 | 307 |
| Anatabine | Anatabine 0.75 mg/kg | Rat Plasma | 4E | 0.5 | 359 |
| Anatabine | Anatabine 0.75 mg/kg | Rat Plasma | 4F | 0.5 | 396 |
| Anatabine | Anatabine 0.75 mg/kg | Rat Plasma | 4A | 1 | 146 |
| Anatabine | Anatabine 0.75 mg/kg | Rat Plasma | 4B | 1 | 207 |
| Anatabine | Anatabine 0.75 mg/kg | Rat Plasma | 4C | 1 | 165 |
| Anatabine | Anatabine 0.75 mg/kg | Rat Plasma | 4A | 1.5 | 136 |
| Anatabine | Anatabine 0.75 mg/kg | Rat Plasma | 4B | 1.5 | 134 |
| Anatabine | Anatabine 0.75 mg/kg | Rat Plasma | 4C | 1.5 | 150 |
| Anatabine | Anatabine 0.75 mg/kg | Rat Plasma | 4A | 2 | 89 |
| Anatabine | Anatabine 0.75 mg/kg | Rat Plasma | 4B | 2 | 113 |

(continued)

| Compound | Dose Group | Tissue | Rat ID | Time Point (hr) | Concentration (ng/mL) |
|---|---|---|---|---|---|
| Anatabine | Anatabine 0.75 mg/kg | Rat Plasma | 4C | 2 | 97 |
| Anatabine | Anatabine 0.75 mg/kg | Rat Plasma | 4A | 4 | 45 |
| Anatabine | Anatabine 0.75 mg/kg | Rat Plasma | 4B | 4 | 50 |
| Anatabine | Anatabine 0.75 mg/kg | Rat Plasma | 4C | 4 | 41 |
| Anatabine | Anatabine 0.75 mg/kg | Rat Plasma | 4A | 6 | 14 |
| Anatabine | Anatabine 0.75 mg/kg | Rat Plasma | 4B | 6 | 23 |
| Anatabine | Anatabine 0.75 mg/kg | Rat Plasma | 4C | 6 | 18 |
| Anatabine | Anatabine 0.75 mg/kg | Rat Plasma | 4G | 6 | 15 |
| Anatabine | Anatabine 0.75 mg/kg | Rat Plasma | 4H | 6 | 38 |
| Anatabine | Anatabine 0.75 mg/kg | Rat Plasma | 41 | 6 | 16 |
| Anatabine | Anatabine 0.75 mg/kg | Rat Plasma | 4A | 8 | 10 |
| Anatabine | Anatabine 0.75 mg/kg | Rat Plasma | 4B | 8 | 12 |
| Anatabine | Anatabine 0.75 mg/kg | Rat Plasma | 4C | 8 | 11 |
| Anatabine | Anatabine 0.75 mg/kg | Rat Plasma | 4A | 24 | <LLQ |
| Anatabine | Anatabine 0.75 mg/kg | Rat Plasma | 4B | 24 | <LLQ |
| Anatabine | Anatabine 0.75 mg/kg | Rat Plasma | 4C | 24 | <LLQ |
| Anatabine | Anatabine 1.0 mg/kg | Rat Plasma | 5A | 0.25 | 296 |
| Anatabine | Anatabine 1.0 mg/kg | Rat Plasma | 5B | 0.25 | 291 |
| Anatabine | Anatabine 1.0 mg/kg | Rat Plasma | 5C | 0.25 | 270 |
| Anatabine | Anatabine 1.0 mg/kg | Rat Plasma | 5A | 0.5 | 288 |
| Anatabine | Anatabine 1.0 mg/kg | Rat Plasma | 5B | 0.5 | 264 |
| Anatabine | Anatabine 1.0 mg/kg | Rat Plasma | 5C | 0.5 | 265 |

(continued)

| Compound | Dose Group | Tissue | Rat ID | Time Point (hr) | Concentration (ng/mL) |
|---|---|---|---|---|---|
| Anatabine | Anatabine 1.0 mg/kg | Rat Plasma | 5D | 0.5 | 447 |
| Anatabine | Anatabine 1.0 mg/kg | Rat Plasma | 5E | 0.5 | 384 |
| Anatabine | Anatabine 1.0 mg/kg | Rat Plasma | 5F | 0.5 | 366 |
| Anatabine | Anatabine 1.0 mg/kg | Rat Plasma | 5A | 1 | 257 |
| Anatabine | Anatabine 1.0 mg/kg | Rat Plasma | 5B | 1 | 225 |
| Anatabine | Anatabine 1.0 mg/kg | Rat Plasma | 5C | 1 | 219 |
| Anatabine | Anatabine 1.0 mg/kg | Rat Plasma | 5A | 1.5 | 163 |
| Anatabine | Anatabine 1.0 mg/kg | Rat Plasma | 5B | 1.5 | 148 |
| Anatabine | Anatabine 1.0 mg/kg | Rat Plasma | 5C | 1.5 | 160 |
| Anatabine | Anatabine 1.0 mg/kg | Rat Plasma | 5A | 2 | 121 |
| Anatabine | Anatabine 1.0 mg/kg | Rat Plasma | 5B | 2 | 129 |
| Anatabine | Anatabine 1.0 mg/kg | Rat Plasma | 5C | 2 | 119 |
| Anatabine | Anatabine 1.0 mg/kg | Rat Plasma | 5A | 4 | 36 |
| Anatabine | Anatabine 1.0 mg/kg | Rat Plasma | 5B | 4 | 51 |
| Anatabine | Anatabine 1.0 mg/kg | Rat Plasma | 5C | 4 | 40 |
| Anatabine | Anatabine 1.0 mg/kg | Rat Plasma | 5A | 6 | 20 |
| Anatabine | Anatabine 1.0 mg/kg | Rat Plasma | 5B | 6 | 19 |
| Anatabine | Anatabine 1.0 mg/kg | Rat Plasma | 5C | 6 | 15 |
| Anatabine | Anatabine 1.0 mg/kg | Rat Plasma | 5G | 6 | 26 |
| Anatabine | Anatabine 1.0 mg/kg | Rat Plasma | 5H | 6 | 38 |
| Anatabine | Anatabine 1.0 mg/kg | Rat Plasma | 51 | 6 | 17 |
| Anatabine | Anatabine 1.0 mg/kg | Rat Plasma | 5A | 8 | 8 |

(continued)

| Compound | Dose Group | Tissue | Rat ID | Time Point (hr) | Concentration (ng/mL) |
|---|---|---|---|---|---|
| Anatabine | Anatabine 1.0 mg/kg | Rat Plasma | 5B | 8 | 9 |
| Anatabine | Anatabine 1.0 mg/kg | Rat Plasma | 5C | 8 | 6 |
| Anatabine | Anatabine 1.0 mg/kg | Rat Plasma | 5A | 24 | <LLQ |
| Anatabine | Anatabine 1.0 mg/kg | Rat Plasma | 5B | 24 | <LLQ |
| Anatabine | Anatabine 1.0 mg/kg | Rat Plasma | 5C | 24 | <LLQ |
| Anatabine | Anatabine 1.0 mg/kg | Rat Plasma | 6A | 0.25 | 293 |
| Anatabine | Anatabine 1.0 mg/kg | Rat Plasma | 6B | 0.25 | 271 |
| Anatabine | Anatabine 1.0 mg/kg | Rat Plasma | 6C | 0.25 | 302 |
| Anatabine | Anatabine 1.0 mg/kg | Rat Plasma | 6A | 0.5 | 222 |
| Anatabine | Anatabine 1.0 mg/kg | Rat Plasma | 6B | 0.5 | 253 |
| Anatabine | Anatabine 1.0 mg/kg | Rat Plasma | 6C | 0.5 | 236 |
| Anatabine | Anatabine 1.0 mg/kg | Rat Plasma | 6D | 0.5 | 347 |
| Anatabine | Anatabine 1.0 mg/kg | Rat Plasma | 6E | 0.5 | 362 |
| Anatabine | Anatabine 1.0 mg/kg | Rat Plasma | 6F | 0.5 | 395 |
| Anatabine | Anatabine 1.0 mg/kg | Rat Plasma | 6A | 1 | 218 |
| Anatabine | Anatabine 1.0 mg/kg | Rat Plasma | 6B | 1 | 225 |
| Anatabine | Anatabine 1.0 mg/kg | Rat Plasma | 6C | 1 | 244 |
| Anatabine | Anatabine 1.0 mg/kg | Rat Plasma | 6A | 1.5 | 196 |
| Anatabine | Anatabine 1.0 mg/kg | Rat Plasma | 6B | 1.5 | 192 |
| Anatabine | Anatabine 1.0 mg/kg | Rat Plasma | 6C | 1.5 | 211 |
| Anatabine | Anatabine 1.0 mg/kg | Rat Plasma | 6A | 2 | 147 |
| Anatabine | Anatabine 1.0 mg/kg | Rat Plasma | 6B | 2 | 170 |

(continued)

| Compound | Dose Group | Tissue | Rat ID | Time Point (hr) | Concentration (ng/mL) |
|---|---|---|---|---|---|
| Anatabine | Anatabine 1.0 mg/kg | Rat Plasma | 6C | 2 | 174 |
| Anatabine | Anatabine 1.0 mg/kg | Rat Plasma | 6A | 4 | 73 |
| Anatabine | Anatabine 1.0 mg/kg | Rat Plasma | 6B | 4 | 52 |
| Anatabine | Anatabine 1.0 mg/kg | Rat Plasma | 6C | 4 | 57 |
| Anatabine | Anatabine 1.0 mg/kg | Rat Plasma | 6A | 6 | 33 |
| Anatabine | Anatabine 1.0 mg/kg | Rat Plasma | 6B | 6 | 34 |
| Anatabine | Anatabine 1.0 mg/kg | Rat Plasma | 6C | 6 | 21 |
| Anatabine | Anatabine 1.0 mg/kg | Rat Plasma | 6G | 6 | 27 |
| Anatabine | Anatabine 1.0 mg/kg | Rat Plasma | 6H | 6 | 24 |
| Anatabine | Anatabine 1.0 mg/kg | Rat Plasma | 61 | 6 | 18 |
| Anatabine | Anatabine 1.0 mg/kg | Rat Plasma | 6A | 8 | 19 |
| Anatabine | Anatabine 1.0 mg/kg | Rat Plasma | 6B | 8 | 19 |
| Anatabine | Anatabine 1.0 mg/kg | Rat Plasma | 6C | 8 | 15 |
| Anatabine | Anatabine 1.0 mg/kg | Rat Plasma | 6A | 24 | <LLQ |
| Anatabine | Anatabine 1.0 mg/kg | Rat Plasma | 6B | 24 | <LLQ |
| Anatabine | Anatabine 1.0 mg/kg | Rat Plasma | 6C | 24 | <LLQ |

Table 15. Mean Concentrations and Descriptive Statistics of Anatabine and Nicotine in Rat Brain Extracts and Plasma Samples at Each Time Point

| Compound | Dose Group | Tissue | Sex | Time Point (hr) | Male or Female N = | Avg. Conc. (ng/ml for plasma and ng/g for brain) | ±STDEV | ±SEM | Combined Male and Female Male and Female Avg. Conc. (ng/ml for plasma and ng/g for brain) | n= | stdev | sem |
|---|---|---|---|---|---|---|---|---|---|---|---|---|
| Nicotine | Nicotine 0.4 mg/kg | Rat Brain | male | 0.5 | 3 | 94.3 | 20.6 | 11.9 | 107.2 | 6 | 19.3 | 7.9 |
| Nicotine | Nicotine 0.4 mg/kg | Rat Brain | male | 6 | 2 | 6.0 | 1.4 | 1.0 | 5.7 | 3 | 1.2 | 0.7 |
| Nicotine | Nicotine 0.4 mg/kg | Rat Brain | male | 24 | 3 | 6.3 | 2.3 | 1.3 | 6.5 | 4 | 1.9 | 1.0 |
| Nicotine | Nicotine 0.4 mg/kg | Rat Brain | female | 0.5 | 3 | 120.0 | 3.0 | 1.7 | | | | |
| Nicotine | Nicotine 0.4 mg/kg | Rat Brain | female | 6 | 1 | 5.0 | ND | ND | | | | |
| Nicotine | Nicotine 0.4 mg/kg | Rat Brain | female | 24 | 1 | 7.0 | ND | ND | | | | |
| Anatabine | Anatabine 0.1 mg/kg | Rat Brain | mate | 0.5 | 2 | 20.0 | 1.4 | 1.0 | 25.8 | 5 | 6.2 | 2.8 |
| Anatabine | Anatabine 0.1 mg/kg | Rat Brain | male | 6 | 1 | 3.0 | ND | ND | 3.5 | 4 | 1.3 | 0.6 |
| Anatabine | Anatabine 0.1 mg/kg | Rat Brain | male | 24 | 1 | 3.0 | ND | ND | 2.5 | 2 | 0.7 | 0.5 |
| Anatabine | Anatabine 0.1 mg/kg | Rat Brain | female | 0.5 | 3 | 29.7 | 4.5 | 2.6 | | | | |
| Anatabine | Anatabine 0.1 mg/kg | Rat Brain | female | 6 | 3 | 3.7 | 1.5 | 0.9 | | | | |
| Anatabine | Anatabine 0.1 mg/kg | Rat Brain | female | 24 | 1 | 2.0 | ND | ND | | | | |
| Anatabine | Anatabine 0.75 mg/kg | Rat Brain | male | 0.5 | 3 | 276.0 | 37.0 | 21.4 | 295.3 | 6 | 53.3 | 21.8 |

(continued)

| | | | | | Male or Female | | | | Combined Male and Female | | | |
|---|---|---|---|---|---|---|---|---|---|---|---|---|
| Compound | Dose Group | Tissue | Sex | Time Point (hr) | N = | Avg. Conc. (ng/ml for plasma and ng/g for brain) | ±STDEV | ±SEM | Male and Female Avg. Conc. (ng/ml for plasma and ng/g for brain) | n= | stdev | sem |
| Anatabine | Anatabine 0.75 mg/kg | Rat Brain | male | 6 | 3 | 21.3 | 5.1 | 3.0 | 21.2 | 6 | 5.1 | 2.1 |
| Anatabine | Anatabine 0.75 mg/kg | Rat Brain | male | 24 | 0 | <LLQ | ND | ND | <LLQ | 0 | ND | ND |
| Anatabine | Anatabine 0.75 mg/kg | Rat Brain | female | 0.5 | 3 | 314.7 | 67.9 | 39.2 | | | | |
| Anatabine | Anatabine 0.75 mg/kg | Rat Brain | female | 6 | 3 | 21.0 | 6.2 | 3.6 | | | | |
| Anatabine | Anatabine 0.75 mg/kg | Rat Brain | female | 24 | 0 | <LLQ | ND | ND | | | | |
| Anatabine | Anatabine 1.0 mg/kg | Rat Brain | male | 0.5 | 3 | 323.0 | 35.9 | 20.7 | 334.5 | 6 | 44.3 | 18.1 |
| Anatabine | Anatabine 1.0 mg/kg | Rat Brain | male | 6 | 3 | 5.0 | 2.6 | 1.5 | 5.4 | 5 | 2.4 | 1.1 |
| Anatabine | Anatabine 1.0 mg/kg | Rat Brain | male | 24 | 2 | 2.5 | 0.7 | 0.5 | 2.5 | 2 | 0.7 | 0.5 |
| Anatabine | Anatabine 1.0 mg/kg | Rat Brain | female | 0.5 | 3 | 346.0 | 56.7 | 32.7 | | | | |
| Anatabine | Anatabine 1.0 mg/kg | Rat Brain | female | 6 | 2 | 6.0 | 2.8 | 2.0 | | | | |
| Anatabine | Anatabine 1.0 mg/kg | Rat Brain | female | 24 | 0 | <LLQ | ND | ND | | | | |
| Nicotine | Nicotine 0.4 mg/kg | Rat Plasma | male | 0.25 | 3 | 165.0 | 25.7 | 14.8 | 166.5 | 6 | 20.8 | 8.5 |
| Nicotine | Nicotine 0.4 mg/kg | Rat Plasma | male | 0.5 | 3 | 99.3 | 20.6 | 11.9 | 104.8 | 6 | 17.2 | 7.0 |

(continued)

| Compound | Dose Group | Tissue | Sex | Time Point (hr) | Male or Female N = | Avg. Conc. (ng/ml for plasma and ng/g for brain) | ±STDEV | ±SEM | Combined Male and Female Male and Female Avg. Conc. (ng/ml for plasma and ng/g for brain) | n= | stdev | sem |
|---|---|---|---|---|---|---|---|---|---|---|---|---|
| Nicotine | Nicotine 0.4 mg/kg | Rat Plasma | male | 0.5 | 3 | 154.0 | 34.6 | 20.0 | 159.8 | 6 | 24.1 | 9.9 |
| Nicotine | Nicotine 0.4 mg/kg | Rat Plasma | male | 1 | 3 | 69.0 | 2.6 | 1.5 | 75.5 | 6 | 15.6 | 6.4 |
| Nicotine | Nicotine 0.4 mg/kg | Rat Plasma | male | 1.5 | 3 | 35.7 | 7.4 | 4.3 | 42.8 | 6 | 12.5 | 5.1 |
| Nicotine | Nicotine 0.4 mg/kg | Rat Plasma | male | 2 | 3 | 24.7 | 6.4 | 3.7 | 28.2 | 6 | 9.6 | 3.9 |
| Nicotine | Nicotine 0.4 mg/kg | Rat Plasma | male | 4 | 0 | <LLQ | ND | ND | 3.5 | 2 | 0.7 | 0.5 |
| Nicotine | Nicotine 0.4 mg/kg | Rat Plasma | male | 6 | 0 | <LLQ | ND | ND | <LLQ | 0 | ND | ND |
| Nicotine | Nicotine 0.4 mg/kg | Rat Plasma | male | 8 | 0 | <LLQ | ND | ND | <LLQ | 0 | ND | ND! |
| Nicotine | Nicotine 0.4 mg/kg | Rat Plasma | male | 24 | 0 | <LLQ | ND | ND | <LLQ | 0 | ND | ND |
| Nicotine | Nicotine 0.4 mg/kg | Rat Plasma | female | 0.25 | 3 | 168.0 | 20.4 | 11.8 | 108.4 | 5 | 83.0 | 37.1 |
| Nicotine | Nicotine 0.4 mg/kg | Rat Plasma | female | 0.5 | 3 | 110.3 | 15.0 | 8.7 | 79.7 | 12 | 65.0 | 18.8 |
| Nicotine | Nicotine 0.4 mg/kg | Rat Plasma | female | 0.5 | 3 | 165.7 | 12.5 | 7.2 | 95.3 | 6 | 77.7 | 31.7 |
| Nicotine | Nicotine 0.4 mg/kg | Rat Plasma | female | 1 | 3 | 82.0 | 21.8 | 12.6 | 50.8 | 6 | 37.6 | 15.3 |
| Nicotine | Nicotine 0.4 mg/kg | Rat Plasma | female | 1.5 | 3 | 50.0 | 13.5 | 7.8 | 33.6 | 5 | 24.4 | 10.9 |

(continued)

| | | | | | Male or Female | | | | Combined Male and Female | | | |
|---|---|---|---|---|---|---|---|---|---|---|---|---|
| Compound | Dose Group | Tissue | Sex | Time Point (hr) | N = | Avg. Conc. (ng/ml for plasma and ng/g for brain) | ±STDEV | ±SEM | Male and Female Avg. Conc. (ng/ml for plasma and ng/g for brain) | n= | stdev | sem |
| Nicotine | Nicotine 0.4 mg/kg | Rat Plasma | female | 2 | 3 | 31.7 | 12.4 | 7.2 | 19.2 | 6 | 15.8 | 6.5 |
| Nicotine | Nicotine 0.4 mg/kg | Rat Plasma | female | 4 | 2 | 3.5 | 0.7 | 0.5 | 3.6 | 5 | 0.9 | 0.4 |
| Nicotine | Nicotine 0.4 mg/kg | Rat Plasma | female | 6 | 0 | <LLQ | ND | ND | 5.5 | 2 | 2.1 | 1.5 |
| Nicotine | Nicotine 0.4 mg/kg | Rat Plasma | female | 6 | 0 | <LLQ | ND | ND | 5.5 | 2 | 2.1 | 1.5 |
| Nicotine | Nicotine 0.4 mg/kg | Rat Plasma | female | 8 | 0 | <LLQ | ND | ND | 4.0 | 1 | ND | ND |
| Nicotine | Nicotine 0.4 mg/kg | Rat Plasma | female | 24 | 0 | <LLQ | ND | ND | <LLQ | 0 | ND | ND |
| Anatabine | Anatabine 0.1 mg/kg | Rat Plasma | male | 0.25 | 3 | 19.3 | 5.0 | 2.9 | 18.0 | 6 | 4.2 | 1.7 |
| Anatabine | Anatabine 0.1 mg/kg | Rat Plasma | male | 0.5 | 3 | 15.3 | 2.1 | 1.2 | 22.9 | 12 | 9.4 | 2.7 |
| Anatabine | Anatabine 0.1 mg/kg | Rat Plasma | male | 0.5 | 3 | 31.7 | 1.5 | 0.9 | 31.7 | 6 | 1.6 | 0.7 |
| Anatabine | Anatabine 0.1 mg/kg | Rat Plasma | male | 1 | 3 | 12.3 | 3.2 | 1.9 | 8.0 | 6 | 5.2 | 2.1 |
| Anatabine | Anatabine 0.1 mg/kg | Rat Plasma | male | 1.5 | 2 | 6.5 | 0.7 | 0.5 | 9.2 | 5 | 2.8 | 1.2 |
| Anatabine | Anatabine 0.1 mg/kg | Rat Plasma | male | 2 | 3 | 6.3 | 1.5 | 0.9 | 8.7 | 6 | 3.6 | 1.5 |
| Anatabine | Anatabine 0.1 mg/kg | Rat Plasma | male | 4 | 2 | 3.0 | 0.0 | 0.0 | 5.2 | 5 | 2.6 | 1.2 |

(continued)

| Compound | Dose Group | Tissue | Sex | Time Point (hr) | Male or Female N = | Avg. Conc. (ng/ml for plasma and ng/g for brain) | ±STDEV | ±SEM | Combined Male and Female Male and Female Avg. Conc. (ng/ml for plasma and ng/g for brain) | n= | stdev | sem |
|---|---|---|---|---|---|---|---|---|---|---|---|---|
| Anatabine | Anatabine 0.1 mg/kg | Rat Plasma | male | 6 | 0 | <LLQ | ND | ND | 4.3 | 6 | 1.5 | 0.6 |
| Anatabine | Anatabine 0.1 mg/kg | Rat Plasma | male | 6 | 3 | 5.0 | 1.7 | 1.0 | 4.5 | 4 | 1.7 | 0.9 |
| Anatabine | Anatabine 0.1 mg/kg | Rat Plasma | male | 8 | 0 | <LLQ | ND | ND | <LLQ | 0 | ND | ND |
| Anatabine | Anatabine 0.1 mg/kg | Rat Plasma | male | 24 | 0 | <LLQ | ND | ND | <LLQ | 0 | ND | ND |
| Anatabine | Anatabine 0.1 mg/kg | Rat Plasma | female | 0.25 | 3 | 16.7 | 3.8 | 2.2 | | | | |
| Anatabine | Anatabine 0.1 mg/kg | Rat Plasma | female | 0.5 | 3 | 13.0 | 3.0 | 1.7 | | | | |
| Anatabine | Anatabine 0.1 mg/kg | Rat Plasma | female | 0.5 | 3 | 31.7 | 2.1 | 1.2 | | | | |
| Anatabine | Anatabine 0.1 mg/kg | Rat Plasma | female | 6 | 3 | 3.7 | 0.6 | 0.3 | | | | |
| Anatabine | Anatabine 0.1 mg/kg | Rat Plasma | female | 1 | 3 | 11.0 | 1.7 | 1.0 | | | | |
| Anatabine | Anatabine 0.1 mg/kg | Rat Plasma | female | 1.5 | 3 | 11.0 | 3.6 | 2.1 | | | | |
| Anatabine | Anatabine 0.1 mg/kg | Rat Plasma | female | 2 | 3 | 6.7 | 2.3 | 1.3 | | | | |
| Anatabine | Anatabine 0.1 mg/kg | Rat Plasma | female | 4 | 2 | 4.0 | 1.4 | 1.0 | | | | |
| Anatabine | Anatabine 0.1 mg/kg | Rat Plasma | female | 6 | 1 | 3.0 | ND | ND | | | | |

(continued)

| Compound | Dose Group | Tissue | Sex | Time Point (hr) | Male or Female N = | Avg. Conc. (ng/ml for plasma and ng/g for brain) | ±STDEV | ±SEM | Combined Male and Female Male and Female Avg. Conc. (ng/ml for plasma and ng/g for brain) | n= | stdev | sem |
|---|---|---|---|---|---|---|---|---|---|---|---|---|
| Anatabine | Anatabine 0.1 mg/kg | Rat Plasma | female | 8 | 0 | <LLQ | ND | ND | | | | |
| Anatabine | Anatabine 0.1 mg/kg | Rat Plasma | female | 24 | 0 | <LLQ | ND | ND | | | | |
| Anatabine | Anatabine 0.75 mg/kg | Rat Plasma | male | 0.25 | 3 | 200.3 | 33.4 | 19.3 | 206.3 | 6 | 23.4 | 9.5 |
| Anatabine | Anatabine 0.75 mg/kg | Rat Plasma | male | 0.5 | 3 | 180.7 | 8.1 | 4.7 | 256.6 | 12 | 82.2 | 23.7 |
| Anatabine | Anatabine 0.75 mg/kg | Rat Plasma | male | 0.5 | 3 | 301.7 | 36.5 | 21.1 | 327.8 | 6 | 46.4 | 18.9 |
| Anatabine | Anatabine 0.75 mg/kg | Rat Plasma | male | 1 | 3 | 151.7 | 5.1 | 3.0 | 162.2 | 6 | 23.1 | 9.4 |
| Anatabine | Anatabine 0.75 mg/kg | Rat Plasma | male | 1.5 | 3 | 126.7 | 8.3 | 4.8 | 133.3 | 6 | 10.6 | 4.3 |
| Anatabine | Anatabine 0.75 mg/kg | Rat Plasma | male | 2 | 3 | 83.7 | 17.6 | 10.2 | 91.7 | 6 | 16.1 | 6.6 |
| Anatabine | Anatabine 0.75 mg/kg | Rat Plasma | male | 4 | 3 | 34.7 | 5.1 | 3.0 | 40.0 | 6 | 7.3 | 3.0 |
| Anatabine | Anatabine 0.75 mg/kg | Rat Plasma | male | 6 | 3 | 14.0 | 1.0 | 0.6 | 19.3 | 12 | 7.8 | 2.2 |
| Anatabine | Anatabine 0.75 mg/kg | Rat Plasma | male | 6 | 3 | 22.0 | 8.2 | 4.7 | 22.5 | 6 | 9.7 | 4.0 |
| Anatabine | Anatabine 0.75 mg/kg | Rat Plasma | male | 8 | 3 | 9.0 | 1.7 | 1.0 | 10.0 | 6 | 1.7 | 0.7 |
| Anatabine | Anatabine 0.75 mg/kg | Rat Plasma | male | 24 | 0 | <LLQ | ND | ND | <LLQ | 0 | ND | ND |

(continued)

| Compound | Dose Group | Tissue | Sex | Time Point (hr) | Male or Female | | | | Combined Male and Female | | | |
|---|---|---|---|---|---|---|---|---|---|---|---|---|
| | | | | | N = | Avg. Conc. (ng/ml for plasma and ng/g for brain) | ±STDEV | ±SEM | Male and Female Avg. Conc. (ng/ml for plasma and ng/g for brain) | n= | stdev | sem |
| Anatabine | Anatabine 0.75 mg/kg | Rat Plasma | female | 0.25 | 3 | 212.3 | 11.9 | 6.9 | | | | |
| Anatabine | Anatabine 0.75 mg/kg | Rat Plasma | female | 0.5 | 3 | 190.0 | 34.1 | 19.7 | | | | |
| Anatabine | Anatabine 0.75 mg/kg | Rat Plasma | female | 0.5 | 3 | 354.0 | 44.7 | 25.8 | | | | |
| Anatabine | Anatabine 0.75 mg/kg | Rat Plasma | female | 1 | 3 | 172.7 | 31.2 | 18.0 | | | | |
| Anatabine | Anatabine 0.75 mg/kg | Rat Plasma | female | 1.5 | 3 | 140.0 | 8.7 | 5.0 | | | | |
| Anatabine | Anatabine 0.75 mg/kg | Rat Plasma | female | 2 | 3 | 99.7 | 12.2 | 7.1 | | | | |
| Anatabine | Anatabine 0.75 mg/kg | Rat Plasma | female | 4 | 3 | 45.3 | 4.5 | 2.6 | | | | |
| Anatabine | Anatabine 0.75 mg/kg | Rat Plasma | female | 6 | 3 | 18.3 | 4.5 | 2.6 | | | | |
| Anatabine | Anatabine 0.75 mg/kg | Rat Plasma | female | 6 | 3 | 23.0 | 13.0 | 7.5 | | | | |
| Anatabine | Anatabine 0.75 mg/kg | Rat Plasma | female | 8 | 3 | 11.0 | 1.0 | 0.6 | | | | |
| Anatabine | Anatabine 0.75 mg/kg | Rat Plasma | female | 24 | 0 | <LLQ | ND | ND | | | | |
| Anatabine | Anatabine 1.0 mg/kg | Rat Plasma | male | 0.25 | 3 | 285.7 | 13.8 | 8.0 | 287.2 | 6 | 13.4 | 5.5 |
| Anatabine | Anatabine 1.0 mg/kg | Rat Plasma | male | 0.5 | 3 | 272.3 | 13.6 | 7.8 | 319.1 | 12 | 73.1 | 21.1 |

(continued)

| Compound | Dose Group | Tissue | Sex | Time Point (hr) | Male or Female N = | Avg. Conc. (ng/ml for plasma and ng/g for brain) | ±STDEV | ±SEM | Combined Male and Female Male and Female Avg. Conc. (ng/ml for plasma and ng/g for brain) | n= | stdev | sem |
|---|---|---|---|---|---|---|---|---|---|---|---|---|
| Anatabine | Anatabine 1.0 mg/kg | Rat Plasma | male | 0.5 | 3 | 399.0 | 42.5 | 24.6 | 383.5 | 6 | 35.4 | 14.5 |
| Anatabine | Anatabine 1.0 mg/kg | Rat Plasma | male | 1 | 3 | 233.7 | 20.4 | 11.8 | 231.3 | 6 | 15.7 | 6.4 |
| Anatabine | Anatabine 1.0 mg/kg | Rat Plasma | male | 1.5 | 3 | 157.0 | 7.9 | 4.6 | 178.3 | 6 | 24.7 | 10.1 |
| Anatabine | Anatabine 1.0 mg/kg | Rat Plasma | male | 2 | 3 | 123.0 | 5.3 | 3.1 | 143.3 | 6 | 24.3 | 9.9 |
| Anatabine | Anatabine 1.0 mg/kg | Rat Plasma | male | 4 | 3 | 42.3 | 7.8 | 4.5 | 51.5 | 6 | 13.2 | 5.4 |
| Anatabine | Anatabine 1.0 mg/kg | Rat Plasma | male | 6 | 3 | 18.0 | 2.6 | 1.5 | 24.3 | 12 | 7.4 | 2.1 |
| Anatabine | Anatabine 1.0 mg/kg | Rat Plasma | male | 6 | 3 | 27.0 | 10.5 | 6.1 | 25.0 | 6 | 7.6 | 3.1 |
| Anatabine | Anatabine 1.0 mg/kg | Rat Plasma | male | 8 | 3 | 7.7 | 1.5 | 0.9 | 12.7 | 6 | 5.8 | 2.3 |
| Anatabine | Anatabine 1.0 mg/kg | Rat Plasma | male | 24 | 0 | <LLQ | ND | ND | <LLQ | 0 | ND | ND |
| Anatabine | Anatabine 1.0 mg/kg | Rat Plasma | female | 0.25 | 3 | 288.7 | 15.9 | 9.2 | | | | |
| Anatabine | Anatabine 1.0 mg/kg | Rat Plasma | female | 0.5 | 3 | 237.0 | 15.5 | 9.0 | | | | |
| Anatabine | Anatabine 1.0 mg/kg | Rat Plasma | female | 0.5 | 3 | 368.0 | 24.6 | 14.2 | | | | |
| Anatabine | Anatabine 1.0 mg/kg | Rat Plasma | female | 1 | 3 | 229.0 | 13.5 | 7.8 | | | | |

(continued)

| | | | | | Male or Female | | | | Combined Male and Female | | | |
|---|---|---|---|---|---|---|---|---|---|---|---|---|
| Compound | Dose Group | Tissue | Sex | Time Point (hr) | N = | Avg. Conc. (ng/ml for plasma and ng/g for brain) | ±STDEV | ±SEM | Male and Female Avg. Conc. (ng/ml for plasma and ng/g for brain) | n= | stdev | sem |
| Anatabine | Anatabine 1.0 mg/kg | Rat Plasma | female | 1.5 | 3 | 199.7 | 10.0 | 5.8 | | | | |
| Anatabine | Anatabine 1.0 mg/kg | Rat Plasma | female | 2 | 3 | 163.7 | 14.6 | 8.4 | | | | |
| Anatabine | Anatabine 1.0 mg/kg | Rat Plasma | female | 4 | 3 | 60.7 | 11.0 | 6.3 | | | | |
| Anatabine | Anatabine 1.0 mg/kg | Rat Plasma | female | 6 | 3 | 29.3 | 7.2 | 4.2 | | | | |
| Anatabine | Anatabine 1.0 mg/kg | Rat Plasma | female | 6 | 3 | 23.0 | 4.6 | 2.6 | | | | |
| Anatabine | Anatabine 1.0 mg/kg | Rat Plasma | female | 8 | 3 | 17.7 | 2.3 | 1.3 | | | | |
| Anatabine | Anatabine 1.0 mg/kg | Rat Plasma | female | 24 | 0 | <LLQ | ND | ND | | | | |

Table 16. Dosing solutions

| Test compound | Percentage content of anatabine or nicotine base | Dose level (mg/kg) | Test compound base concentration (mg/mL) | Injection volume (mL/kg) |
|---|---|---|---|---|
| Anatabine | 41.6 | 0.10 | 0.048 | 5 |
| Anatabine | 41.6 | 0.75 | 0.36 | 5 |
| Anatabine | 41.6 | 1.5 | 0.72 | 5 |
| Nicotine | 35.1 | 0.75 | 0.36 | 5 |

Table 17. Study Outline

| Test compound | Route | Dose level (mg/kg) | Number of animals (M/F) | Observations, body weight and food consumption frequency |
|---|---|---|---|---|
| Vehicle | i.v. | 0.0 | 5/5 | Daily for 14 days |
| Anatabine | i.v. | 0.10 | 5/5 | Daily for 14 days |
| Anatabine | i.v. | 0.75 | 5/5 | Daily for 14 days |
| Anatabine | i.v. | 1.5 | 5/5 | Daily for 14 days |
| Nicotine | i.v. | 1.5 | 5/5 | Not applicable |
| Nicotine | i.v. | 0.75 | 5/5 | Daily for 14 days |

Table 18. Test Performed and Tissues Collected

| Parameter | Tests performed/tissues collected |
|---|---|
| Hematology | Hematocrit, Hemoglobin, MCH, MCH Concentration, MCV, RBC, Reticulocyte count, Platelet count, WBC, WBC differential, blood smear evaluation |
| Clinical Chemistry | A/G ratio (calculated). ALT. Albumin, Alkaline phosphatase, AST, Bilirubin, Calcium, Chloride, Cholesterol (total), Creatinine, Globulin (calculated), Glucose, Phosphorus (inorganic), Potassium, Sodium. Total protein, BUN |
| Coagulation | Activated partial thromboplastin time, Prothrombin time |
| Urinalysis | Bilirubin, Blood, color and appearance, Glucose, Ketones, pH. Protein, Specific gravity, Urobilinogen. Volume |
| Necropsy | Adrenal glands[1], Brain, Heart. Kidneys. Liver. Lungs, Ovaries and Uterus, Pituitary gland[1], Prostate gland, Spleen, Testes, Thymus, Thyroid and Parathyroid glands[1], section of small intestines |
| [1] Not weighed; placed in cassettes. | |

Table 19. Dosing Solution Analysis

| Compound | Dosing Date | Dose (mg/kg) | Expected Conc. (mg/ml) | Actual Concentration (mg/mL) | Actual Concentration relative to Expected % |
|---|---|---|---|---|---|
| Nicotine | Jun 16 | 0.4 | 0.081 | 0.096 | 118.5 |
| Anatabine | Jun 09 | 0.1 | 0.02 | 0.016 | 80 |
| Anatabine | Jun 09 | 0.75 | 0.15 | 0.127 | 85 |
| Anatabine | Jun 09 | 1.5 | 0.2 | 0.239 | 119.5 |

**Table 20. Mean Increase in Body Weights and Daily Food Consumption (descriptive statistics)**

| Test Compound | Dose (mg/kg) | Gender | Number of animals | Mean weight increase by day 14 (g) | Standard Deviation | *p* (comparison to Vehicle) | Mean food consumption/ day (g) | Standard Deviation | *p* (comparison to Vehicle) |
|---|---|---|---|---|---|---|---|---|---|
| Vehicle | - | Male | 5 | 130.6 | 16.8 | | 27.7 | 2.8 | |
| | | Female | 5 | 49.7 | 6.4 | | 20.3 | 2.7 | |
| Anatabine | 0.1 | Male | 5 | 132.5 | 17.1 | 0.864 | 31.0 | 4.0 | **0.001** |
| | | Female | 5 | 39.0 | 10.3 | 0.083 | 20.8 | 3.6 | 0.535 |
| Anatabine | 0.75 | Male | 5 | 111.6 | 8.9 | 0.055 | 27.8 | 3.2 | 0.936 |
| | | Female | 5 | 43.1 | 9.3 | 0.229 | 19.7 | 2.8 | 0.434 |
| Anatabine | 1.5 | Male | 5 | 137.9 | 14.0 | 0.475 | 30.8 | 4.0 | **0.001** |
| | | Female | 5 | 38.6 | 13.0 | 0.125 | 19.8 | 3.0 | 0.504 |
| Nicotine | 0.75 | Male | 4 | 98.5 | 15.8 | **0.0001** | 26.4 | 3.2 | 0.098 |
| | | Female | 3 | 40.8 | 3.4 | 0.071 | 20.6 | 2.6 | 0.598 |

**Table 21. Mean Organ Weights (descriptive statistics) for Male Animals by Treatment Group**

| Test Compound | Dose (mg/kg) | | Organ Weight (g) | | | | | | | | | |
|---|---|---|---|---|---|---|---|---|---|---|---|---|
| | | | Thymus | Heart | Lungs | Liver | Kidneys | Spleen | Small intestine | Prostate | Testes | Brain |
| | | | | | | | | | | | | |
| Vehicle | - | Mean | 0.77 | 1.66 | 1.90 | 15.81 | 3.53 | 1.03 | 0.64 | 0.49 | 3.39 | 2.02 |
| | n=5 | Std Dev | 0.04 | 0.18 | 0.21 | 1.44 | 0.30 | 0.24 | 0.43 | 0.11 | 0.07 | 0.12 |
| | | Mean | 0.75 | 1.74 | 1.98 | 16.81 | 3.57 | 0.82 | 0.66 | 0.59 | 3.42 | 2.15 |
| Anatabine | 0.1 | Std Dev | 0.04 | 0.16 | 0.22 | 0.96 | 0.29 | 0.11 | 0.26 | 0.12 | 0.24 | 0.18 |
| | n=5 | *p*[a] | ns[b] | ns | ns | ns | ns | ns | ns | ns | ns | ns |
| | | Mean | 0.67 | 1.43 | 1.82 | 14.50 | 3.15 | 0.74 | 0.68 | 0.51 | 3.21 | 2.07 |
| Anatabine | 0.75 | StdDev | 0.22 | 0.11 | 0.16 | 0.50 | 0.32 | 0.12 | 0.41 | 0.08 | 0.42 | 0.08 |
| | n=5 | *p* | ns | **0.04** | ns | ns | ns | **0.04** | ns | ns | ns | ns |
| | | Mean | 0.72 | 1.52 | 1.94 | 16.55 | 3.45 | 0.86 | 0.68 | 0.47 | 3.24 | 1.93 |
| Anatabine | 1.5 | Std Dev | 0.14 | 0.06 | 0.11 | 1.55 | 0.28 | 0.10 | 0.22 | 0.03 | 0.28 | 0.14 |
| | n=5 | *p* | ns | ns | ns | ns | ns | ns | ns | ns | ns | ns |
| | | Mean | 0.58 | 1.25 | 1.65 | 12.69 | 3.03 | 0.77 | 0.55 | 0.36 | 3.03 | 1.94 |
| Nicotine | 0.75 | Std Dev | 0.13 | 0.11 | 0.14 | 1.04 | 0.13 | 0.10 | 0.13 | 0.09 | 0.09 | 0.09 |
| | n=4 | *p* | ns | **0.006** | ns | **0.008** | **0.017** | ns | ns | ns | ns | ns |

[a] *p*. probability relative to Vehicle control. [b] ns, not significant

**Table 22 Mean Organ Weights (descriptive statistics) for Female Animals by Treatment Group**

| Test Compound | Dose (mg/kg) | | Organ Weight (g) | | | | | | | | |
|---|---|---|---|---|---|---|---|---|---|---|---|
| | | | Thymus | Heart | Lungs | Liver | Kidneys | Spleen | Small intestine | Ovaries/ uterus | Brain |
| | | | | | | | | | | | |
| Vehicle | - | Mean | 0.69 | 1.27 | 1.52 | 10.94 | 2.34 | 0.61 | 0.82 | 1.10 | 1.93 |
| | n=5 | Std Dev | 0.10 | 0.09 | 0.13 | 1.18 | 0.40 | 0.09 | 0.39 | 0.53 | 0.15 |
| Anatabine | 0.1 | Mean | 0.64 | 1.13 | 1.41 | 10.84 | 2.18 | 0.65 | 0.65 | 1.14 | 1.86 |
| | | Std Dev | 0.10 | 0.14 | 0.17 | 1.38 | 0.18 | 0.10 | 0.15 | 0.45 | 0.06 |
| | n=5 | *p*[a] | ns[b] | ns | ns | ns | ns | ns | ns | ns | ns |
| Anatabine | 0.75 | Mean | 0.56 | 1.11 | 1.45 | 10.42 | 2.30 | 0.60 | 0.75 | 1.58 | 1.90 |
| | | Std Dev | 0.04 | 0.11 | 0.12 | 1.09 | ns | 0.09 | 0.38 | 1.20 | 0.13 |
| | n=5 | *p* | **0.026** | **0.033** | ns | ns | ns | ns | ns | ns | ns |
| Anatabine | 1.5 | Mean | 0.57 | 1.00 | 1.46 | 10.04 | 2.28 | 0.60 | 0.70 | 1.03 | 1.95 |
| | | Std Dev | 0.05 | 0.09 | 0.14 | 1.16 | 0.29 | 0.08 | 0.23 | 0.24 | 0.08 |
| | n=5 | *p* | ns | **0.001** | ns | ns | ns | ns | ns | ns | ns |
| Nicotine | 0.75 | Mean | 0.50 | 0.99 | 1.32 | 9.59 | 2.31 | 0.56 | 0.36 | 1.28 | 1.86 |
| | | Std Dev | 0.03 | 0.14 | 0.14 | 0.47 | 0.03 | 0.17 | 0.07 | 0.34 | 0.08 |
| | n=3 | *p* | **0.022** | **0.013** | ns | ns | ns | ns | ns | ns | ns |

[a] *p*. probability relative to Vehicle control
[b] ns, not significant

**Table 23A. Hematology Parameters (descriptive statistics) (Part 1) by Treatment Group and Gender**

| Treatment Group | Gender | | WBC x $10^3/\mu L$ | RBC x $10^6/\mu L$ | HGB g/dL | HCT % | MCV fL | MCH pg | MCHC % | RETICULOCYTE COUNT % | PLATELET COUNT x $10^3/\mu L$ |
|---|---|---|---|---|---|---|---|---|---|---|---|
| Vehicle | M | n | 5 | 5 | 5 | 5 | 5 | 5 | 5 | 5 | 4 |
| | | Mean | 10.5 | 7.3 | 15.2 | 45.4 | 62.2 | 20.7 | 33.4 | 6.1 | 1287.3 |
| | | Std Dev | 1.4 | 0.2 | 0.5 | 1.5 | 1.5 | 0.6 | 0.4 | 1.0 | 210.8 |
| | F | N | 5 | 5 | 5 | 5 | 5 | 5 | 5 | 5 | 5 |
| | | Mean | 9.3 | 7.8 | 16.4 | 47.3 | 61.0 | 21.0 | 34.5 | 5.0 | 1198.4 |
| | | Std Dev | 2.8 | 0.7 | 1.7 | 4.7 | 1.2 | 0.6 | 0.8 | 1.1 | 200.2 |
| Anatabine 0.1 mg/kg | M | n | 5 | 5 | 5 | 5 | 5 | 5 | 5 | 5 | 5 |
| | | Mean | 15.6 | 7.4 | 15.8 | 47.1 | 63.4 | 21.2 | 33.5 | 5.5 | 1248.6 |
| | | Std Dev | 4.7 | 0.4 | 1.2 | 2.9 | 1.8 | 0.6 | 0.9 | 0.5 | 358.1 |
| | | *p*[a] | ns[b] | ns | ns | ns | ns | ns | ns | ns | ns |
| | F | n | 5 | 5 | 5 | 5 | 5 | 5 | 5 | 5 | 3 |
| | | Mean | 9.3 | 7.8 | 15.6 | 46.4 | 60.0 | 20.2 | 33.7 | 4.5 | 997.3 |
| | | Std Dev | 3.4 | 0.7 | 1.0 | 3.2 | 2.0 | 0.8 | 0.3 | 1.5 | 165.8 |
| | | *p* | ns | ns | ns | ns | ns | ns | **0.042**[c] | ns | ns |
| | | n | 5 | 5 | 5 | 5 | 5 | 5 | 5 | 5 | 4 |

(continued)

| Treatment Group | Gender | | WBC x $10^3/\mu L$ | RBC x $10^6/\mu L$ | HGB g/dL | HCT % | MCV fL | MCH pg | MCHC % | RETICULOCYTE COUNT % | PLATELET COUNT x $10^3/\mu L$ |
|---|---|---|---|---|---|---|---|---|---|---|---|
| Anatabine 0.75 mg/kg | M | Mean | 12.1 | 7.5 | 15.6 | 45.8 | 61.4 | 20.9 | 34.0 | 4.4 | 1325.8 |
| | | Std Dev | 5.7 | 0.7 | 1.5 | 4.0 | 1.5 | 0.8 | 0.7 | 0.6 | 193.7 |
| | | *p* | ns | ns | ns | ns | ns | ns | ns | **0.012**[c] | ns |
| | F | n | 5 | 5 | 5 | 5 | 5 | 5 | 5 | 5 | 5 |
| | | Mean | 10.3 | 7.5 | 15.4 | 45.2 | 59.8 | 20.4 | 34.1 | 3.3 | 1269.6 |
| | | Std Dev | 4.0 | 0.3 | 0.5 | 1.5 | 2.3 | 0.8 | 0.3 | 0.9 | 356.4 |
| | | *p* | ns | ns | ns | ns | ns | ns | ns | **0.022**[c] | ns |

**Table 23B. Hematology Parameters (descriptive statistics) (Part 1) by Treatment Group and Gender - cont'd**

| Treatment Group | Gender | | WBC x $10^3/\mu L$ | RBC x $10^6/\mu L$ | HGB g/dL | HCT % | MCV fL | MCH pg | MCHC % | RETICULOCYTE COUNT % | PLATELET COUNT x $10^3/\mu L$ |
|---|---|---|---|---|---|---|---|---|---|---|---|
| Anatabine 1.5 mg/kg | M | n | 5 | 5 | 5 | 5 | 5 | 5 | 5 | 5 | 5 |
| | | Mean | 13.1 | 7.5 | 15.4 | 46.1 | 62.0 | 20.6 | 33.4 | 4.2 | 1222.5 |
| | | Std Dev | 3.1 | 0.2 | 0.6 | 2.2 | 1.9 | 0.5 | 0.4 | 0.8 | 248.4 |
| | | *p* | ns | ns | ns | ns | ns | ns | ns | **0.011**[c] | ns |
| | F | n | 5 | 5 | 5 | 5 | 5 | 5 | 5 | 5 | 5 |
| | | Mean | 9.2 | 7.7 | 15.2 | 45.4 | 58.8 | 19.6 | 33.4 | 3.2 | 1127.4 |
| | | Std Dev | 2.5 | 0.4 | 0.7 | 2.7 | 0.8 | 0.4 | 0.6 | 0.9 | 503.7 |
| | | *p* | ns | ns | ns | ns | **0.011**[c] | **0.002**[c] | **0.036**[c] | **0.021**[c] | ns |
| Nicotine 0.75 mg/kg | M | n | 4 | 4 | 4 | 4 | 4 | 4 | 4 | 4 | 4 |
| | | Mean | 11.4 | 7.6 | 15.8 | 46.6 | 61.5 | 20.9 | 34.0 | 5.2 | 1355.8 |
| | | Std Dev | 3.2 | 0.5 | 0.7 | 2.4 | 1.0 | 0.6 | 0.5 | 0.8 | 83.0 |
| | | *p* | ns | ns | ns | ns | ns | ns | ns | ns | ns |
| | F | n | 3 | 3 | 3 | 3 | 3 | 3 | 3 | 3 | 2 |
| | | Mean | 10.2 | 8.0 | 16.0 | 46.7 | 58.7 | 19.9 | 34.2 | 5.1 | 1363.0 |
| | | Std Dev | 3.1 | 0.5 | 0.7 | 2.2 | 1.2 | 0.5 | 0.2 | 0.7 | 248.9 |
| | | *p* | ns | ns | ns | ns | **0.038**[c] | **0.038**[c] | ns | ns | ns |

[a] *p*. probability relative to Vehicle control
[b] ns, not significant
[c] Mean within normal range

**Table 24. Hematology Parameters (descriptive statistics) (Part 2) by Treatment Group and Gender**

| Treatment Group | Gender | | **NEUTROPHIL SEG %** | **LYMPHOCYTE %** | **MONOCYTE %** | **EOSINOPHIL %** | **BASOPHIL %** |
|---|---|---|---|---|---|---|---|
| Vehicle | M | n | 5 | 5 | 5 | 5 | 5 |
| | | Mean | 8.8 | 89.6 | 1.6 | 0.0 | 0.0 |
| | | Std Dev | 2.2 | 2 3 | 1.5 | 0.0 | 0.0 |
| | F | N | 5 | 5 | 5 | 5 | 5 |
| | | Mean | 12.6 | 85.2 | 2.2 | 0.0 | 0.0 |
| | | Std | 2.6 | 2.9 | 0.4 | 0.0 | 0.0 |
| Anatabine 0.1 mg/kg | M | n | 5 | 5 | 5 | 5 | 5 |
| | | Mean | 8.6 | 89.2 | 2.0 | 0.2 | 0.0 |
| | | Std Dev | 3.6 | 2.9 | 1.0 | 0.4 | 0.0 |
| | | *p*[a] | ns[b] | ns | ns | ns | ns |
| | F | n | 5 | 5 | 5 | 5 | 5 |
| | | Mean | 8.0 | 90.2 | 1.8 | 0.0 | 0.0 |
| | | Std Dev | 1.9 | 1.9 | 0.8 | 0.0 | 0.0 |
| | | *p* | ns | ns | ns | ns | ns |
| Anatabine 0 75 mg/kg | M | n | 5 | 5 | 5 | 5 | 5 |
| | | Mean | 9.8 | 88.8 | 1.4 | 0.0 | 0.0 |
| | | Std Dev | 2.5 | 2.2 | 0.5 | 0.0 | 0.0 |
| | | *p* | ns | ns | ns | ns | ns |
| | F | n | 5 | 5 | 5 | 5 | 5 |
| | | Mean | 8.6 | 90.6 | 0.8 | 0.0 | 0.0 |
| | | Std Dev | 2.3 | 2.6 | 0.8 | 0.0 | 0.0 |
| | | *p* | ns | ns | ns | ns | ns |
| | M | n | 5 | 5 | 5 | 5 | 5 |
| | | Mean | 14.6 | 83.8 | 1.6 | 0.0 | 0.0 |
| | | Std Dev | 7.7 | 8.0 | 0.9 | 0.0 | 0.0 |

(continued)

| Treatment Group | Gender | | **NEUTROPHIL SEG %** | **LYMPHOCYTE %** | **MONOCYTE %** | **EOSINOPHIL %** | **BASOPHIL %** |
|---|---|---|---|---|---|---|---|
| Anatabine 1.5 mg/kg | | *p* | ns | ns | ns | ns | ns |
| | F | n | 5 | 5 | 5 | 5 | 5 |
| | | Mean | 12.6 | 86.6 | 0.8 | 0.0 | 0.0 |
| | | Std Dev | 4.4 | 3.8 | 0.8 | 0.0 | 0.0 |
| | | *p* | ns | ns | ns | ns | ns |
| Nicotine 0.75 mg/kg | M | n | 4 | 4 | 4 | 4 | 4 |
| | | Mean | 11.8 | 85.8 | 2.5 | 0.0 | 0.0 |
| | | Std Dev | 3.3 | 4.2 | 1.0 | 0.0 | 0.0 |
| | | *p* | ns | ns | ns | ns | ns |
| | F | n | 3 | 3 | 3 | 3 | 3 |
| | | Mean | 12.7 | 86.3 | 1.0 | 0.0 | 0.0 |
| | | Std Dev | 2.1 | 3.1 | 1.0 | 0.0 | 0.0 |
| | | *p* | ns | ns | ns | ns | |

[a] *p.* probability relative to vehicle control
[b] ns. not significant

**Table 25. Coagulation Parameters (descriptive statistics) by Treatment Group and Gender**

| Treatment Group | Gender | | ACTIVATED PARTIAL THROMBOPLASTIN TIME (seconds) | PROTHROMBIN TIME (seconds) |
|---|---|---|---|---|
| Vehicle | M | n | 5 | 5 |
| | | Mean | 32.0 | 12.9 |
| | | Sid Dev | 2.2 | 0.5 |
| | F | n | 5 | 5 |
| | | Mean | 35.4 | 12.7 |
| | | Std Dev | 4.1 | 0.3 |
| Anatabine 0.1 mg/kg | M | n | 5 | 5 |
| | | Mean | 32.4 | 12.8 |
| | | Sid Dev | 3.3 | 0.7 |
| | | *p*[a] | ns[b] | ns |
| | F | n | 5 | 5 |
| | | Mean | 29.4 | 12.1 |
| | | Sid Dev | 3.4 | 0.4 |
| | | *p* | ns | ns |
| Anatabine 0.75 mg/kg | M | n | 5 | 5 |
| | | Mean | 38.7 | 13.3 |
| | | Sid Dev | 9.1 | 2.6 |
| | | *p* | ns | ns |
| | F | n | 5 | 5 |
| | | Mean | 33.3 | 14.1 |
| | | Std Dev | 2.8 | 5 |
| | | *p* | ns | ns |
| Anatabine 1.5 mg/kg | M | n | 5 | 5 |
| | | Mean | 30.6 | 13.2 |
| | | Sid Dev | 3.1 | 0.4 |
| | | *p* | ns | ns |
| | F | n | 5 | 5 |
| | | Mean | 32.5 | 13.3 |
| | | Std Dev | 2.9 | 0.2 |
| | | *p* | ns | ns |
| | | n | 4 | 4 |

(continued)

| Treatment Group | Gender | | ACTIVATED PARTIAL THROMBOPLASTIN TIME (seconds) | PROTHROMBIN TIME (seconds) |
|---|---|---|---|---|
| Nicotine 0.75 mg/kg | M | Mean | 16.3 | 13.6 |
| | | Std Dev | 1.1 | 0.6 |
| | | *p* | **<0.001[c]** | ns |
| | F | n | 2 | 2 |
| | | Mean | 16.9 | 14.1 |
| | | Sld Dev | 2.6 | 0.2 |
| | | *p* | **0.002[c]** | ns |

[a]*p*. probability relalive to Vehicle control
[b] ns. not significant
[c] Mean within normal range

**Table 26A. Clinical Chemistry Parameters (descriptive statistics) (Part 1) by Treatment Group and Gender**

| Treatment Group | Gender | | ALK PHOSPHATASE IU/L | ALT IU/L | AST IU/L | ALBUMIN g/dL | TOT PROTEIN g/dL | GLOBULIN g/dL | TOT BILIRUBIN mg/dL | DIR BILIRUBIN mg/dL | BUN mg/dL |
|---|---|---|---|---|---|---|---|---|---|---|---|
| Vehicle | M | n | 5 | 5 | 5 | 5 | 5 | 5 | 5 | 5 | 5 |
| | | Mean | 370.80 | 59.40 | 99.00 | 3.12 | 5.62 | 2.50 | 0.00 | 0.00 | 18.60 |
| | | Std Dev | 7247 | 2.07 | 22.86 | 0 04 | 004 | 000 | 0.00 | 0.00 | 1.14 |
| | F | N | 5 | 5 | 5 | 5 | 5 | 5 | 5 | 5 | 5 |
| | | Mean | 230.20 | 58 20 | 80.80 | 3.40 | 6.24 | 2.84 | 0.00 | 0.000 | 18.20 |
| | | Std Dev | 54.56 | 444 | 11 32 | 0.07 | 0.09 | 0 11 | 000 | 0.00 | 4.44 |
| Anatabine 0.1 mg/kg | M | n | 5 | 5 | 5 | 5 | 5 | 5 | 5 | 5 | 5 |
| | | Mean | 407.40 | 70.60 | 96.00 | 3.24 | 6.10 | 2.86 | 0.00 | 0.00 | 18.00 |
| | | Std Dev | 145.89 | 17 77 | 7.5 | 0.15 | 023 | 0.18 | 0.00 | 0.00 | 2.55 |
| | | *p*[a] | ns[b] | ns | ns | ns | **0.002[c]** | **0.002[c]** | ns | ns | ns |
| | F | n | 5 | 5 | 5 | 5 | 5 | 5 | 5 | 5 | 5 |
| | | Mean | 248.40 | 49.60 | 77.00 | 3.62 | 6.64 | 3.02 | 0.00 | 0.00 | 19.80 |
| | | Std Dev | 101.28 | 9.63 | 742 | 0.11 | 0.22 | 0.13 | 000 | 0.00 | 1.10 |
| | | *p* | ns | ns | ns | **0.005[c]** | **0.005** | **0.049[c]** | ns | ns | ns |

(continued)

| Treatment Group | Gender | | ALK PHOSPHATASE IU/L | ALT IU/L | AST IU/L | ALBUMIN g/dL | TOT PROTEIN g/dL | GLOBULIN g/dL | TOT BILIRUBIN mg/dL | DIR BILIRUBIN mg/dL | BUN mg/dL |
|---|---|---|---|---|---|---|---|---|---|---|---|
| Anatabine 0 75 mg/kg | M | n | 5 | 5 | 5 | 5 | 5 | 5 | 5 | 5 | 5 |
| | | Mean | 370.80 | 66.80 | 89.60 | 3.28 | 6.04 | 2.76 | 0.02 | 0.02 | 16.60 |
| | | Std Dev | 74 89 | 14 64 | 14.12 | 0.13 | 0.24 | 0.11 | 0.04 | 004 | 3.36 |
| | | *p* | ns | ns | ns | **0.032[c]** | **0.005[c]** | **0.001[c]** | ns | ns | ns |
| | F | n | 5 | 5 | 5 | 5 | 5 | 5 | 5 | 5 | 5 |
| | | Mean | 158.00 | 54.60 | 7320 | 3.52 | 6.38 | 2.86 | 0.00 | 0.00 | 15.40 |
| | | Std Dev | 45.27 | 590 | 14 75 | 0 08 | 0 16 | 0 13 | 0.00 | 000 | 0.55 |
| | | *p* | ns | ns | ns | **0.040[c]** | ns | ns | ns | ns | ns |

**Table 26B. Clinical Chemistry Parameters (descriptive statistics) (Part 1) by Treatment Group and Gender - cont'd**

| Treatment Group | Gender | | ALK PHOSPHATASE IU/L | ALT IU/L | AST IU/L | ALBUMIN g/dL | TOT PROTEIN g/dL | GLOBULIN g/dL | TOT BILIRUBIN mg/dL | DIR BILIRUBIN mg/dL | BUN mg/dL |
|---|---|---|---|---|---|---|---|---|---|---|---|
| Anatabine 1.5mg/kg | M | n | 5 | 5 | 5 | 5 | 5 | 5 | 5 | 5 | 5 |
| | | Mean | 411.40 | 7040 | 97.00 | 3.32 | 6.00 | 268 | 000 | 0.00 | 17.20 |
| | | Std Dev | 133.64 | 2061 | 26.88 | 0.13 | 0.25 | 0.13 | 0.00 | 0.00 | 1 30 |
| | | *p* | ns | ns | ns | **0.012[c]** | **0.011[c]** | **0.015[c]** | ns | ns | ns |
| | F | n | 5 | 5 | 5 | 5 | 5 | 5 | 5 | 5 | 5 |
| | | Mean | 194 00 | 49.20 | 76.60 | 3.48 | 6.36 | 288 | 002 | 0.02 | 16 20 |
| | | Std Dev | 58.97 | 6 18 | 9.21 | 0.15 | 0.28 | 0.16 | 0.04 | 0.04 | 2.17 |
| | | *p* | ns | ns | ns | ns | ns | ns | ns | ns | ns |
| Nicotine 0.75 mg/kg | M | n | 4 | 4 | 4 | 4 | 4 | 4 | 4 | 4 | 4 |
| | | Mean | 291.00 | 55.75 | 82.25 | 3.08 | 6.00 | 2.93 | 0.05 | 0.00 | 17.50 |
| | | Std Dev | 51 59 | 8.46 | 763 | 0 05 | 0.16 | 0 17 | 006 | 000 | 1.73 |
| | | *p* | ns | ns | ns | ns | **0.001[c]** | **0.001[c]** | ns | ns | ns |
| | F | n | 3 | 3 | 3 | 3 | 3 | 3 | 3 | 3 | 3 |
| | | Mean | 189.67 | 68.33 | 103.00 | 3.30 | 6.50 | 3.20 | 0.10 | 0.03 | 19.00 |
| | | Std Dev | 74.33 | 24 83 | 29.05 | 0.00 | 0.10 | 0 10 | 0.00 | 0.06 | 1.73 |
| | | *p* | ns | ns | ns | ns | **0.009[c]** | **0.004[c]** | ns | ns | ns |

[a]*p*. probability relative to Vehicle control
[b]ns, not significant
' Mean within normal range

**Table 27A. Clinical Chemistry Parameters (descriptive statistics) (Part 2) by Treatment Group and Gender**

| Treatment Group | Gender | | CREATININE mg/dL | CHOLESTEROL mg/dL | GLUCOSE mg/dL | CALCIUM mg/dL | PHOSPHORUS mg/dL | CHLORIDE mEq/L | POTASSIUM mEq/L | SODIUM mEq/L | A/G RA-TIO |
|---|---|---|---|---|---|---|---|---|---|---|---|
| Vehicle | M | n | 5 | 5 | 5 | 5 | 5 | 5 | 5 | 5 | 5 |
| | | Mean | 036 | 60.00 | 222.00 | 12.12 | 10.82 | 98.60 | 630 | 145.40 | 1.22 |
| | | Std Dev | 0 05 | 4.06 | 47.00 | 0.31 | 0.51 | 1 52 | 0 29 | 1.14 | 0.04 |
| | F | n | 5 | 5 | 5 | 5 | 5 | 5 | 5 | 5 | 5 |
| | | Mean | 0.42 | 67.40 | 198.20 | 11.66 | 9.06 | 99.80 | 5.92 | 145.60 | 118 |
| | | Std Dev | 004 | 11.48 | 13 03 | 0.34 | 0.61 | 0.45 | 0.50 | 0.55 | 0.08 |
| Anatabine 01 mg/kg | M | n | 5 | 5 | 5 | 5 | 5 | 5 | 5 | 5 | 5 |
| | | Mean | 0.36 | 67.20 | 219.00 | 12.00 | 10.82 | 98.00 | 6.40 | 146.20 | 1.14 |
| | | Std Dev | 0.05 | 8.35 | 23.73 | 0.39 | 0.54 | 1.00 | 0.40 | 1.48 | 0.09 |
| | | *p*[a] | ns[b] | ns | ns | ns | ns | ns | ns | ns | ns |
| | F | n | 5 | 5 | 5 | 5 | 5 | 5 | 5 | 5 | 5 |
| | | Mean | 0.44 | 76.40 | 207.60 | 11.72 | 8.08 | 100.20 | 5.82 | 146.80 | 1.22 |
| | | Std Dev | 0.05 | 11.63 | 24.11 | 0.58 | 0.48 | 2.17 | 0.65 | 1.30 | 0.04 |
| | | *p* | ns | ns | ns | ns | ns | ns | ns | ns | ns |
| Anatabine 0.75 mg/kg | M | | 5 | 5 | 5 | 5 | 5 | 5 | 5 | 5 | 5 |
| | | Mean | 0.36 | 62.80 | 202.00 | 12.04 | 10.92 | 98.60 | 6.12 | 147.80 | 1.20 |
| | | Std Dev | 0.05 | 6.38 | 25.25 | 0.38 | 0.85 | 1.14 | 0.45 | 1.48 | 000 |
| | | *p* | ns | ns | ns | ns | ns | ns | ns | **0.021[c]** | ns |
| | F | n | 5 | 5 | 5 | 5 | 5 | 5 | 5 | 5 | 5 |
| | | Mean | 0.44 | 69.80 | 220.80 | 11.68 | 8.64 | 98.00 | 5.88 | 145.00 | 1.24 |
| | | Std Dev | 0.05 | 9.88 | 21.73 | 0.29 | 1.22 | 2.00 | 0.30 | 1.22 | 0.05 |
| | | *p* | ns | ns | ns | ns | ns | ns | ns | ns | ns |

**Table 27B. Clinical Chemistry Parameters (descriptive statistics) (Part 2) by Treatment Group and Gender - cont'd**

| Treatment Group | Gender | | CREATININE mg/dL | CHOLESTEROL mg/dL | GLUCOSE mg/dL | CALCIUM mg/dL | PHOSPHORUS mg/dL | CHLORIDE mEq/L | POTASSIUM mEq/L | SODIUM mEq/L | A/G RA-TIO |
|---|---|---|---|---|---|---|---|---|---|---|---|
| Anatabine 1.5 mg/kg | M | n | 5 | 5 | 5 | 5 | 5 | 5 | 5 | 5 | 5 |
| | | Mean | 0.36 | 62.40 | 205.60 | 11.88 | 11.00 | 99.80 | 6.02 | 147.80 | 1.24 |
| | | Std Dev | 0 05 | 439 | 6.11 | 0 51 | 0 83 | 0.84 | 077 | 1 30 | 005 |
| | | *p* | ns | ns | ns | ns | ns | ns | ns | **0.015[c]** | ns |
| | F | | 5 | 5 | 5 | 5 | 5 | 5 | 5 | 5 | 5 |
| | | Mean | 0 40 | 70 00 | 188 40 | 11.36 | 8.24 | 100.80 | 5.74 | 147.20 | 1.24 |
| | | Std Dev | 0.00 | 9.92 | 9.07 | 0.35 | 0.59 | 1.92 | 0 59 | 0.84 | 0.05 |
| | | *p* | ns | ns | ns | ns | ns | ns | ns | **0.007[c]** | ns |
| Nicotine 0 75 mg/kg | M | n | 4 | 4 | 4 | 4 | 4 | 4 | 4 | 4 | 4 |
| | | Mean | 0.35 | 61.00 | 201.00 | 11.05 | 10,38 | 9975 | 6.20 | 146-75 | 105 |
| | | Std Dev | 0.06 | 8.49 | 10 42 | 0.06 | 0 58 | 0.96 | 0 50 | 0.96 | 0.06 |
| | | *p* | ns | ns | ns | **<0.001[c]** | ns | ns | ns | ns | **0.002[c]** |
| | F | n | 3 | 3 | 3 | 3 | 3 | 3 | 3 | 3 | 3 |
| | | Mean | 0.43 | 65.00 | 238.67 | 11.03 | 8.37 | 98.67 | 6.27 | 145.00 | 1.3 |
| | | Sit Dev | 0.06 | 14.93 | 53.72 | 0 21 | 1.2 | 1.53 | 0.50 | 1 73 | 0.06 |
| | | *p* | ns | ns | ns | ns | ns | ns | ns | ns | **0.038'** |

[a]*p*. probability relative to Vehicle control
[b] ns, not significant
[c] Mean within normal range

Table 28A. Dosing Calculations and Body Weights, days 1-5 (anatabine)

| Group | Rat | M/F | B.W. (g) | Dose volume (mL) | time of dosing | day 1 B. W. (g) | day 2 B. W. (g) | day 3 B. W. (g) | day 4 B. W. (g) | day 5 B. W. (g) |
|---|---|---|---|---|---|---|---|---|---|---|
| **A-1** | 1 | M | 250 | 1.25 | 10:39 | 258.0 | 270.0 | 280.3 | 296.0 | 306.6 |
| vehicle | 2 | M | 228 | 1.15 | 10:41 | 238.0 | 251.0 | 257.8 | 273.7 | 286.0 |
| 5 mL/kg | 3 | M | 235 | 1.18 | 10:43 | 241.0 | 250.0 | 260.2 | 278.0 | 287.0 |
| **A-2** | 4 | M | 224 | 1.13 | 10:44 | 230.0 | 239.0 | 247.6 | 262.6 | 267.0 |
| | 5 | M | 236 | 1.18 | 10:45 | 241.0 | 253.1 | 261.7 | 281.0 | 289.0 |
| **A-3** | 6 | F | 207 | 1.03 | 10:49 | 210.2 | 220.5 | 218.1 | 224.2 | 227.0 |
| | 7 | F | 221 | 1.1 | 10:50 | 221.9 | 222.0 | 225.1 | 229.2 | 235.7 |
| | 8 | F | 209 | 1.05 | 10:51 | 210.0 | 214.0 | 216.9 | 219.5 | 220.0 |
| **A-4** | 9 | F | 201 | 1 | 10:51 | 200.0 | 200.1 | 205.0 | 216.7 | 212.0 |
| | 10 | F | 211 | 1.05 | 10:52 | 208.4 | 215.0 | 216.1 | 227.9 | 225.0 |
| **B-1** | 1 | M | 237 | 1.18 | 10:48 | 245.0 | 257.0 | 270.0 | 286.0 | 296.5 |
| Anatabine | 2 | M | 227 | 1.13 | 10:49 | 233.7 | 235.1 | 251.0 | 270.0 | 273.8 |
| 0.1 mg/kg | 3 | M | 230 | 1.15 | 10:50 | 235.0 | 231.0 | 252.0 | 271.2 | 278.5 |
| **B-2** | 4 | M | 243 | 1.23 | 10:52 | 243.5 | 253.5 | 259.4 | 280.5 | 288.3 |
| | 5 | M | 235 | 1.18 | 10:55 | 239.9 | 247.3 | 256.6 | 273.7 | 286.6 |
| **B-3** | 6 | F | 226 | 1.13 | 10:56 | 223.0 | 223.9 | 230.8 | 242.3 | 246.9 |
| | 7 | F | 212 | 1.05 | 10:57 | 211.1 | 215.1 | 213.2 | 222.4 | 226.4 |
| | 8 | F | 207 | 1.03 | 10:58 | 207.0 | 208.1 | 209.1 | 220.7 | 219.3 |
| **B-4** | 9 | F | 205 | 1.03 | 10:58 | 201.0 | 208.8 | 209.1 | 211.4 | 221.0 |
| | 10 | F | 215 | 1.08 | 10:59 | 210.9 | 212.8 | 219.3 | 224.7 | 229.5 |
| Group | Rat | M/F | B.W. (g) | Dose volume (mL) | time | day 1 B. W. (g) | day 2 B.W. (g) | day 3 B. W. (g) | day 4 B. W. (g) | day 4 B. W. (g) |
| **C-1** | 1 | M | 239 | 1.2 | 10:58 | 242.7 | 248.9 | 261.1 | 275.7 | 282.3 |
| Anatabine | 2 | M | 241 | 1.2 | 11:00 | 251.4 | 259.7 | 269.1 | 286.0 | 287.6 |
| 0.75 mg/kg | 3 | M | 229 | 1.15 | 11:02 | 231.9 | 240.0 | 252.0 | 268.0 | 269.9 |
| C-2 | 4 | M | 223 | 1.13 | 11:04 | 226.8 | 232.1 | 243.0 | 256.3 | 262.0 |
| | 5 | M | 240 | 1.2 | 11:06 | 239.8 | 247.2 | 256.1 | 271.2 | 275.7 |
| C-3 | 6 | F | 214 | 1.08 | 11:01 | 208.9 | 206.2 | 214.0 | 230.0 | 225.4 |
| | 7 | F | 206 | 1.03 | 11:02 | 207.7 | 210.0 | 214.0 | 214.7 | 219.3 |
| | 8 | F | 215 | 1.08 | 11:03 | 219.1 | 225.5 | 224.8 | 232.4 | 237.1 |
| C-4 | 9 | F | 207 | 1.03 | 11:05 | 196.7 | 210.0 | 213.6 | 222.1 | 220.8 |
| | 10 | F | 212 | 1.05 | 11:06 | 199.5 | 210.1 | 210.8 | 220.0 | 227.6 |
| **D-1** | 1 | M | 230 | 1.15 | 11:07 | 234.0 | 240.4 | 258.4 | 244.4 | 286.3 |
| Anatabine | 2 | M | 248 | 1.25 | 11:21 | 242.6 | 254.0 | 267.9 | 255.1 | 268.1 |
| 1.5 mg/kg | 3 | M | 228 | 1.15 | 11:23 | 231.0 | 240.3 | 252.5 | 247.3 | 269.8 |
| **D-2** | 4 | M | 239 | 1.2 | 11:26 | 245.3 | 257.0 | 272.5 | 290.2 | 295.8 |
| | 5 | M | 227 | 1.15 | 11:28 | 227.8 | 237.3 | 250.1 | 262.0 | 270.5 |

(continued)

| Group | Rat | M/F | B.W. (g) | Dose volume (mL) | time | day 1 B. W. (g) | day 2 B.W. (g) | day 3 B. W. (g) | day 4 B. W. (g) | day 4 B. W. (g) |
|---|---|---|---|---|---|---|---|---|---|---|
| **D-3** | 6 | F | 216 | 1.08 | 11:08 | 213.9 | 212.0 | 220.0 | 226.2 | 228.0 |
| | 7 | F | 206 | 1.03 | 11:09 | 204.0 | 206.2 | 210.3 | 216.8 | 217.5 |
| | 8 | F | 219 | 1.1 | 11:20 | 219.6 | 221.6 | 224.3 | 234.7 | 230.9 |
| **D-4** | 9 | F | 231 | 1.15 | 11:22 | 229.5 | 233.9 | 237.0 | 248.1 | 247.5 |
| | 10 | F | 206 | 1.03 | 11:23 | 206.1 | 208.6 | 211.4 | 221.8 | 218.9 |

Table 28B. Dosing Calculations and Body Weights, days 6-12 (anatabine)

| Group | Rat | M/F | day 6 B. W. (g) | day 7 B. W. (g) | day 8 B. W. (g) | day 9 B. W. (g) | day 10 B. W. (g) | day 11 B. w. (g) | day 12 B. W. (g) |
|---|---|---|---|---|---|---|---|---|---|
| **A-1** | 1 | M | 308 | 317.0 | 332.0 | 338.0 | 348.7 | 364.2 | 369.5 |
| vehicle | 2 | M | 283.7 | 300.0 | 310.0 | 315.0 | 327.2 | 342.6 | 342.6 |
| 5 mL/kg | 3 | M | 287.9 | 300.0 | 314.0 | 317.0 | 332.0 | 349.1 | 354.1 |
| A-2 | 4 | M | 268.8 | 273.5 | 286.0 | 286.9 | 296.1 | 312.7 | 316.7 |
| | 5 | M | 294.6 | 301.1 | 315.0 | 315.2 | 332.3 | 339.7 | 351.0 |
| A-3 | 6 | F | 228.2 | 231.7 | 235.5 | 237.4 | 246.7 | 253.8 | 248.9 |
| | 7 | F | 235.3 | 236.8 | 260.8 | 249.8 | 252.8 | 261.3 | 256.3 |
| | 8 | F | 221.9 | 225.9 | 226.4 | 230.1 | 236.4 | 248.0 | 240.9 |
| A-4 | 9 | F | 216 | 217.4 | 218.0 | 224.8 | 228.5 | 237.1 | 238.8 |
| | 10 | F | 232.4 | 232.5 | 233.0 | 238.3 | 241.9 | 256.7 | 256.9 |
| | | | | | | | | | |
| **B-1** | 1 | M | 301.8 | 318.2 | 327.0 | 336.8 | 346.4 | 365.7 | 359.9 |
| Anatabine | 2 | M | 278 | 293.8 | 302.0 | 303.4 | 312.9 | 331.0 | 320.0 |
| 0.1 mg/kg | 3 | M | 284.3 | 297.6 | 309.0 | 310.0 | 319.9 | 343.8 | 341.9 |
| B-2 | 4 | M | 288.3 | 301.2 | 310.0 | 318.7 | 322.3 | 346.9 | 347.3 |
| | 5 | M | 219.6 | 300.7 | 311.0 | 317.4 | 330.4 | 348.8 | 360.9 |
| B-3 | 6 | F | 241.1 | 246.5 | 252.0 | 255.1 | 261.9 | 274.8 | 277.2 |
| | 7 | F | 235.5 | 226.4 | 232.0 | 239.9 | 234.5 | 238.5 | 246.3 |
| | 8 | F | 220.1 | 221.7 | 227.0 | 224.1 | 226.0 | 233.9 | 242.0 |
| B-4 | 9 | F | 218.8 | 222.1 | 223.0 | 236.0 | 232.5 | 245.6 | 240.0 |
| | 10 | F | 222.9 | 232.1 | 235.0 | 232.0 | 233.7 | 245.4 | 241.6 |
| Group | Ral | M/F | B. W. (g) | B. W. (g) | B. W. (g) | B. W. (g) | B. W. | B. W. (g) | B. W. (g) |
| **C-1** | 1 | M | 282.4 | 294.5 | 300.0 | 307.6 | 317.2 | 334.6 | 327.6 |
| Anatabine | 2 | M | 287.6 | 302.9 | 310.6 | 319.8 | 331.9 | 339.8 | 325.4 |
| 0.75 mg/kg | 3 | M | 268.2 | 281.8 | 293.4 | 298.8 | 307.9 | 329.4 | 322.5 |
| C-2 | 4 | M | 268.2 | 274.1 | 284.2 | 296.2 | 303.8 | 316.1 | 324.8 |
| | 5 | M | 281.3 | 287.1 | 292.0 | 298.5 | 306.8 | 321.9 | 331.1 |
| C-3 | 6 | F | 224.4 | 227.4 | 232.0 | 237.3 | 239.3 | 250.9 | 249.7 |

(continued)

| Group | Ral | M/F | B. W. (g) | B. W. (g) | B. W. (g) | B. W. (g) | B. W. | B. W. (g) | B. W. (g) |
|---|---|---|---|---|---|---|---|---|---|
| | 7 | F | 217.8 | 223 | 220.0 | 226.3 | 232.2 | 239.1 | 233.5 |
| | 8 | F | 236.2 | 243.2 | 243.0 | 247.6 | 252.7 | 268.8 | 259.6 |
| C-4 | 9 | F | 214.7 | 222 | 224.6 | 229.1 | 230.9 | 240.9 | 241.6 |
| | 10 | F | 221.4 | 219.3 | 227.4 | 230.6 | 233.5 | 244.1 | 247.9 |
| | | | | | | | | | |
| **D-1** | I | M | 279.1 | 292 | 300.0 | 302.1 | 317.1 | 333.0 | 341.1 |
| Anatabine | 2 | M | 296.9 | 305.6 | 319.0 | 323.1 | 331.8 | 359.6 | 360.3 |
| 1.5 mg/kg | 3 | M | 280.4 | 288.2 | 298.4 | 303.8 | 318.3 | 335.4 | 327.9 |
| D-2 | 4 | M | 302.3 | 316.7 | 326.0 | 337.3 | 347.0 | 369.5 | 380.4 |
| | 5 | M | 274.8 | 283.5 | 297.0 | 301.9 | 310.0 | 332.6 | 336.3 |
| D-3 | 6 | F | 221.2 | 229 | 229.6 | 232.8 | 232.8 | 242.1 | 236.9 |
| | 7 | F | 204.6 | 208.1 | 216.4 | 218.7 | 229.0 | 233.0 | 227.4 |
| | 8 | F | 229.3 | 235.7 | 244.0 | 243.9 | 252.1 | 259.7 | 257.1 |
| D-4 | 9 | F | 246 | 249.9 | 256.7 | 258.3 | 260.9 | 266.9 | 276.3 |
| | 10 | F | 216.9 | 224 | 225.0 | 225.8 | 234.5 | 243.5 | 240.9 |

Table 28C. Dosing Calculations and Body Weights, days 13-14 (anatabine)

| Group | Rat | M/F | day 13 B. W. (g) | day 14 B. W. (g) |
|---|---|---|---|---|
| **A-1** vehicle 5 mL/kg | 1 | M | 379.1 | 389.0 |
| | 2 | M | 349 | 363.0 |
| | 3 | M | 356 | 377.0 |
| A-2 | 4 | M | 317 | 325.0 |
| | 5 | M | 356 | 371.9 |
| A-3 | 6 | F | 258 | 265.2 |
| | 7 | F | 249.8 | 268.2 |
| | 8 | F | 245.6 | 252.7 |
| A-4 | 9 | F | 235 | 245.8 |
| | 10 | F | 253 | 265.7 |
| **B-1** Anatabine 0.1 mg/kg | 1 | M | 367 | 391.0 |
| | 2 | M | 331 | 347.3 |
| | 3 | M | 349 | 368.8 |
| B-2 | 4 | M | 334.4 | 353.5 |
| | 5 | M | 360 | 373.8 |
| B-3 | 6 | F | 271.8 | 272.7 |
| | 7 | F | 244 | 248.5 |
| | 8 | F | 241 | 243.9 |
| B-4 | 9 | F | 242.1 | 255.6 |
| | 10 | F | 244 | 239.2 |

| Group | Rat | M/F | day 13 B. W. (g) | day 14 B. W. (g) |
|---|---|---|---|---|
| **C-1** Anatabine 0.75 mg/kg | 1 | M | 335.6 | 351.9 |
| | 2 | M | 338 | 357.9 |
| | 3 | M | 330 | 345.1 |
| C-2 | 4 | M | 326 | 339 |
| | 5 | M | 326 | 335.9 |
| C-3 | 6 | F | 248 | 251.5 |
| | 7 | F | 237.2 | 242.8 |
| | 8 | F | 268 | 274.1 |
| C-4 | 9 | F | 234 | 250.7 |
| | 10 | F | 243 | 250.6 |
| **D-1** Anatabine 1.5 mg/kg | 1 | M | 339.8 | 359.8 |
| | 2 | M | 370 | 389.7 |
| | 3 | M | 340 | 357.1 |
| D-2 | 4 | M | 379 | 399.9 |
| | 5 | M | 337 | 355 |
| D-3 | 6 | F | 242.4 | 249.9 |
| | 7 | F | 223 | 224.4 |
| | 8 | F | 257 | 264.7 |
| D-4 | 9 | F | 275.8 | 283.1 |
| | 10 | F | 240.3 | 248.8 |

Table 28D. Dosing Calculations and Body Weights, days 1-5 (nicotine)

| Group | Rat | M/F | B.W. (g) | Dose volume (mL) | time | day 1 B. W. (g) | day 2 B. W. (g) | day 3 B. W. (g) | day 4 B. W. (g) | day 5 B. W. (g) |
|---|---|---|---|---|---|---|---|---|---|---|
| **E-1** Nicotine 0.75 mg/kg | 1 | M | 218 | 1.1 | 8:45 | 228 | 239.1 | 245.1 | 263.4 | 269.1 |
| | 2 | M | 218 | 1.1 | 8:48 | 223 | 229.5 | 241.1 | 257.6 | 260.7 |
| | 3 | M | 207 | 1.03 | 8:50 | 219 | 229.1 | 238.4 | 252.9 | 258.7 |
| **E-2** | 4 | M | 214 | 1.08 | 8:51 | | | | | |
| | 5 | M | 221 | 1.1 | 8:53 | 227 | 234.9 | 243 | 260.5 | 272.1 |
| **E-3** | 6 | F | 187 | 0.93 | 8:55 | 191.6 | 190.5 | 198 | 207.8 | 212.3 |
| | 7 | F | 195 | 0.98 | 8:57 | | | | | |
| | 8 | F | 193 | 0.98 | 8:59 | | | | | |
| **E-4** | 9 | F | 207 | 1.05 | 9:01 | 206.1 | 213.4 | 217.1 | 223.8 | 226.3 |
| | 10 | F | 192 | 0.95 | 9:03 | 192.8 | 195.7 | 199.1 | 205.6 | 206.4 |

Table 28E. Dosing Calculations and Body Weights, days 6-12 (nicotine)

| Group | Rat | M/F | day 6 B. W. (g) | day 7 B. W. (g) | day 8 B. W. (g) | day 9 B. W. (g) | day 10 B. W. (g) | day 11 B. W. (g) | day 12 B. W. (g) |
|---|---|---|---|---|---|---|---|---|---|
| E-1 | 1 | M | 270 | 288 | 285.6 | 285.6 | 301 | 300.1 | 303 |
| Nicotine | 2 | M | 262.8 | 277.8 | 278 | 274.6 | 283.4 | 291.5 | 287.3 |
| 0.75 mg/kg | 3 | M | 259 | 279.9 | 284 | 274.9 | 286.1 | 292.2 | 290.7 |
| E-2 | 4 | M | [redacted] | | | | | | |
| | 5 | M | 275.4 | 295.8 | 293.7 | 303.5 | 314.2 | 317.7 | 329.5 |
| E-3 | 6 | F | 207 | 213 | 218 | 219.1 | 221.8 | 222.8 | 232.1 |
| | 7 | F | [redacted] | | | | | | |
| | 8 | F | [redacted] | | | | | | |
| E-4 | 9 | F | 228.9 | 240.1 | 237.4 | 236.4 | 240.1 | 244.5 | 248.9 |
| | 10 | F | 205.5 | 218.3 | 217.4 | 213.2 | 220.5 | 226 | 227.7 |

Table 28F. Dosing Calculations and Body Weights, days 13-14 (nicotine)

| Group | Rat | M/F | day 13 B. W. (g) | day 14 B. W. (g) |
|---|---|---|---|---|
| E-1 | 1 | M | 312.3 | 316.1 |
| Nicotine | 2 | M | 292.4 | 297.6 |
| 0.75 mg/kg | 3 | M | 299.4 | 305 |
| E-2 | 4 | M | [redacted] | |
| | 5 | M | 330 | 339.2 |
| E-3 | 6 | F | 232.6 | 230.4 |
| | 7 | F | [redacted] | |
| | 8 | F | [redacted] | |
| E-4 | 9 | F | 251.8 | 249 |
| | 10 | F | 227.3 | 229 |

Table 29A: **Average Daily Food Consumption per Rat (grams)**

| | | | | | Anatabine | | | | | |
|---|---|---|---|---|---|---|---|---|---|---|
| | | | **A- Vehicle** | | **B - 0.1 mg/kg** | | **C - 0.75 mg/kg** | | **D - 1.5 mg/kg** | |
| Date | cage# | # of rats | Male | Female | Male | Female | Male | Female | Male | Female |
| day 1 | 1 | 3 | 25.1 | 14.1 | 25.8 | 16.4 | 23.9 | 17.1 | 22.7 | 16.1 |
| | 2 | 2 | 21.1 | 15.4 | 28.0 | 14.4 | 19.2 | 17.1 | 21.4 | 13.6 |
| day 2 | 1 | 3 | 27.4 | 16.3 | 33.8 | 20.2 | 26.3 | 22.5 | 24.2 | 20.5 |
| | 2 | 2 | 24.5 | 20.4 | 29.1 | 20.4 | 29.5 | 11.3 | 30.2 | 19.8 |
| day 3 | 1 | 3 | 25.4 | 16.8 | 24.8 | 19.4 | 28.1 | 21.0 | 30.1 | 19.6 |
| | 2 | 2 | 25.3 | 20.3 | 22.0 | 18.2 | 26.3 | 17.4 | 31.2 | 20.9 |
| day 4 | 1 | 3 | 26.8 | 17.6 | 34.1 | 23.2 | 28.3 | 21.0 | 22.1 | 20.8 |
| | 2 | 2 | 26.4 | 20.2 | 28.4 | 18.0 | 29.8 | 18.7 | 30.0 | 21.6 |
| day 5 | 1 | 3 | 28.5 | 17.2 | 30.6 | 22.4 | 24.4 | 19.1 | 30.0 | 18.5 |
| | 2 | 2 | 28.3 | 22.4 | 28.6 | 21.4 | 27.0 | 21.7 | 29.0 | 18.5 |

(continued)

| | | | Anatabine | | | | | | | |
|---|---|---|---|---|---|---|---|---|---|---|
| | | | A- Vehicle | | B - 0.1 mg/kg | | C - 0.75 mg/kg | | D - 1.5 mg/kg | |
| Date | cage# | # of rats | Male | Female | Male | Female | Male | Female | Male | Female |
| day 6 | 1 | 3 | 26.7 | 20.6 | 33.6 | 26.8 | 25.6 | 18.8 | 33.6 | 14.2 |
| | 2 | 2 | 26.0 | 19.2 | 28.0 | 18.2 | 29.5 | 16,4 | 35.4 | 18.4 |
| day 7 | 1 | 3 | 30.1 | 21.6 | 35.7 | 21.7 | 29.0 | 24.0 | 34.1 | 21.2 |
| | 2 | 2 | 27.9 | 20.5 | 31.8 | 22.4 | 31.5 | 17.4 | 33.5 | 28.8 |
| day 8 | 1 | 3 | 33.2 | 23.7 | 33.2 | 26.5 | 31.3 | 23.2 | 31.7 | 22.7 |
| | 2 | 2 | 31.5 | 23.8 | 31.9 | 21.3 | 32.6 | 22.8 | 34.4 | 19.8 |
| day 9 | 1 | 3 | 29.1 | 24.1 | 35.5 | 21.1 | 29.1 | 23.7 | 33.9 | 21.7 |
| | 2 | 2 | 27.7 | 24.9 | 36.8 | 23.6 | 30.3 | 23.0 | 34.5 | 23.1 |
| day 10 | 1 | 3 | 30.4 | 22.3 | 34.7 | 20.4 | 28.7 | 20.0 | 33.5 | 21.7 |
| | 2 | 2 | 29.4 | 20.5 | 26.7 | 16.9 | 28.0 | 21.0 | 32.4 | 21.7 |
| day 11 | 1 | 3 | 28.0 | 19.9 | 29.1 | 19.0 | 24.4 | 20.5 | 28.3 | 15.6 |
| | 2 | 2 | 24.4 | 22.9 | 30.6 | 19.1 | 26.7 | 18.6 | 29.9 | 19.0 |
| day 12 | 1 | 3 | 27.4 | 20.3 | 31.6 | 27.9 | 21.7 | 16.6 | 30.1 | 18.4 |
| | 2 | 2 | 25.6 | 18.8 | 34.9 | 16.7 | 31.0 | 20.4 | 33.1 | 20.8 |
| day 13 | 1 | 3 | 33.6 | 22.4 | 39.9 | 29.6 | 33.9 | 22.0 | 36.8 | 19.6 |
| | 2 | 2 | 30.8 | 20.9 | 27.1 | 19.3 | 27.2 | 17.7 | 34.7 | 20.5 |
| day 14 | 1 | 3 | 28.7 | 20.9 | 32.3 | 18.8 | 27.8 | 17.6 | 29.9 | 17.1 |
| | 2 | 2 | 26.4 | 19.6 | 28.8 | 19.1 | 26.4 | 20.7 | 31.4 | 19.2 |

Table 29B: **Average Daily Food Consumption per Rat (grams)**

| | | E- Nicotine 0.75 mp/kg | | | |
|---|---|---|---|---|---|
| | | Males | | Female | |
| Date | cage# | # of rats | grams | # of rats | grams |
| day 1 | 1 | 3 | 20.8 | I | 23.7 |
| | 2 | I | 19.3 | 2 | 14.8 |
| day 2 | 1 | 3 | 23.5 | 1 | 24.9 |
| | 2 | I | 26.2 | 2 | 18.4 |
| day 3 | 1 | 3 | 26.6 | I | 22.2 |
| | 2 | I | 26.2 | 2 | 22.3 |
| day 4 | 1 | 3 | 26.2 | I | 19.4 |
| | 2 | I | 23.7 | 2 | 15.8 |
| day 5 | 1 | 3 | 25.1 | 1 | 18.7 |
| | 2 | I | 21.9 | 2 | 16.2 |
| day 6 | 1 | 3 | 23.8 | 1 | 20.0 |
| | 2 | I | 25.9 | 2 | 17.2 |

(continued)

| | | E- Nicotine 0.75 mp/kg | | | |
|---|---|---|---|---|---|
| | | Males | | Female | |
| Date | cage# | # of rats | grams | # of rats | grams |
| day 7 | 1 | 3 | 26.5 | 1 | 19.4 |
| | 2 | I | 27.9 | 2 | 21.7 |
| day 8 | 1 | 3 | 24.0 | 1 | 20.1 |
| | 2 | I | 28.2 | 2 | 20.6 |
| day 9 | 1 | 3 | 27.3 | 1 | 24.4 |
| | 2 | I | 26.1 | 2 | 21.5 |
| day 10 | 1 | 3 | 29.6 | I | 22.6 |
| | 2 | I | 30.9 | 2 | 20.4 |
| day 11 | 1 | 3 | 30.1 | 1 | 20.4 |
| | 2 | 1 | 29.1 | 2 | 20.3 |
| day 12 | 1 | 3 | 26.5 | I | 22.5 |
| | 2 | I | 23.4 | 2 | 21.7 |
| day 13 | 1 | 3 | 31.9 | I | 23.7 |
| | 2 | I | 28.4 | 2 | 21.2 |
| day 14 | 1 | 3 | 32.7 | I | 24.4 |
| | 2 | I | 26.4 | 2 | 19.6 |

Table 30. Hematology/Coagulation Parameters: Normal Ranges in the Rat

| | **Unit of Measure** | **Range** |
|---|---|---|
| WBC | x $10^3/\mu L$ | 3.0 - 17.0 |
| RBC | x $10^6/\mu L$ | 5 - 10 |
| HGB | g/μL | 11 - 19 |
| HCT | % | 35 - 57 |
| MCV | fL | 46 - 65 |
| MCH | pg | 18 - 23 |
| MCHC | g/dL | 31 - 40 |
| RETICULOCYTE COUNT | % | 0 - 25 |
| NEUTROPHIL SEG | % | 7 - 15 |
| LYMPHOCYTE | % | 77 - 89 |
| MONOCYTE | % | 0 - 5 |
| EOSINOPHIL | % | 0 - 4 |
| BASOPHIL | % | 0 - 1 |
| PLATELET COUNT | x $10^3/\mu L$ | 200 - 1500 |
| aPTT | sec | 13.2 - 22.4 |
| PT | sec | 11.0 - 15.6 |

Table 31. Hematology Parameters

| Animal ID | Sex | Anatabine Dose (mg/kg) | WBC | RBC | HGB | HCT | MCV | MCH | MCHC | RETICULOCYTE COUNT | PLATELET COUNT |
|---|---|---|---|---|---|---|---|---|---|---|---|
| | | | x $10^3$/μL | x $10^6$/μL | gm/dL | % | $U^3$ | UUG | % | % | x $10^3$/μL |
| A1 | M | 0 | 9.4 | 7.33 | 14.5 | 43.6 | 60 | 19.7 | 33.1 | 4.6 | 1494 |
| A2 | M | 0 | 11.8 | 7.22 | 15.1 | 44.9 | 62 | 20.9 | 33.6 | 6.1 | 1124 |
| A3 | M | 0 | 12.2 | 7.26 | 15.4 | 45.5 | 63 | 21.2 | 33.9 | 6.8 | 1088 |
| A4 | M | 0 | 9.9 | 7.69 | 15.8 | 47.8 | 62 | 20.5 | 33 | 5.9 | TNP[1] |
| A5 | M | 0 | 9.4 | 7.13 | 15.2 | 45.4 | 64 | 21.3 | 33.4 | 7.3 | 1443 |
| A6 | F | 0 | 5.8 | 8.4 | 18.3 | 51 | 61 | 21.7 | 35.8 | 5.6 | 1500 |
| A7 | F | 0 | 11.6 | 7.2 | 15.6 | 45 | 63 | 21.6 | 34.6 | 3.4 | 1057 |
| A8 | F | 0 | 12 | 7.35 | 15.2 | 44.3 | 60 | 20.6 | 34.2 | 6.3 | 1263 |
| A9 | F | 0 | 7 | 8.71 | 18.1 | 53.5 | 61 | 20.8 | 33.8 | 4.7 | 986 |
| A10 | F | 0 | 10.1 | 7.16 | 14.6 | 42.7 | 60 | 20.4 | 34.3 | 5.2 | 1186 |
| B1 | M | 0.1 | 23.5 | 8.05 | 17.6 | 52.1 | 65 | 21.9 | 33.8 | 6.1 | 701 |
| B2 | M | 0.1 | 15 | 7.38 | 15.5 | 45.7 | 62 | 21.1 | 34 | 5.4 | 1339 |
| B3 | M | 0.1 | 11.1 | 7.4 | 16.1 | 47.5 | 64 | 21.8 | 34 | 4.7 | 1601 |
| B4 | M | 0.1 | 13.2 | 7.42 | 15.2 | 45 | 61 | 20.5 | 33.8 | 5.6 | 1107 |
| B5 | M | 0.1 | 15.4 | 6.96 | 14.5 | 45.3 | 65 | 20.8 | 32 | 5.9 | 1495 |
| B6 | F | 0.1 | 4.7 | 7.7 | 15.8 | 46.7 | 61 | 20.5 | 33.8 | 4.8 | 1145 |
| B7 | F | 0.1 | 10.7 | 8.71 | 17 | 51.3 | 59 | 19.6 | 33.2 | 5.4 | TNP[1] |
| B8 | F | 0.1 | 8 | 7.1 | 14.8 | 44 | 62 | 20.9 | 33.6 | 6.1 | TNP[1] |
| B9 | F | 0.1 | 14 | 7.06 | 14.6 | 43.1 | 61 | 20.8 | 34 | 3.7 | 1029 |
| B10 | F | 0.1 | 9.2 | 8.22 | 15.8 | 46.8 | 57 | 19.2 | 33.7 | 2.4 | 818 |

| Animal ID | Sex | Anatabine Dose (mg/kg) | WBC | RBC | HGB | HCT | MCV | MCH | MCHC | RETICULOCYTE COUNT | PLATELET COUNT |
|---|---|---|---|---|---|---|---|---|---|---|---|
| | | | x $10^3/\mu L$ | x $10^6/\mu L$ | gm/dL | % | $U^3$ | UUG | % | % | x $10^3/\mu L$ |
| C1 | M | 0.75 | 9.9 | 8.23 | 16.8 | 49.6 | 60 | 20.3 | 33.8 | 3.6 | 1446 |
| C2 | M | 0.75 | 11.1 | 7.51 | 15.8 | 45.5 | 61 | 21 | 34.7 | 4.2 | 1478 |
| C3 | M | 0.75 | 22.1 | 7.63 | 16.8 | 48.4 | 63 | 22 | 34.7 | 4.1 | 1327 |
| C4 | M | 0.75 | 7.5 | 6.23 | 13.1 | 39.2 | 63 | 21.1 | 33.5 | 4.7 | TNP[1] |
| C5 | M | 0.75 | 9.9 | 7.73 | 15.4 | 46.3 | 60 | 19.9 | 33.2 | 5.3 | 1052 |
| C6 | F | 0.75 | 13.5 | 7.42 | 15 | 44.7 | 60 | 20.2 | 33.6 | 3.4 | 653 |
| C7 | F | 0.75 | 14.5 | 7.78 | 14.9 | 43.9 | 56 | 19.2 | 34.1 | 1.8 | 1475 |
| C8 | F | 0.75 | 10.9 | 7.11 | 15.1 | 44.2 | 62 | 21.2 | 34.2 | 3.2 | 1304 |
| C9 | F | 0.75 | 8 | 7.58 | 15.6 | 45.4 | 60 | 20.5 | 34.3 | 3.8 | 1540 |
| C10 | F | 0.75 | 4.8 | 7.81 | 16.2 | 47.6 | 61 | 20.8 | 34.1 | 4.1 | 1376 |
| D1 | M | 1.5 | 12.4 | 7.82 | 15.8 | 48 | 62 | 20.2 | 32.9 | 4.4 | TNP2 |
| D2 | M | 1.5 | 15.3 | 7.43 | 15.4 | 46.1 | 62 | 20.7 | 33.4 | 3.3 | 1350 |
| D3 | M | 1.5 | 8.4 | 7.49 | 16.1 | 48.5 | 65 | 21.5 | 33.1 | 3.6 | 1407 |
| D4 | M | 1.5 | 13 | 7.16 | 14.6 | 43.1 | 60 | 20.4 | 33.8 | 5.1 | 1274 |
| D5 | M | 1.5 | 16.4 | 7.37 | 15 | 44.8 | 61 | 20.4 | 33.6 | 4.8 | 859 |
| D6 | F | 1.5 | 6.4 | 8.17 | 15.8 | 47.1 | 58 | 19.3 | 33.6 | 3.2 | 1399 |
| D7 | F | 1.5 | 7 | 7.77 | 15.1 | 45.9 | 59 | 19.4 | 32.8 | 2.8 | 1346 |
| D8 | F | 1.5 | 12.1 | 7.65 | 14.8 | 44.3 | 58 | 19.3 | 33.4 | 4.6 | 1312 |
| D9 | F | 1.5 | 10.9 | 8.01 | 15.9 | 48.2 | 60 | 19.8 | 32.9 | 3.4 | 228 |
| D10 | F | 1.5 | 9.5 | 7.05 | 14.2 | 41.4 | 59 | 20.2 | 34.4 | 2.1 | 1352 |

Hematology Parameters

| Animal ID | Sex | Anatabine Dose (mg/kg) | WBC | RBC | HGB | HCT | MCV | MCH | MCHC | RETICULOCYTE COUNT | PLATELET COUNT |
|---|---|---|---|---|---|---|---|---|---|---|---|
| | | | Hematology Parameters | | | | | | | | |
| | | | x $10^3$/μL | x $10^6$/μL | gm/dL | % | $U^3$ | UUG | % | % | x $10^3$/μL |
| E1 | M | 0.75[1] | 9.2 | 7.43 | 15.2 | 45.6 | 61 | 20.5 | 33.4 | 4.3 | 1458 |
| E2 | M | 0.75[1] | 13.6 | 7.61 | 15.9 | 46.2 | 61 | 20.8 | 34.4 | 4.8 | 1343 |
| E3 | M | 0.75[1] | 8.2 | 8.26 | 16.8 | 50 | 61 | 20.4 | 33.7 | 5.6 | 1366 |
| E5 | M | 0.75[1] | 14.6 | 7.04 | 15.3 | 44.5 | 63 | 21.7 | 34.3 | 6.1 | 1256 |
| E6 | F | 0.75[1] | 11.2 | 8.25 | 16.2 | 47.7 | 58 | 19.6 | 34 | 5.8 | 1539 |
| E9 | F | 0.75[1] | 6.7 | 7.41 | 15.2 | 44.2 | 60 | 20.5 | 34.4 | 4.9 | 1187 |
| E10 | F | 0.75[1] | 12.7 | 8.36 | 16.5 | 48.3 | 58 | 19.7 | 34.1 | 4.5 | TNP[2] |

1. Dose is 0.75 mg/kg nicotine
2. TNP: Test not performed due to clot in EDTA tube

| | | Anatabine | Hematology Parameters | | | | | | | |
|---|---|---|---|---|---|---|---|---|---|---|
| | | | NEUTROPHIL SEG | LYMPHOCYTE | MONOCYTE | EOSINOPHIL | BASOPHIL | PLATELET EST | POLYCHROMASIA | ANISOCYTOSIS |
| Animal ID | Sex | Dose (mg/kg) | % | % | % | % | % | | | |
| A1 | M | 0 | 9 | 90 | 1 | 0 | 0 | ADEQUATE | SLIGHT | SLIGHT |
| A2 | M | 0 | 9 | 87 | 4 | 0 | 0 | ADEQUATE | SLIGHT | SLIGHT |
| A3 | M | 0 | 12 | 88 | 0 | 0 | 0 | ADEQUATE | SLIGHT | SLIGHT |
| A4 | M | 0 | 6 | 93 | 1 | 0 | 0 | DECREASED | SLIGHT | SLIGHT |
| A5 | M | 0 | 8 | 90 | 2 | 0 | 0 | ADEQUATE | SLIGHT | SLIGHT |
| A6 | F | 0 | 9 | 89 | 2 | 0 | 0 | INCREASED | SLIGHT | SLIGHT |
| A7 | F | 0 | 12 | 86 | 2 | 0 | 0 | ADEQUATE | SLIGHT | SLIGHT |
| A8 | F | 0 | 14 | 84 | 2 | 0 | 0 | ADEQUATE | DNR | DNR |
| A9 | F | 0 | 12 | 86 | 2 | 0 | 0 | ADEQUATE | SLIGHT | SLIGHT |
| A10 | F | 0 | 16 | 81 | 3 | 0 | 0 | ADEQUATE | SLIGHT | SLIGHT |
| B1 | M | 0 1 | 12 | 87 | 1 | 0 | 0 | ADEQUATE | SLIGHT | SLIGHT |
| B2 | M | 0 1 | 3 | 94 | 3 | 0 | 0 | ADEQUATE | SLIGHT | SLIGHT |
| B3 | M | 0 1 | 7 | 90 | 3 | 0 | 0 | INCREASED | SLIGHT | SLIGHT |
| B4 | M | 0 1 | 10 | 87 | 2 | 1 | 0 | ADEQUATE | SLIGHT | SLIGHT |
| B5 | M | 0 1 | 11 | 88 | 1 | 0 | 0 | ADEQUATE | SLIGHT | SLIGHT |
| B6 | F | 0 1 | 8 | 89 | 3 | 0 | 0 | ADEQUATE | SLIGHT | SLIGHT |
| B7 | F | 0 1 | 8 | 90 | 2 | 0 | 0 | ADEQUATE | SLIGHT | SLIGHT |
| B8 | F | 0 1 | 7 | 91 | 2 | 0 | 0 | DECREASED | SLIGHT | SLIGHT |
| B9 | F | 0 1 | 6 | 93 | 1 | 0 | 0 | ADEQUATE | SLIGHT | SLIGHT |
| B10 | F | 0 1 | 11 | 88 | 1 | 0 | 0 | ADEQUATE | SLIGHT | SLIGHT |

DNR Did not report - insufficient sample

| | | Anatabine | Hematology Parameters | | | | | | | |
|---|---|---|---|---|---|---|---|---|---|---|
| | | | NEUTROPHIL SEG | LYMPHOCYTE | MONOCYTE | EOSINOPHIL | BASOPHIL | PLATELET EST | POLYCHROMASIA | ANISOCYTOSIS |
| Animal ID | Sex | Dose (mg/kg) | % | % | % | % | % | | | |
| C1 | M | 0.75 | 13 | 86 | 1 | 0 | 0 | ADEQUATE | SLIGHT | SLIGHT |
| C2 | M | 0.75 | 10 | 89 | 1 | 0 | 0 | ADEQUATE | SLIGHT | SLIGHT |
| C3 | M | 0.75 | 6 | 92 | 2 | 0 | 0 | ADEQUATE | SLIGHT | SLIGHT |
| C4 | M | 0.75 | 10 | 89 | 1 | 0 | 0 | DECREASED | SLIGHT | SLIGHT |
| C5 | M | 0.75 | 10 | 88 | 2 | 0 | 0 | ADEQUATE | DNR | DNR |
| C6 | F | 0.75 | 6 | 93 | 1 | 0 | 0 | ADEQUATE | SLIGHT | SLIGHT |
| C7 | F | 0.75 | 12 | 87 | 1 | 0 | 0 | ADEQUATE | SLIGHT | SLIGHT |
| C8 | F | 0.75 | 9 | 91 | 0 | 0 | 0 | ADEQUATE | SLIGHT | SLIGHT |
| C9 | F | 0.75 | 9 | 89 | 2 | 0 | 0 | INCREASED | SLIGHT | SLIGHT |
| C10 | F | 0.75 | 7 | 93 | 0 | 0 | 0 | ADEQUATE | SLIGHT | SLIGHT |
| D1 | M | 1.5 | 20 | 77 | 3 | 0 | 0 | DECREASED | SLIGHT | SLIGHT |
| D2 | M | 1.5 | 6 | 93 | 1 | 0 | 0 | ADEQUATE | SLIGHT | SLIGHT |
| D3 | M | 1.5 | 12 | 86 | 2 | 0 | 0 | ADEQUATE | SLIGHT | SLIGHT |
| D4 | M | 1.5 | 25 | 74 | 1 | 0 | 0 | ADEQUATE | SLIGHT | SLIGHT |
| D5 | M | 1.5 | 10 | 89 | 1 | 0 | 0 | ADEQUATE | SLIGHT | SLIGHT |
| D6 | F | 1.5 | 11 | 88 | 1 | 0 | 0 | ADEQUATE | SLIGHT | SLIGHT |
| D7 | F | 1.5 | 13 | 87 | 0 | 0 | 0 | ADEQUATE | SLIGHT | SLIGHT |
| D8 | F | 1.5 | 20 | 80 | 0 | 0 | 0 | ADEQUATE | SLIGHT | SLIGHT |
| D9 | F | 1.5 | 10 | 89 | 1 | 0 | 0 | ADEQUATE | SLIGHT | SLIGHT |
| D10 | F | 1.5 | 9 | 89 | 2 | 0 | 0 | ADEQUATE | SLIGHT | SLIGHT |

DNR Did not report- insufficient sample

| | | Anatabine | Hematology Parameters | | | | | | | |
|---|---|---|---|---|---|---|---|---|---|---|
| | | | NEUTROPHIL SEG | LYMPHOCYTE | MONOCYTE | EOSINOPHIL | BASOPHIL | PLATELET EST | POLYCHROMASIA | ANISOCYTOSIS |
| Animal ID | Sex | Dose (mg/kg) | % | % | % | % | % | | | |
| E1 | M | 0.75[1] | 12 | 86 | 2 | 0 | 0 | ADEQUATE | SLIGHT | SLIGHT |
| E2 | M | 0.75[1] | 11 | 87 | 2 | 0 | 0 | ADEQUATE | SLIGHT | SLIGHT |
| E3 | M | 0.75[1] | 16 | 80 | 4 | 0 | 0 | ADEQUATE | SLIGHT | SLIGHT |
| E5 | M | 0.75[1] | 8 | 90 | 2 | 0 | 0 | ADEQUATE | SLIGHT | SLIGHT |
| E6 | F | 0.75[1] | 15 | 83 | 2 | 0 | 0 | INCREASED | SLIGHT | SLIGHT |
| E9 | F | 0.75[1] | 12 | 87 | 1 | 0 | 0 | ADEQUATE | SLIGHT | SLIGHT |
| E10 | F | 0.75[1] | 11 | 89 | 0 | 0 | 0 | DECREASED | SLIGHT | SLIGHT |

1. Dose is 0.75 mg/kg nicotine

**Table 32. Clinical Chemistry Parameters: Normal Ranges in the Rat**

| | Unit of Measure | Range |
|---|---|---|
| ALKALINE PHOSPHATASE | IU/L | 160 - 500 |
| ALT (SGPT) | IU/L | 35 - 80 |
| AST (SGOT) | IU/L | 33 - 53 |
| GLOBULIN | g/dL | 1.4 - 5.0 |
| ALBUMIN | g/dL | 2.9 - 5.9 |
| TOTAL PROTEIN | g/dL | 4.5 - 8.4 |
| TOTAL BILIRUBIN | mg/dL | 0 - 0.64 |
| BLOOD UREA NITROGEN (BUN) | mg/dL | 11 - 23 |
| CREATININE | mg/dL | 0.4 - 3.8 |
| CHOLESTEROL | mg/dL | 35 - 75 |
| GLUCOSE | mg/dL | 80 - 300 |
| CALCIUM | mg/dL | 9.1 - 15.1 |
| PHOSPHORUS | mg/dL | 4.7 - 16.0 |
| CHLORIDE | mEq/L | 79 - 111 |
| POTASSIUM | mEq/L | 3.6 - 9.2 |
| SODIUM | mEq/L | 142 - 154 |

Table 33. Clinical Chemistry Parameters

| | | | Clinical Chemistry Parameters | | | | | | | | |
|---|---|---|---|---|---|---|---|---|---|---|---|
| | | Anatabine | ALK PHOSPHATASE | ALT | AST | ALBUMIN | TOT PROTEIN | GLOBULIN | TOT BILIRUBIN | DIR BILIRUBIN | BUN |
| Animal ID | Sex | Dose (mg/kg) | IU/L | IU/L | IU/L | g/dL | g/dL | g/dL | mg/dL | mg/dL | mg/dL |
| A1 | M | 0 | 463 | 62 | 85 | 3.1 | 5.6 | 2.5 | 0 | 0 | 19 |
| A2 | M | 0 | 398 | 59 | 78 | 3.1 | 5.6 | 2.5 | 0 | 0 | 17 |
| A3 | M | 0 | 275 | 61 | 96 | 3.1 | 5.6 | 2.5 | 0 | 0 | 19 |
| A4 | M | 0 | 393 | 57 | 137 | 3.2 | 5.7 | 2.5 | 0 | 0 | 18 |
| A5 | M | 0 | 325 | 58 | 99 | 3.1 | 5.6 | 2.5 | 0 | 0 | 20 |
| A6 | F | 0 | 248 | 55 | 71 | 3.4 | 6.4 | 3 | 0 | 0 | 17 |
| A7 | F | 0 | 265 | 61 | 81 | 3.3 | 6.2 | 2.9 | 0 | 0 | 14 |
| A8 | F | 0 | 292 | 62 | 75 | 3.4 | 6.2 | 2.8 | 0 | 0 | 20 |
| A9 | F | 0 | 176 | 52 | 77 | 3.5 | 6.2 | 2.7 | 0 | 0 | 15 |
| A10 | F | 0 | 170 | 61 | 100 | 3.4 | 6.2 | 2.8 | 0 | 0 | 25 |
| | | | | | | | | | | | |
| B1 | M | 0.1 | 470 | 92 | 97 | 3.4 | 6.3 | 2.9 | 0 | 0 | 18 |
| B2 | M | 0.1 | 227 | 65 | 99 | 3.2 | 5.8 | 2.6 | 0 | 0 | 18 |
| B3 | M | 0.1 | 364 | 74 | 93 | 3.1 | 5.9 | 2.8 | 0 | 0 | 21 |
| B4 | M | 0.1 | 358 | 44 | 85 | 3.1 | 6.2 | 3.1 | 0 | 0 | 14 |
| B5 | M | 0.1 | 618 | 78 | 106 | 3.4 | 6.3 | 2.9 | 0 | 0 | 19 |
| B6 | F | 0.1 | 261 | 57 | 78 | 3.6 | 6.7 | 3.1 | 0 | 0 | 21 |
| B7 | F | 0.1 | 140 | 43 | 69 | 3.5 | 6.3 | 2.8 | 0 | 0 | 19 |
| B8 | F | 0.1 | 209 | 47 | 70 | 3.6 | 6.7 | 3.1 | 0 | 0 | 21 |
| B9 | F | 0.1 | 412 | 62 | 86 | 3.8 | 6.9 | 3.1 | 0 | 0 | 19 |
| B10 | F | 0.1 | 220 | 39 | 82 | 3.6 | 6.6 | 3 | 0 | 0 | 19 |
| | | | | | | | | | | | |
| | | | | | | | | | | | |

| Animal ID | Sex | Anatabine Dose (mg/kg) | Clinical Chemistry Parameters: ALK PHOSPHATASE IU/L | ALT IU/L | AST IU/L | ALBUMIN g/dL | TOT PROTEIN g/dL | GLOBULIN g/dL | TOT BILIRUBIN mg/dL | DIR BILIRUBIN mg/dL | BUN mg/dL |
|---|---|---|---|---|---|---|---|---|---|---|---|
| C1 | M | 0.75 | 479 | 75 | 103 | 3.4 | 6.2 | 2.8 | 0 | 0 | 15 |
| C2 | M | 0.75 | 297 | 57 | 66 | 3.1 | 5.7 | 2.6 | 0 | 0 | 18 |
| C3 | M | 0.75 | 325 | 65 | 97 | 3.2 | 5.9 | 2.7 | 0 | 0 | 12 |
| C4 | M | 0.75 | 337 | 50 | 90 | 3.3 | 6.1 | 2.8 | 0 | 0 | 17 |
| C5 | M | 0.75 | 416 | 87 | 92 | 3.4 | 6.3 | 2.9 | 0.1 | 0.1 | 21 |
| C6 | F | 0.75 | 123 | 63 | 95 | 3.5 | 6.3 | 2.8 | 0 | 0 | 15 |
| C7 | F | 0.75 | 142 | 49 | 69 | 3.6 | 6.3 | 2.7 | 0 | 0 | 16 |
| C8 | F | 0.75 | 235 | 55 | 76 | 3.4 | 6.2 | 2.8 | 0 | 0 | 15 |
| C9 | F | 0.75 | 130 | 49 | 54 | 3.6 | 6.6 | 3 | 0 | 0 | 16 |
| C10 | F | 0.75 | 160 | 57 | 72 | 3.5 | 6.5 | 3 | 0 | 0 | 15 |
| | | | | | | | | | | | |
| D1 | M | 1.5 | 389 | 107 | 144 | 3.4 | 6.2 | 2.8 | 0 | 0 | 18 |
| D2 | M | 1.5 | 644 | 61 | 83 | 3.2 | 5.7 | 2.5 | 0 | 0 | 17 |
| D3 | M | 1.5 | 304 | 65 | 91 | 3.5 | 6.3 | 2.8 | 0 | 0 | 18 |
| D4 | M | 1.5 | 357 | 58 | 77 | 3.2 | 5.8 | 2.6 | 0 | 0 | 18 |
| D5 | M | 1.5 | 363 | 61 | 90 | 3.3 | 6 | 2.7 | 0 | 0 | 15 |
| D6 | F | 1.5 | 189 | 47 | 78 | 3.7 | 6.6 | 2.9 | 0 | 0 | 18 |
| D7 | F | 1.5 | 218 | 53 | 82 | 3.5 | 6.5 | 3 | 0 | 0 | 17 |
| D8 | F | 1.5 | 181 | 43 | 70 | 3.5 | 6.5 | 3 | 0 | 0 | 15 |
| D9 | F | 1.5 | 272 | 58 | 88 | 3.4 | 6.3 | 2.9 | 0.1 | 0.1 | 18 |
| D10 | F | 1.5 | 110 | 45 | 65 | 3.3 | 5.9 | 2.6 | 0 | 0 | 13 |

| | | | Clinical Chemistry Parameters | | | | | | | | |
|---|---|---|---|---|---|---|---|---|---|---|---|
| | | Anatabine | ALK PHOSPHATASE | ALT | AST | ALBUMIN | TOT PROTEIN | GLOBULIN | TOT BILIRUBIN | DIR BILIRUBIN | BUN |
| Animal ID | Sex | Dose (mg/kg) | IU/L | IU/L | IU/L | g/dL | g/dL | g/dL | mg/dL | mg/dL | mg/dL |
| E1 | M | 0.75[1] | 241 | 54 | 89 | 3.1 | 6 | 2.9 | 0.1 | 0 | 15 |
| E2 | M | 0.75[1] | 257 | 59 | 81 | 3.1 | 6.2 | 3.1 | 0 | 0 | 18 |
| E3 | M | 0.75[1] | 313 | 45 | 72 | 3.1 | 5.8 | 2.7 | 0 | 0 | 19 |
| E5 | M | 0.75[1] | 353 | 65 | 87 | 3 | 6 | 3 | 0.1 | 0 | 18 |
| E6 | F | 0.75[1] | 274 | 97 | 133 | 3.3 | 6.4 | 3.1 | 0.1 | 0 | 20 |
| E9 | F | 0.75[1] | 138 | 54 | 75 | 3.3 | 6.6 | 3.3 | 0.1 | 0.1 | 17 |
| E10 | F | 0.75[1] | 154 | 54 | 101 | 3.3 | 6.5 | 3.2 | 0.1 | 0 | 20 |

1 Dose is 0.75 mg/kg nicotine

| | | | Clinical Chemistry Parameters | | | | | | | | |
|---|---|---|---|---|---|---|---|---|---|---|---|
| | | Anatabine | CREATININE | CHOLESTEROL | GLUCOSE | CALCIUM | PHOSPHORUS | CHLORIDE | POTASSIUM | SODIUM | A/G RATIO |
| Animal ID | Sex | Dose (mg/kg) | mg/dL | mg/dL | mg/dL | mg/dL | mg/dL | mEq/L | mEq/L | mEq/L | |
| A1 | M | 0 | 0.3 | 54 | 183 | 12.3 | 10.9 | 97 | 6.8 | 144 | 1.2 |
| A2 | M | 0 | 0.4 | 62 | 215 | 12.1 | 11 | 97 | 6.1 | 145 | 1.2 |
| A3 | M | 0 | 0.4 | 60 | 217 | 12.2 | 10.8 | 99 | 6.2 | 147 | 1.2 |
| A4 | M | 0 | 0.3 | 65 | 302 | 12.4 | 11.4 | 100 | 6.3 | 145 | 1.3 |
| A5 | M | 0 | 0.4 | 59 | 193 | 11.6 | 10 | 100 | 6.1 | 146 | 1.2 |
| A6 | F | 0 | 0.4 | 85 | 205 | 11.9 | 10 | 100 | 5.5 | 145 | 1.1 |
| A7 | F | 0 | 0.4 | 72 | 176 | 11.3 | 9 | 99 | 5.5 | 146 | 1.1 |
| A8 | F | 0 | 0.4 | 56 | 206 | 11.4 | 9.1 | 100 | 5.8 | 146 | 1.2 |
| A9 | F | 0 | 0.4 | 64 | 197 | 11.6 | 8.9 | 100 | 6.7 | 146 | 1.3 |
| A10 | F | 0 | 0.5 | 60 | 207 | 12.1 | 8.3 | 100 | 6.1 | 145 | 1.2 |
| B1 | M | 0.1 | 0.4 | 65 | 258 | 12.7 | 10.3 | 98 | 5.9 | 148 | 1.2 |
| B2 | M | 0.1 | 0.4 | 64 | 219 | 11.8 | 10.3 | 97 | 6.5 | 144 | 1.2 |
| B3 | M | 0.1 | 0.4 | 75 | 214 | 11.9 | 11.2 | 99 | 6.6 | 146 | 1.1 |
| B4 | M | 0.1 | 0.3 | 56 | 194 | 11.8 | 11.5 | 97 | 6.9 | 147 | 1 |
| B5 | M | 0.1 | 0.3 | 76 | 210 | 11.8 | 10.8 | 99 | 6.1 | 146 | 1.2 |
| B6 | F | 0.1 | 0.4 | 90 | 224 | 12 | 8.5 | 99 | 5.4 | 147 | 1.2 |
| B7 | F | 0.1 | 0.5 | 74 | 170 | 11.4 | 8.2 | 102 | 6.3 | 148 | 1.3 |
| B8 | F | 0.1 | 0.5 | 65 | 227 | 11.9 | 7.4 | 102 | 5.2 | 148 | 1.2 |
| B9 | F | 0.1 | 0.4 | 66 | 220 | 12.4 | 8.5 | 97 | 5.5 | 145 | 1.2 |
| B10 | F | 0.1 | 0.4 | 87 | 197 | 10.9 | 7.8 | 101 | 6.7 | 146 | 1.2 |

| Animal ID | Sex | Anatabine Dose (mg/kg) | CREATININE | CHOLESTEROL | GLUCOSE | CALCIUM | PHOSPHORUS | CHLORIDE | POTASSIUM | SODIUM | A/G RATIO |
|---|---|---|---|---|---|---|---|---|---|---|---|
| | | | Clinical Chemistry Parameters | | | | | | | | |
| | | | mg/dL | mg/dL | mg/dL | mg/dL | mg/dL | mEq/L | mEq/L | mEq/L | |
| C1 | M | 0.75 | 0.4 | 69 | 211 | 12.3 | 11 | 98 | 6.3 | 148 | 1.2 |
| C2 | M | 0.75 | 0.4 | 60 | 222 | 12.2 | 10.7 | 99 | 6.3 | 146 | 1.2 |
| C3 | M | 0.75 | 0.3 | 54 | 219 | 11.8 | 10 | 100 | 5.4 | 147 | 1.2 |
| C4 | M | 0.75 | 0.3 | 62 | 160 | 11.5 | 10.6 | 99 | 6 | 150 | 1.2 |
| C5 | M | 0.75 | 0.4 | 69 | 198 | 12.4 | 12.3 | 97 | 6.6 | 148 | 1.2 |
| C6 | F | 0.75 | 0.5 | 66 | 237 | 11.8 | 9.7 | 97 | 5.5 | 145 | 1.3 |
| C7 | F | 0.75 | 0.4 | 64 | 247 | 11.3 | 7.7 | 96 | 5.9 | 144 | 1.3 |
| C8 | F | 0.75 | 0.4 | 63 | 192 | 11.6 | 9.6 | 99 | 6 | 145 | 1.2 |
| C9 | F | 0.75 | 0.4 | 69 | 211 | 12.1 | 9.2 | 97 | 5.7 | 144 | 1.2 |
| C10 | F | 0.75 | 0.5 | 87 | 217 | 11.6 | 7 | 101 | 6.3 | 147 | 1.2 |
| D1 | M | 1.5 | 0.4 | 57 | 209 | 12.2 | 10.9 | 99 | 7.2 | 146 | 1.2 |
| D2 | M | 1.5 | 0.4 | 68 | 204 | 11 | 9.6 | 100 | 5.4 | 149 | 1.3 |
| D3 | M | 1.5 | 0.4 | 64 | 207 | 12.2 | 11.6 | 99 | 6.3 | 148 | 1.3 |
| D4 | M | 1.5 | 0.3 | 64 | 196 | 12.1 | 11.6 | 100 | 5.3 | 149 | 1.2 |
| D5 | M | 1.5 | 0.3 | 59 | 212 | 11.9 | 11.3 | 101 | 5.9 | 147 | 1.2 |
| D6 | F | 1.5 | 0.4 | 68 | 196 | 11.8 | 8.4 | 102 | 6.1 | 148 | 1.3 |
| D7 | F | 1.5 | 0.4 | 78 | 173 | 11.6 | 8.5 | 100 | 6.3 | 147 | 1.2 |
| D8 | F | 1.5 | 0.4 | 81 | 192 | 11.3 | 7.2 | 101 | 5.2 | 148 | 1.2 |
| D9 | F | 1.5 | 0.4 | 67 | 193 | 11.2 | 8.6 | 98 | 5 | 147 | 1.2 |
| D10 | F | 1.5 | 0.4 | 56 | 188 | 10.9 | 8.5 | 103 | 6.1 | 146 | 1.3 |

| Animal ID | Sex | Anatabine Dose (mg/kg) | Clinical Chemistry Parameters: CREATININE mg/dL | CHOLESTEROL mg/dL | GLUCOSE mg/dL | CALCIUM mg/dL | PHOSPHORUS mg/dL | CHLORIDE mEq/L | POTASSIUM mEq/L | SODIUM mEq/L | A/G RATIO |
|---|---|---|---|---|---|---|---|---|---|---|---|
| E1 | M | 0.75[1] | 0.3 | 53 | 199 | 11.1 | 10.5 | 100 | 6.7 | 147 | 1.1 |
| E2 | M | 0.75[1] | 0.3 | 71 | 204 | 11.1 | 10.4 | 99 | 6 | 148 | 1 |
| E3 | M | 0.75[1] | 0.4 | 55 | 213 | 11 | 9.6 | 99 | 5.6 | 146 | 1.1 |
| E5 | M | 0.75[1] | 0.4 | 65 | 188 | 11 | 11 | 101 | 6.5 | 146 | 1 |
| E6 | F | 0.75[1] | 0.5 | 76 | 299 | 11.1 | 8.8 | 97 | 6.8 | 144 | 1.1 |
| E9 | F | 0.75[1] | 0.4 | 71 | 196 | 11.2 | 9.1 | 99 | 6.2 | 144 | 1 |
| E10 | F | 0.75[1] | 0.4 | 48 | 221 | 10.8 | 7.2 | 100 | 5.8 | 147 | 1 |

1. Dose is 0.75 mg/kg nicotine

Table 34. Coagulation Parameters

| Animal ID | Sex | Anilabine Dose (mg/kg) | Coagulation Parameters: ACTIVE PARTIAL THROMBOPLASTIN TIME | PROTHROMBIN TIME |
|---|---|---|---|---|
| | | | seconds | seconds |
| A1 | M | 0 | 33.5 | 13.3 |
| A2 | M | 0 | 31.7 | 13.3 |
| A3 | M | 0 | 29.6 | 12.5 |
| A4 | M | 0 | 30.4 | 12.3 |
| A5 | M | 0 | 34.9 | 13.1 |
| A6 | F | 0 | 36.7 | 12.8 |
| A7 | F | 0 | 36.2 | 12.7 |
| A8 | F | 0 | 40.6 | 13.2 |
| A9 | F | 0 | 34.5 | 12.5 |
| A10 | F | 0 | 29.2 | 12.5 |
| B1 | M | 0.1 | 34.9 | 13.6 |
| B2 | M | 0.1 | 36.4 | 13.4 |
| B3 | M | 0.1 | 29.6 | 12.5 |
| B4 | M | 0.1 | 28.9 | 12.3 |
| B5 | M | 0.1 | 32 | 12.1 |
| B6 | F | 0.1 | 31.1 | 12.3 |
| B7 | F | 0.1 | 34.2 | 11.3 |
| B8 | F | 0.1 | 26 | 12.2 |
| B9 | F | 0.1 | 26.7 | 12.3 |
| B10 | F | 0.1 | 28.9 | 12.3 |
| C1 | M | 0.75 | 39.7 | 12.4 |
| C2 | M | 0.75 | 43.9 | 12.1 |
| C3 | M | 0.75 | 27.9 | 12.1 |
| C4 | M | 0.75 | 50.4 | 17.9 |
| C5 | M | 0.75 | 31.7 | 12.1 |
| C6 | F | 0.75 | 31.4 | 11.4 |
| C7 | F | 0.75 | 32.5 | 12.1 |
| C8 | F | 0.75 | 31.5 | 11.6 |
| C9 | F | 0.75 | 32.7 | 11.4 |
| C10 | F | 0.75 | 38.2 | 23.8 |
| D1 | M | 1.5 | 34.1 | 13.8 |
| D2 | M | 1.5 | 33.8 | 13.1 |
| D3 | M | 1.5 | 28.3 | 12.9 |
| D4 | M | 1.5 | 27.9 | 12.9 |
| D5 | M | 1.5 | 29.1 | 13.5 |
| D6 | F | 1.5 | 37.2 | 13.2 |
| D7 | F | 1.5 | 31.7 | 13.2 |
| D8 | F | 1.5 | 29.3 | 13 |
| D9 | F | 1.5 | 32.3 | 13.6 |
| D10 | F | 1.5 | 32 | 13.3 |

(continued)

| Animal ID | Sex | Anatabine Dose (mg/kg) | ACTIVE PARTIAL THROMBOPLASTIN TIME | PROTHROMBIN TIME |
|---|---|---|---|---|
| | | | seconds | seconds |
| E1 | M | 0.75' | 15 | 13.8 |
| E2 | M | 0.75' | 16.3 | 13.2 |
| E3 | M | 0.75' | 17.6 | 14.2 |
| E5 | M | 0.75' | 16.2 | 13 |
| E6 | F | 0.75' | 18.7 | 13.9 |
| E9 | F | 0.75' | 15 | 14.2 |
| E10 | F | 0.75' | UNABLE TO OBTAIN RESULTS DUE TO FIBRIN CLOTS | UNABLE TO OBTAIN RESULTS DUE TO FIBRIN CLOTS |

I Dose is 0 75 mg/kg nicotine

Table 35. Urinalysis Results

| Animal ID | Sex | Anatabine Dose (mg/kg) | Urinalysis: VOLUME | COLOR | CLARITY | SPEC GRAVITY | pH | PROTEIN | GLUCOSE | KETONES | UROBILINOGEN | BILIRUBIN | BLOOD |
|---|---|---|---|---|---|---|---|---|---|---|---|---|---|
| A1 | M | 0 | <0.5 mL | YELLOW | HAZY | 1.046 | 7 | TRACE | NEGATIVE | 1+ | NORMAL | NEGATIVE | NEGATIVE |
| A2 | M | 0 | <0.5 mL | YELLOW | HAZY | 1.046 | 7 | TRACE | NEGATIVE | 1+ | NORMAL | NEGATIVE | NEGATIVE |
| A3 | M | 0 | <0.5 mL | YELLOW | HAZY | 1.046 | 7.5 | TRACE | NEGATIVE | 1+ | NORMAL | 1+ | NEGATIVE |
| A4 | M | 0 | <0.5 mL | YELLOW | HAZY | 1.031 | 7.5 | TRACE | NEGATIVE | NEGATIVE | NORMAL | NEGATIVE | 2+ |
| A5 | M | 0 | <0.5 mL | YELLOW | HAZY | 1.026 | 7.5 | TRACE | NEGATIVE | NEGATIVE | NORMAL | NEGATIVE | TRACE |
| A6 | F | 0 | <0.5 mL | YELLOW | HAZY | 1.034 | 6.5 | TRACE | NEGATIVE | NEGATIVE | NORMAL | NEGATIVE | TRACE |
| A7 | F | 0 | <0.5 mL | YELLOW | HAZY | 1.034 | 7 | TRACE | NEGATIVE | NEGATIVE | NORMAL | NEGATIVE | TRACE |
| A8 | F | 0 | <0.5 mL | YELLOW | HAZY | 1.024 | 8.5 | A | NEGATIVE | NEGATIVE | NORMAL | NEGATIVE | NEGATIVE |
| A9 | F | 0 | <0.5 mL | YELLOW | HAZY | 1.021 | 8 | 1+ | NEGATIVE | NEGATIVE | NORMAL | NEGATIVE | NEGATIVE |
| A10 | F | 0 | <0.25 mL | YELLOW | HAZY | 1.047 | 7 | A | NEGATIVE | NEGATIVE | NORMAL | NEGATIVE | TRACE |
| B1 | M | 0.1 | <0.5 mL | YELLOW | HAZY | 1.027 | 8.5 | TRACE | NEGATIVE | 1+ | NORMAL | NEGATIVE | TRACE |
| B2 | M | 0.1 | A | DNR | DNR | DNR | DNR | DNR | DNR | DNR | DNR | DNR | DNR |
| B3 | M | 0.1 | <0.5 mL | YELLOW | HAZY | 1.039 | 7.5 | TRACE | NEGATIVE | NEGATIVE | NORMAL | NEGATIVE | TRACE |
| B4 | M | 0.1 | <0.5 mL | YELLOW | HAZY | 1.027 | 7 | NEGATIVE | NEGATIVE | 1+ | NORMAL | NEGATIVE | NEGATIVE |
| B5 | M | 0.1 | <0.5 mL | YELLOW | HAZY | 1.048 | 7.5 | TRACE | NEGATIVE | 1+ | NORMAL | NEGATIVE | 1+ |
| B6 | F | 0.1 | <0.5 mL | YELLOW | HAZY | 1.039 | 7.5 | A | NEGATIVE | NEGATIVE | NORMAL | NEGATIVE | NEGATIVE |
| B7 | F | 0.1 | <0.5 mL | YELLOW | HAZY | 1.036 | 7.5 | A | NEGATIVE | 1+ | NORMAL | NEGATIVE | TRACE |
| B8 | F | 0.1 | <0.5 mL | YELLO | HAZY | 1.045 | 7 | NEGATIV | NEGATIV | 1+ | NORMAL | 1+ | NEGATIV |

| | | | | | | | | | | | | | | |
|---|---|---|---|---|---|---|---|---|---|---|---|---|---|---|
| | | | | W | | | | E | E | | | | E |
| B9 | F | 0 1 | 0 5 | YELLO W | HAZY | 1 017 | 8 | NEGATIV E | NEGATIV E | NEGATIV E | NORMAL | NEGATIVE | NEGATIV E |
| B10 | F | 0 1 | A | DNR | DNR | DNR | DN R | DNR | DNR | DNR | DNR | DNR | DNR |

**DNR - did not report - insufficient sample**

A. Sample quantity was not sufficient for complete testing

| Animal ID | Sex | Anatabine Dose (mg/kg) | Urinalysis | | | | | | | | | | |
|---|---|---|---|---|---|---|---|---|---|---|---|---|---|
| | | | VOLUME | COLOR | CLARITY | SPEC GRAVITY | pH | PROTEIN | GLUCOSE | KETONES | UROBILINOGEN | BILIRUBIN | BLOOD |
| C1 | M | 0 75 | No Urine Sample Submitted | | | | | | | | | | |
| C2 | M | 0 75 | <0 5 mL | YELLOW | HAZY | 1 05 | 7 | TRACE | NEGATIVE | 1+ | NORMAL | NEGATIVE | TRACE |
| C3 | M | 0 75 | <0 5 mL | YELLOW | HAZY | 1 051 | 7 | TRACE | NEGATIVE | 1+ | NORMAL | NEGATIVE | NEGATIVE |
| C4 | M | 0 75 | <0 5 mL | YELLOW | HAZY | 1 043 | 8 | TRACE | NEGATIVE | NEGATIVE | NORMAL | 1+ | NEGATIVE |
| C5 | M | 0 75 | <0 5 mL | YELLOW | HAZY | 1 049 | 7 | TRACE | NEGATIVE | 1+ | NORMAL | NEGATIVE | NEGATIVE |
| C6 | F | 0 75 | <0 5 mL | YELLOW | HAZY | 1 016 | 7 5 | A | NEGATIVE | NEGATIVE | NORMAL | NEGATIVE | 3+ |
| C7 | F | 0 75 | <0 5 mL | YELLOW | HAZY | 1 039 | 6 | A | NEGATIVE | NEGATIVE | NORMAL | NEGATIVE | TRACE |
| C8 | F | 0 75 | <0 5 mL | YELLOW | HAZY | 1 036 | 8 | A | NEGATIVE | 1+ | NORMAL | NEGATIVE | NEGATIVE |
| C9 | F | 0 75 | <0 25 mL | YELLOW | HAZY | A | DNR | DNR | NEGATIVE | 1+ | DNR | NEGATIVE | DNR |
| C10 | F | 0 75 | <0 5 mL | YELLOW | HAZY | 1 014 | 8 | 1+ | NEGATIVE | NEGATIVE | NORMAL | NEGATIVE | 2+ |
| D1 | M | 1 5 | <0 25 mL | YELLOW | HAZY | 1 031 | 8 | A | NEGATIVE | 1+ | NORMAL | NEGATIVE | 2+ |
| D2 | M | 1 5 | <0 5 mL | STRAW | CLOUDY | 1 034 | 7 5 | A | NEGATIVE | 1+ | NORMAL | NEGATIVE | 2+ |
| D3 | M | 1 5 | <0 25 mL | YELLOW | HAZY | 1 034 | 7 5 | A | NEGATIVE | 1+ | NORMAL | NEGATIVE | 2+ |
| D4 | M | 1 5 | <0 5 mL | YELLOW | HAZY | 1 045 | 7 5 | 2+ | NEGATIVE | 1+ | NORMAL | NEGATIVE | 2+ |
| D5 | M | 1 5 | <0 5 mL | YELLOW | HAZY | 1 047 | 8 5 | TRACE | NEGATIVE | 1+ | NORMAL | NEGATIVE | NEGATIVE |
| D6 | F | 1 5 | <0 5 mL | YELLOW | HAZY | 1 039 | 8 | 1+ | NEGATIVE | NEGATIVE | NORMAL | NEGATIVE | TRACE |
| D7 | F | 1 5 | <0 5 mL | YELLOW | HAZY | 1 038 | 8 5 | NEGATIVE | NEGATIVE | 1+ | NORMAL | NEGATIVE | NEGATIVE |
| D8 | F | 1 5 | <0 5 mL | YELLO | HAZY | 1 039 | 7 | NEGATIV | NEGATIV | NEGATIV | NORMAL | NEGATIVE | NEGATIV |

| | | | | | | | |
|---|---|---|---|---|---|---|---|
| | | | W | E | E | E | E |
| D9 | F | 1.5 | | No Urine Sample Submitted | | | |
| D10 | F | 1.5 | | No Urine Sample Submitted | | | |

**DNR - did not report - insufficient sample**

A Sample quantity was not sufficient for complete testing

| Animal ID | Sex | Anatabine Dose (mg/kg) | Urinalysis VOLUME | COLOR | CLARITY | SPEC GRAVITY | pH | PROTEIN | GLUCOSE | KETONES | UROBILINOGEN | BILIRUBIN | BLOOD |
|---|---|---|---|---|---|---|---|---|---|---|---|---|---|
| E1 | M | 0.75[1] | <0.5 mL | YELLOW | HAZY | 1.054 | 6 | NEGATIVE | NEGATIVE | NEGATIVE | NORMAL | NEGATIVE | NEGATIVE |
| E2 | M | 0.75[1] | <0.5 mL | YELLOW | HAZY | 1.058 | 6.5 | TRACE | NEGATIVE | NEGATIVE | NORMAL | NEGATIVE | 1+ |
| E3 | M | 0.75[1] | | | | | | No Urine Sample Submitted | | | | | |
| E5 | M | 0.75[1] | <0.5 mL | YELLOW | HAZY | 1.046 | 7 | NEGATIVE | NEGATIVE | NEGATIVE | NORMAL | NEGATIVE | NEGATIVE |
| E6 | F | 0.75[1] | 10UL | YELLOW | HAZY | 1.047 | 6.5 | NEGATIVE | NEGATIVE | NEGATIVE | NORMAL | NEGATIVE | 2+ |
| E9 | F | 0.75[1] | <0.5 mL | YELLOW | HAZY | 1.052 | 7.5 | NEGATIVE | NEGATIVE | NEGATIVE | NORMAL | NEGATIVE | NEGATIVE |
| E10 | F | 0.75[1] | | | | | | No Urine Sample Submitted | | | | | |

1. Dose is 0.75 mg/kg nicotine

A. Sample quantity was not sufficient for complete testing. DNR did not report due to insufficient sample

Table 36. Tissue Collection Weights (g)

## Group A: Vehicle (Males)

| 1 | | 2 | | 3 | | 4 | | 5 | |
|---|---|---|---|---|---|---|---|---|---|
| **Tissue** | **weights** | **Tissue** | **weights** | **Tissue** | **weights** | **Tissue** | **weights** | **Tissue** | **weights** |
| Thymus | 0.80 | Thymus | 0.80 | Thymus | 0.80 | Thymus | 0.70 | Thymus | 0.76 |
| Heart | 1.65 | Heart | 1.78 | Heart | 1.87 | Heart | 1.39 | Heart | 1.62 |
| Lungs | 2.02 | Lungs | 1.67 | Lungs | 2.16 | Lungs | 1.70 | Lungs | 1.97 |
| Thyroid/ para thyroid | Cass | Thyroid/ para thyroid | Cass | Thyroid/ para thyroid | Cass | Thyroid/ para thyroid | Cass | Thyroid/ para thyroid | Cass |
| Liver | 17.19 | Liver | 17.01 | Liver | 15.67 | Liver | 13.61 | Liver | 15.58 |
| Adrenals | Cass | Adrenals | Cass | Adrenals | Cass | Adrenals | Cass | Adrenals | Cass |
| Kidneys | 3.86 | Kidneys | 3.51 | Kidneys | 3.67 | Kidneys | 3.06 | Kidneys | 3.54 |
| Spleen | 1.12 | Spleen | 0.86 | Spleen | 1.41 | Spleen | 0.89 | Spleen | 0.88 |
| Small intestine | 0.53 | Small intestine | 1.4 | Small intestine | 0.47 | Small intestine | 0.41 | Small intestine | 0.41 |
| Prostate | 0.31 | Prostate | 0.56 | Prostate | 0.56 | Prostate | 0.57 | Prostate | 0.43 |
| Testes | 3.40 | Testes | 3.33 | Testes | 3.31 | Testes | 3.46 | Testes | 3.47 |
| Brain | 1.82 | Brain | 2.04 | Brain | 2.04 | Brain | 2.04 | Brain | 2.14 |
| Pituitary | Cass | Pituitary | cass | Pituitary | cass | Pituitary | Cass | Pituitary | Cass |
| Marrow | √ | Marrow | √ | Marrow | √ | Marrow | √ | Marrow | √ |

## Group A Vehicle (Females)

| 6 | | 7 | | 8 | | 9 | | 10 | |
|---|---|---|---|---|---|---|---|---|---|
| Tissue | weights | Tissue | weights | Tissue | weights | Tissue | weights | Tissue | weights |
| Thymus | 0.66 | Thymus | 0.79 | Thymus | 0.78 | Thymus | 0.55 | Thymus | 0.66 |
| Heart | 1.33 | Heart | 1.31 | Heart | 1.34 | Heart | 1.14 | Heart | 1.22 |
| Lungs | 1.67 | Lungs | 1.60 | Lungs | 1.36 | Lungs | 1.41 | Lungs | 1.56 |
| Thyroid/ para thyroid | Cass | Thyroid/ para thyroid | Cass | Thyroid/ para thyroid | Cass | Thyroid/ para thyroid | Cass | Thyroid/ para thyroid | Cass |
| Liver | 12.40 | Liver | 11.24 | Liver | 10.19 | Liver | 9.36 | Liver | 11.49 |
| Adrenals | Cass | Adrenals | Cass | Adrenals | Cass | Adrenals | Cass | Adrenals | Cass |
| Kidneys | 2.66 | Kidneys | 2.72 | Kidneys | 2.14 | Kidneys | 1.75 | Kidneys | 2.42 |
| Spleen | 0.72 | Spleen | 0.68 | Spleen | 0.56 | Spleen | 0.50 | Spleen | 0.61 |
| Small intestine | 1.33 | Small intestine | 0.41 | Small intestine | 0.90 | Small intestine | 0.44 | Small intestine | 1.02 |
| Ovaries/ uterus | 0.76 | Ovaries/ uterus | 1.42 | Ovaries/ uterus | 0.83 | Ovaries/ uterus | 1.88 | Ovaries/ uterus | 0.61 |
| Brain | 1.87 | Brain | 2.06 | Brain | 1.85 | Brain | 2.12 | Brain | 1.76 |
| Pituitary | cass | Pituitary | cass | Pituitary | cass | Pituitary | cass | Pituitary | cass |
| Marrow | √ | Marrow | √ | Marrow | √ | Marrow | √ | Marrow | √ |

## Group B: Anatabine 0.1 mg/kg (Males)

| 1 | | 2 | | 3 | | 4 | | 5 | |
|---|---|---|---|---|---|---|---|---|---|
| Tissue | weights | Tissue | weights | Tissue | weights | Tissue | weights | Tissue | weights |
| Thymus | 0.74 | Thymus | 0.74 | Thymus | 0.76 | Thymus | 0.71 | Thymus | 0.81 |
| Heart | 1.71 | Heart | 1.59 | Heart | 1.97 | Heart | 1.60 | Heart | 1.85 |
| Lungs | 2.31 | Lungs | 1.77 | Lungs | 2.08 | Lungs | 1.91 | Lungs | 1.83 |
| Thyroid/ para thyroid | Cass | Thyroid/ para thyroid | Cass | Thyroid/ para thyroid | Cass | Thyroid/ para thyroid | Cass | Thyroid/ para thyroid | Cass |
| Liver | 17.92 | Liver | 15.45 | Liver | 16.98 | Liver | 16.32 | Liver | 17.38 |
| Adrenals | Cass | Adrenals | Cass | Adrenals | Cass | Adrenals | Cass | Adrenals | Cass |
| Kidneys | 3.55 | Kidneys | 3.33 | Kidneys | 3.70 | Kidneys | 3.27 | Kidneys | 3.99 |
| Spleen | 0.91 | Spleen | 0.66 | Spleen | 0.82 | Spleen | 0.93 | Spleen | 0.76 |
| Small intestine | 0.61 | Small intestine | 0.96 | Small intestine | 0.88 | Small intestine | 0.50 | Small intestine | 0.35 |
| Prostate | 0.72 | Prostate | 0.49 | Prostate | 0.73 | Prostate | 0.53 | Prostate | 0.48 |
| Testes | 3.69 | Testes | 3.64 | Testes | 3.14 | Testes | 3.35 | Testes | 3.29 |
| Brain | 2.35 | Brain | 2.16 | Brain | 2.24 | Brain | 2.11 | Brain | 1.88 |
| Pituitary | cass | Pituitary | cass | Pituitary | cass | Pituitary | cass | Pituitary | cass |
| Marrow | √ | Marrow | √ | Marrow | √ | Marrow | √ | Marrow | √ |

## Group B: Anatabine 0.1 mg/kg (Females)

| 6 | | 7 | | 8 | | 9 | | 10 | |
|---|---|---|---|---|---|---|---|---|---|
| Tissue | weights | Tissue | weights | Tissue | weights | Tissue | weights | Tissue | weights |
| Thymus | 0.68 | Thymus | 0.59 | Thymus | 0.49 | Thymus | 0.71 | Thymus | 0.73 |
| Heart | 1.29 | Heart | 1.12 | Heart | 0.95 | Heart | 1.05 | Heart | 1.25 |
| Lungs | 1.42 | Lungs | 1.39 | Lungs | 1.15 | Lungs | 1.52 | Lungs | 1.59 |
| Thyroid/ para thyroid | Cass | Thyroid/ para thyroid | Cass | Thyroid/ para thyroid | Cass | Thyroid/ para thyroid | Cass | Thyroid/ para thyroid | Cass |
| Liver | 11.54 | Liver | 10.09 | Liver | 9.94 | Liver | 12.94 | Liver | 9.67 |
| Adrenals | Cass | Adrenals | Cass | Adrenals | Cass | Adrenals | Cass | Adrenals | Cass |
| Kidneys | 2.46 | Kidneys | 2.02 | Kidneys | 2.02 | Kidneys | 2.14 | Kidneys | 2.25 |
| Spleen | 0.79 | Spleen | 0.62 | Spleen | 0.51 | Spleen | 0.67 | Spleen | 0.65 |
| Small intestine | 0.87 | Small intestine | 0.71 | Small intestine | 0.55 | Small intestine | 0.49 | Small intestine | 0.63 |
| Ovaries/ uterus | 0.96 | Ovaries/ uterus | 1.79 | Ovaries/ uterus | 0.84 | Ovaries/ uterus | 1.42 | Ovaries/ uterus | 0.70 |
| Brain | 1.91 | Brain | 1.89 | Brain | 1.75 | Brain | 1.86 | Brain | 1.89 |
| Pituitary | cass | Pituitary | cass | Pituitary | cass | Pituitary | cass | Pituitary | cass |
| Marrow | √ | Marrow | √ | Marrow | √ | Marrow | √ | Marrow | √ |

## Group C: Anatabine 0.75 mg/kg (Males)

**1**

| Tissue | weights |
|---|---|
| Thymus | 1.02 |
| Heart | 1.52 |
| Lungs | 1.86 |
| Thyroid/ para thyroid | Cass |
| Liver | 14.92 |
| Adrenals | Cass |
| Kidneys | 3.45 |
| Spleen | 0.71 |
| Small intestine | 0.54 |
| Prostate | 0.49 |
| Testes | 3.19 |
| Brain | 2.01 |
| Pituitary | cass |
| Marrow | √ |

**2**

| Tissue | weights |
|---|---|
| Thymus | 0.52 |
| Heart | 1.38 |
| Lungs | 1.97 |
| Thyroid/ para thyroid | Cass |
| Liver | 13.91 |
| Adrenals | Cass |
| Kidneys | 2.77 |
| Spleen | 0.63 |
| Small intestine | 1.41 |
| Prostate | 0.49 |
| Testes | 3.84 |
| Brain | 2.19 |
| Pituitary | cass |
| Marrow | √ |

**3**

| Tissue | weights |
|---|---|
| Thymus | 0.67 |
| Heart | 1.56 |
| Lungs | 1.72 |
| Thyroid/ para thyroid | Cass |
| Liver | 14.70 |
| Adrenals | Cass |
| Kidneys | 3.49 |
| Spleen | 0.79 |
| Small intestine | 0.56 |
| Prostate | 0.65 |
| Testes | 3.03 |
| Brain | 2.12 |
| Pituitary | cass |
| Marrow | √ |

**4**

| Tissue | weights |
|---|---|
| Thymus | 0.69 |
| Heart | 1.39 |
| Lungs | 1.95 |
| Thyroid/ para thyroid | Cass |
| Liver | 14.01 |
| Adrenals | Cass |
| Kidneys | 3.16 |
| Spleen | 0.93 |
| Small intestine | 0.41 |
| Prostate | 0.46 |
| Testes | 3.28 |
| Brain | 2.00 |
| Pituitary | cass |
| Marrow | √ |

**5**

| Tissue | weights |
|---|---|
| Thymus | 0.47 |
| Heart | 1.29 |
| Lungs | 1.58 |
| Thyroid/ para thyroid | Cass |
| Liver | 14.94 |
| Adrenals | Cass |
| Kidneys | 2.89 |
| Spleen | 0.64 |
| Small intestine | 0.47 |
| Prostate | 0.45 |
| Testes | 2.70 |
| Brain | 2.02 |
| Pituitary | cass |
| Marrow | √ |

## Group C: Anatabine 0.75 mg/kg (Females)

| 6 | | 7 | | 8 | | 9 | | 10 | |
|---|---|---|---|---|---|---|---|---|---|
| **Tissue** | **weights** | **Tissue** | **weights** | **Tissue** | **weights** | **Tissue** | **weights** | **Tissue** | **weights** |
| Thymus | 0.56 | Thymus | 0.53 | Thymus | 0.63 | Thymus | 0.52 | Thymus | 0.54 |
| Heart | 1.09 | Heart | 1.21 | Heart | 1.23 | Heart | 1.03 | Heart | 0.99 |
| Lungs | 1.46 | Lungs | 1.39 | Lungs | 1.62 | Lungs | 1.30 | Lungs | 1.49 |
| Thyroid/ para thyroid | Cass | Thyroid/ para thyroid | Cass | Thyroid/ para thyroid | Cass | Thyroid/ para thyroid | Cass | Thyroid/ para thyroid | Cass |
| Liver | 10.71 | Liver | 8.65 | Liver | 11.37 | Liver | 11.20 | Liver | 10.19 |
| Adrenals | Cass | Adrenals | Cass | Adrenals | Cass | Adrenals | Cass | Adrenals | Cass |
| Kidneys | 2.08 | Kidneys | 2.20 | Kidneys | 2.68 | Kidneys | 2.18 | Kidneys | 2.36 |
| Spleen | 0.70 | Spleen | 0.65 | Spleen | 0.65 | Spleen | 0.51 | Spleen | 0.49 |
| Small intestine | 0.72 | Small intestine | 0.37 | Small intestine | 0.85 | Small intestine | 0.47 | Small intestine | 1.34 |
| Ovaries/ uterus | 0.81 | Ovaries/ uterus | 1.50 | Ovaries/ uterus | 0.72 | Ovaries/ uterus | 1.23 | Ovaries/ uterus | 3.65 |
| Brain | 1.99 | Brain | 1.93 | Brain | 1.73 | Brain | 2.06 | Brain | 1.81 |
| Pituitary | cass | Pituitary | cass | Pituitary | cass | Pituitary | cass | Pituitary | cass |
| Marrow | √ | Marrow | √ | Marrow | √ | Marrow | √ | Marrow | √ |

## Group D: Anatabine 1.5 mg/kg (Males)

| 1 | | 2 | | 3 | | 4 | | 5 | |
|---|---|---|---|---|---|---|---|---|---|
| Tissue | weights | Tissue | weights | Tissue | weights | Tissue | weights | Tissue | weights |
| Thymus | 0.83 | Thymus | 0.77 | Thymus | 0.54 | Thymus | 0.86 | Thymus | 0.59 |
| Heart | 1.50 | Heart | 1.53 | Heart | 1.47 | Heart | 1.61 | Heart | 1.47 |
| Lungs | 1.77 | Lungs | 2.06 | Lungs | 1.93 | Lungs | 2.02 | Lungs | 1.90 |
| Thyroid/ para thyroid | Cass | Thyroid/ para thyroid | Cass | Thyroid/ para thyroid | Cass | Thyroid/ para thyroid | Cass | Thyroid/ para thyroid | Cass |
| Liver | 15.86 | Liver | 17.03 | Liver | 17.35 | Liver | 18.27 | Liver | 14.26 |
| Adrenals | Cass | Adrenals | Cass | Adrenals | Cass | Adrenals | Cass | Adrenals | Cass |
| Kidneys | 3.48 | Kidneys | 3.54 | Kidneys | 3.02 | Kidneys | 3.79 | Kidneys | 3.43 |
| Spleen | 0.71 | Spleen | 0.83 | Spleen | 0.86 | Spleen | 0.97 | Spleen | 0.92 |
| Small intestine | 0.43 | Small intestine | 0.77 | Small intestine | 0.56 | Small intestine | 1.00 | Small intestine | 0.66 |
| Prostate | 0.50 | Prostate | 0.45 | Prostate | | Prostate | 0.49 | Prostate | 0.45 |
| Testes | 2.78 | Testes | 3.28 | Testes | 3.51 | Testes | 3.41 | Testes | 3.23 |
| Brain | 1.75 | Brain | 1.81 | Brain | 2.02 | Brain | 2.00 | Brain | 2.08 |
| Pituitary | cass | Pituitary | cass | Pituitary | cass | Pituitary | cass | Pituitary | cass |
| Marrow | √ | Marrow | √ | Marrow | √ | Marrow | √ | Marrow | √ |

## Group D: Anatabine 1.5 mg/kg (Females)

| 6 | | 7 | | 8 | | 9 | | 10 | |
|---|---|---|---|---|---|---|---|---|---|
| Tissue | weights | Tissue | weights | Tissue | weights | Tissue | weights | Tissue | weights |
| Thymus | 0.59 | Thymus | 0.63 | Thymus | 0.52 | Thymus | 0.61 | Thymus | 0.51 |
| Heart | 1.12 | Heart | 0.88 | Heart | 1.04 | Heart | 0.97 | Heart | 0.98 |
| Lungs | 1.29 | Lungs | 1.46 | Lungs | 1.37 | Lungs | 1.60 | Lungs | 1.59 |
| Thyroid/ para thyroid | Cass | Thyroid/ para thyroid | Cass | Thyroid/ para thyroid | Cass | Thyroid/ para thyroid | Cass | Thyroid/ para thyroid | Cass |
| Liver | 9.65 | Liver | 8.83 | Liver | 11.46 | Liver | 11.05 | Liver | 9.21 |
| Adrenals | Cass | Adrenals | Cass | Adrenals | Cass | Adrenals | Cass | Adrenals | Cass |
| Kidneys | 2.17 | Kidneys | 2.08 | Kidneys | 2.52 | Kidneys | 2.64 | Kidneys | 1.97 |
| Spleen | 0.53 | Spleen | 0.65 | Spleen | 0.64 | Spleen | 0.68 | Spleen | 0.51 |
| Small intestine | 0.49 | Small intestine | 0.60 | Small intestine | 1.07 | Small intestine | 0.56 | Small intestine | 0.77 |
| Ovaries/ uterus | 0.84 | Ovaries/ uterus | 1.20 | Ovaries/ uterus | 0.98 | Ovaries/ uterus | 1.34 | Ovaries/ uterus | 0.78 |
| Brain | 1.90 | Brain | 1.91 | Brain | 1.97 | Brain | 2.08 | Brain | 1.91 |
| Pituitary | cass | Pituitary | cass | Pituitary | cass | Pituitary | cass | Pituitary | cass |
| Marrow | √ | Marrow | √ | Marrow | √ | Marrow | √ | Marrow | √ |

## Group E: Nicotine 0.75 mg/kg (Males)

| 1 | | 2 | | 3 | | 5 | |
|---|---|---|---|---|---|---|---|
| Tissue | weights | Tissue | weights | Tissue | weights | Tissue | weights |
| Thymus | 0.54 | Thymus | 0.46 | Thymus | 0.56 | Thymus | 0.76 |
| Heart | 1.20 | Heart | 1.17 | Heart | 1.21 | Heart | 1.42 |
| Lungs | 1.84 | Lungs | 1.49 | Lungs | 1.62 | Lungs | 1.66 |
| Thyroid/ para thyroid | Cass | Thyroid/ para thyroid | Cass | Thyroid/ para thyroid | Cass | Thyroid/ para thyroid | Cass |
| Liver | 11.97 | Liver | 12.52 | Liver | 12.06 | Liver | 14.20 |
| Adrenals | Cass | Adrenals | Cass | Adrenals | Cass | Adrenals | Cass |
| Kidneys | 3.19 | Kidneys | 3.04 | Kidneys | 2.87 | Kidneys | 3.01 |
| Spleen | 0.81 | Spleen | 0.62 | Spleen | 0.78 | Spleen | 0.86 |
| Small intestine | 0.71 | Small intestine | 0.56 | Small intestine | 0.53 | Small intestine | 0.40 |
| Prostate | 0.41 | Prostate | 0.28 | Prostate | 0.45 | Prostate | 0.28 |
| Testes | 3.05 | Testes | 3.13 | Testes | 3.02 | Testes | 2.92 |
| Brain | 2.06 | Brain | 1.90 | Brain | 1.84 | Brain | 1.94 |
| Pituitary | Cass | Pituitary | Cass | Pituitary | Cass | Pituitary | Cass |
| Marrow | √ | Marrow | √ | Marrow | √ | Marrow | √ |

**Group E: Nicotine 0.75 mg/kg (Females)**

| 6 | | 9 | | 10 | |
|---|---|---|---|---|---|
| Tissue | weights | Tissue | weights | Tissue | weights |
| Thymus | 0.54 | Thymus | 0.48 | Thymus | 0.48 |
| Heart | 1.09 | Heart | 1.06 | Heart | 0.83 |
| Lungs | 1.20 | Lungs | 1.48 | Lungs | 1.28 |
| Thyroid/ para thyroid | Cass | Thyroid/ para thyroid | Cass | Thyroid/ para thyroid | Cass |
| Liver | 9.48 | Liver | 10.11 | Liver | 9.18 |
| Adrenals | Cass | Adrenals | Cass | Adrenals | Cass |
| Kidneys | 2.34 | Kidneys | 2.29 | Kidneys | 2.29 |
| Spleen | 0.46 | Spleen | 0.76 | Spleen | 0.46 |
| Small intestine | 0.41 | Small intestine | 0.40 | Small intestine | 0.28 |
| Ovaries/ uterus | 1.59 | Ovaries/ uterus | 0.92 | Ovaries/ uterus | 1.32 |
| Brain | 1.77 | Brain | 1.93 | Brain | 1.87 |
| Pituitary | Cass | Pituitary | Cass | Pituitary | Cass |
| Marrow | √ | Marrow | √ | Marrow | √ |

**Table 37. Dosing solutions**

| Test compound | Percentage content of anatabine | Dose level (mg/kg) | Test compound concentration (total) (mg/mL) | Test compound concentration (base) (mg/mL) | Injection volume (mL/kg) |
|---|---|---|---|---|---|
| Anatabine | 5.18 | 0.20 | 0.772 | 0.04 | 5 |
| Anatabine | 5.18 | 2.0 | 7.72 | 0.4 | 5 |

**Table 38. Dosing**

| Test compound | Route | Sex | Concentration mg/kg/ dose | # of animals | Dosing times (minutes) |
|---|---|---|---|---|---|
| Anatabine | p.o. | M | 0.2 | 4 | 0, 240, 480 |
| | | F | 0.2 | 4 | |
| | | M | 2.0 | 4 | |
| | | F | 2.0 | 4 | |

**Table 39. Blood Collection Times**

| Test compound | Route | Sex | Concentration mg/kg/ dose | # of animals | Blood collection (minutes) |
|---|---|---|---|---|---|
| Anatabine | p.o. | M | 0.2 | 4 | 30, 60, 235 (pre-dose), 270, 300, 475 (pre-dose), 540, 600, 720, 1440 |
| | | F | 0.2 | 4 | |
| | | M | 2.0 | 4 | |
| | | F | 2.0 | 4 | |

**Table 40. Calibration Curve Concentrations**

| nominal concentration (nM) | stock concentration (μM) |
|---|---|
| 5000 | 250 |
| 1667 | 83.3 |
| 555.5 | 27.8 |
| 185.2 | 9.3 |
| 61.7 | 3.1 |
| 20.6 | 1.0 |
| 6.9 | 0.34 |
| 2.3 | 0.11 |
| 0.76 | 0.038 |
| 0.25 | 0.013 |

**Table 41. LC/MS/MS ionization conditions and identity of parent and product ions.**

| Compound | MW | Polarization | Precursor m/z | Product m/z | Collision energy (V) |
|---|---|---|---|---|---|
| **Anatabine** | 160.2 | Positive | 161.1 | 115.1 | 28 |
| **(*R,S*)-Antabine-2,4,5,6-$d_4$** | 164.24 | Positive | 165.1 | 148.1 | 20 |

**Table 42. Limits of Detection and Calibration Curves**

| Sample | Limit of Detection (LOD) (ng/mL) | Lower Limit of Quantitation (LLQ) (ng/mL) | Upper Limit of Quantitation (ULQ) (ng/mL) |
|---|---|---|---|
| **Anatabine in rat plasma** | 0.37 | 1.1 | ≥801 |

Table 43. Dosing Solution Analysis

| Compound | Dose (mg/kg) | Expected Concentration (mg/mL) | Actual Concentration {mg/mL) | Actual Concentration relative to Expected (%) |
|---|---|---|---|---|
| **anatabine** | 0.2 | 0.04 | 0.025 | 63% |
| **anatabine** | 2.0 | 0.4 | 0.335 | 84% |

**Table 44. Comparison of pharmacokinetic parameters ($T_{max}$, $C_{p.max}$ and $C_{p.min}$) between male and female rats in each of the two treatment groups.**

| Parameter | Anatabine (0.6 mg/kg) | | | | | | | Anatabine (6.0 mg/kg) | | | | | | |
|---|---|---|---|---|---|---|---|---|---|---|---|---|---|---|
| | Male | | | Female | | | *p* | Male | | | Female | | | *p* |
| | n | Mean | SD | n | Mean | SD | | n | Mean | SD | n | Mean | SD | |
| $T_{max\,(1)}$ (hr) | 4 | 0.75 | 0.29 | 4 | 0.63 | 0.25 | 0.537 | 4 | 0.63 | 0.25 | 4 | 0.50 | 0.00 | 0.356 |
| $T_{max\,(2)}$ (hr) | 4 | 0.63 | 0.25 | 4 | 0.88 | 0.25 | 0.207 | 4 | 0.63 | 0.25 | 4 | 0.63 | 0.25 | 1.000 |
| $T_{max\,(3)}$ (hr) | 4 | 1.00 | 0 | 4 | 1.25 | 0.50 | 0.356 | 4 | 2.00 | 1.41 | 4 | 1.25 | 0.50 | 0.356 |
| $C_{p.\,max\,(1)}$ (ng/mL) | 4 | 34.3 | 2.1 | 4 | 38.3 | 6.1 | 0.262 | 4 | 244 | 86 | 4 | 260 | 35 | 0.738 |
| $C_{p.\,max\,(2)}$ (ng/mL) | 4 | 30.3 | 3.8 | 4 | 31.3 | 14.2 | 0.896 | 4 | 305 | 53 | 4 | 312 | 77 | 0.889 |
| $C_{p.\,max\,(3)}$ (ng/mL) | 3 | 37.3 | 8.1 | 4 | 35.5 | 10.6 | 0.814 | 4 | 283 | 94 | 4 | 375 | 123 | 0.281 |
| $C_{p.\,min\,(1)}$ (ng/mL) | 3 | 11.0 | 2.0 | 4 | 10.3 | 6.4 | 0.856 | 4 | 75 | 26 | 4 | 52 | 26 | 0.254 |
| $C_{p.\,min\,(2)}$ (ng/mL) | 3 | 15.3 | 5.5 | 4 | 7.5 | 1.7 | **0.040** | 4 | 93 | 16 | 4 | 180 | 31 | **0.002** |

Table 45. Comparison of pharmacokinetic parameters ($C_{p.\,max}$ and $C_{p.\,min}$) over time for male and female rats in each of the two treatment groups.

| Parameter | Anatabine (0.6 mg/kg) | | | | | | Anatabine (6.0 mg/kg) | | | | | |
|---|---|---|---|---|---|---|---|---|---|---|---|---|
| | Male | | | Female | | | Male | | | Female | | |
| | Mean | SD | *p* | Mean | SD | *p* | Mean | SD | *p* | Mean | SD | *p* |
| $C_{p.\,max\,(1)}$ (ng/mL) | 34.3 | 2.1 | | 38.3 | 6.1 | | 287 | 11 | | 259.8 | 35.4 | |
| $C_{p.\,max\,(2)}$ (ng/mL) | 30.3 | 3.8 | 0.195 | 31.3 | 14.2 | 0.570 | 305 | 53 | 0.897 | 312.0 | 76.8 | 0.142 |
| $C_{p.\,max\,(3)}$ (ng/mL) | 37.3 | 8.1 | | 35.5 | 10.6 | | 283 | 94 | | 374.8 | 122.9 | |
| $C_{p.\,min\,(1)}$ (ng/mL) | 11.0 | 2.0 | 0.177 | 10.3 | 6.4 | 0.428 | 75 | 26 | 0.131 | 51.5 | 26.0 | **0.015** |
| $C_{p.\,min\,(2)}$ (ng/mL) | 15.3 | 5.5 | | 7.5 | 1.7 | | 93 | 16 | | 180.0 | 30.7 | |

**Table 46. Comparison of pharmacokinetic parameters ($AUC_{0\rightarrow\infty}$, $t_{1/2,\ 0\rightarrow4}$, $t_{1/2,\ terminal}$, $MTT_{0\rightarrow4}$ and $MAT_{0\rightarrow4}$) between male and female rats in each of the two treatment groups.**

| Parameter | Anatabine (0.6 mg/kg) | | | | | | | Anatabine (6.0 mg/kg) | | | | | | |
|---|---|---|---|---|---|---|---|---|---|---|---|---|---|---|
| | Male | | | Female | | | *p* | Male | | | Female | | | *p* |
| | n | Mean | SD | n | Mean | SD | | n | Mean | SD | n | Mean | SD | |
| $AUC_{0\rightarrow\infty}$ (ng·hr/mL) | 3 | 280 | 102 | 4 | 290 | 71 | 0.880 | 4 | 3257 | 480 | 4 | 3735 | 587 | 0.255 |
| $t_{1/2.\ 0\rightarrow4}$ (hr) | 4 | 2.05 | 0.43 | 4 | 2.06 | 1.20 | 0.986 | 3 | 1.76 | 0.39 | 4 | 1.82 | 0.81 | 0.918 |
| $t_{1/2.\ terminal}$ (hr) | 3 | 1.78 | 0.68 | 4 | 1.80 | 0.72 | 0.970 | 4 | 5.07 | 2.05 | 4 | 3.99 | 1.52 | 0.430 |
| $MTT_{0\rightarrow4}$ (hr) | 3 | 3.17 | 0.51 | 4 | 2.98 | 1.84 | 0.873 | 4 | 3.18 | 1.29 | 4 | 2.76 | 1.39 | 0.668 |
| $MAT_{0\rightarrow4}$ (hr) | 3 | 0.71 | 1.34 | 4 | 0.53 | 1.84 | 0.873 | 4 | 0.73 | 1.29 | 4 | 0.30 | 1.39 | 0.668 |

**Table 47. Comparison of pharmacokinetic parameters ($AUC_{0\to\infty}$, $t_{1/2.\,0\to4}$, $t_{1/2.\,terminal}$, $MTT_{0\to4}$ and $MAT_{0\to4}$) between treatment groups.**

| Parameter | Anatabine (0.6 mg/kg) | | | Anatabine(6.0 mg/kg) | | | p |
|---|---|---|---|---|---|---|---|
| | n | Mean | SD | n | Mean | SD | |
| $AUC_{0\to\infty}$ (ng hr/mL) | 7 | 285 | 77 | 8 | 3496 | 559 | **<0.001** |
| $t_{1/2.0\to4}$ (hr) | 8 | 2.05 | 0.83 | 7 | 1.79 | 0.62 | 0.514 |
| $t_{1/2.\,terminal}$ (hr) | 7 | 1.79 | 0.64 | 8 | 4.53 | 1.77 | **0.002** |
| $MTT_{0\to4}$ (hr) | 7 | 3.06 | 1.34 | 8 | 2.97 | 1.26 | 0.898 |
| $MAT_{0\to4}$ (hr) | 7 | 0.61 | 1.34 | 8 | 0.52 | 1.26 | 0.898 |

**Table 48. Comparison of pharmacokinetic parameters ($t_{1/2.\,0\to4}$, $MTT_{0\to4}$ and $MAT_{0\to4}$) between male and female rats in both treatment groups, combined, and all data combined.**

| Parameter | Male | | | Female | | | ρ | Overall | | |
|---|---|---|---|---|---|---|---|---|---|---|
| | n | Mean | SD | n | Mean | SD | | n | Mean | SD |
| $t_{1/2.0\to4}$ (hr) | 7 | 1.92 | 0.41 | 8 | 1.94 | 0.96 | 0.973 | 15 | 1.93 | 0.73 |
| $MTT_{0\to4}$ (hr) | 7 | 3.18 | 0.96 | 8 | 2.87 | 1.52 | 0.650 | 15 | 3.01 | 1.25 |
| $MAT_{0\to4}$ (hr) | 7 | 0.72 | 0.96 | 8 | 0.42 | 1.52 | 0.650 | 15 | 0.56 | 1.25 |

**Table 49. Animal Weights and Dosing Times**

| | | | Dose | | Time Points for Samples (hrs) - retro-orbital | | | | | | | | | | | |
|---|---|---|---|---|---|---|---|---|---|---|---|---|---|---|---|---|
| Cmpnd | Rat | B W (g) | volume (ml) | time | 0 5 | 1 | 4 (pre) | dose (2) | 4 5 | 5 | 8 (pre) | dose (3) | 9 | 10 | 12 | 24 |
| 1 | A | 246 | 1 2 | 6 06 | 6 36 | 7 06 | 10 01 | 10 06 | 10 36 | 11 06 | 2 01 | 2 06 | 3 06 | 4 06 | 6 06 | 6 06 |
| MALE | B | 231 | 1 2 | 6 07 | 6 37 | 7 07 | 10 02 | 10 07 | 10 37 | 11 07 | 2 02 | 2 07 | 3 07 | 4 07 | 6 07 | 6 07 |
| | C | 244 | 1 2 | 6 08 | 6 38 | 7 08 | 10 03 | 10 08 | 10 38 | 11 08 | 2 03 | 2 08 | 3 08 | 4 08 | 6 08 | 6 08 |
| Anatabine 0 2 MPK/ dose | D | 242 | 1 2 | 6 09 | 6 39 | 7 09 | 10 04 | 10 09 | 10 39 | 11 09 | 2 04 | 2 09 | 3 09 | 4 09 | 6 09 | 6 09 |
| 2 | A | 203 | 1 0 | 6 10 | 6 40 | 7 10 | 10 05 | 10 10 | 10 40 | 11 10 | 2 05 | 2 10 | 3 10 | 4 10 | 6 10 | 6 10 |
| FEMALE | B | 200 | 1 0 | 6 11 | 6 41 | 7 11 | 10 06 | 10 11 | 10 41 | 11 11 | 2 06 | 2 11 | 3 11 | 4 11 | 6 11 | 6 11 |
| | C | 212 | 1 1 | 6 12 | 6 42 | 7 12 | 10 07 | 10 12 | 10 42 | 11 12 | 2 07 | 2 12 | 3 12 | 4 12 | 6 12 | 6 12 |
| Anatabino 0 2 MPK/dose | D | 201 | 1 0 | 6 13 | 6 43 | 7 13 | 10 08 | 10 13 | 10 43 | 11 13 | 2 08 | 2 13 | 3 13 | 4 13 | 6 13 | 6 13 |
| 3 | A | 240 | 1 2 | 6 14 | 6 44 | 7 14 | 10 09 | 10 14 | 10 44 | 11 14 | 2 09 | 2 14 | 3 14 | 4 14 | 6 14 | 6 14 |
| MALE | B | 239 | 1 2 | 6 15 | 6 45 | 7 15 | 10 10 | 10 15 | 10 45 | 11 15 | 2 10 | 2 15 | 3 15 | 4 15 | 6 15 | 6 15 |
| | C | 241 | 1 2 | 6 16 | 6 46 | 7 16 | 10 11 | 10 16 | 10 46 | 11 16 | 2 11 | 2 16 | 3 16 | 4 16 | 6 16 | 6 16 |
| Anatabine 2 0 MPK/dose | D | 240 | 1 2 | 6 17 | 6 47 | 7 17 | 10 12 | 10 17 | 10 47 | 11 17 | 2 12 | 2 17 | 3 17 | 4 17 | 6 17 | 6 17 |
| 4 | A | 208 | 1 0 | 6 18 | 6 48 | 7 18 | 10 13 | 10 18 | 10 48 | 11 18 | 2 13 | 2 18 | 3 18 | 4 18 | 6 18 | 6 18 |
| FEMALE | B | 215 | 1 1 | 6 19 | 6 49 | 7 19 | 10 14 | 10 19 | 10 49 | 11 19 | 2 14 | 2 19 | 3 19 | 4 19 | 6 19 | 6 19 |
| | C | 207 | 1 0 | 6 20 | 6 50 | 7 20 | 10 15 | 10 20 | 10 50 | 11 20 | 2 15 | 2 20 | 3 20 | 4 20 | 6 20 | 6 20 |
| Anatabine 2 0 MPK/dose | D | 201 | 1 00 | 6 21 | 6 51 | 7 21 | 10 16 | 10 21 | 10 51 | 11 21 | 2 16 | 2 21 | 3 21 | 4 21 | 6 21 | 6 21 |

**Table 50. Measured Concentrations of Anatabine in Rat Plasma Samples at Each Time Point**

| Dose (mg/kg) | Animal ID | Sex | Time Point (min) | Anatabine Concentration (ng/mL) |
|---|---|---|---|---|
| 0.2 | 1A | male | 30 | 34 |
| | | | 60 | 26 |
| | | | 235 | 11 |
| | | | 270 | 35 |
| | | | 300 | 22 |
| | | | 475 | 9 |
| | | | 540 | 4 |
| | | | 600 | <LOQ |
| | | | 720 | <LOQ |
| | | | 1440 | <LOQ |
| | 1B | male | 30 | <LOQ |
| | | | 60 | 32 |
| | | | 235 | <LOQ |
| | | | 270 | 31 |
| | | | 300 | 26 |
| | | | 475 | <LOQ |
| | | | 540 | 28 |
| | | | 600 | 17 |
| | | | 720 | 5 |
| | | | 1440 | <LOQ |
| | 1C | male | 30 | 29 |
| | | | 60 | 34 |
| | | | 235 | 13 |
| | | | 270 | 20 |
| | | | 300 | 29 |
| | | | 475 | 18 |
| | | | 540 | 41 |
| | | | 600 | 35 |
| | | | 720 | 12 |
| | | | 1440 | <LOQ |

(continued)

| Dose (mg/kg) | Animal ID | Sex | Time Point (min) | Anatabine Concentration (ng/mL) |
|---|---|---|---|---|
| | 1D | male | 30 | 37 |
| | | | 60 | 24 |
| | | | 235 | 9 |
| | | | 270 | 26 |
| | | | 300 | 22 |
| | | | 475 | 19 |
| | | | 540 | 43 |
| | | | 600 | 34 |
| | | | 720 | 19 |
| | | | 1440 | <LOQ |
| | 2A | female | 30 | 34 |
| | | | 60 | 29 |
| | | | 235 | 4 |
| | | | 270 | 14 |
| | | | 300 | 18 |
| | | | 475 | 7 |
| | | | 540 | 24 |
| | | | 600 | 12 |
| | | | 720 | 18 |
| | | | 1440 | <LOQ |
| | 2B | female | 30 | 34 |
| | | | 60 | 33 |
| | | | 235 | 18 |
| | | | 270 | 11 |
| | | | 300 | 31 |
| | | | 475 | 7 |
| | | | 540 | 44 |
| | | | 600 | 18 |
| | | | 720 | 15 |
| | | | 1440 | <LOQ |

(continued)

| Dose (mg/kg) | Animal ID | Sex | Time Point (min) | Anatabine Concentration (ng/mL) |
|---|---|---|---|---|
| | 2C | female | 30 | 42 |
| | | | 60 | 47 |
| | | | 235 | 6 |
| | | | 270 | 17 |
| | | | 300 | 25 |
| | | | 475 | 6 |
| | | | 540 | 29 |
| | | | 600 | 20 |
| | | | 720 | 7 |
| | | | 1440 | <LOQ |
| | 2D | female | 30 | 38 |
| | | | 60 | 25 |
| | | | 235 | 13 |
| | | | 270 | 51 |
| | | | 300 | 44 |
| | | | 475 | 10 |
| | | | 540 | 29 |
| | | | 600 | 45 |
| | | | 720 | 15 |
| | | | 1440 | <LOQ |

(continued)

| Dose (mg/kg) | Animal ID | Sex | Time Point (min) | Anatabine Concentration (ng/mL) |
|---|---|---|---|---|
| 2 | 3A | male | 30 | 298 |
| | | | 60 | 153 |
| | | | 235 | 84 |
| | | | 270 | 131 |
| | | | 300 | 312 |
| | | | 475 | 82 |
| | | | 540 | 223 |
| | | | 600 | 269 |
| | | | 720 | 133 |
| | | | 1440 | 12 |
| | 3B | male | 30 | 288 |
| | | | 60 | 106 |
| | | | 235 | 46 |
| | | | 270 | 236 |
| | | | 300 | 232 |
| | | | 475 | 79 |
| | | | 540 | 214 |
| | | | 600 | 173 |
| | | | 720 | 401 |
| | | | 1440 | <LOQ |
| | 3C | male | 30 | 269 |
| | | | 60 | 272 |
| | | | 235 | 63 |
| | | | 270 | 364 |
| | | | 475 | 97 |
| | | | 540 | 290 |
| | | | 600 | 130 |
| | | | 720 | 137 |
| | | | 1440 | 42 |
| | | | 300 | 116 |

(continued)

| Dose (mg/kg) | Animal ID | Sex | Time Point (min) | Anatabine Concentration (ng/mL) |
|---|---|---|---|---|
| | 3D | male | 30 | 116 |
| | | | 60 | 116 |
| | | | 235 | 105 |
| | | | 270 | 309 |
| | | | 300 | 202 |
| | | | 475 | 114 |
| | | | 540 | 173 |
| | | | 600 | 150 |
| | | | 720 | 71 |
| | | | 1440 | 36 |
| | 4A | female | 30 | 245 |
| | | | 60 | 81 |
| | | | 235 | 75 |
| | | | 270 | 237 |
| | | | 300 | 216 |
| | | | 475 | 144 |
| | | | 540 | 231 |
| | | | 600 | 197 |
| | | | 720 | 186 |
| | | | 1440 | 42 |
| | 4B | female | 30 | 78 |
| | | | 60 | 95 |
| | | | 235 | 36 |
| | | | 270 | 324 |
| | | | 300 | 97 |
| | | | 475 | 219 |
| | | | 540 | 314 |
| | | | 600 | 207 |
| | | | 720 | 165 |
| | | | 1440 | 8 |

(continued)

| Dose (mg/kg) | Animal ID | Sex | Time Point (min) | Anatabine Concentration (ng/mL) |
|---|---|---|---|---|
| | 4C | female | 30 | 218 |
| | | | 60 | 127 |
| | | | 235 | 23 |
| | | | 270 | 273 |
| | | | 300 | 191 |
| | | | 475 | 178 |
| | | | 540 | 369 |
| | | | 600 | 480 |
| | | | 720 | 244 |
| | | | 1440 | 36 |
| | 4D | female | 30 | 98 |
| | | | 60 | 165 |
| | | | 235 | 72 |
| | | | 270 | 350 |
| | | | 300 | 414 |
| | | | 475 | 179 |
| | | | 540 | 474 |
| | | | 600 | 288 |
| | | | 720 | 217 |
| | | | 1440 | <LOQ |

**Table 51. Mean Concentrations and Descriptive Statistics of Anatabine in Plasma Samples at Each Time Point**

| | | | Male or Female | | | | Combined Male and Female | | | |
|---|---|---|---|---|---|---|---|---|---|---|
| Dose Group | Sex | Time Point (min) | N= | Avg. Conc. (ng/ml) | ±SD | ±SEM | Male and Female Avg. Conc. (ng/ml) | n= | ±SD | ±SEM |
| 0.2 | male | 30 | 3 | 33 | 4.2 | 2.4 | 36 | 7 | 4.3 | 1.6 |
| | | 60 | 4 | 30 | 4.6 | 2.3 | 31 | 8 | 7.5 | 2.6 |
| | | 235 | 3 | 11 | 1.6 | 0.9 | 10 | 7 | 4.7 | 1.8 |
| | | 270 | 4 | 26 | 6.9 | 3.4 | 26 | 8 | 13.3 | 4.7 |
| | | 300 | 4 | 26 | 3.0 | 1.5 | 27 | 8 | 7.9 | 2.8 |
| | | 475 | 3 | 18 | 5.3 | 3.0 | 11 | 7 | 5.4 | 2.0 |
| | | 540 | 4 | 38 | 17.9 | 8.9 | 30 | 8 | 13.1 | 4.6 |
| | | 600 | 3 | 29 | 10.4 | 6.0 | 26 | 7 | 12.3 | 4.3 |
| | | 720 | 3 | 12 | 6.5 | 3.8 | 13 | 7 | 5.2 | 1.8 |
| | | 1440 | 0 | n/a | n/a | n/a | n/a | 0 | n/a | n/a |
| | | 30 | 4 | 37 | 3.9 | 2.0 | | | | |
| | | 60 | 4 | 35 | 9.7 | 4.9 | | | | |

(continued)

| Dose Group | Sex | Time Point (min) | Male or Female N= | Avg. Conc. (ng/ml) | ±SD | ±SEM | Combined Male and Female: Male and Female Avg. Conc. (ng/ml) | n= | ±SD | ±SEM |
|---|---|---|---|---|---|---|---|---|---|---|
| 0.2 | female | 235 | 4 | 12 | 6.4 | 3.2 | | | | |
| | | 270 | 4 | 26 | 18.7 | 9.4 | | | | |
| | | 300 | 4 | 34 | 10.9 | 5.5 | | | | |
| | | 475 | 4 | 8 | 1.7 | 0.9 | | | | |
| | | 540 | 4 | 34 | 8.7 | 4.4 | | | | |
| | | 600 | 4 | 28 | 14.7 | 7.3 | | | | |
| | | 720 | 4 | 12 | 4.8 | 2.4 | | | | |
| | | 1440 | 0 | n/a | n/a | n/a | | | | |
| 2 | male | 30 | 4 | 243 | 85.6 | 42.8 | 201 | 8 | 90.1 | 31.9 |
| | | 60 | 4 | 165 | 76.1 | 38.0 | 139 | 8 | 60.4 | 21.3 |
| | | 235 | 4 | 72 | 25.7 | 12.9 | 63 | 8 | 26.9 | 9.5 |
| | | 270 | 4 | 303 | 100.9 | 50.5 | 278 | 8 | 76.4 | 27.0 |
| | | 300 | 4 | 183 | 80.7 | 40.3 | 222 | 8 | 102.3 | 36.2 |
| | | 475 | 4 | 97 | 16.2 | 8.1 | 136 | 8 | 51.7 | 18.3 |
| | | 540 | 4 | 226 | 48.3 | 24.1 | 286 | 8 | 98.7 | 34.9 |
| | | 600 | 4 | 151 | 61.7 | 30.8 | 237 | 8 | 112.2 | 39.7 |
| | | 720 | 4 | 203 | 146.6 | 73.3 | 194 | 8 | 99.1 | 35.0 |
| | | 1440 | 3 | 39 | 15.9 | 9.2 | 29 | 6 | 15.5 | 6.3 |
| 2 | female | 30 | 4 | 160 | 83.8 | 41.9 | | | | |
| | | 60 | 4 | 129 | 37.3 | 18.6 | | | | |
| | | 235 | 4 | 44 | 25.8 | 12.9 | | | | |
| | | 270 | 4 | 316 | 50.8 | 25.4 | | | | |
| | | 300 | 4 | 234 | 133.3 | 66.6 | | | | |
| | | 475 | 4 | 192 | 30.7 | 15.3 | | | | |
| | | 540 | 4 | 386 | 102.1 | 51.1 | | | | |
| | | 600 | 4 | 325 | 131.0 | 65.5 | | | | |
| | | 720 | 4 | 208 | 34.7 | 17.3 | | | | |
| | | 1440 | 3 | 22 | 18.6 | 10.7 | | | | |

**Claims**

**1.** A composition comprising an isolated form of a compound of Formula I or a pharmaceutically acceptable salt thereof:

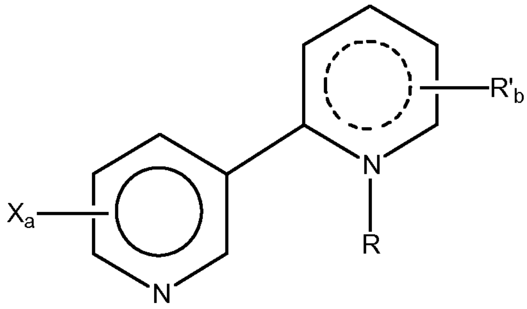

Formula I

wherein:

R represents hydrogen or $C_1$ - $C_5$ alkyl;
R' represents hydrogen or $C_1$ - $C_7$ alkyl;
X represents halogen or $C_1$ - $C_7$ alkyl;
the dotted line within the piperidine ring represents a carbon/carbon or
carbon/nitrogen double bond within that ring, or two conjugated double bonds within that ring; wherein one of the two conjugated double bonds is a carbon/nitrogen double bond, or both of the conjugated double bonds are carbon/carbon double bonds;
wherein when a carbon/nitrogen double bond is present, R is absent; and either (i) "a" is an integer ranging from 1-4 and "b" is an integer ranging from 0-8, or (ii) "a" is an integer ranging from 0-4 and "b" is an integer ranging from 1-8; and when a carbon/nitrogen double bond is not present, R is present; "a" is an integer ranging from 0-4 and "b" is an integer ranging from 0-8,
for use in a method of reducing a symptom of a disorder in an individual, wherein the disorder is selected from the group consisting of Autism Spectrum Disorders and seizure disorders.

2. A composition comprising an isolated form of a compound of Formula I or a pharmaceutically acceptable salt thereof:

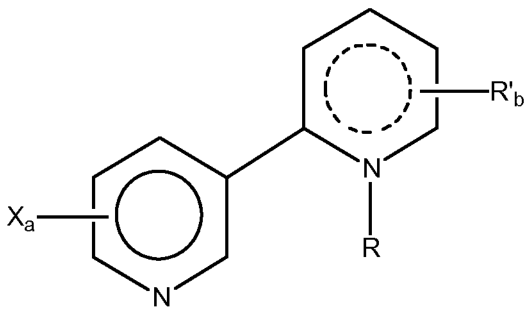

Formula I

wherein:

R represents hydrogen or $C_1$ - $C_5$ alkyl;
R' represents hydrogen or $C_1$ - $C_7$ alkyl;
X represents halogen or $C_1$ - $C_7$ alkyl;
the dotted line within the piperidine ring represents a carbon/carbon or
carbon/nitrogen double bond within that ring, or two conjugated double bonds within that ring; wherein one of the two conjugated double bonds is a carbon/nitrogen double bond, or both of the conjugated double bonds are carbon/carbon double bonds;
wherein when a carbon/nitrogen double bond is present, R is absent; and either (i) "a" is an integer ranging from 1-4 and "b" is an integer ranging from 0-8, or (ii) "a" is an integer ranging from 0-4 and "b" is an integer ranging from 1-8; and when a carbon/nitrogen double bond is not present, R is present; "a" is an integer ranging from 0-4 and "b" is an integer ranging from 0-8,
for use in a method of reducing a risk in an individual of developing a disorder, wherein the disorder is selected

from the group consisting of Autism Spectrum Disorders and seizure disorders.

3. The composition comprising the isolated form of a compound of Formula 1 or a pharmaceutically acceptable salt thereof for use according to claim 1 or claim 2 wherein the disorder is an Autism Spectrum Disorder (ASD).

4. The composition comprising the isolated form of a compound of Formula 1 or a pharmaceutically acceptable salt thereof for use according to claim 3 wherein the ASD is selected from the group consisting of autism, Asperger's syndrome, Rett syndrome, childhood disintegrative disorder, and atypical autism ("pervasive developmental disorder not otherwise specified," PDD-NOS).

5. The composition comprising the isolated form of a compound of Formula 1 or a pharmaceutically acceptable salt thereof for use according to claim 1 or claim 2 wherein the disorder is a seizure disorder.

6. The composition comprising the isolated form of a compound of Formula 1 or a pharmaceutically acceptable salt thereof for use according to claim 5 wherein the seizure disorder is epilepsy, a generalized seizure or a partial seizure.

7. The composition comprising the isolated form of a compound of Formula 1 or a pharmaceutically acceptable salt thereof for use according to any of claims 1-6 wherein the compound of Formula I is present as a racemic mixture.

8. The composition comprising the isolated form of a compound of Formula 1 or a pharmaceutically acceptable salt thereof for use according to any of claims 1-6 wherein the compound of Formula I is present as an isolated enantiomer of Formula IA:

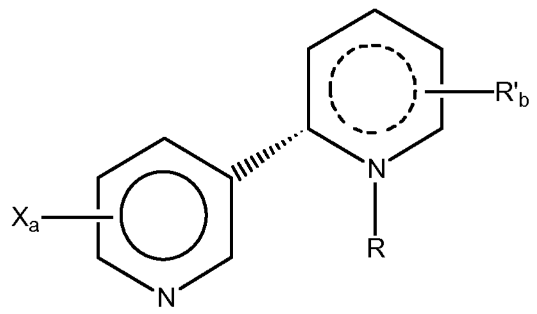

Formula IA.

9. The composition comprising the isolated form of a compound of Formula 1 or a pharmaceutically acceptable salt thereof for use according to any of claims 1-6 wherein the compound of Formula I is present as an isolated enantiomer of Formula IB:

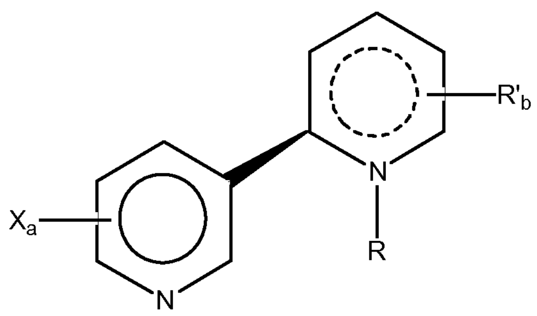

Formula IB.

10. The composition comprising the isolated form of a compound of Formula 1 or a pharmaceutically acceptable salt thereof for use according to any of claims 1-9 wherein the compound is anatabine.

**11.** The composition comprising the isolated form of a compound of Formula 1 or a pharmaceutically acceptable salt thereof for use according to any of claims 1-9 wherein the composition comprises a pharmaceutically acceptable salt of anatabine.

**12.** The composition comprising the isolated form of a compound of Formula 1 or a pharmaceutically acceptable salt thereof for use according to any of claims 1-9 wherein the compound is S-(-)-anatabine.

**13.** The composition comprising the isolated form of a compound of Formula 1 or a pharmaceutically acceptable salt thereof for use according to any of claims 1-9 wherein the composition comprises a pharmaceutically acceptable salt of S-(-)-anatabine.

**14.** The composition comprising the isolated form of a compound of Formula 1 or a pharmaceutically acceptable salt thereof for use according to any of claims 1-13 wherein the compound is in an extended release formulation.

**15.** The composition comprising the isolated form of a compound of Formula I or a pharmaceutically acceptable salt thereof for use according to any of claims 1-20 wherein the compound is in a dosage form suitable to provide from about 0.1 to about 1.5 mg/kg body weight.

## Patentansprüche

**1.** Zusammensetzung umfassend eine isolierte Form einer Verbindung der Formel I oder eines pharmazeutisch geeigneten Salzes davon:

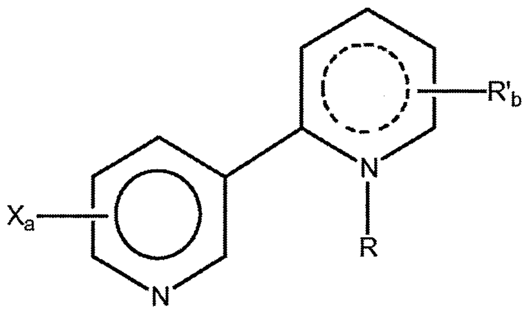

Formel I

wobei:

R für Wasserstoff oder $C_1$-$C_5$-Alkyl steht;
R' für Wasserstoff oder $C_1$-$C_7$-Alkyl steht;
X für Halogen oder $C_1$-$C_7$-Alkyl steht;
die gepunktete Linie innerhalb des Piperidinrings für eine Kohlenstoff/Kohlenstoff-oder Kohlenstoff/Stickstoff-Doppelbindung innerhalb dieses Rings oder für zwei konjugierte Doppelbindungen innerhalb dieses Rings steht; wobei eine der beiden konjugierten Doppelbindungen eine Kohlenstoff/Stickstoff-Doppelbindung ist oder beide der konjugierten Doppelbindungen Kohlenstoff/Kohlenstoff-Doppelbindun-gen sind; wobei wenn eine Kohlenstoff/Stickstoff-Doppelbindung vorhanden ist, R abwesend ist; und entweder (i) "a" eine ganze Zahl von 1-4 ist und "b" eine ganze Zahl von 0-8 ist, oder (ii) "a" eine ganze Zahl von 0-4 ist und "b" eine ganze Zahl von 1-8 ist; und wenn keine Kohlenstoff/Stickstoff-Doppelbindung vorhanden ist, R vorhanden ist; "a" eine ganze Zahl von 0-4 ist und "b" eine ganze Zahl von 0-8 ist,

zur Verwendung in einem Verfahren zum Verringern eines Symptoms einer Erkrankung in einem Individuum, wobei die Erkrankung aus der Gruppe ausgewählt ist bestehend aus dem Spektrum autistischer Erkrankungen und Anfallsleiden.

**2.** Zusammensetzung umfassend eine isolierte Form einer Verbindung der Formel I oder eines pharmazeutisch geeigneten Salzes davon:

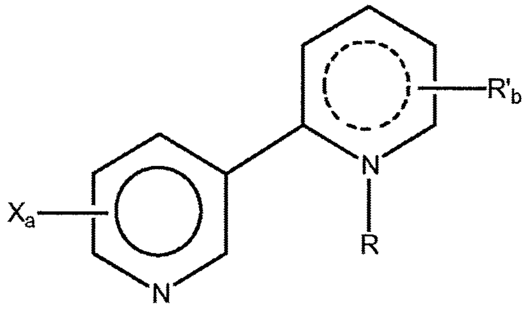

Formel I

wobei:

R für Wasserstoff oder $C_1$-$C_5$-Alkyl steht;
R' für Wasserstoff oder $C_1$-$C_7$-Alkyl steht;
X für Halogen oder $C_1$-$C_7$-Alkyl steht;
die gepunktete Linie innerhalb des Piperidinrings für eine Kohlenstoff/Kohlenstoff- oder Kohlenstoff/Stickstoff-Doppelbindung innerhalb dieses Rings oder für zwei konjugierte Doppelbindungen innerhalb dieses Rings steht; wobei eine der beiden konjugierten Doppelbindungen eine Kohlenstoff/Stickstoff-Doppelbindung ist oder beide der konjugierten Doppelbindungen Kohlenstoff/Kohlenstoff-Doppelbindun-gen sind; wobei wenn eine Kohlenstoff/Stickstoff-Doppelbindung vorhanden ist, R abwesend ist; und entweder (i) "a" eine ganze Zahl von 1-4 ist und "b" eine ganze Zahl von 0-8 ist, oder (ii) "a" eine ganze Zahl von 0-4 ist und "b" eine ganze Zahl von 1-8 ist; und wenn keine Kohlenstoff/Stickstoff-Doppelbindung vorhanden ist, R vorhanden ist; "a" eine ganze Zahl von 0-4 ist und "b" eine ganze Zahl von 0-8 ist,

zur Verwendung in einem Verfahren zum Verringern eines Risikos in einem Individuum für die Entwicklung einer Erkrankung, wobei die Erkrankung aus der Gruppe ausgewählt ist bestehend aus dem Spektrum autistischer Erkrankungen und Anfallsleiden.

**3.** Zusammensetzung umfassend die isolierte Form einer Verbindung der Formel 1 oder eines pharmazeutisch geeigneten Salzes davon zur Verwendung nach Anspruch 1 oder Anspruch 2 wobei die Erkrankung eine Autismus-Spektrum-Störung (Autism Spectrum Disorder (ASD)) ist.

**4.** Zusammensetzung umfassend die isolierte Form einer Verbindung der Formel 1 oder eines pharmazeutisch geeigneten Salzes davon zur Verwendung nach Anspruch 3 wobei die ASD aus der Gruppe ausgewählt ist bestehend aus Autismus, Asperger-Syndrom, Rett-Syndrom, desintegrative Störung des Kindesalters und atypischer Autismus ("tiefgreifende Entwicklungsstörung - nicht anders bezeichnet" PDD-NOS).

**5.** Zusammensetzung umfassend die isolierte Form einer Verbindung der Formel 1 oder eines pharmazeutisch geeigneten Salzes davon zur Verwendung nach Anspruch 1 oder Anspruch 2 wobei die Erkrankung ein Anfallsleiden ist.

**6.** Zusammensetzung umfassend die isolierte Form einer Verbindung der Formel 1 oder eines pharmazeutisch geeigneten Salzes davon zur Verwendung nach Anspruch 5 wobei das Anfallsleiden Epilepsie, ein generalisierter Anfall oder ein partieller Anfall ist.

**7.** Zusammensetzung umfassend die isolierte Form einer Verbindung der Formel 1 oder eines pharmazeutisch geeigneten Salzes davon zur Verwendung nach einem der Ansprüche 1-6 wobei die Verbindung der Formel I als racemische Mischung vorhanden ist.

**8.** Zusammensetzung umfassend die isolierte Form einer Verbindung der Formel 1 oder eines pharmazeutisch geeigneten Salzes davon zur Verwendung nach einem der Ansprüche 1-6 wobei die Verbindung der Formel I als ein isoliertes Enantiomer der Formel IA vorhanden ist:

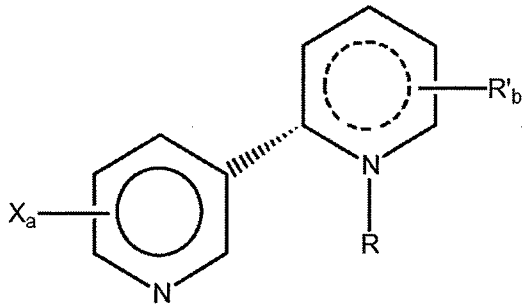

Formel IA .

9. Zusammensetzung umfassend die isolierte Form einer Verbindung der Formel 1 oder eines pharmazeutisch geeigneten Salzes davon zur Verwendung nach einem der Ansprüche 1-6 wobei die Verbindung der Formel I als ein isoliertes Enantiomer der Formel IB vorhanden ist:

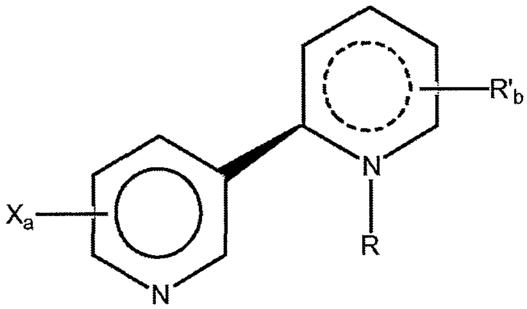

Formel 1B.

10. Zusammensetzung umfassend die isolierte Form einer Verbindung der Formel 1 oder eines pharmazeutisch geeigneten Salzes davon zur Verwendung nach einem der Ansprüche 1-9 wobei die Verbindung Anatabin ist.

11. Zusammensetzung umfassend die isolierte Form einer Verbindung der Formel 1 oder eines pharmazeutisch geeigneten Salzes davon zur Verwendung nach einem der Ansprüche 1-9 wobei die Zusammensetzung ein pharmazeutisch geeignetes Salz von Anatabin umfasst.

12. Zusammensetzung umfassend die isolierte Form einer Verbindung der Formel 1 oder eines pharmazeutisch geeigneten Salzes davon zur Verwendung nach einem der Ansprüche 1-9 wobei die Verbindung S-(-)-Anatabin ist.

13. Zusammensetzung umfassend die isolierte Form einer Verbindung der Formel 1 oder eines pharmazeutisch geeigneten Salzes davon zur Verwendung nach einem der Ansprüche 1-9 wobei die Zusammensetzung ein pharmazeutisch geeignetes Salz von S-(-)-Anatabin umfasst.

14. Zusammensetzung umfassend die isolierte Form einer Verbindung der Formel 1 oder eines pharmazeutisch geeigneten Salzes davon zur Verwendung nach einem der Ansprüche 1-13 wobei die Verbindung in einer Formulierung mit verlängerter Freisetzung vorliegt.

15. Zusammensetzung umfassend die isolierte Form einer Verbindung der Formel I oder eines pharmazeutisch geeigneten Salzes davon zur Verwendung nach einem der Ansprüche 1-20 wobei die Verbindung in einer Dosierungsform vorliegt, die für eine Bereitstellung von ungefähr 0,1 bis ungefähr 1,5 mg/kg Körpergewicht geeignet ist.

**Revendications**

1. Composition comprenant une forme isolée d’un composé de Formule I ou sel pharmaceutiquement acceptable de

celle-ci :

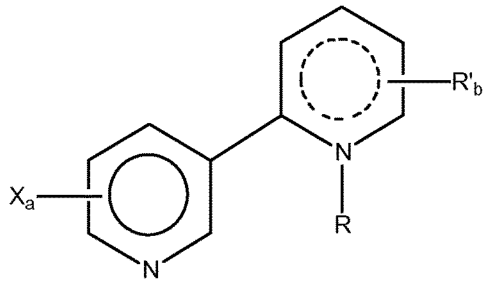

Formule I

dans laquelle :

R représente un hydrogène ou un C1-C5 alkyle ;
R' représente un hydrogène ou un C1-C7 alkyle ;
X représente un halogène ou un C1-C7 alkyle ;
la ligne en pointillés dans le cycle de pipéridine représente une double liaison carbone/carbone ou carbone/azote au sein de ce cycle, ou deux doubles liaisons conjuguées au sein de ce cycle ; dans laquelle une des deux doubles liaisons conjuguées est une double liaison carbone/azote ou les deux doubles liaisons conjuguées sont des doubles liaisons carbone/carbone ; dans laquelle quand une double liaison carbone/azote est présente, R est absent ; et soit (i) « a » est un nombre entier allant de 1-4 et « b » est un nombre entier allant de 0-8, soit (ii) « a » est un nombre entier allant de 0-4 et « b » est un nombre entier allant de 1-8 ; et quand une double liaison carbone/azote n'est pas présente, R est présent ; « a » est un nombre entier allant de 0-4 et « b » est un nombre entier allant de 0-8,
pour son utilisation dans un procédé de réduction d'un symptôme d'un trouble chez un individu, dans laquelle le trouble est sélectionné dans le groupe constitué des troubles du spectre autistique et des troubles convulsifs.

**2.** Composition comprenant une forme isolée d'un composé de Formule I ou sel pharmaceutiquement acceptable de celle-ci :

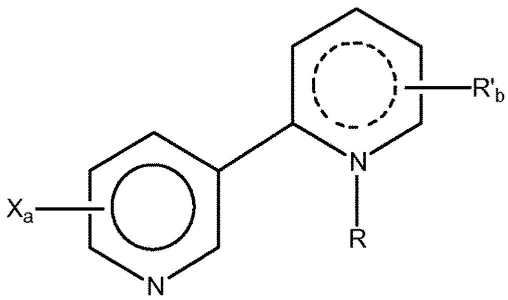

Formule I

dans laquelle :

R représente un hydrogène ou un C1-C5 alkyle ;
R' représente un hydrogène ou un C1-C7 alkyle ;
X représente un halogène ou un C1-C7 alkyle ;
la ligne en pointillés dans le cycle de pipéridine représente une double liaison carbone/carbone ou carbone/azote, au sein de ce cycle, ou deux doubles liaisons conjuguées au sein de ce cycle ; dans laquelle une des deux doubles liaisons conjuguées est une double liaison carbone/azote ou les deux doubles liaisons conjuguées sont des doubles liaisons carbone/carbone ; dans laquelle, quand une double liaison carbone/azote est présente, R est absent ; et soit (i) « a » est un nombre entier allant de 1-4 et « b » est un nombre entier allant de 0-8, soit

(ii) « a » est un nombre entier allant de 0-4 et « b » est un nombre entier allant de 1-8 ; et quand une double liaison carbone/azote n'est pas présente, R est présent ; « a » est un nombre entier allant de 0-4 et « b » est un nombre entier allant de 0-8,
pour son utilisation dans un procédé de réduction du risque chez un individu de développer un trouble, dans laquelle le trouble est sélectionné dans le groupe constitué des troubles du spectre autistique et des troubles convulsifs.

**3.** Composition comprenant la forme isolée d'un composé de formule 1 ou sel pharmaceutiquement acceptable de celle-ci pour son utilisation selon la revendication 1 ou la revendication 2, dans laquelle le trouble est un trouble du spectre autistique (TSA).

**4.** Composition comprenant la forme isolée d'un composé de formule 1 ou sel pharmaceutiquement acceptable de celle-ci pour son utilisation selon la revendication 3, dans laquelle le TSA est sélectionné dans le groupe constitué de l'autisme, du syndrome d'Asperger, du syndrome de Rett, du trouble désintégratif de l'enfance, et de l'autisme atypique (« trouble envahissant du développement non spécifié », PDD-NOS).

**5.** Composition comprenant la forme isolée d'un composé de Formule 1 ou sel pharmaceutiquement acceptable de celle-ci pour son utilisation selon la revendication 1 ou la revendication 2, dans laquelle le trouble est un trouble convulsif.

**6.** Composition comprenant la forme isolée d'un composé de formule 1 ou sel pharmaceutiquement acceptable de celle-ci pour son utilisation selon la revendication 5, dans laquelle le trouble convulsif est l'épilepsie, une convulsion généralisée ou une convulsion partielle.

**7.** Composition comprenant la forme isolée d'un composé de Formule 1 ou sel pharmaceutiquement acceptable de celle-ci pour son utilisation selon l'une quelconque des revendications 1-6, dans laquelle le composé de Formule I est présent sous la forme d'un mélange racémique.

**8.** Composition comprenant la forme isolée d'un composé de Formule 1 ou sel pharmaceutiquement acceptable de celle-ci pour son utilisation selon l'une quelconque des revendications 1-6, dans laquelle le composé de Formule I est présent sous la forme d'un énantiomère isolé de Formule 1A :

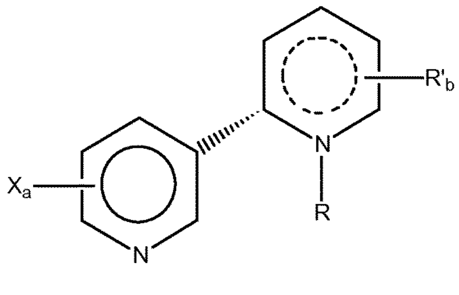

Formule IA

**9.** Composition comprenant la forme isolée d'un composé de Formule 1 ou sel pharmaceutiquement acceptable de celle-ci pour son utilisation selon l'une quelconque des revendications 1-6, dans laquelle le composé de Formule I est présent sous la forme d'un énantiomère isolé de Formule 1B :

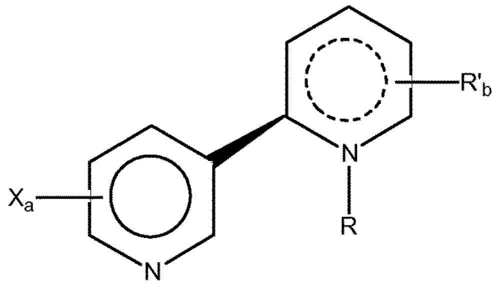

Formule IB

10. Composition comprenant la forme isolée d'un composé de Formule 1 ou sel pharmaceutiquement acceptable de celle-ci pour son utilisation selon l'une quelconque des revendications 1-9, dans laquelle le composé est l'anatabine.

11. Composition comprenant la forme isolée d'un composé de Formule 1 ou sel pharmaceutiquement acceptable de celle-ci pour son utilisation selon l'une quelconque des revendications 1-9, dans laquelle la composition comprend un sel pharmaceutiquement acceptable d'anatabine.

12. Composition comprenant la forme isolée d'un composé de Formule 1 ou sel pharmaceutiquement acceptable de celle-ci pour son utilisation selon l'une quelconque des revendications 1-9, dans laquelle le composé est la S-(-)-anatabine.

13. Composition comprenant la forme isolée d'un composé de Formule 1 ou sel pharmaceutiquement acceptable de celle-ci pour son utilisation selon l'une quelconque des revendications 1-9, dans laquelle la composition comprend un sel pharmaceutiquement acceptable de S-(-)-anatabine

14. Composition comprenant la forme isolée d'un composé de Formule 1 ou sel pharmaceutiquement acceptable de celle-ci pour son utilisation selon l'une quelconque des revendications 1-13, dans laquelle le composé est une formulation à libération prolongée.

15. Composition comprenant la forme isolée d'un composé de Formule 1 ou sel pharmaceutiquement acceptable de celle-ci pour son utilisation selon l'une quelconque des revendications 1-20, dans laquelle le composé est dans une forme de dosage adaptée pour fournir d'environ 0,1 à environ 1,5 mg/kg de poids corporel.

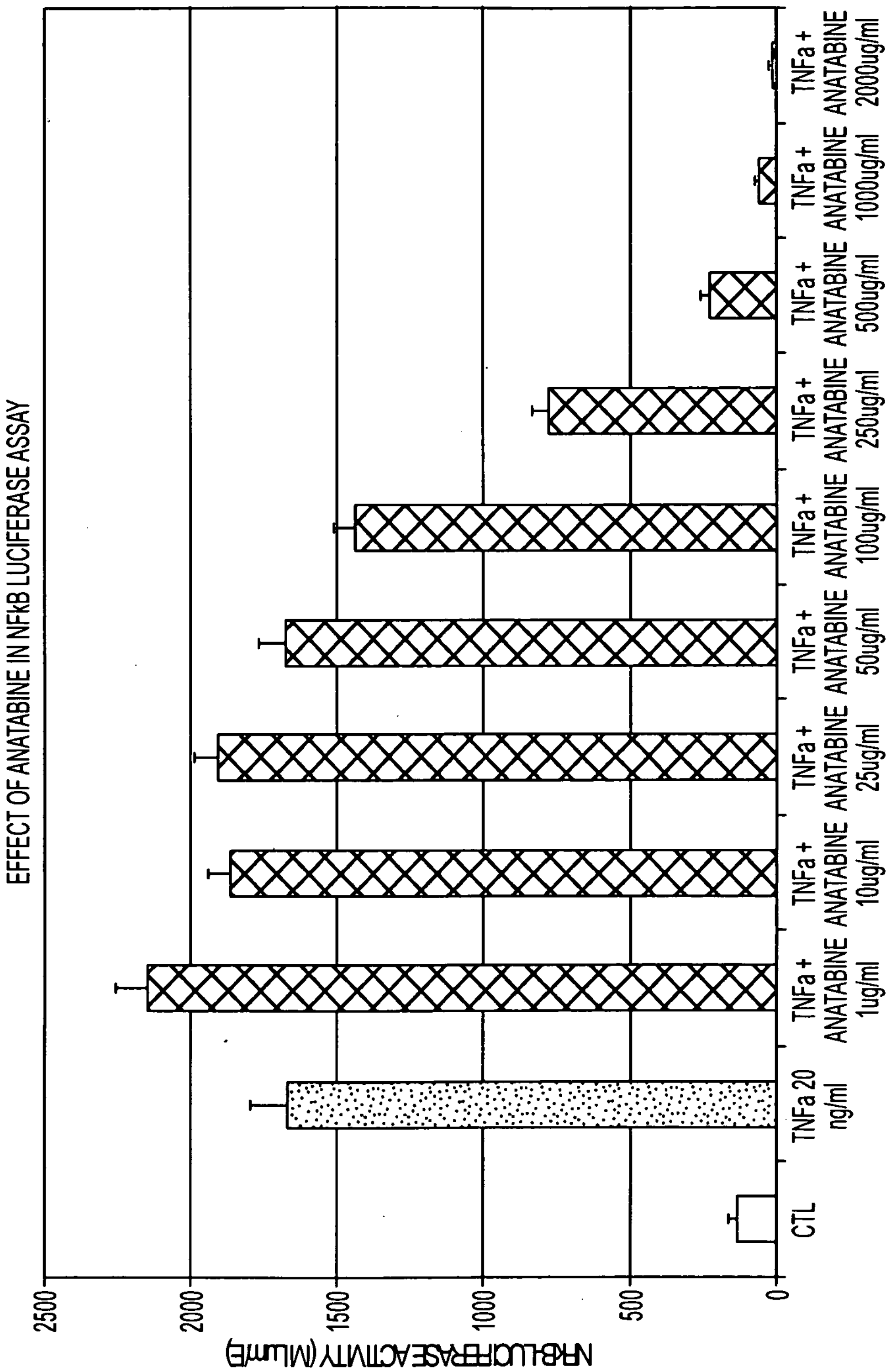
EFFECT OF ANATABINE IN NFkB LUCIFERASE ASSAY
NFkB-LUCIFERASE ACTIVITY (MLum/E)
0
500
1000
1500
2000
2500
CTL
TNFa 20 ng/ml
TNFa + ANATABINE 1ug/ml
TNFa + ANATABINE 10ug/ml
TNFa + ANATABINE 25ug/ml
TNFa + ANATABINE 50ug/ml
TNFa + ANATABINE 100ug/ml
TNFa + ANATABINE 250ug/ml
TNFa + ANATABINE 500ug/ml
TNFa + ANATABINE 1000ug/ml
TNFa + ANATABINE 2000ug/ml

FIG. 1

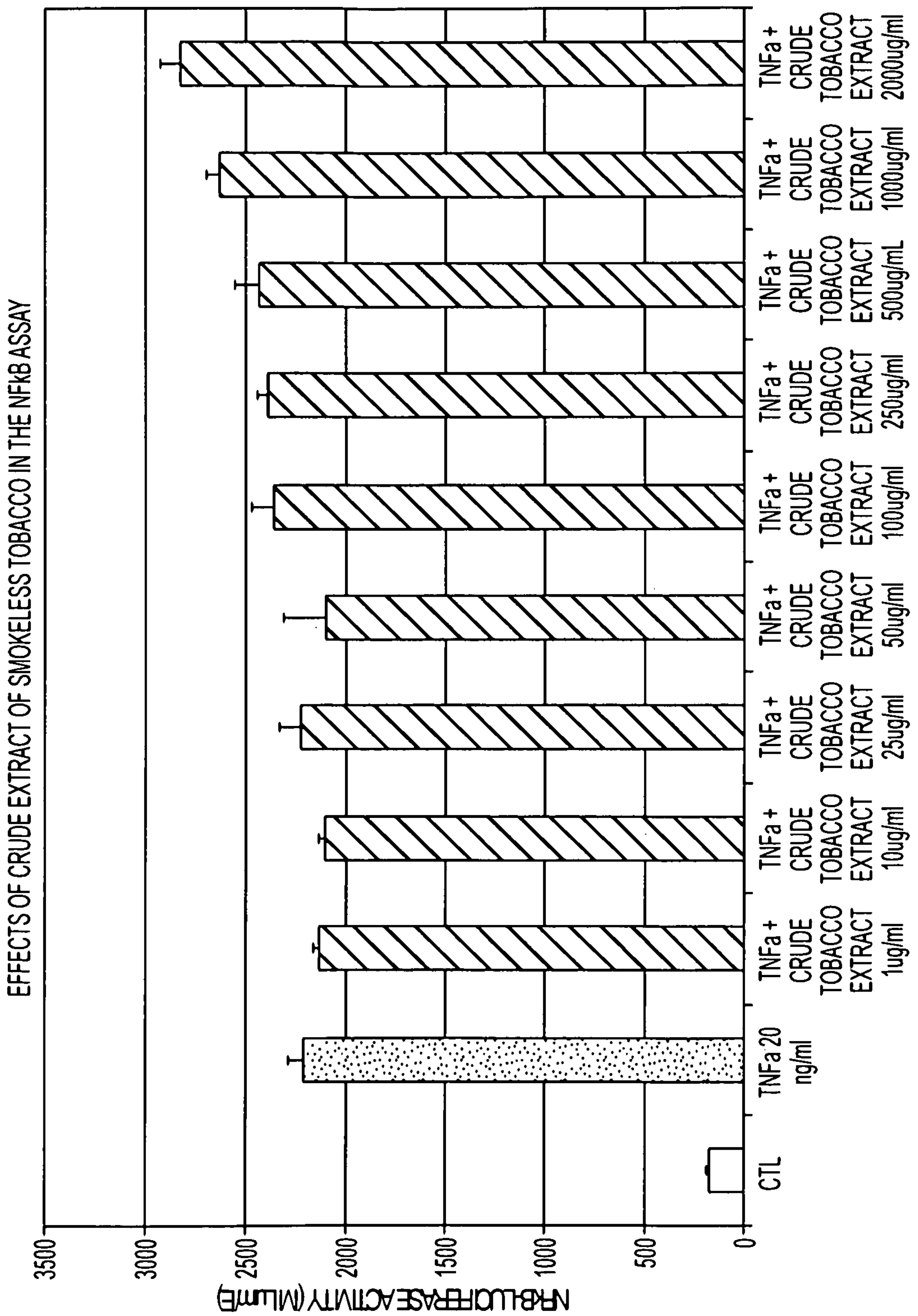
EFFECTS OF CRUDE EXTRACT OF SMOKELESS TOBACCO IN THE NFkB ASSAY
3500
3000
2500
2000
1500
1000
500
0
CTL
TNFa 20 ng/ml
TNFa + CRUDE TOBACCO EXTRACT 1ug/ml
TNFa + CRUDE TOBACCO EXTRACT 10ug/ml
TNFa + CRUDE TOBACCO EXTRACT 25ug/ml
TNFa + CRUDE TOBACCO EXTRACT 50ug/ml
TNFa + CRUDE TOBACCO EXTRACT 100ug/ml
TNFa + CRUDE TOBACCO EXTRACT 250ug/ml
TNFa + CRUDE TOBACCO EXTRACT 500ug/mL
TNFa + CRUDE TOBACCO EXTRACT 1000ug/ml
TNFa + CRUDE TOBACCO EXTRACT 2000ug/ml

FIG. 2

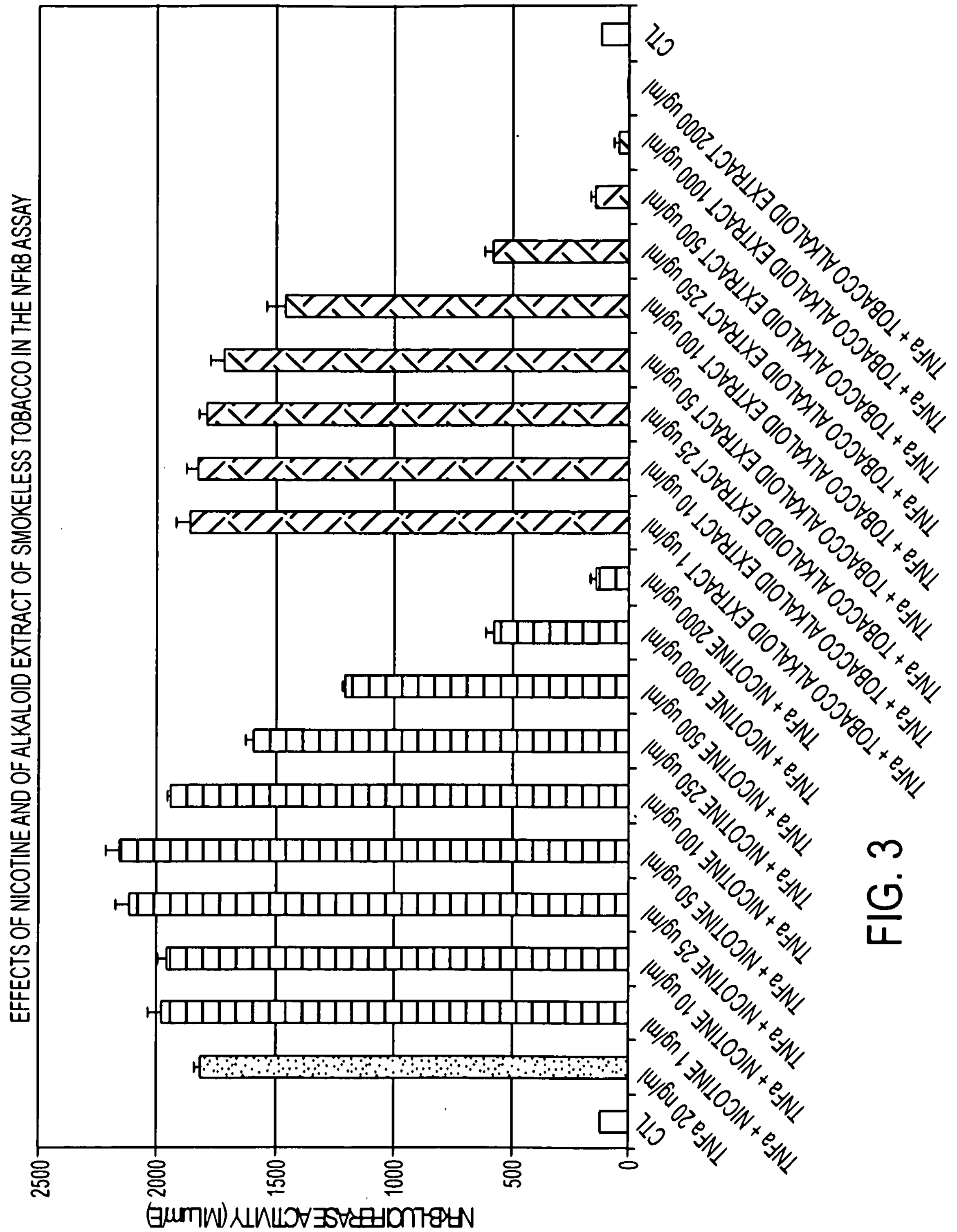
EFFECTS OF NICOTINE AND OF ALKALOID EXTRACT OF SMOKELESS TOBACCO IN THE NFkB ASSAY
NFkB-LUCIFERASE ACTIVITY (MLumE)
2500
2000
1500
1000
500
0
CTL
TNFa 20 ng/ml
TNFa + NICOTINE 1 ug/ml
TNFa + NICOTINE 10 ug/ml
TNFa + NICOTINE 25 ug/ml
TNFa + NICOTINE 50 ug/ml
TNFa + NICOTINE 100 ug/ml
TNFa + NICOTINE 250 ug/ml
TNFa + NICOTINE 500 ug/ml
TNFa + NICOTINE 1000 ug/ml
TNFa + NICOTINE 2000 ug/ml
TNFa + TOBACCO ALKALOID EXTRACT 1 ug/ml
TNFa + TOBACCO ALKALOID EXTRACT 10 ug/ml
TNFa + TOBACCO ALKALOID EXTRACT 25 ug/ml
TNFa + TOBACCO ALKALOID EXTRACT 50 ug/ml
TNFa + TOBACCO ALKALOID EXTRACT 100 ug/ml
TNFa + TOBACCO ALKALOID EXTRACT 250 ug/ml
TNFa + TOBACCO ALKALOID EXTRACT 500 ug/ml
TNFa + TOBACCO ALKALOID EXTRACT 1000 ug/ml
TNFa + TOBACCO ALKALOID EXTRACT 2000 ug/ml
CTL

FIG. 3

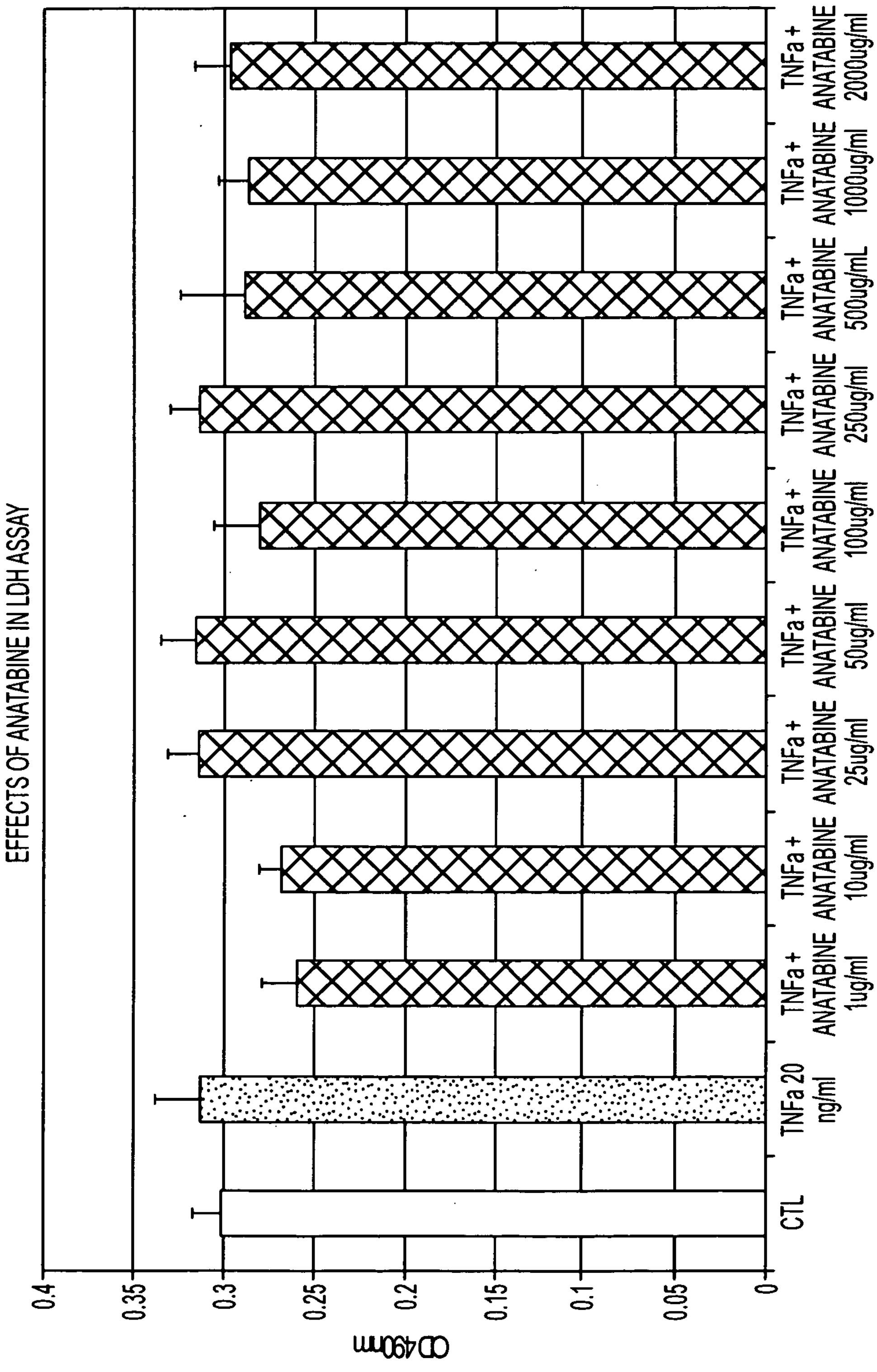
EFFECTS OF ANATABINE IN LDH ASSAY
OD490nm
0
0.05
0.1
0.15
0.2
0.25
0.3
0.35
0.4
CTL
TNFa 20 ng/ml
TNFa + ANATABINE 1ug/ml
TNFa + ANATABINE 10ug/ml
TNFa + ANATABINE 25ug/ml
TNFa + ANATABINE 50ug/ml
TNFa + ANATABINE 100ug/ml
TNFa + ANATABINE 250ug/ml
TNFa + ANATABINE 500ug/mL
TNFa + ANATABINE 1000ug/ml
TNFa + ANATABINE 2000ug/ml

FIG. 4

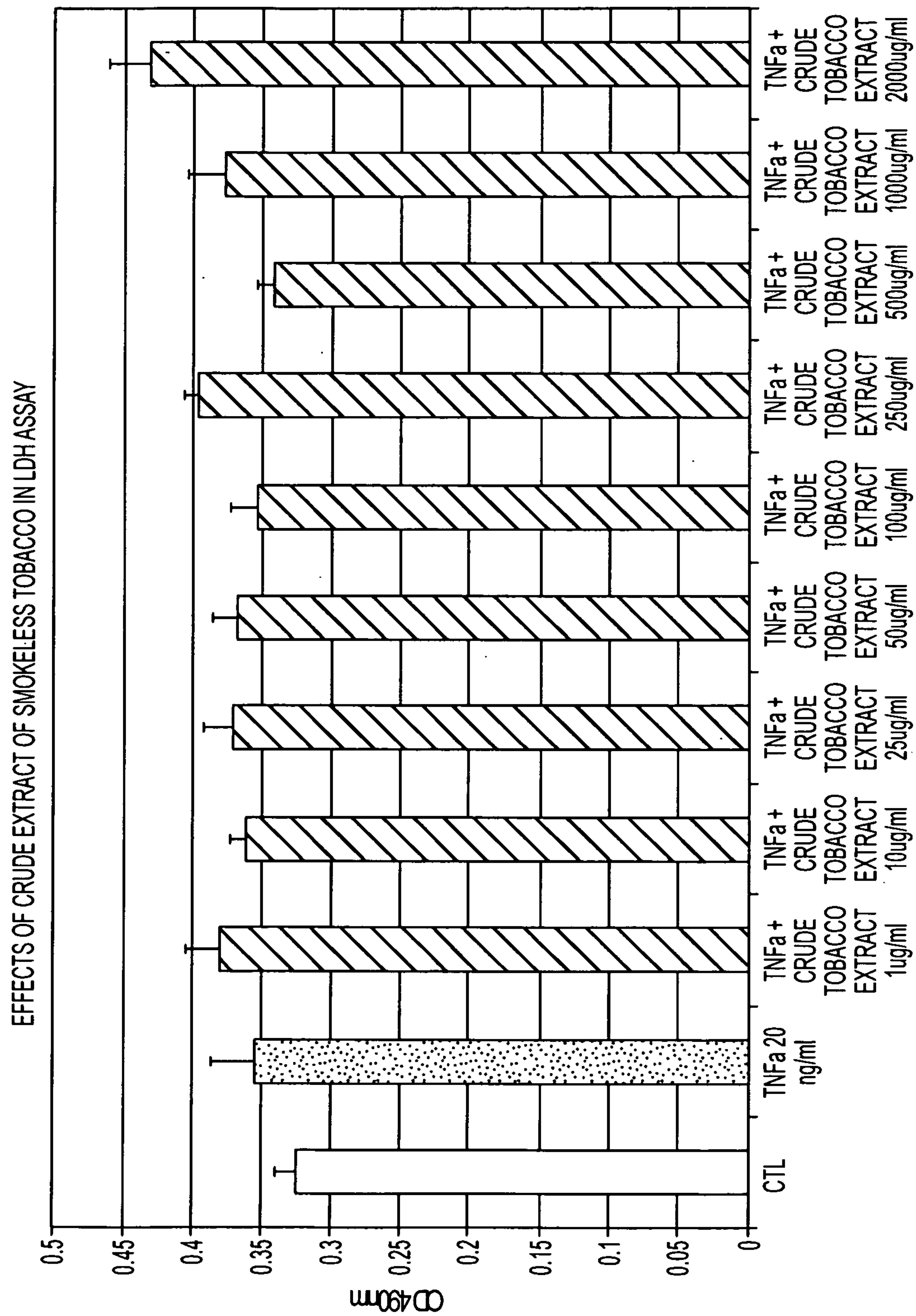
EFFECTS OF CRUDE EXTRACT OF SMOKELESS TOBACCO IN LDH ASSAY
OD490nm
0.5
0.45
0.4
0.35
0.3
0.25
0.2
0.15
0.1
0.05
0
CTL
TNFa 20 ng/ml
TNFa + CRUDE TOBACCO EXTRACT 1ug/ml
TNFa + CRUDE TOBACCO EXTRACT 10ug/ml
TNFa + CRUDE TOBACCO EXTRACT 25ug/ml
TNFa + CRUDE TOBACCO EXTRACT 50ug/ml
TNFa + CRUDE TOBACCO EXTRACT 100ug/ml
TNFa + CRUDE TOBACCO EXTRACT 250ug/ml
TNFa + CRUDE TOBACCO EXTRACT 500ug/ml
TNFa + CRUDE TOBACCO EXTRACT 1000ug/ml
TNFa + CRUDE TOBACCO EXTRACT 2000ug/ml

FIG. 5

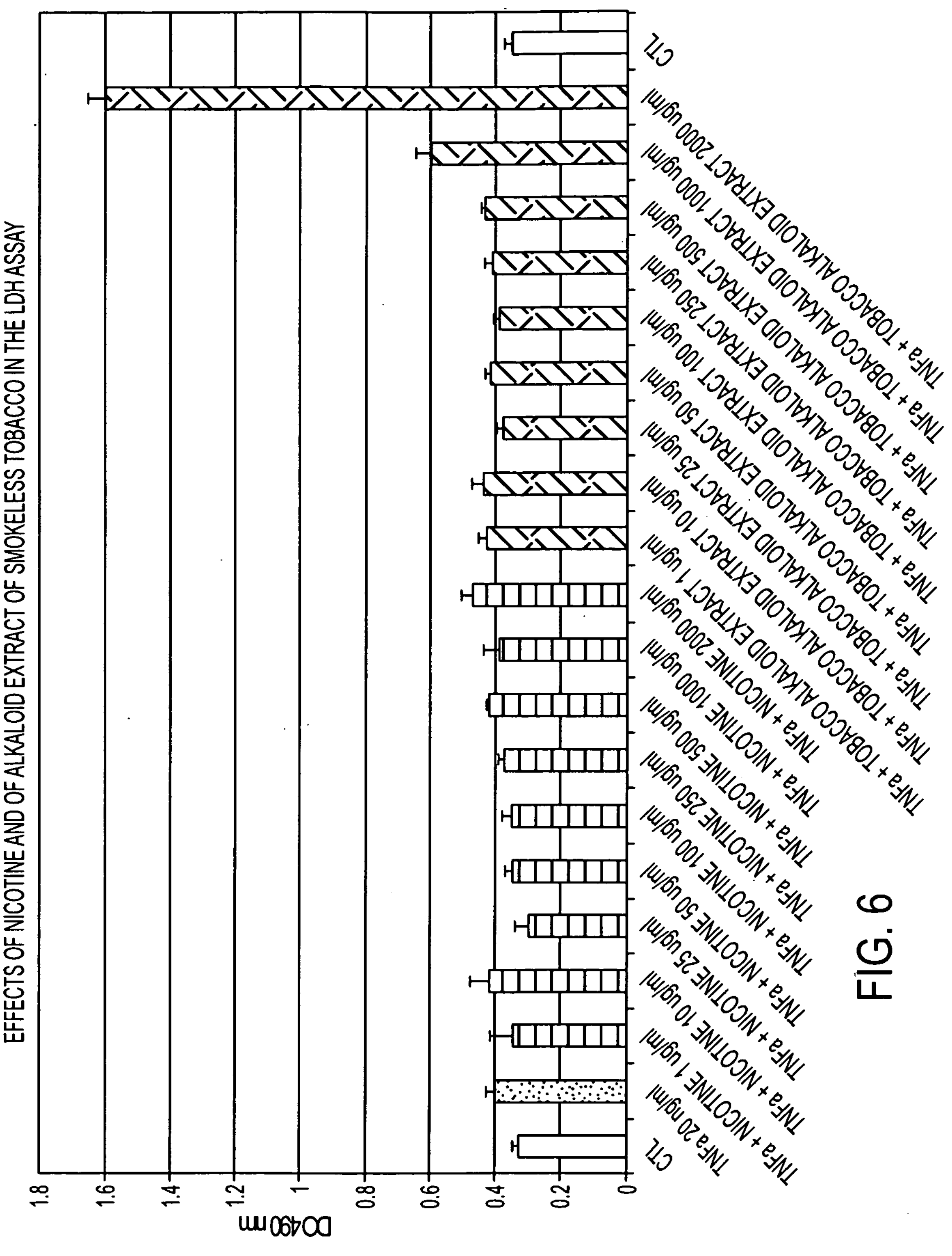
EFFECTS OF NICOTINE AND OF ALKALOID EXTRACT OF SMOKELESS TOBACCO IN THE LDH ASSAY
DO490 nm
1.8
1.6
1.4
1.2
1
0.8
0.6
0.4
0.2
0
CTL
TNFa 20 ng/ml
TNFa + NICOTINE 1 ug/ml
TNFa + NICOTINE 10 ug/ml
TNFa + NICOTINE 25 ug/ml
TNFa + NICOTINE 50 ug/ml
TNFa + NICOTINE 100 ug/ml
TNFa + NICOTINE 250 ug/ml
TNFa + NICOTINE 500 ug/ml
TNFa + NICOTINE 1000 ug/ml
TNFa + NICOTINE 2000 ug/ml
TNFa + TOBACCO ALKALOID EXTRACT 1 ug/ml
TNFa + TOBACCO ALKALOID EXTRACT 10 ug/ml
TNFa + TOBACCO ALKALOID EXTRACT 25 ug/ml
TNFa + TOBACCO ALKALOID EXTRACT 50 ug/ml
TNFa + TOBACCO ALKALOID EXTRACT 100 ug/ml
TNFa + TOBACCO ALKALOID EXTRACT 250 ug/ml
TNFa + TOBACCO ALKALOID EXTRACT 500 ug/ml
TNFa + TOBACCO ALKALOID EXTRACT 1000 ug/ml
TNFa + TOBACCO ALKALOID EXTRACT 2000 ug/ml
CTL

FIG. 6

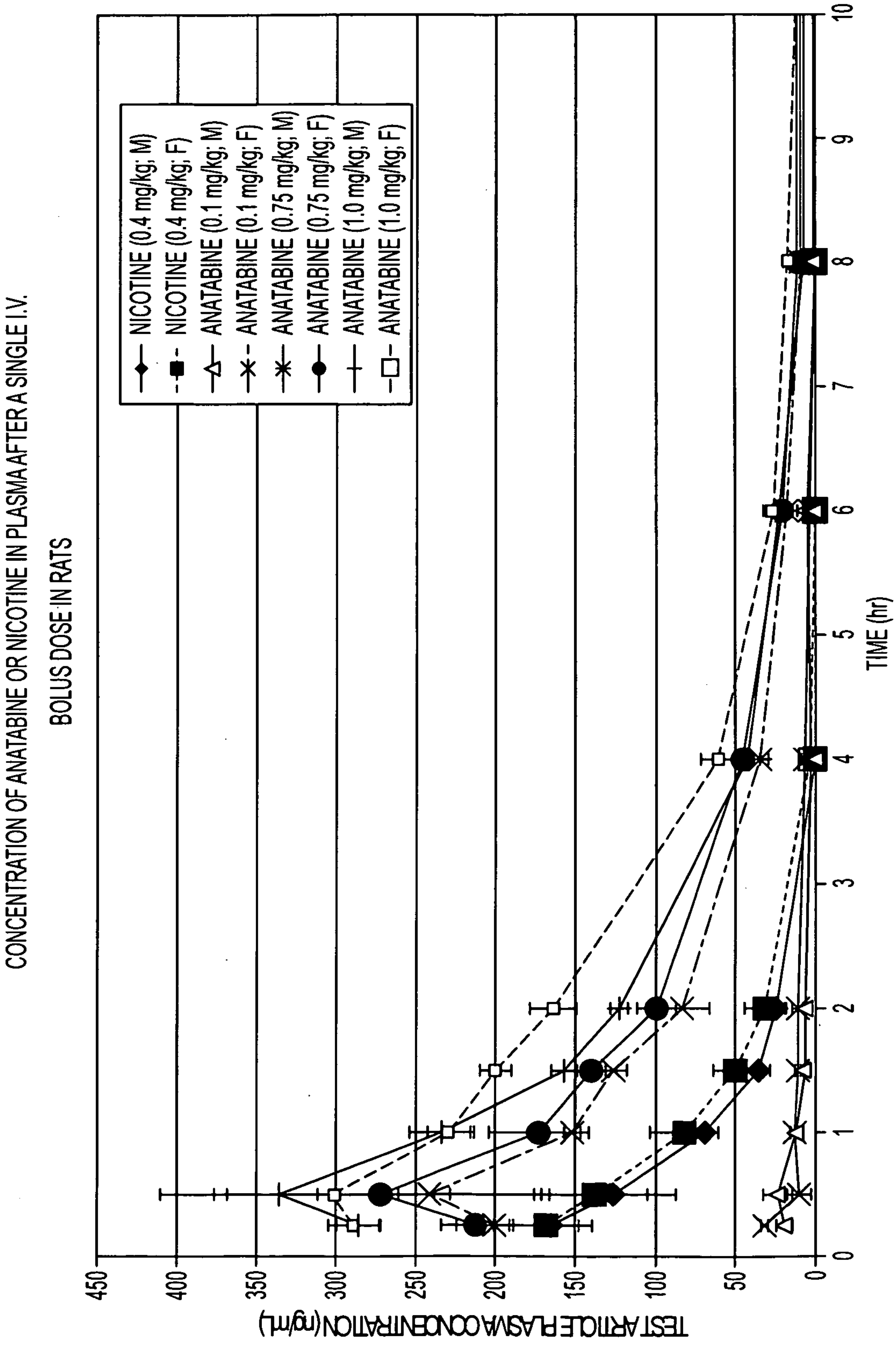
CONCENTRATION OF ANATABINE OR NICOTINE IN PLASMA AFTER A SINGLE I.V.
BOLUS DOSE IN RATS
NICOTINE (0.4 mg/kg; M)
NICOTINE (0.4 mg/kg; F)
ANATABINE (0.1 mg/kg; M)
ANATABINE (0.1 mg/kg; F)
ANATABINE (0.75 mg/kg; M)
ANATABINE (0.75 mg/kg; F)
ANATABINE (1.0 mg/kg; M)
ANATABINE (1.0 mg/kg; F)
TEST ARTICLE PLASMA CONCENTRATION (ng/mL)
0
50
100
150
200
250
300
350
400
450
TIME (hr)
0
1
2
3
4
5
6
7
8
9
10

FIG. 7

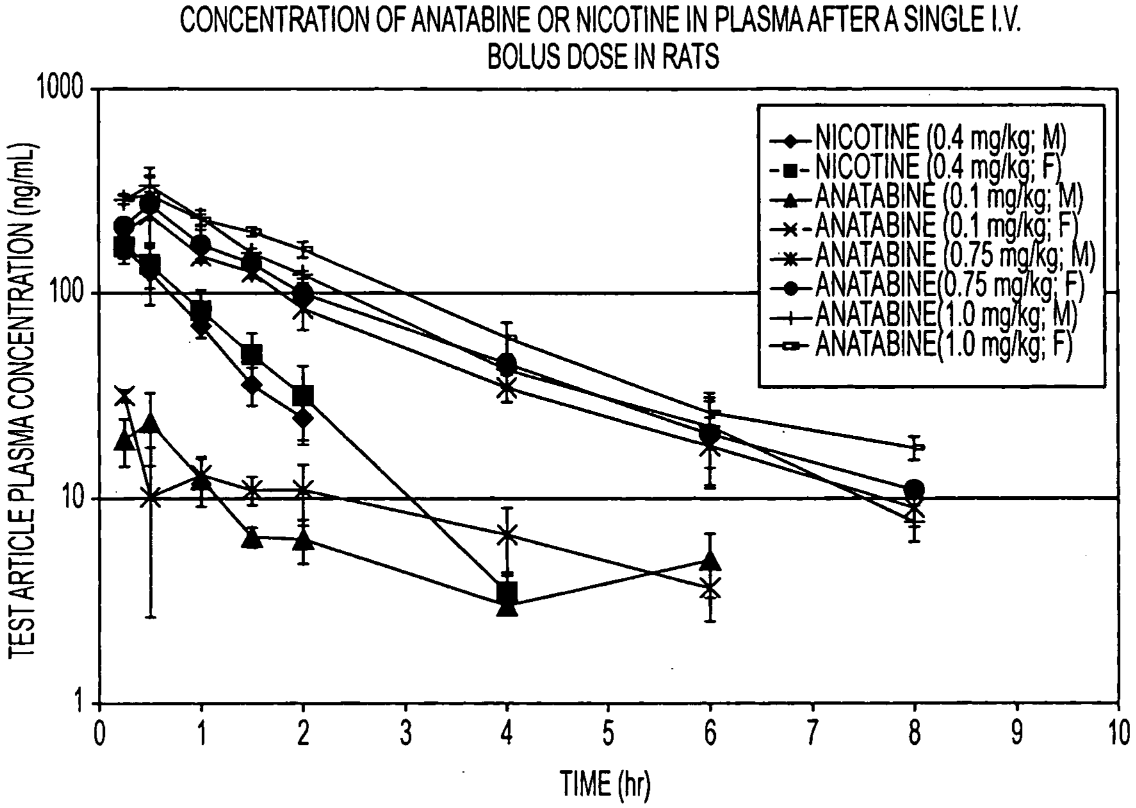
CONCENTRATION OF ANATABINE OR NICOTINE IN PLASMA AFTER A SINGLE I.V. BOLUS DOSE IN RATS
TEST ARTICLE PLASMA CONCENTRATION (ng/mL)
1000
100
10
1
TIME (hr)
0
1
2
3
4
5
6
7
8
9
10
NICOTINE (0.4 mg/kg; M)
NICOTINE (0.4 mg/kg; F)
ANATABINE (0.1 mg/kg; M)
ANATABINE (0.1 mg/kg; F)
ANATABINE (0.75 mg/kg; M)
ANATABINE(0.75 mg/kg; F)
ANATABINE(1.0 mg/kg; M)
ANATABINE(1.0 mg/kg; F)

FIG. 8

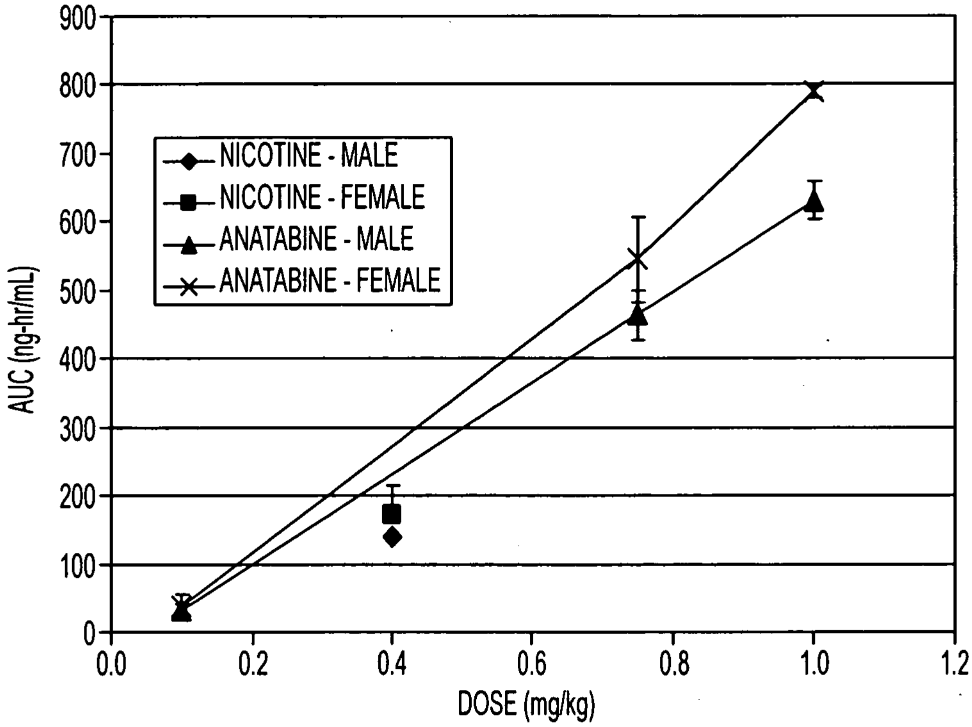
900
800
700
600
500
400
300
200
100
0
AUC (ng-hr/mL)
0.0
0.2
0.4
0.6
0.8
1.0
1.2
DOSE (mg/kg)
NICOTINE - MALE
NICOTINE - FEMALE
ANATABINE - MALE
ANATABINE - FEMALE

FIG. 9

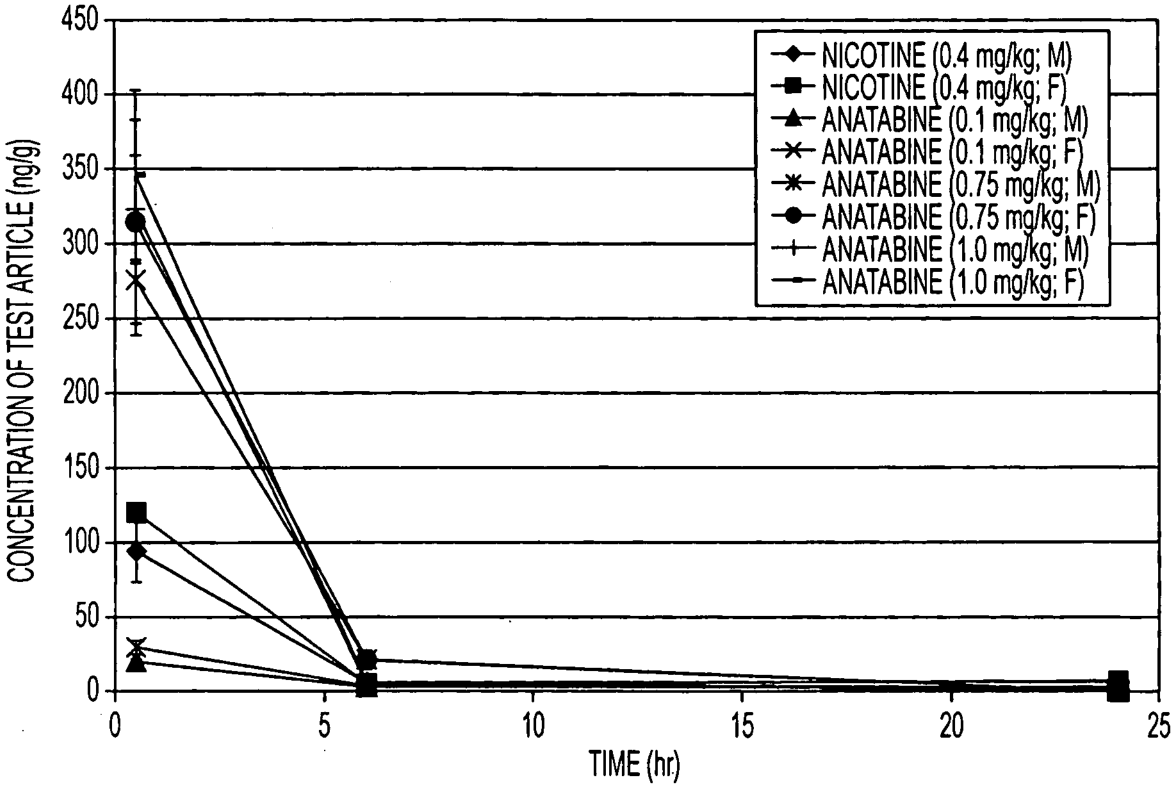
450
400
350
300
250
200
150
100
50
0
CONCENTRATION OF TEST ARTICLE (ng/g)
0
5
10
15
20
25
TIME (hr)
NICOTINE (0.4 mg/kg; M)
NICOTINE (0.4 mg/kg; F)
ANATABINE (0.1 mg/kg; M)
ANATABINE (0.1 mg/kg; F)
ANATABINE (0.75 mg/kg; M)
ANATABINE (0.75 mg/kg; F)
ANATABINE (1.0 mg/kg; M)
ANATABINE (1.0 mg/kg; F)

FIG. 10

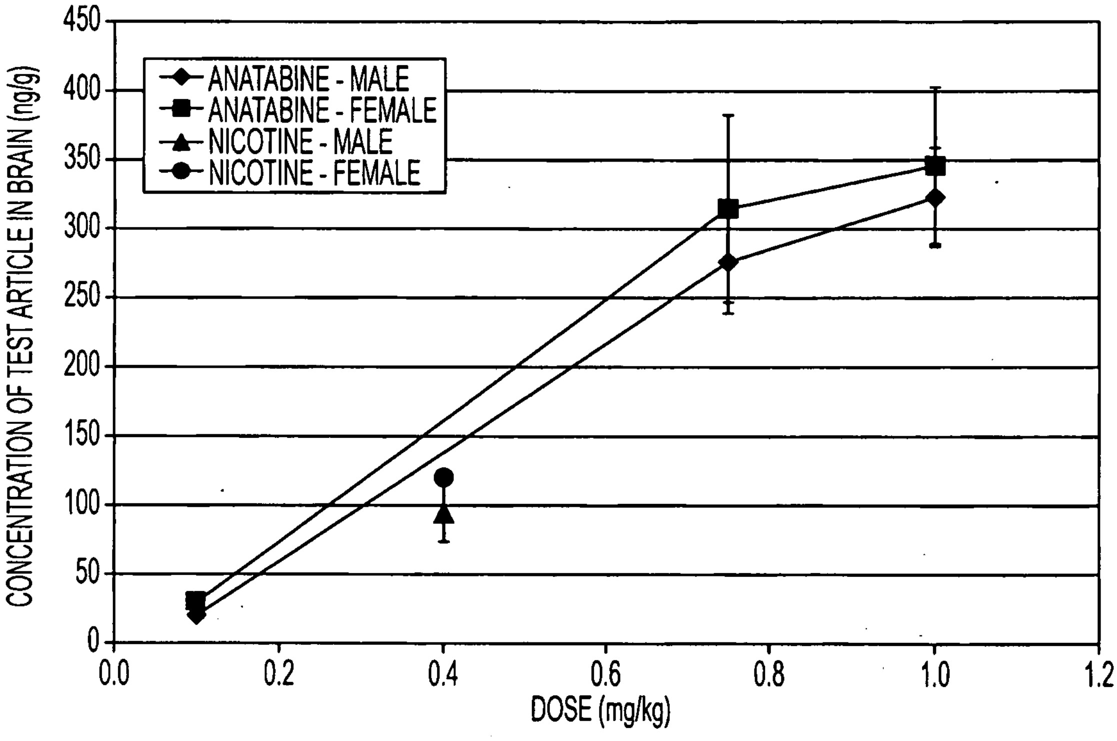
CONCENTRATION OF TEST ARTICLE IN BRAIN (ng/g)
DOSE (mg/kg)
ANATABINE - MALE
ANATABINE - FEMALE
NICOTINE - MALE
NICOTINE - FEMALE
0
50
100
150
200
250
300
350
400
450
0.0
0.2
0.4
0.6
0.8
1.0
1.2

FIG. 11

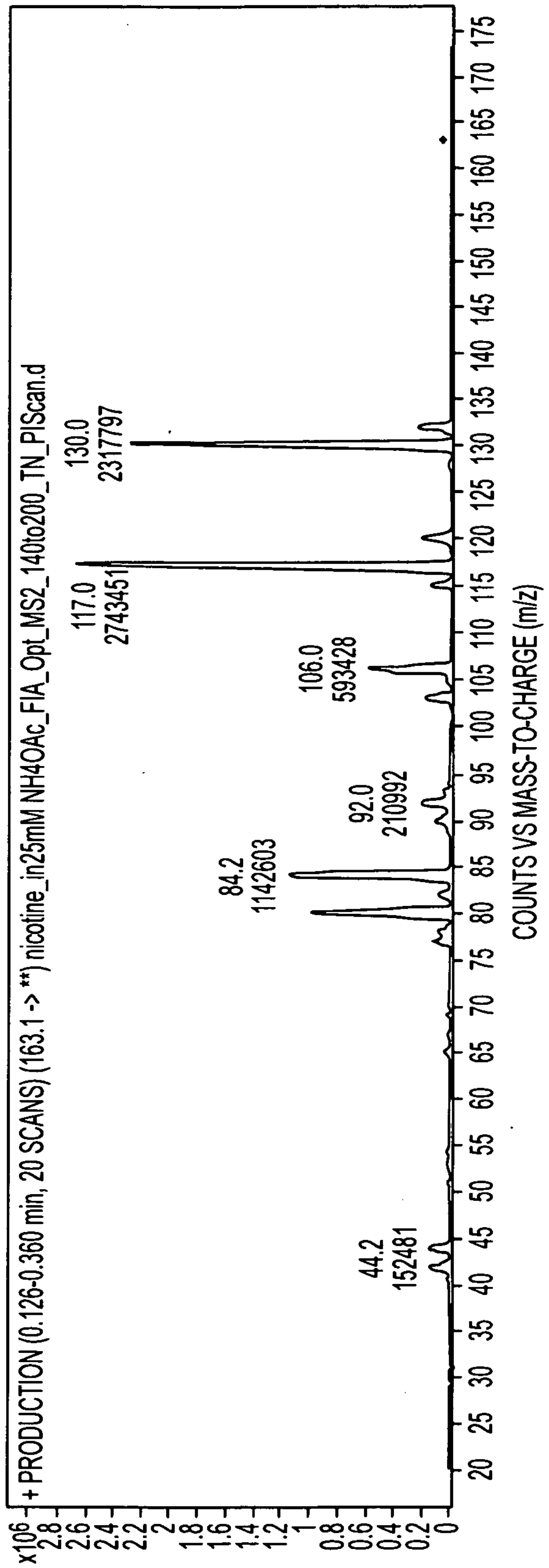
+ PRODUCTION (0.126-0.360 min, 20 SCANS) (163.1 -> **) nicotine_in25mM NH4OAc_FIA_Opt_MS2_140to200_TN_PIScan.d
x10^6
2.8
2.6
2.4
2.2
2
1.8
1.6
1.4
1.2
1
0.8
0.6
0.4
0.2
0
44.2
152481
84.2
1142603
92.0
210992
106.0
593428
117.0
2743451
130.0
2317797
20 25 30 35 40 45 50 55 60 65 70 75 80 85 90 95 100 105 110 115 120 125 130 135 140 145 150 155 160 165 170 175
COUNTS VS MASS-TO-CHARGE (m/z)

FIG. 12

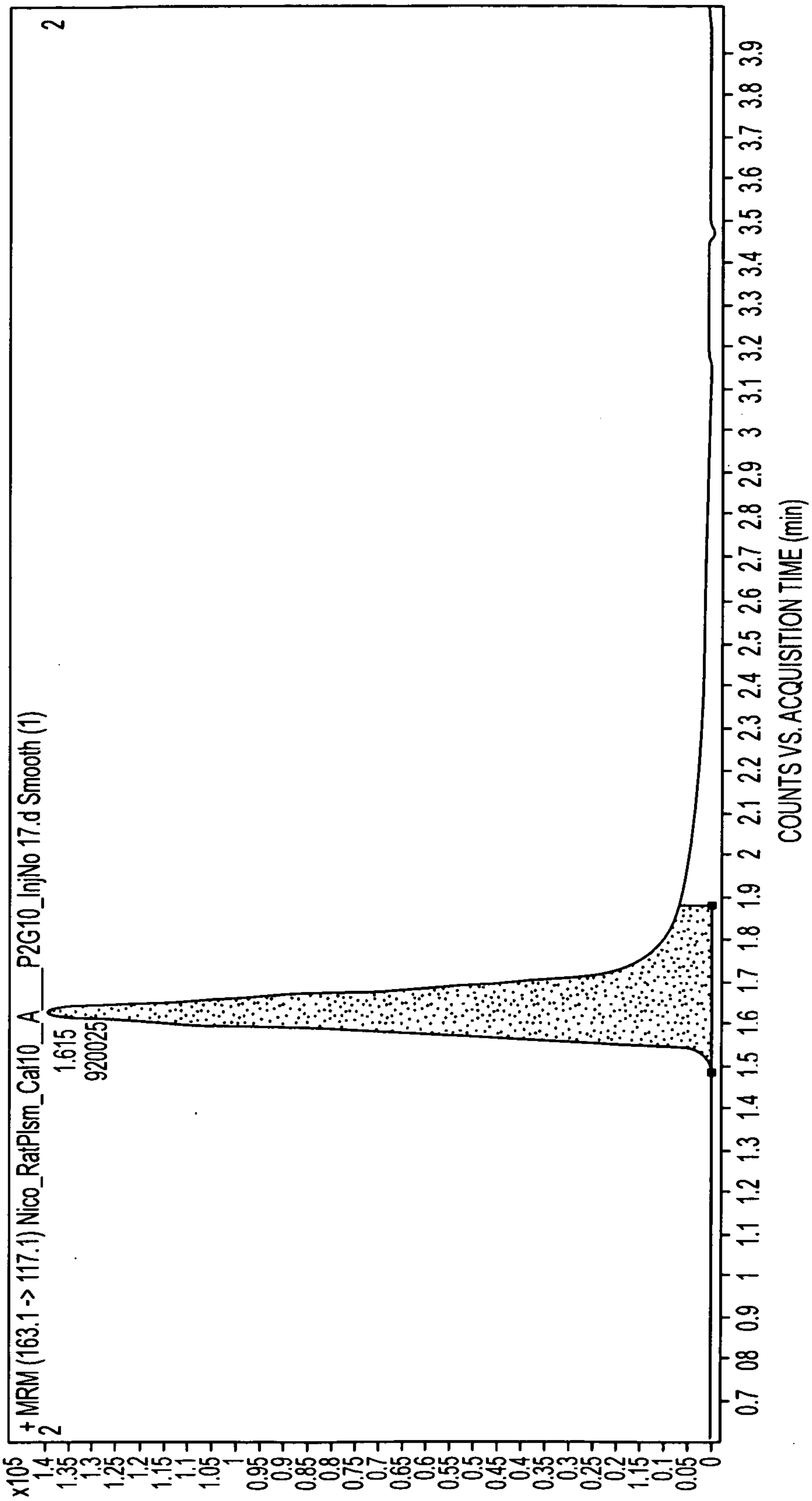
+ MRM (163.1 -> 117.1) Nico_RatPlsm_Cal10__A____P2G10_InjNo 17.d Smooth (1)
x10^5
1.615
920025
COUNTS VS. ACQUISITION TIME (min)

FIG. 13

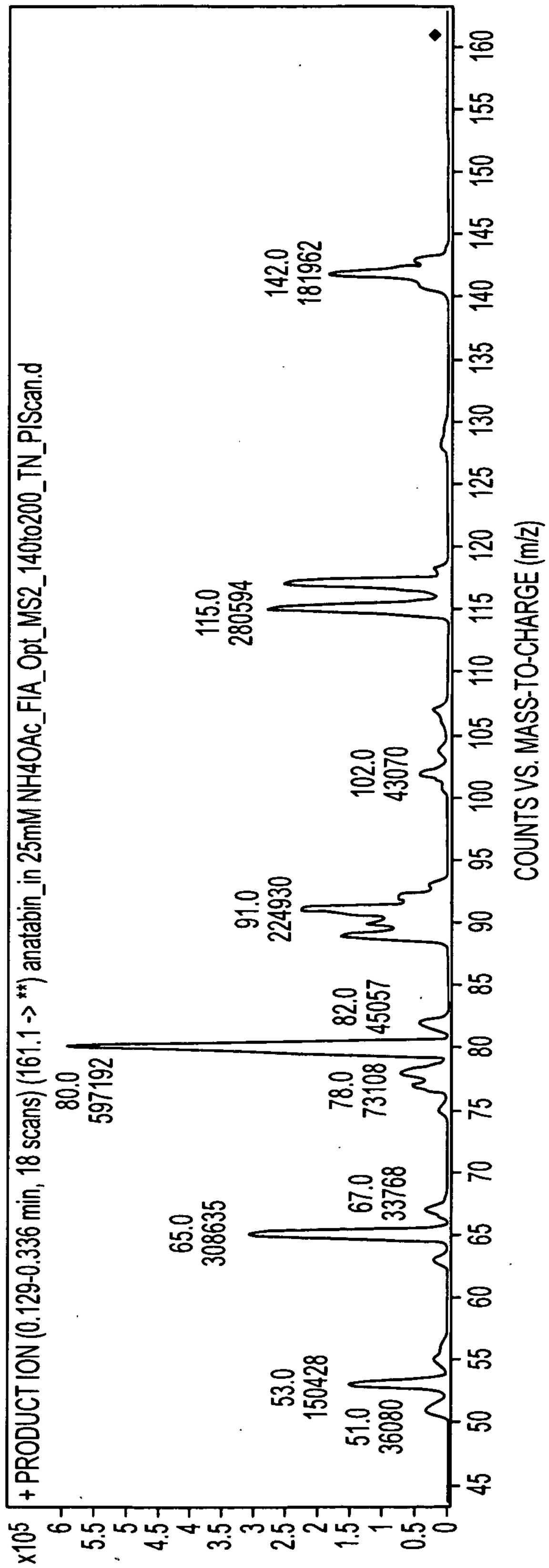
+ PRODUCT ION (0.129-0.336 min, 18 scans) (161.1 -> **) anatabin_in 25mM NH4OAc_FIA_Opt_MS2_140to200_TN_PIScan.d
x10^5
6
5.5
5
4.5
4
3.5
3
2.5
2
1.5
1
0.5
0
51.0
36080
53.0
150428
65.0
308635
67.0
33768
78.0
73108
80.0
597192
82.0
45057
91.0
224930
102.0
43070
115.0
280594
142.0
181962
45
50
55
60
65
70
75
80
85
90
95
100
105
110
115
120
125
130
135
140
145
150
155
160
COUNTS VS. MASS-TO-CHARGE (m/z)

FIG. 14

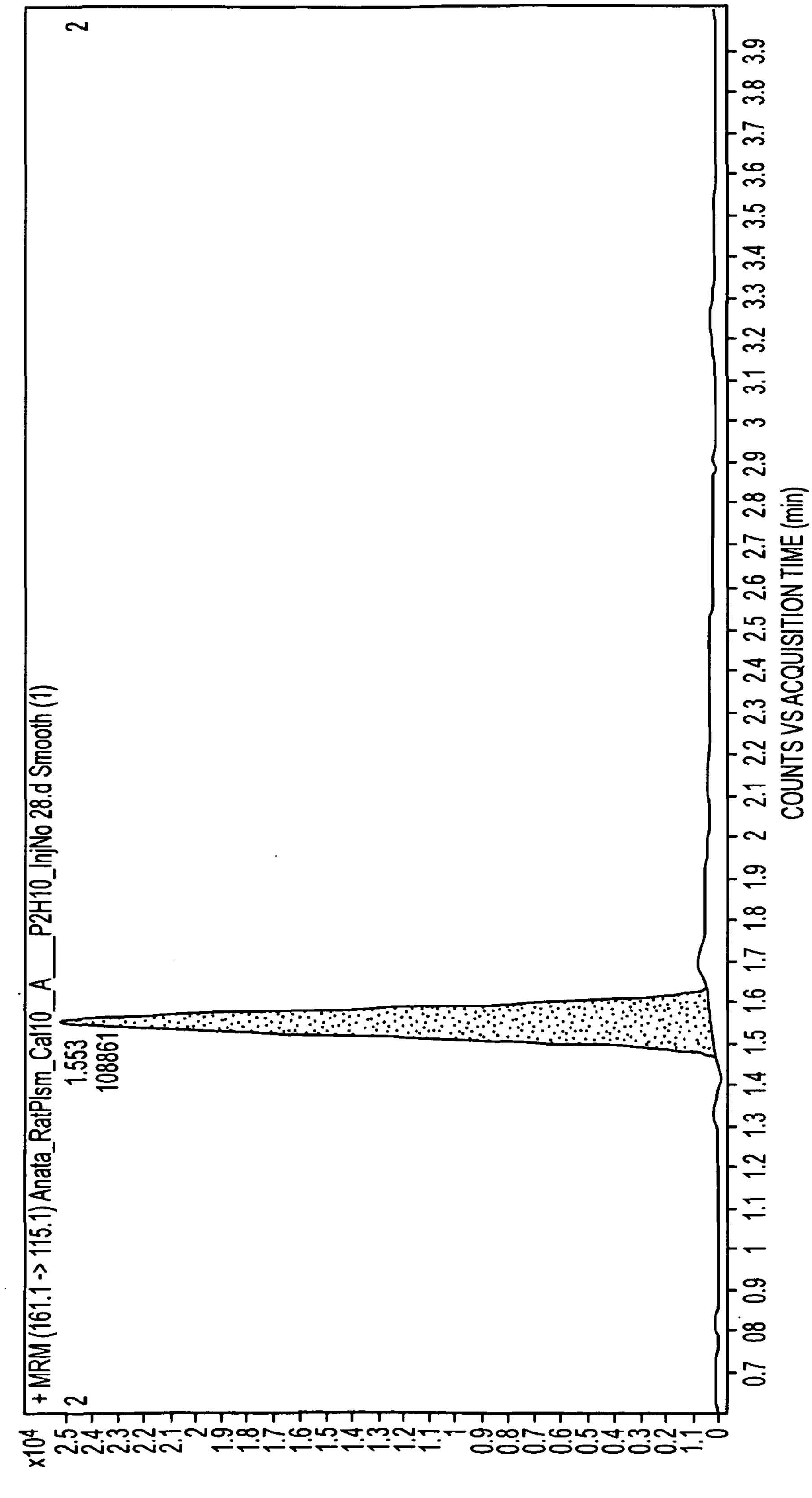
+ MRM (161.1 -> 115.1) Anata_RatPlsm_Cal10__A____P2H10_InjNo 28.d Smooth (1)
x10^4
1.553
108861
2
2
COUNTS VS ACQUISITION TIME (min)

FIG. 15

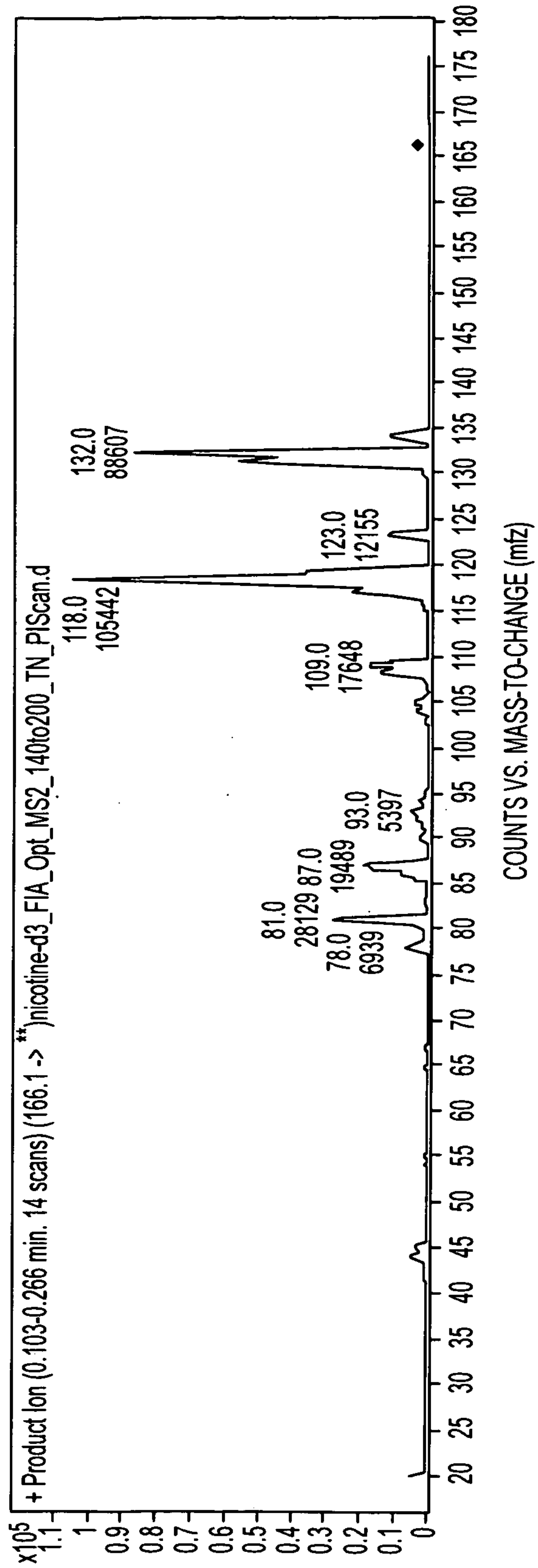
+ Product Ion (0.103-0.266 min. 14 scans) (166.1 -> **)nicotine-d3_FIA_Opt_MS2_140to200_TN_PIScan.d
x10^5
1.1
1
0.9
0.8
0.7
0.6
0.5
0.4
0.3
0.2
0.1
0
78.0
6939
81.0
28129
87.0
19489
93.0
5397
109.0
17648
118.0
105442
123.0
12155
132.0
88607
20 25 30 35 40 45 50 55 60 65 70 75 80 85 90 95 100 105 110 115 120 125 130 135 140 145 150 155 160 165 170 175 180
COUNTS VS. MASS-TO-CHANGE (m/z)

FIG. 16

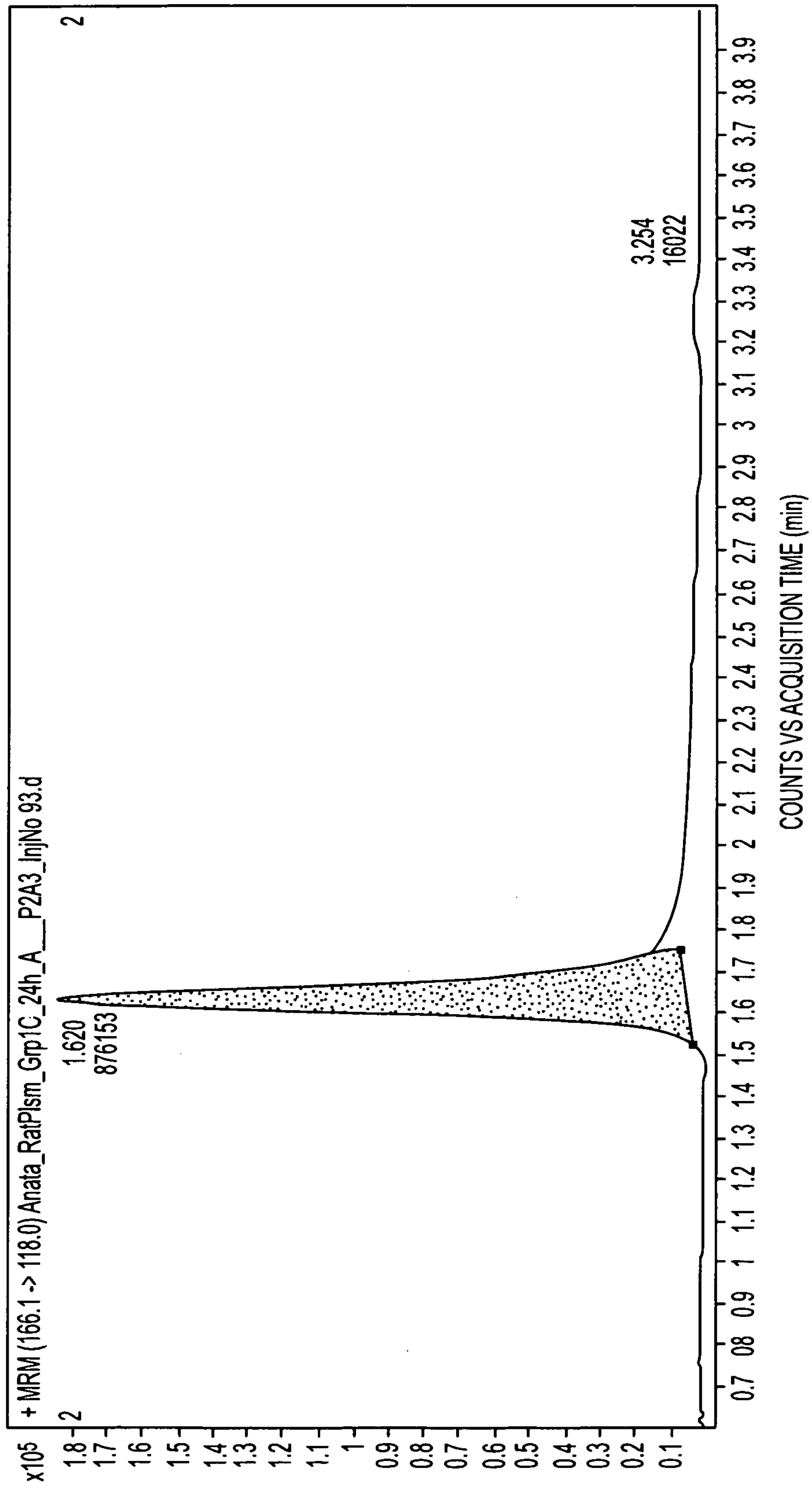
+ MRM (166.1 -> 118.0) Anata_RatPlsm_Grp1C_24h_A___P2A3_InjNo 93.d
x10^5
1.8
1.7
1.6
1.5
1.4
1.3
1.2
1.1
1
0.9
0.8
0.7
0.6
0.5
0.4
0.3
0.2
0.1
2
1.620
876153
3.254
16022
0.7 08 0.9 1 1.1 1.2 1.3 1.4 1.5 1.6 1.7 1.8 1.9 2 2.1 2.2 2.3 2.4 2.5 2.6 2.7 2.8 2.9 3 3.1 3.2 3.3 3.4 3.5 3.6 3.7 3.8 3.9
COUNTS VS ACQUISITION TIME (min)

FIG. 17

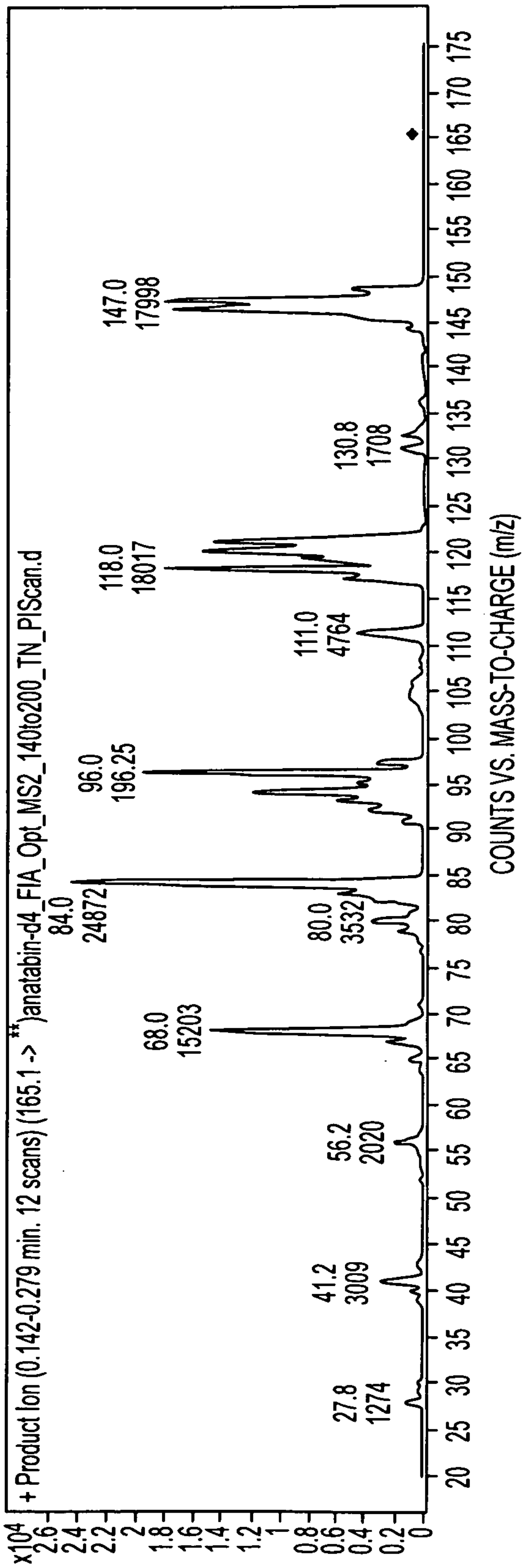
+ Product Ion (0.142-0.279 min. 12 scans) (165.1 -> **)anatabin-d4_FIA_Opt_MS2_140to200_TN_PIScan.d
x10^4
2.6
2.4
2.2
2
1.8
1.6
1.4
1.2
1
0.8
0.6
0.4
0.2
0
27.8 1274
41.2 3009
56.2 2020
68.0 15203
80.0 3532
84.0 24872
96.0 196.25
111.0 4764
118.0 18017
130.8 1708
147.0 17998
20 25 30 35 40 45 50 55 60 65 70 75 80 85 90 95 100 105 110 115 120 125 130 135 140 145 150 155 160 165 170 175
COUNTS VS. MASS-TO-CHARGE (m/z)

FIG. 18

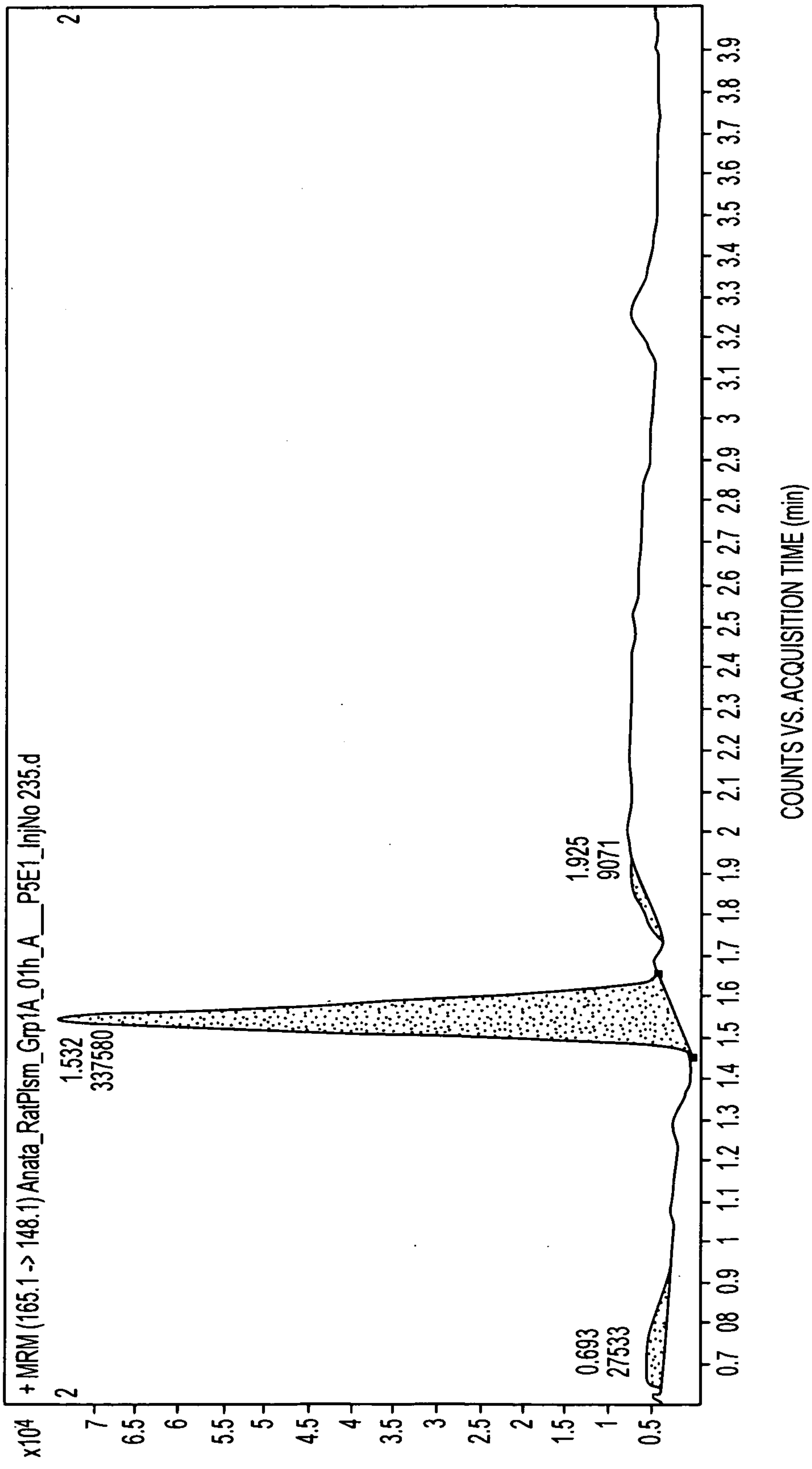
+ MRM (165.1 -> 148.1) Anata_RatPlsm_Grp1A_01h_A___P5E1_InjNo 235.d
x10^4
7
6.5
6
5.5
5
4.5
4
3.5
3
2.5
2
1.5
1
0.5
2
2
0.693
27533
1.532
337580
1.925
9071
0.7 08 0.9 1 1.1 1.2 1.3 1.4 1.5 1.6 1.7 1.8 1.9 2 2.1 2.2 2.3 2.4 2.5 2.6 2.7 2.8 2.9 3 3.1 3.2 3.3 3.4 3.5 3.6 3.7 3.8 3.9
COUNTS VS. ACQUISITION TIME (min)

FIG. 19

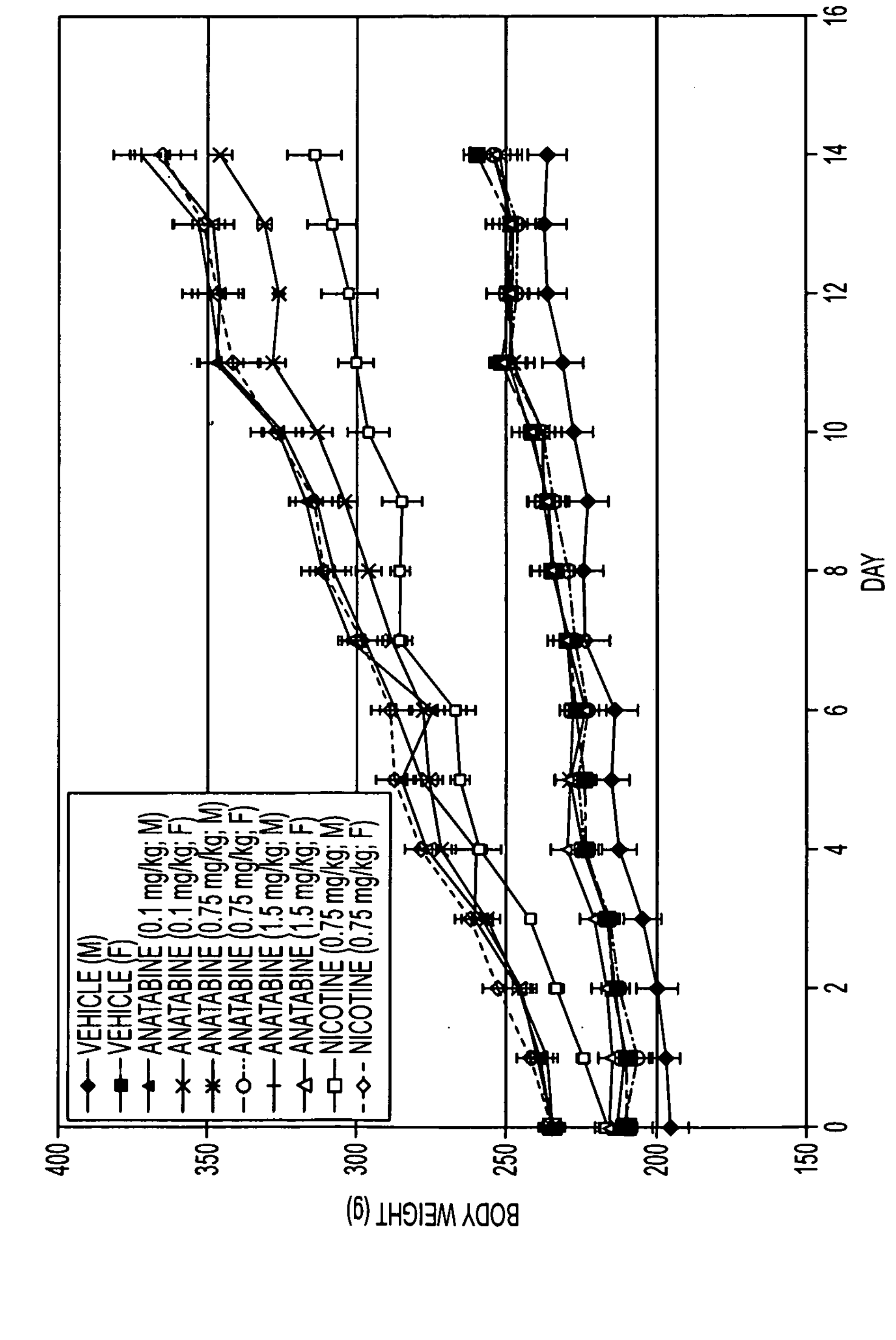

VEHICLE (M)
VEHICLE (F)
ANATABINE (0.1 mg/kg; M)
ANATABINE (0.1 mg/kg; F)
ANATABINE (0.75 mg/kg; M)
ANATABINE (0.75 mg/kg; F)
ANATABINE (1.5 mg/kg; M)
ANATABINE (1.5 mg/kg; F)
NICOTINE (0.75 mg/kg; M)
NICOTINE (0.75 mg/kg; F)
BODY WEIGHT (g)
400
350
300
250
200
150
DAY
0
2
4
6
8
10
12
14
16


FIG. 20

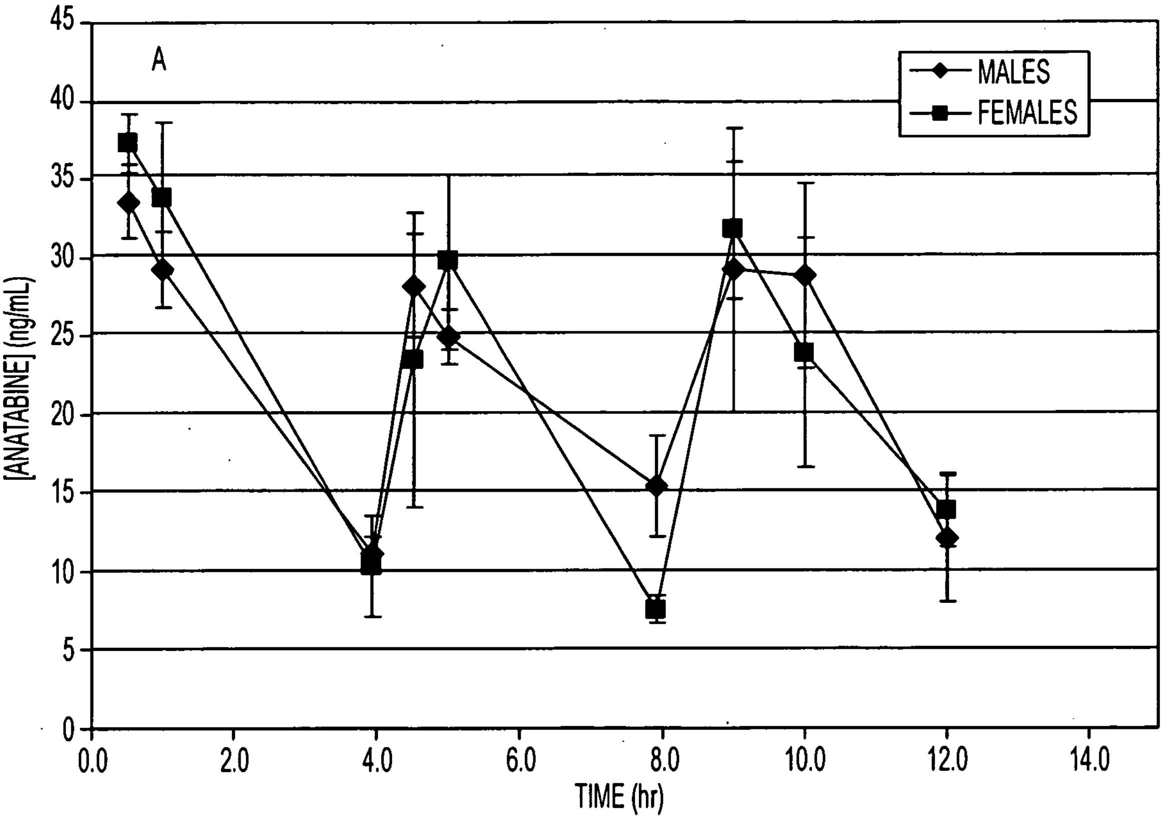

A
MALES
FEMALES
[ANATABINE] (ng/mL)
TIME (hr)
0
5
10
15
20
25
30
35
40
45
0.0
2.0
4.0
6.0
8.0
10.0
12.0
14.0


FIG. 21A

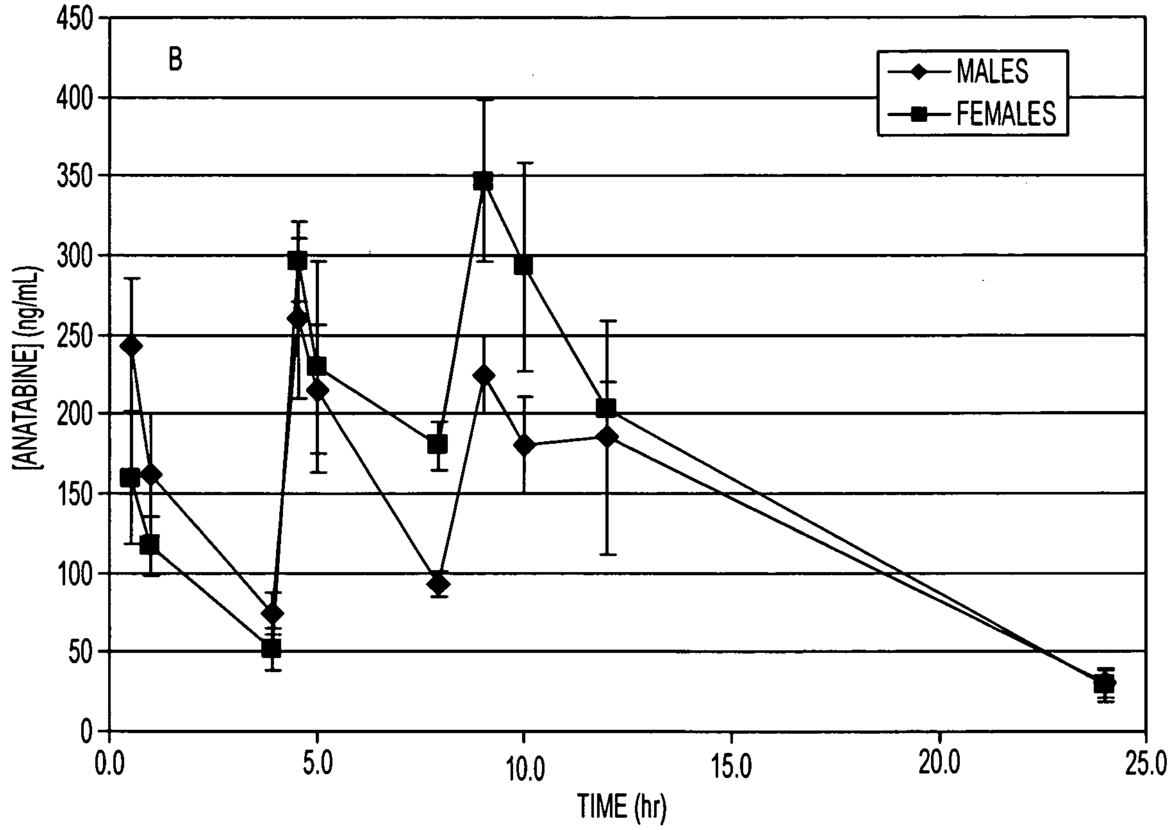
B
MALES
FEMALES
[ANATABINE] (ng/mL)
450
400
350
300
250
200
150
100
50
0
0.0
5.0
10.0
15.0
20.0
25.0
TIME (hr)

FIG. 21B

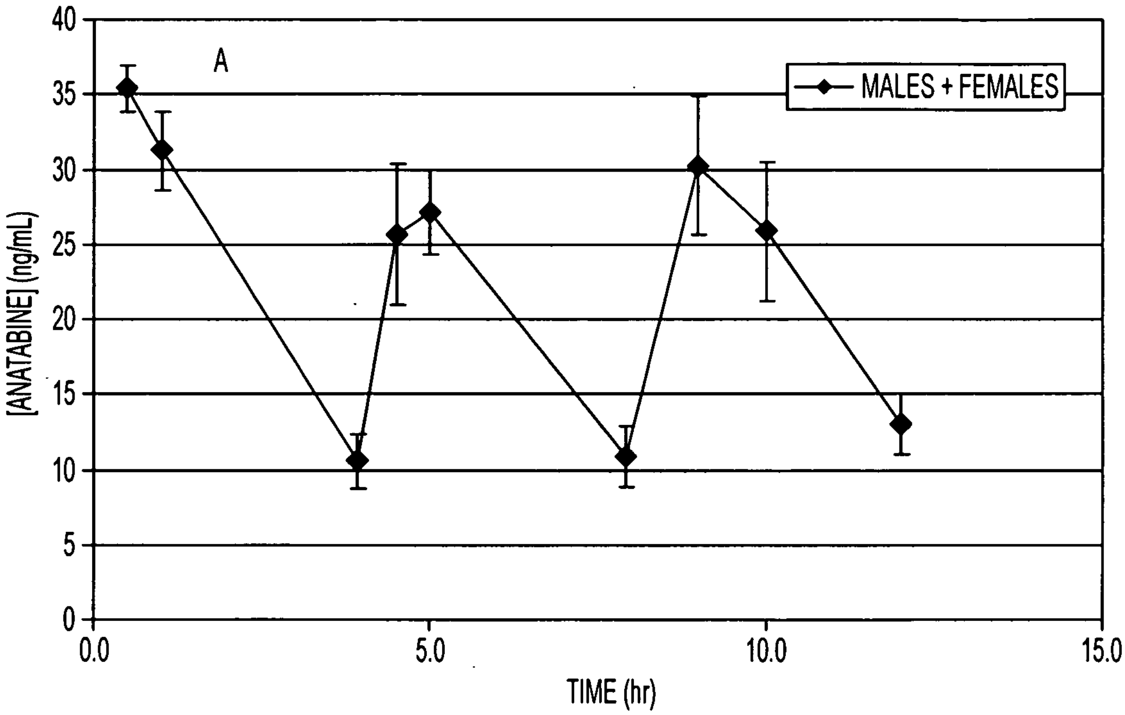
40
35
30
25
20
15
10
5
0
[ANATABINE] (ng/mL)
A
MALES + FEMALES
0.0
5.0
10.0
15.0
TIME (hr)

FIG. 22A

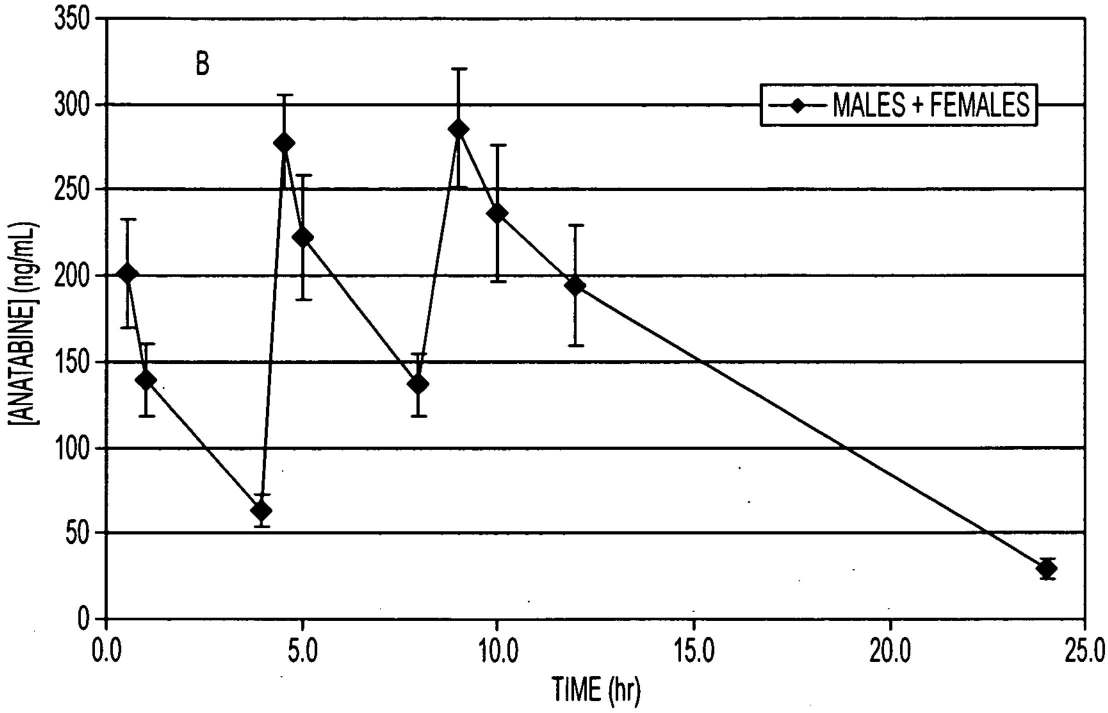
350
300
250
200
150
100
50
0
B
MALES + FEMALES
[ANATABINE] (ng/mL)
0.0
5.0
10.0
15.0
20.0
25.0
TIME (hr)

FIG. 22B

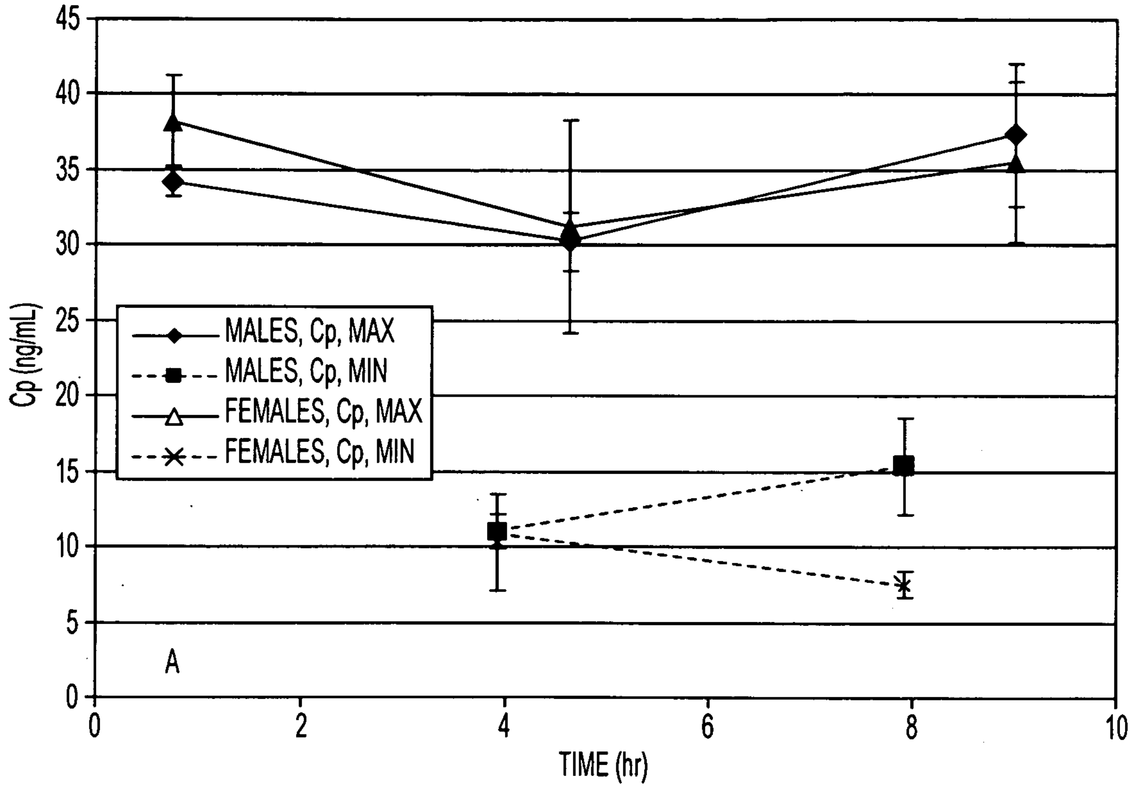
45
40
35
30
25
20
15
10
5
0
Cp (ng/mL)
MALES, Cp, MAX
MALES, Cp, MIN
FEMALES, Cp, MAX
FEMALES, Cp, MIN
A
0
2
4
6
8
10
TIME (hr)

FIG. 23A

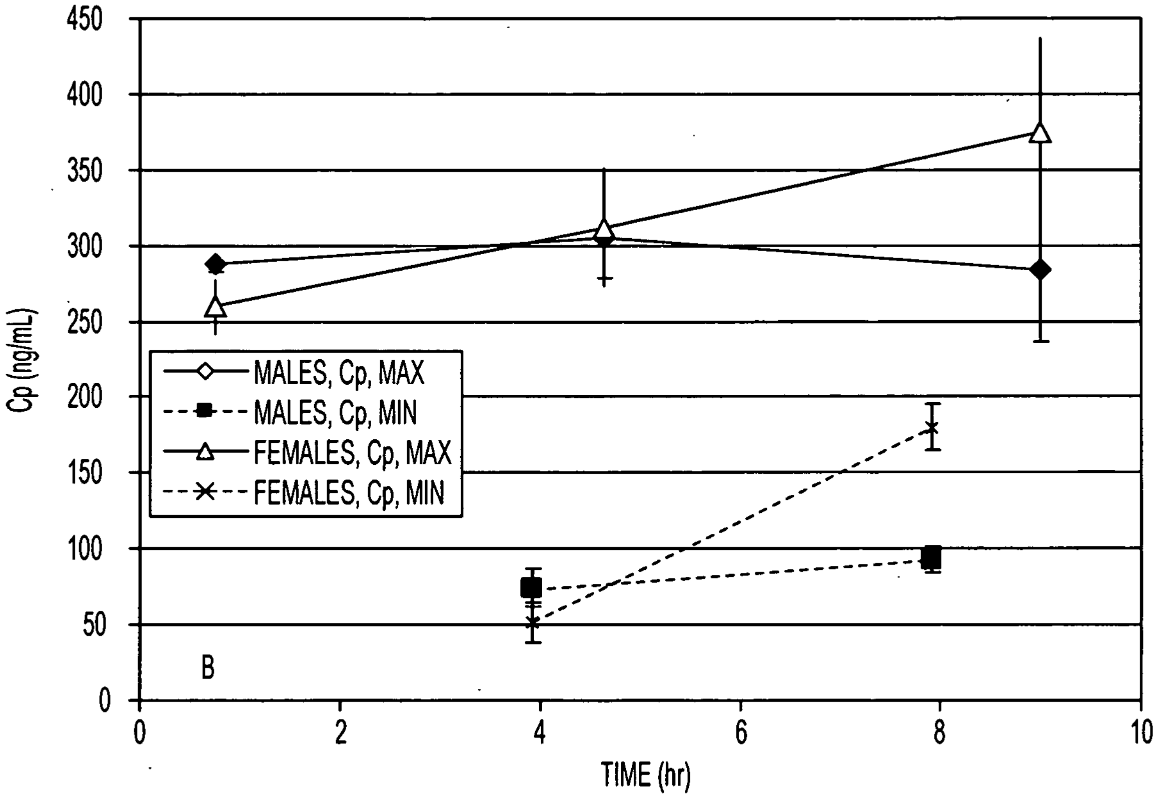
450
400
350
300
250
200
150
100
50
0
Cp (ng/mL)
MALES, Cp, MAX
MALES, Cp, MIN
FEMALES, Cp, MAX
FEMALES, Cp, MIN
B
0
2
4
6
8
10
TIME (hr)

FIG. 23B

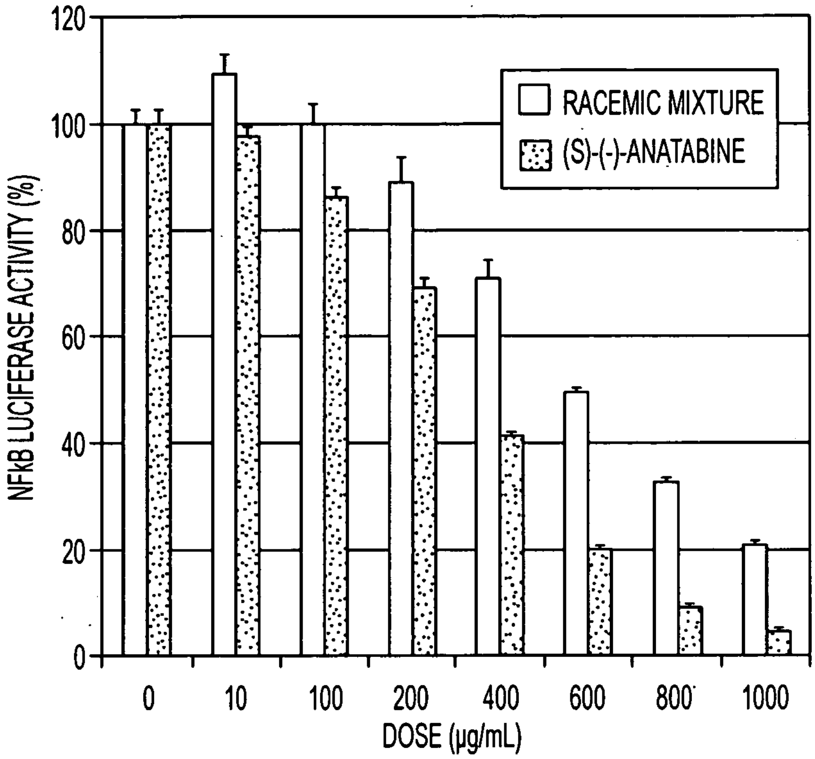
120
100
80
60
40
20
0
NFkB LUCIFERASE ACTIVITY (%)
RACEMIC MIXTURE
(S)-(-)-ANATABINE
0
10
100
200
400
600
800
1000
DOSE (µg/mL)

FIG. 24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 1129613 W
- US 12729346 B
- US 5942244 A


### Non-patent literature cited in the description


- **MINGHETTI.** *Curr Opin Neurol,* 2005, vol. 18, 315-21
- **MRAK ; GRIFFIN.** *Neurobiol Aging,* 2005, vol. 26, 349-54
- **HU et al.** *Bioorgan Med Chem Lett,* 2007, vol. 17, 414-18
- **RALAY et al.** *J Neurosci,* 2006, vol. 26, 662-70
- **CRAFT et al.** *Exp Opin Therap Targets,* 2005, vol. 9, 887-900
- **SHENG et al.** *Neurobiol Aging,* 1996, vol. 17, 761-66
- **VEZZANI et al.** *Epilepsia,* 2002, vol. 43, S30-S35
- **VEZZANI ; GRANATA.** *Epilepsia,* 2005, vol. 46, 1724-43
- The Pharmacological Basis of Therapeutics. Mc-Graw-Hill
- **P. H. STAHL et al.** Handbook of Pharmaceutical Salts. Properties, Selection and Use. Wiley-VCH/VH-CA, 2002
- **WAKEFIELD et al.** *Lancet,* 1998, vol. 351, 351-52
- **WAKEFIELD et al.** *Lancet,* 1998, vol. 351, 637-41
- **VARGAS et al.** *Ann Neurol,* 2004, vol. 57, 67-81